# EUROPEAN PATENT SPECIFICATION

(11) **EP 3 138 840 B1**
(45) Date of publication and mention of the grant of the patent: **23.09.2020**
(21) Application number: 15786304.4
(22) Date of filing: 21.04.2015
(51) Int. Cl.: C07D 417/14, C07D 417/04, A61K 31/4439, A61P 31/14

(54) **THIADIAZOLIDINE DERIVATIVE AS ANTI-ENTEROVIRUS 71**
THIADIAZOLIDINDERIVAT ALS ANTI-ENTEROVIRUS 71
DÉRIVÉ DE THIADIAZOLIDINE COMME ANTI-ENTÉROVIRUS 71

(30) Priority: 28.04.2014 CN 201410176161; 14.10.2014 CN 201410543064; 16.04.2015 CN 201510182757
(43) Date of publication of application: 08.03.2017
(73) Proprietor: Jiangsu Kanion Pharmaceutical Co., Ltd., Jiangsu 222001 (CN)
(72) Inventor: LI, Peng, Shanghai 200131 (CN); HE, Haiying, Shanghai 200131 (CN); LI, Ning, Shanghai 200131 (CN); LI, Jian, Shanghai 200131 (CN); CHEN, Shuhui, Shanghai 200131 (CN); YANG, Bailing, Shanghai 200131 (CN); SHEN, Wang, Lianyungang Jiangsu 222001 (CN); XIAO, Wei, Lianyungang Jiangsu 222001 (CN)
(74) Representative: Cabinet Bleger-Rhein-Poupon
(86) International application number: PCT/CN2015/077043
(87) International publication number: WO 2015/165340

(56) References cited:
- EP-A1- 0 501 568
- WO-A2-2012/134233
- US-A1- 2004 116 476
- US-B2- 6 706 739
- HOLMES, IAN P. ET AL.: 'The identification of a selective dopamine D2 partial agonist, D3 antagonist displaying high levels of brain exposure' BIOORGANIC & MEDICAL CHEMISTRY LETTERS vol. 20, no. 6, 25 January 2010, ISSN 0960-894X pages 2013 - 2016, XP027546795

## Description

### FIELD OF TECHNOLOGY

The present invention relates to a novel anti-enterovirus 71 (EV71) 1,2,5-thiadiazolidine-1,1-dioxide derivative or a pharmaceutically-acceptable salt(s) thereof, specifically a compound(s) represented by Formula (II) or a pharmaceutically-acceptable salt(s) thereof.

### BACKGROUND

The enterovirus 71 is a member of small RNA virus family and one of the most common etiologies of hand-foot-mouth disease. It can also cause various diseases related to nervous system such as herpes pharyngitis, aseptic meningitis, encephalitis, and poliomyelitis-like paralysis and is possibly accompanied with severe central nervous system complications or neuritic pulmonary edema.

The hand-foot-mouth disease features are strong epidemic intensity, strong infectivity, and complex transmission route. There is no specific anti-enterovirus 71 medicine yet.

Although the existing technologies such as the patents of US20030087936, US6706739, US20040116476, US20050267164, and US20070049623 have disclosed a serie of structures, for example the structures represented by Formula (B-I), it still needs in urgency to develop a novel compound with better activity that is more beneficial to make medicine.

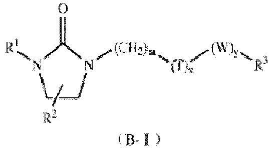

### SUMMARY

The present invention provides a compound represented by Formula (II) or a pharmaceutically-acceptable salt(s) thereof; 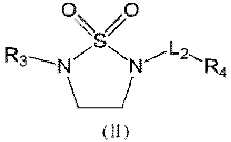 Wherein:
R₃ is any selected from 5-membered heterocycles , C_{6∼12} aryl groups, C_{6∼12} aralkyl groups, C_{5∼12} heteroaromatic rings and C_{5∼12} heteroaryl groups substituted with R₀₁;
L₂ is independently selected respectively from 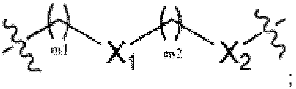
m₁ and m₂ are independently selected respectively from 1, 2 or 3;
X₁ and X₂ are independently selected respectively from -C(R_{d1})(R_{d2})-, -C(=O)N(R_{d3})-, -N(R_{d4})-, -C(=NR_{d5})-, -S(=O)₂N(R_{d6})-, -S(=O)N(R_{d7})-, -O-, -S-, -C(=O)O-, -C(=O)-, -C(=S)-, -S(= O)- or -S(=O)₂;
R₄ is selected from 5∼14-membered cyclic hydrocarbyl or heterocycloalkyl groups;
R_{d1} and R_{d2} are independently selected respectively from H, F, Cl, Br, I, CN, OH, SH, NH2, CHO, COOH or any selected from C₁₋₁₀ alkyl or heteroalkyl groups and C₃₋₁₀ cyclic hydrocarbyl or heterocycloalkyl groups substituted with R₀₁, C₁₋₁₀ alkyl or heteroalkyl groups substituted with C₃₋₁₀ hydrocarbyl or heterocycloalkyl groups;
R₀₁ is selected from F, Cl, Br, I, CN, OH, SH, NH₂, CHO, COOH and R₀₂;
R₀₂ is selected from C₁₋₁₀ alkal groups, C₁₋₁₀ alkylamino groups, N,N-di(C₁₋₁₀ alkayl) amino groups, C₁₋₁₀ alkoxy groups, C₁₋₁₀ alkanoyl groups, C₁₋₁₀ alkoxycarbonyl groups, C₁₋₁₀ alkylsulfonyl groups, C₁₋₁₀ alkylsulfinyl groups, C₃₋₁₀ cycloalkyl groups, C₃₋₁₀ cycloalkylamino groups, C₃₋₁₀ heterocyclic alkyl amino groups, C₃₋₁₀ cycloalkoxy groups, C₃₋₁₀ cycloalkyl acyl groups, C₃₋₁₀ cycloalkoxy-carbonyl groups, C₃₋₁₀ cycloalkylsulfonyl groups and C₃₋₁₀ cycloalkylsulfinyl groups;
The "hetero" denotes a hetero atom or hetero atom radical selected from -C(=O)N(R_{d3})-, -N(R_{d4})-, -C(=NR_{d5})-, -S(=O)₂N(R_{d6})-, -S(=O)N(R_{d7})-, -O-, -S-, =O, =S, -C(=O)O-, -C(=O)-, -C(=S)-, -S(=O)- and/or -S(=O)₂-;
R_{d3}-_{d7} are independently selected respectively from H and R₀₃;
R₀₃ is selected from C₁₋₁₀ alkyl groups, C₁₋₁₀ alkanoyl groups, C₁₋₁₀ alkoxycarbonyl groups, C₁₋₁₀ alkylsulfonyl groups, C₁₋₁₀ alkylsulfinyl groups, C₃₋₁₀ cycloalkyl groups, C₃₋₁₀ cycloalkyl acyl groups, C₃₋₁₀ cycloalkoxy-carbonyl groups, C₃₋₁₀ cycloalkylsulfonyl groups and C₃₋₁₀ cycloalkylsulfinyl groups.
R₀₂ and R₀₃ are optionally substituted with R₀₀₁;
R₀₀₁ is selected from F, Cl, Br, I, CN, OH, N(CH₃)₂, NH(CH₃), NH₂, CHO, COOH, trifluoromethyl group, aminomethyl group, hydroxymethyl group, methyl group, methoxy group, formyl group, methoxycarbonyl group, methylsulfonyl group and methylsulfinyl group;
The number of R₀₁, R₀₀₁ and hetero atom or hetero atom radical is independently selected respectively from 0, 1, 2 or 3;
Optionally, R_{d1} and R_{d2} interconnect to a 3 or 4-membered carbocyclic or heterocyclic ring.

In some schemes according to the present invention, R₃ is any selected from substituted 5 ∼ 6 membered aryl and substituted heteroaryl group.

In some schemes according to the present invention, R₃ is any selected from pyridyl group, phenyl group, furanyl group, pyrazolyl group, pyrrolyl group, thiazolyl group, pyridazinyl group, pyrimidinyl group, and thienyl group substituted with R₀₁ and the definition of R₀₁ is as above and the number of R₀₁ is selected from 0, 1, 2 or 3.

In some schemes according to the present invention, R₀₁ is selected from F, Cl, Br, I, CN, OH, SH, NH₂, CHO, COOH, Me,

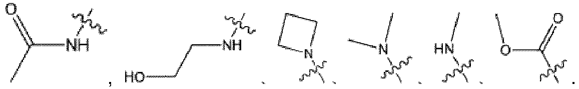

In some schemes according to the present invention, R₃ is selected from the following:

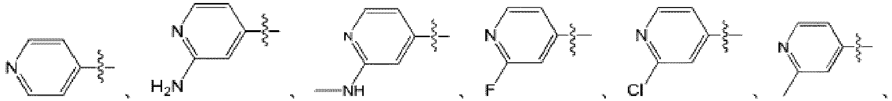

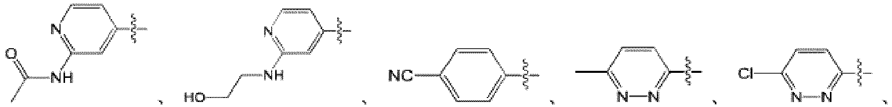

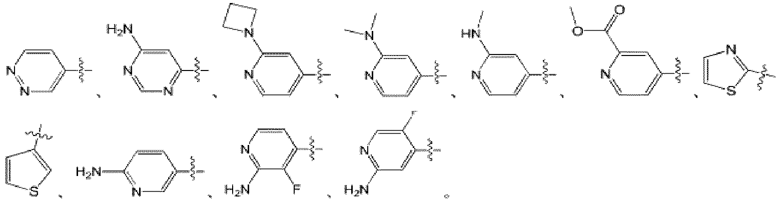

In some schemes according to the present invention, X₁ and X₂ are independently selected respectively from, -O-, -C(=O)-, -CH(CH₃)-, -C(CH₃)₂-, -CF₂-, -CH(F)-, 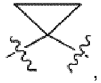 -CH(OH), -CH₂-, -NH- and -N(CH₃)-.

In some schemes according to the present invention, L₂ is selected from the following:

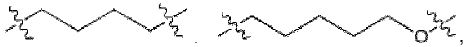

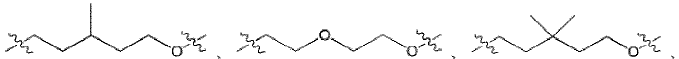

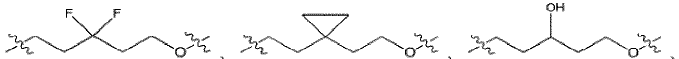

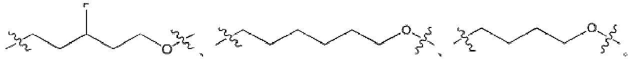

In some schemes according to the present invention, R₄ is selected from the following: 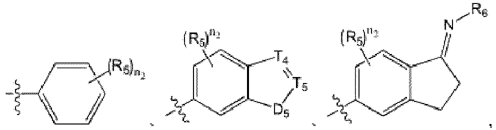 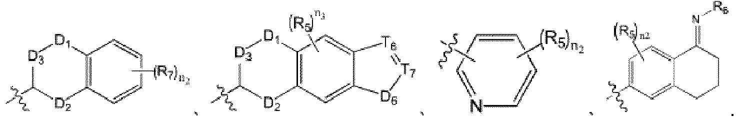
T₄₋₇ are independently selected respectively from N or C(Rₜ).
D₁₋₃, D₅ and D₆ are independently selected respectively from single bond, -C(R_{d1})(R_{d2})-, -C(= O)N(R_{d3})-, -N(R_{d4})-, -C(=NR_{d5})-, -S(=O)₂N(R_{d6})-, -S(=O)N(R_{d7})-, -O-, -S-, -C(=O)O-, -C(=O)-, -C(=S)-, -S(=O)- or -S(=O)₂-,
n₂ is selected from 0, 1, 2 or 3,
n₃ is selected from 0, 1 or 2,
R₅, R₆, R₇, Rₜ, R_{d1} and R_{d2} are independently selected respectively from H, F, Cl, Br, I, CN, OH, SH, NH₂, CHO and COOH or any selected from C₁₋₁₀ alkyl or heteroalkyl groups and C₃₋₁₀ cyclic hydrocarbyl or heterocycloalkyl groups substituted with R₀₁, C₁₋₁₀ alkyl or heteroalkyl groups substituted with C₃₋₁₀ hydrocarbyl or heterocycloalkyl groups. Other variables are defined as in claim 1.

In some schemes according to the present invention, R₄ is selected from the following: 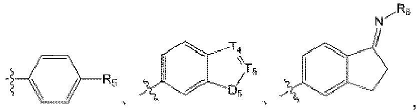 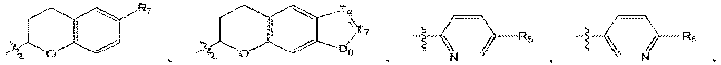 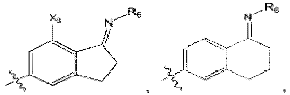 X₃ is selected from F, Cl, Br, I, CN, OH, SH, NH₂, CHO, COOH or any selected from C₁₋₁₀ alkyl or heteroalkyl groups and C₃₋₁₀ cyclic hydrocarbyl or heterocycloalkyl groups substituted with R₀₁, C₁₋₁₀ alkyl or heteroalkyl groups substituted with C₃₋₁₀ hydrocarbyl or heterocycloalkyl groups.

In some schemes according to the present invention, R₅₋₇ are independently selected respectively from the following: 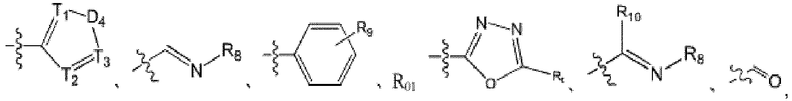 wherein:
T₁₋₃ are independently selected respectively from N or C(Rₜ);
D₄ is selected from -C(R_{d1})(R_{d2})-, -C(=O)N(R_{d3})-, -N(R_{d4})-, -C(=NR_{d5})-, -S(=O)₂N(R_{d6})-, -S(= O)N(R_{d7})-, -O-, -S-, -C(=O)O-, -C(=O)-, -C(=S)-, -S(=O)- or -S(=O)₂-;
R₈, R₉ and Rₜ are independently selected respectively from H, F, Cl, Br, I, CN, OH, SH, NH₂, CHO, COOH or any selected from C₁₋₁₀ alkyl or heteroalkyl groups and C₃₋₁₀ cyclic hydrocarbyl or heterocycloalkyl groups substituted with R₀₁, C₁₋₁₀ alkyl or heteroalkyl groups substituted with C₃₋₁₀ hydrocarbyl or heterocycloalkyl groups;
R₁₀ is selected from F, Cl, Br, I, CN, OH, SH, NH₂, CHO, COOH or any selected from C₁₋₁₀ alkyl or heteroalkyl groups and C₃₋₁₀ cyclic hydrocarbyl or heterocycloalkyl groups substituted with R₀₁, C₁₋₁₀ alkyl or heteroalkyl groups substituted with C₃₋₁₀ hydrocarbyl or heterocycloalkyl groups.

In some schemes according to the present invention, R₅₋₉, R₀₁ and Rₜ are independently selected respectively from the following: 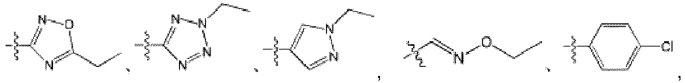 F, Cl, Br, CF₃, -C(C₂H₅)-, -C(OC₂H₅)-, methyl, ethyl, propyl, methoxy, ethoxy, propoxy and

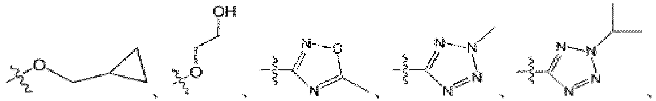

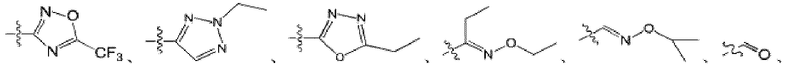

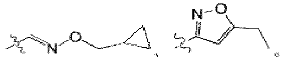

In some schemes according to the present invention, R₄ is selected from the following:

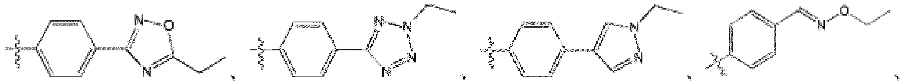

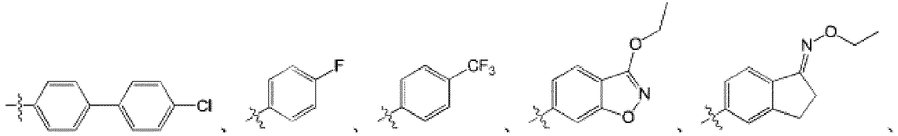

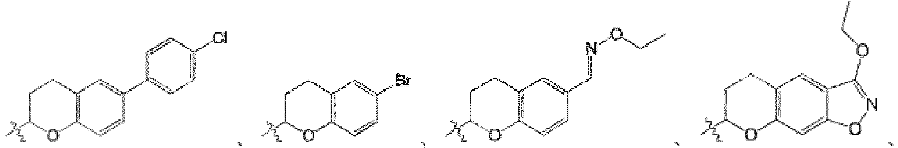

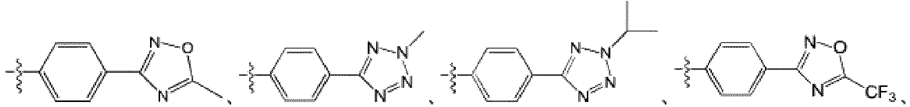

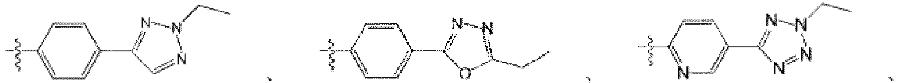

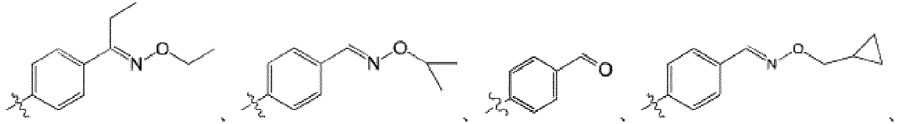

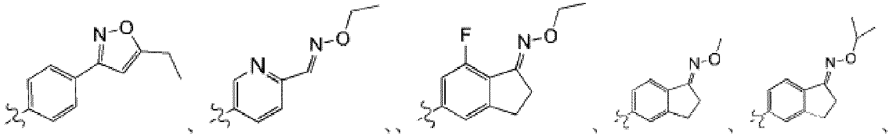

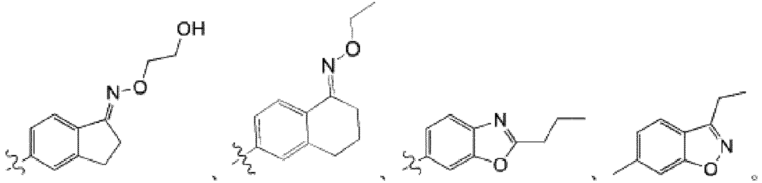

Specifically, the present invention selects it from the following:

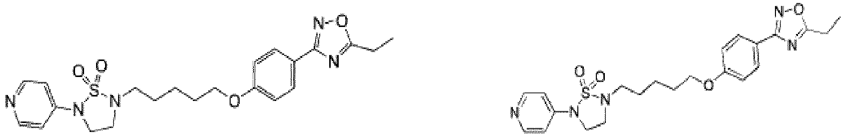

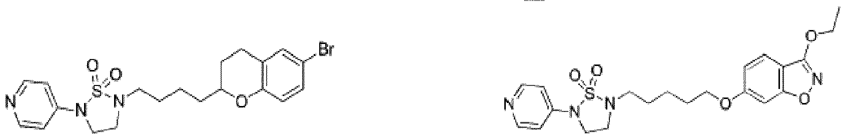

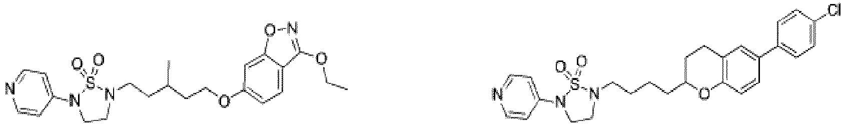

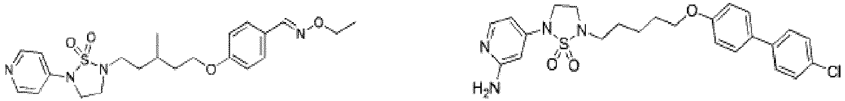

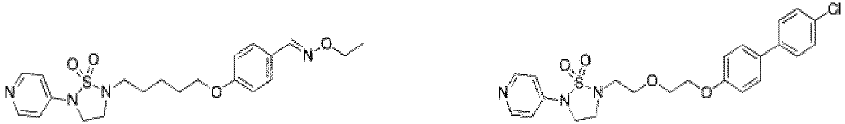

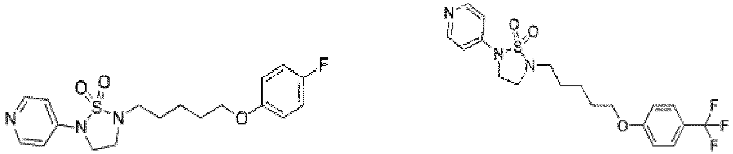

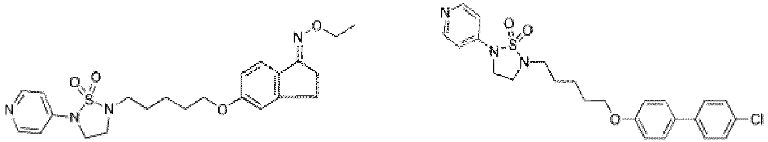

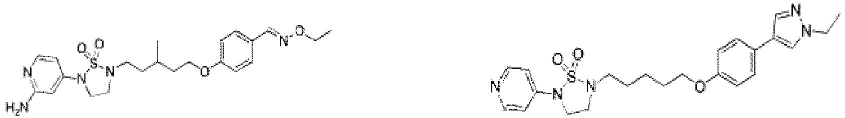

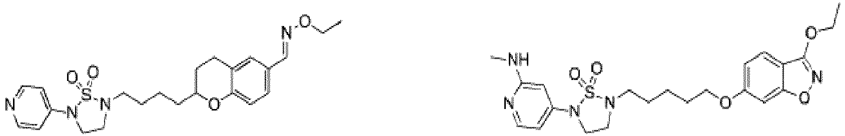

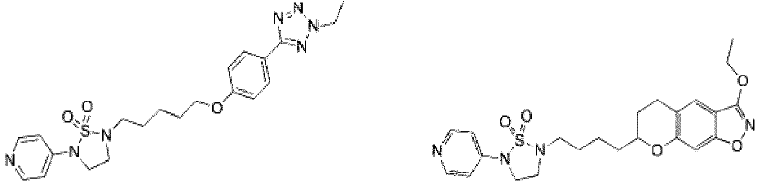

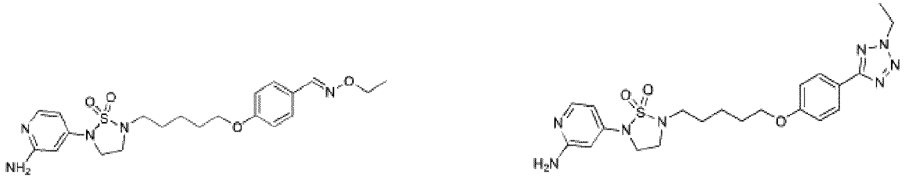

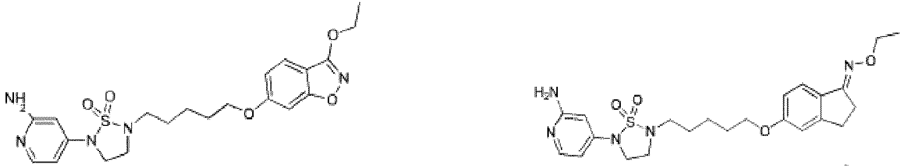

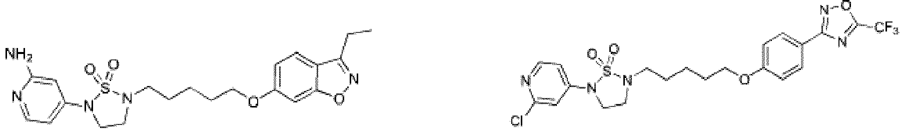

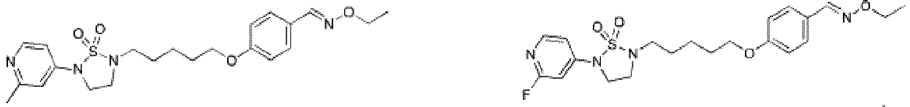

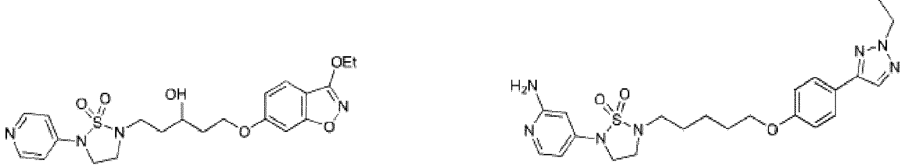

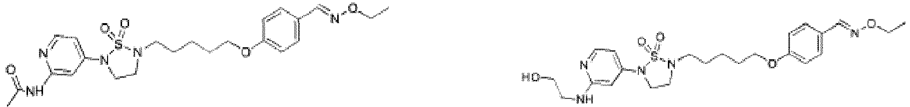

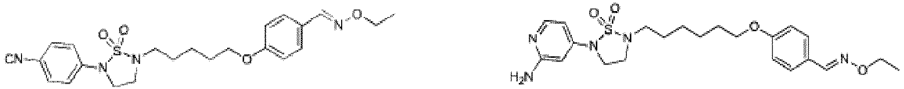

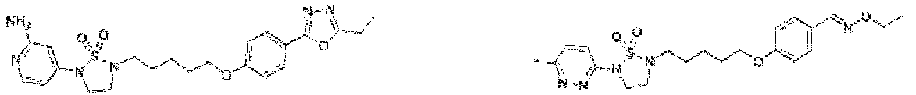

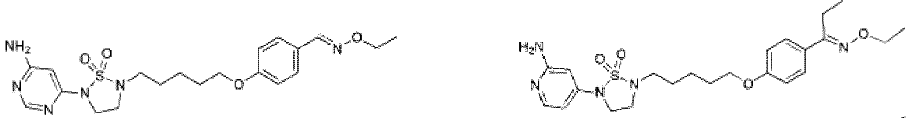

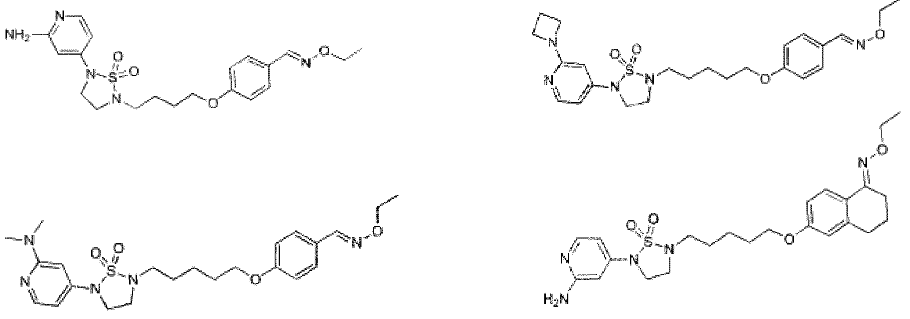

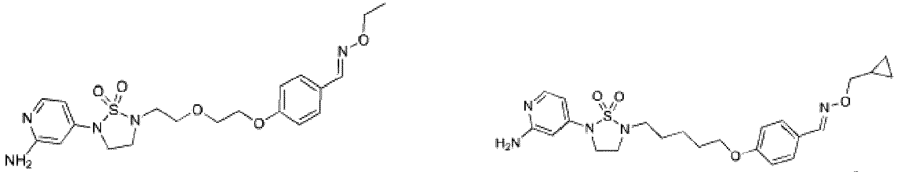

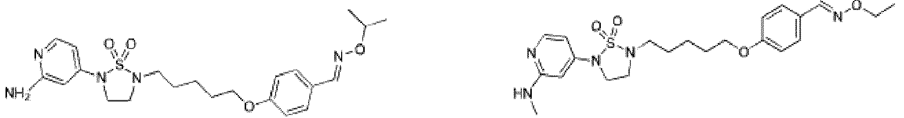

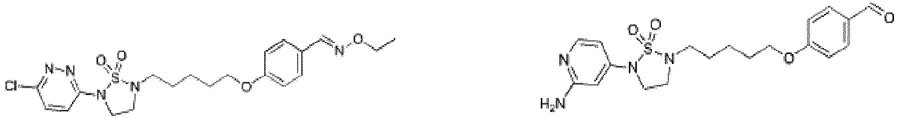

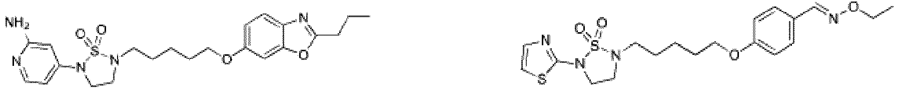

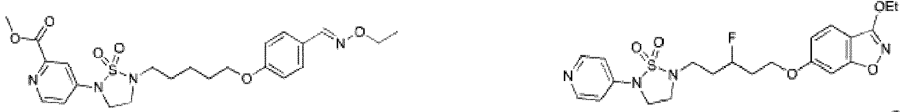

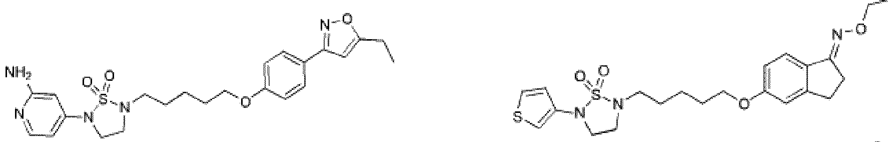

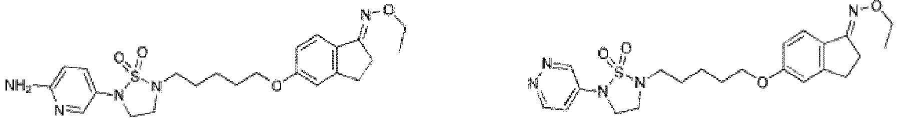

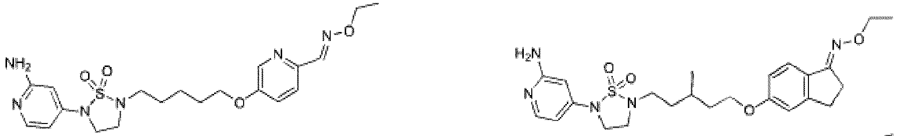

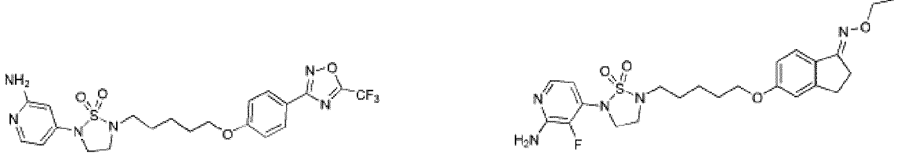

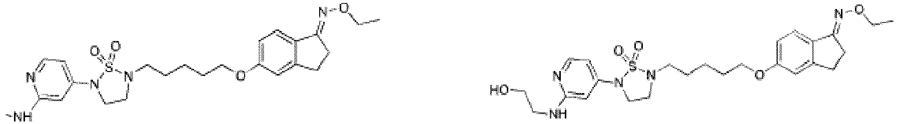

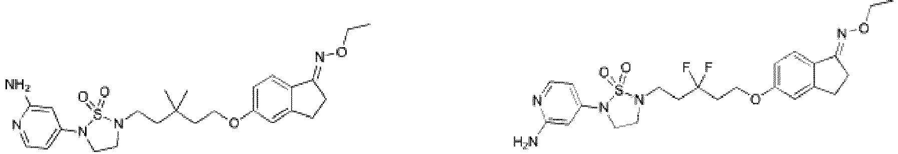

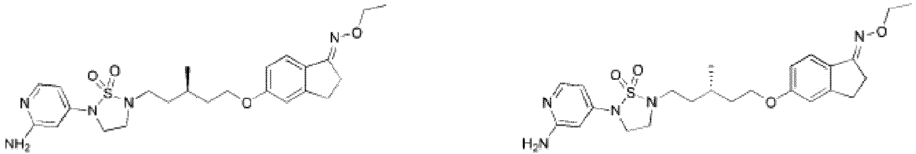

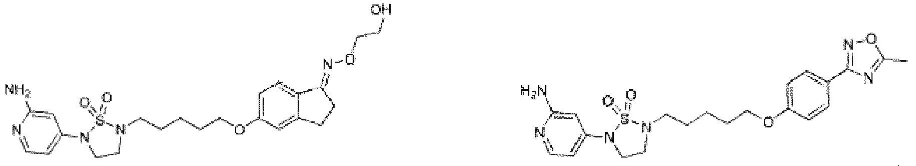

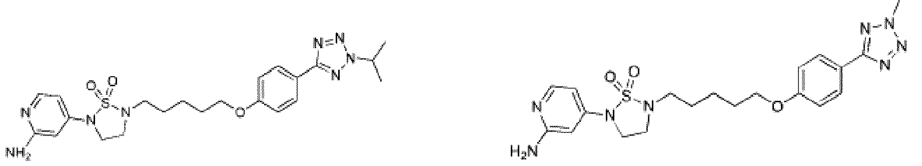

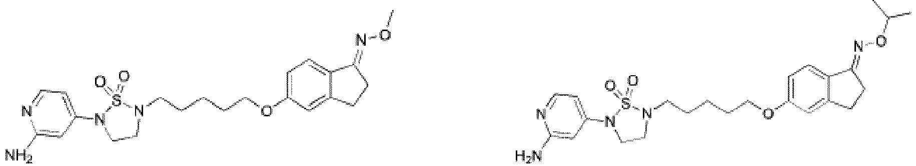

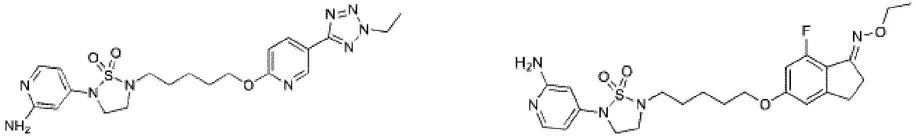

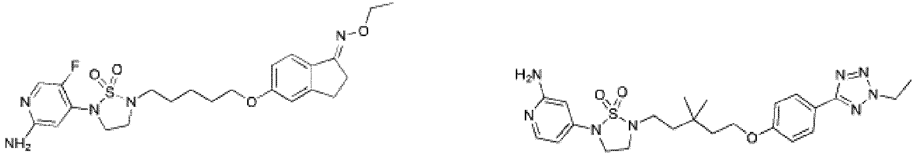

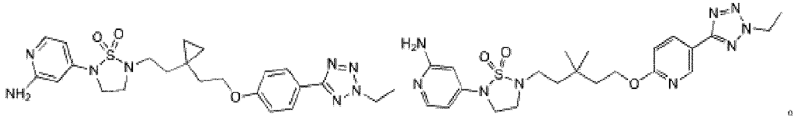

### Relevant definitions:

Unless indicated otherwise, the following terms and phrases used here are intended to have the following meanings. Any specific term or phrase without specific definition should not be regarded as uncertain or indistinctive but should be understood as its common meaning. Any trade name appearing in the present invention denotes the corresponding product it refers to or its active ingredient.

C₁₋₁₂ are selected from C₁, C₂, C₃, C₄, C₅, C₆, C₇, C₈, C₉, C₁₀, C₁₁ and C₁₂; C₃₋₁₂ are selected from C₃, C₄, C₅, C₆, C₇, C₈, C₉, C₁₀, C₁₁ and C₁₂.

C₁₋₁₂ alkyl or heteroalkyl group, C₃₋₁₂ cyclic or heterocycloalkyl group and C₁₋₁₂ alkyl or heteroalkyl group substituted with C₃₋₁₂ cycloalkyl or heterocycloalkyl group include but not limited to:

C₁₋₁₂ alkal groups, C₁₋₁₂ alkylamino groups, N,N-di(C₁₋₁₂ alkayl) amino groups, C₁₋₁₂ alkoxy groups, C₁₋₁₂ alkanoyl groups, C₁₋₁₂ alkoxycarbonyl groups, C₁₋₁₂ alkylsulfonyl groups, C₁₋₁₂ alkylsulfinyl groups, C₃₋₁₂ cycloalkyl groups, C₃₋₁₂ cycloalkylamino groups, C₃₋₁₂ heterocyclic alkyl amino groups, C₃₋₁₂ cycloalkoxy groups, C₃₋₁₂ cycloalkyl acyl groups, C₃₋₁₂ cycloalkoxy-carbonyl groups, C₃₋₁₂ cycloalkylsulfonyl groups and C₃₋₁₂ cycloalkylsulfinyl groups, 5 ∼ 12 membered aryl or heteroaryl groups and 5 ∼ 12 membered aralkyl or heteroaralkyl groups;

methyl group, ethyl group, n-propyl group, isopropyl group, -CH₂C(CH₃)(CH₃)(OH), cyclopropyl group, cyclobutyl group, propyl methylene, cyclopropylmethyl group, benzyl group, trifluoromethyl group, aminomethyl group, hydroxymethyl group, methoxy group, formyl group, methoxycarbonyl group, methylsulfonyl group, methylsulfinyl group, ethoxy group, acetyl group, ethanesulfonyl group, ethoxycarbonyl group, dimethyl amino group, diethylamino group, dimethylamino-carbonyl group and diethyl-aminocarbonyl group; N(CH₃)₂,NH(CH₃),-CH₂CF₃,-CH2CH₂CF₃,-CH₂CH₂F,-CH₂CH₂S(= O)₂CH₃,-CH₂CH₂CN, 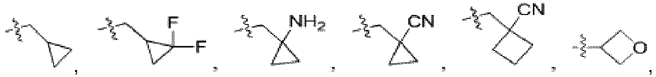 -CH₂CH(OH)(CH3)₂, -CH₂CH(F)(CH₃)₂, -CH₂CH₂F, -CH₂CF₃, -CH₂CH₂CF₃, -CH₂CH₂NH₂, -CH₂CH₂OH, -CH₂CH₂OCH₃, -CH₂CH₂CH₂OCH₃, -CH₂CH₂N(CH₃)₂, -S(=O)₂CH₃, -CH₂CH₂S(= O)₂CH₃, 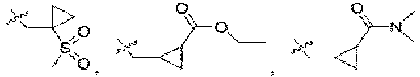 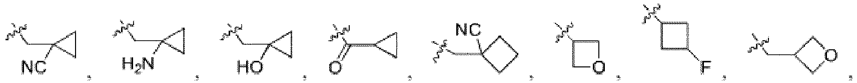 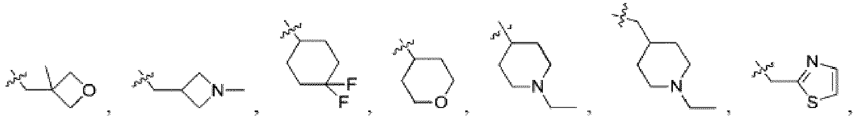 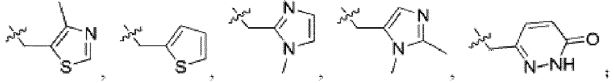 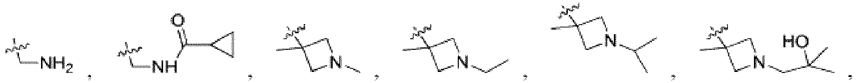 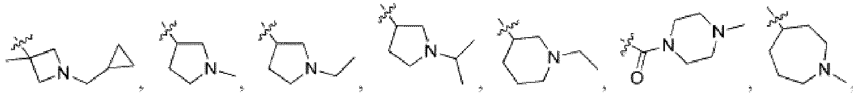 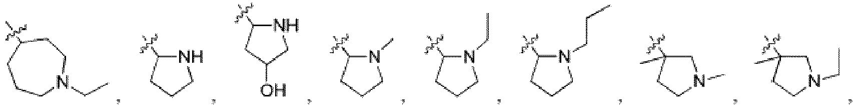 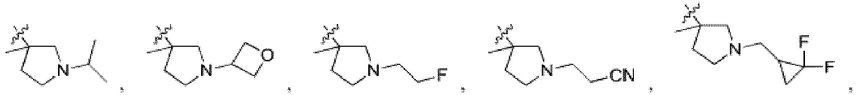 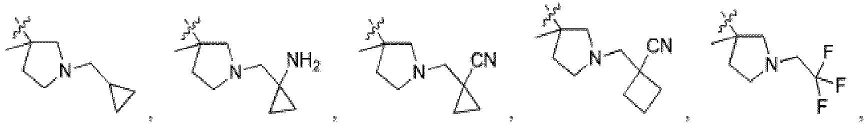 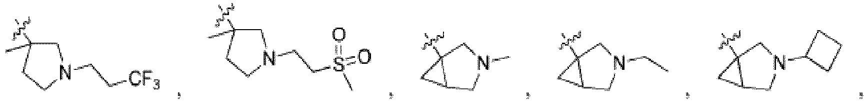 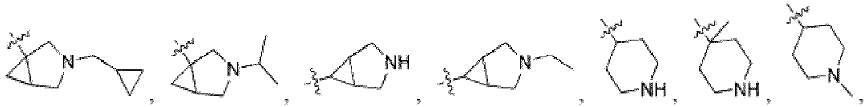 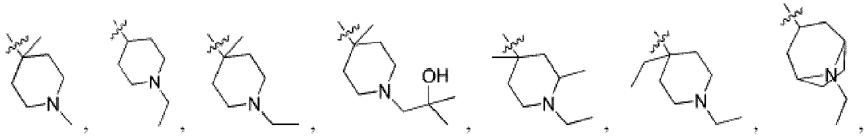 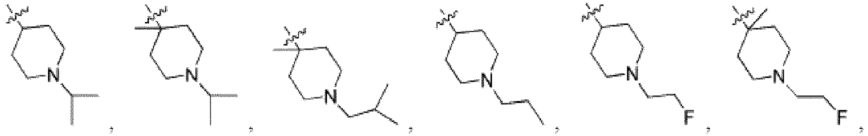 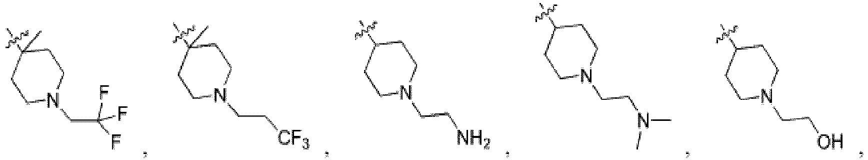 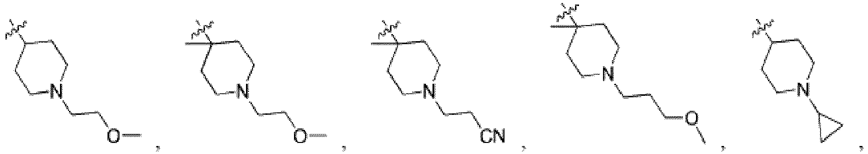 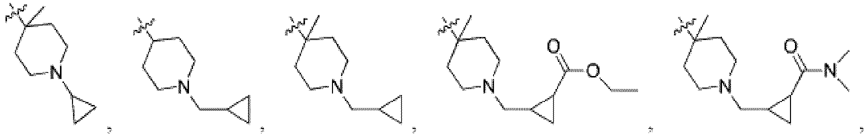 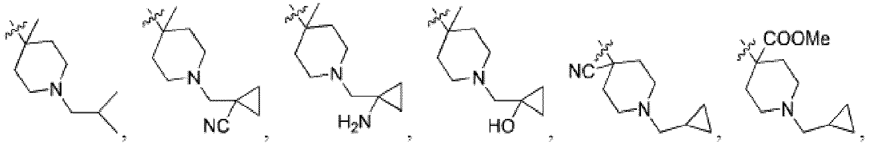 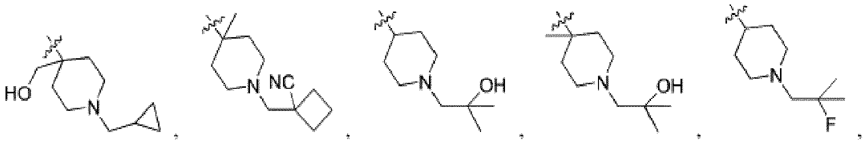 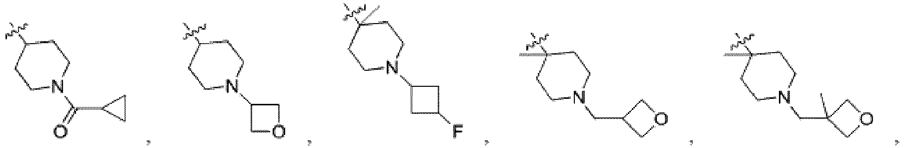 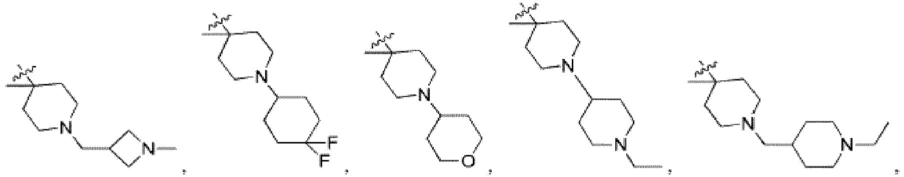 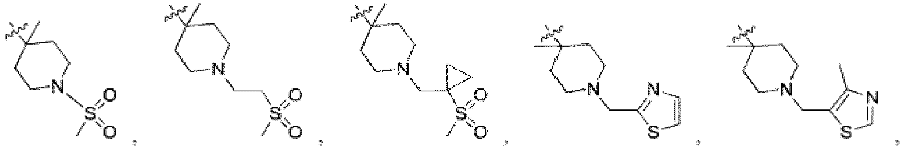 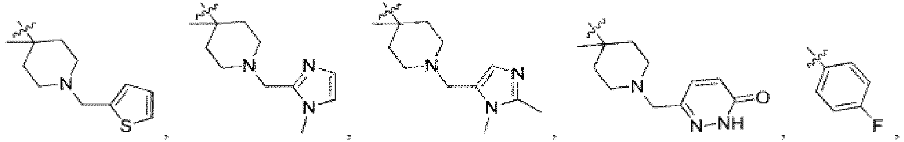 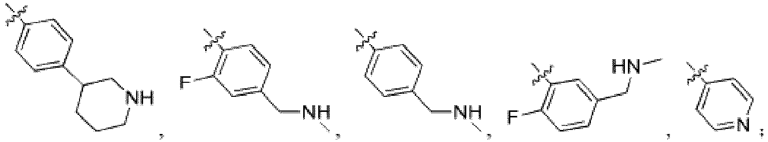 and phenyl group, thiazolyl group, biphenyl group, naphthyl group, cyclopentyl group, furyl group, 3-pyrrolinyl group, pyrrolidinyl group, 1,3-dioxolan, pyrazolyl group, 2-pyrazoline-yl group, pyrazolidinyl group, imidazolyl group, oxazolyl group, thiazolyl group, 1,2,3-oxadiazolyl group, 1,2,3-triazolyl group, 1,2,4-triazolyl group, 1,3,4 thiadiazolyl group, 4H-pyranyl group, pyridinyl group, piperidinyl group, 1,4-dioxan, morpholinyl group, pyridazinyl group, pyrimidinyl group, pyrazinyl group, piperazinyl group, 1,3,5-trithianyl group, 1,3,5-triazinyl group, benzofuranyl group, benzothienyl group, indolyl group, benzimidazolyl group, benzothiazolyl group, purinyl group, quinolyl group, isoquinolinyl group, cinnolinyl group or quinoxalinyl group;

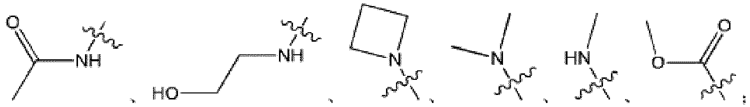

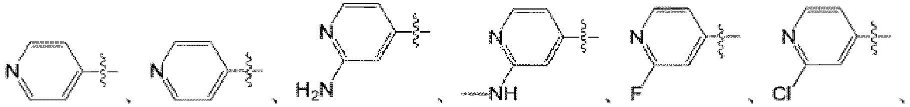

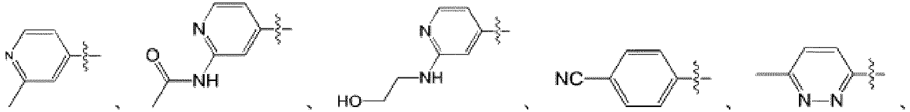

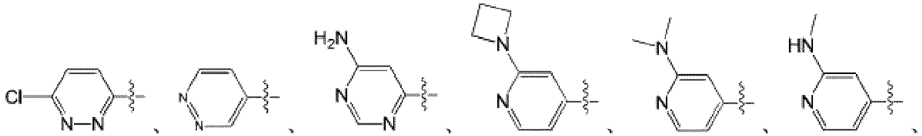

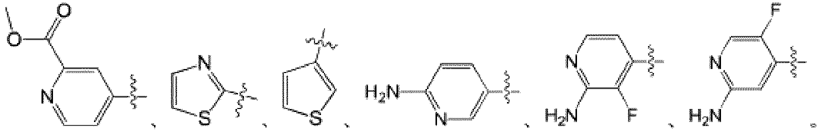

The term "pharmaceutically-acceptable" used here refers to the compound(s), material(s), composition(s) and/or dosage form(s) that is suitable within the scope of reliable medical judgments to be used in contact with the human and animal tissues, without posing excessive toxicity, irritation, allergic reactions or other issues or complications and with reasonable benefit / risk ratio.

The term "pharmaceutically-acceptable salt(s)" refers to the salt(s) of the compound(s) according to the present invention that is prepared from the compound(s) with specific substituting group developed according to the present invention and relatively-nontoxic acid or base. If any compound according to the present invention contains a relatively-acidic functional group, an adequate amount of base can be added in its pure solution or appropriate inert solvent to contact with such compound in neutral form to yield a salt of base addition. The pharmaceutically-acceptable base(s) includes the salts of sodium, potassium, calcium, ammonia, organic ammonium or magnesium or similar salts. If any compound according to the present invention contains a relatively-basic functional group, an adequate amount of acid can be added in its pure solution or appropriate inert solvent to contact with such compound in neutral form to yield a salt of acid addition. The examples of the pharmaceutically-acceptable salt(s) include the inorganic acid salt(s) that includes hydrochloric acid, hydrobromic acid, nitric acid, carbonate, bicarbonate, phosphate, hydrogen phosphate, phosphate, dihydrogen phosphate, sulfate, bisulfate, hydroiodic, phosphorous, etc, and the organic acid salt(s) that includes acetic acid, propionic acid, isobutyric acid, maleic acid, propionic acid, benzoic acid, succinic acid, suberic acid, fumaric acid, lactic acid, mandelic acid, phthalic acid, benzenesulfonic acid, p-toluene sulfonic acid, citric acid, tartaric acid, methanesulfonic acid, etc, and also the salt(s) of amino acid(s) (such as arginine) as well as the salt(s) of organic acid(s) such as glucuronic acid (see Berge et al.,"Pharmaceutical Salts", Journal of Pharmaceutical Science 66:1-19(1977)). Some specific compounds according to the present invention contain basic and acidic functional group(s) and hence can be transformed into any salt of basic or acid addition.

Preferentially, the salt contacts with base or acid in a conventional way before the parent compounds are separated to regenerate the neutral form of compounds. The differences between the compounds in parent form and in the form of various salts lie in some physical properties, e.g. the different solubility in polar solvent.

The "pharmaceutically-acceptable salt(s)" used here is (are) the derivative(s) of the compound according to the present invention, wherein said parent compound(s) is (are) modified by the means of salification with acid or salification with base. The examples of pharmaceutically-acceptable salt(s) include but are not limited to inorganic acid salt(s) or organic acid salt of basic group such as amine and the alkali metal or organic salt(s) of acid group such as carboxylic acid, etc. The pharmaceutically-acceptable salt(s) includes the conventional non-toxic salt(s) or the quaternary ammonium salts of parent compound, e.g. the salt(s) produced from non-toxic inorganic acid or organic acid. The conventional non-toxic salt(s) includes but is not limited to those salts derived from inorganic acid and organic acid that is selected from 2-acetoxybenzoic acid, 2-hydroxyethane sulfonic, acetic, ascorbic, benzenesulfonic, benzoic, bicarbonate radical, carbonate, citric acid, edetic acid, ethane disulfonic acid, ethanesulfonic acid, fumaric acid, glucoheptonic acid, gluconic acid, glutamic acid, glycolic acid, hydrobromic acid, hydrochloric acid, hydroiodide acid, hydroxyl, hydroxynaphthoic acid, isethionic acid, lactic acid, lactobionic acid, dodecyl sulfonic acid, maleic acid, malic acid, mandelic acid, methane sulfonic acid, nitric acid, oxalic acid, pamoic acid, pantothenic acid, phenylacetic acid, phosphoric acid, poly-galacturonic acid, propionic acid, salicylic acid, stearic acid, sub-acetic acid, succinic acid, sulfamic acid, sulfanilic acid, sulfuric acid, tannin, tartaric acid and p-toluenesulfonic acid.

The pharmaceutically-acceptable salt according to the present invention can be synthesized with the conventional chemical method(s) from the parent compounds containing acidic or basic group(s). Generally, the methods of preparing such salt are as follows: in water or organic solvent or in a mixture of them, these compounds in the form of free acid or base react with appropriate base or acid in stoichiometry. Usually, it is preferable to select a non-aqueous media such as ether, ethyl acetate, ethanol, isopropanol, or acetonitrile.

In addition to the form of salt, the compound(s) provided according to the present invention also include the form of prodrug. The prodrug of a compound(s) described here can easily undergo chemical change under physiological conditions so as to transform to the compound(s) according to the present invention. In addition, the prodrug can be transformed into the compound(s) according to the present invention via a chemical or biochemical method in an in-vivo environment.

Some compounds according to the present invention can exist in the form of non-solvation or solvation, including the form of hydrate. In general, the form of solvation and the form of non-solvation are equivalent to each other and both covered within the scope of the present invention. Some compounds according to the present invention can exist in the form of polycrystal or amorphism.

Some compounds according to the present invention can contain asymmetric carbon atoms (optical center) or double bond. The raceme, diastereoisomer, geometric isomer and single isomer are all covered within the scope of the present invention.

The graphic method for the pure compounds of racemate, ambiscalemic and scalemic or antipode is sourced from Maehr, J. Chem. Ed. 1985, 62:114-120. 1985, 62: 114-120. Unless indicated otherwise, the wedge key and the dotted-line key are used to indicate the absolute configuration of a stereocenter. If said compounds here contain olefinic double bond or other asymmetric geometry center, they should include the geometric isomers of *E* and *Z.* Similarly, all the tautomeric forms shall be covered within the scope of the present invention.

The compound(s) according to the present invention can present in the form of specific geometric isomer or stereoisomer. All these compounds proposed by the present invention, including the cis- and trans- isomers, (-)- and (+) - enantiomers, (*R*)- and (*S*)- enantiomers, diastereoisomer, (*D*)-isomer, (*L*)- isomer and their racemic mixtures and other mixtures, e.g. the mixture enriched in enantiomer or non-enantiomer, are all covered within the scope of the present invention. The substituting group such as alkyl may contain other asymmetric atoms. All these isomers and their mixtures are covered within the scope of the present invention.

The chiral synthesis or chiral reagent or other routine techniques can be used to prepare the (*R*)- and (*S*)-isomers as well as *D* and *L* isomers with optical activity. If an enantiomer of some compound according to the present invention is desired, it can be prepared through asymmetric synthesis or the derivatization effect of chiral auxiliary reagent, where the prepared non-enantiomer mixture is separated and the auxiliary group is split to provide the necessary enantiomer. Or, if a molecule contains basic function group (e.g. amino) or acid functional group (e.g. carboxyl), it can form the salt of non-enantiomer with appropriate acid or base with optical activity before the non-enantiomer is separated via the fractional crystallization process or chromatography generally known by those skilled in the art and then recovered to obtain the pure enantiomer. In addition, the separation between enantiomer and non-enantiomer is usually finished via chromatography, which utilizes chiral stationary phase and necessarily combines with chemical derivatization method (e.g. an amine is used to produce carbamate).

The compound(s) according to the present invention may contain a non-natural ratio of isotope of one or more atoms constituting the compound. e.g., the radioactive isotope labeled compound such as tritium (³H), I-125 (¹²⁵I) or C-14(¹⁴C) can be used. All the transformations of the isotope composition of a compound according to the present invention shall, regardless of being radioactive or not, be covered within the scope of the present invention.

The term "pharmaceutically-acceptable carrier" refers to any preparation or carrier medium that can deliver an effective dose of active substance according to the present invention, not interfere with the biological activity of the active substance and pose no toxic and side effect to host or patient. The representative carriers include water, oil, vegetable and mineral, paste, lotion base and ointment base, etc. These bases include suspending agent, tackifier and transdermal enhancer, etc. Their preparations are known by the technicians in cosmetics and toiletries or in the partial pharmaceutical field. As for other information of the carriers, the following reference is available "Remington: The Science and Practice of Pharmacy, 21st Ed., Lippincott, Williams & Wilkins (2005)", whose content are incorporated into this patent by means of referring.

The term "excipient" usually refers to the carriers, diluents, and/or medium necessary to prepare effective pharmaceutical composition.

With regard to a drug or a pharmacological active agent, the term "effective dose" or "therapeutically effective dose" refers to the level of a drug or agent that is non-toxic but adequate to achieve expected effect. As for the oral dosage form in the present invention, the "effective dose" of an active substance in the composition refers to the level necessary to achieve the expected effect in the case of combination with other active substance. The determination of such effective dose varies from person to person, depending on the age of receptor and the general conditions as well as the specific active substance. The individual appropriate effective dose can be determined according to the routine experiments by those skilled in the art.

The terms "active ingredient", "therapeutic agent", "active substance" or "active agent" refer to a chemical entity that can effectively cure a targeted disorder, disease or symptom.

The term "substituted" refers to that any one or several hydrogen atoms bonding to specific atom are replaced by substituting groups including deuterium or the variants of hydrogen as long as the valence state of the specific atom is normal and the compound after substitution is stable. If the substituting group is a ketone group (i.e., =O), it means that two hydrogen atoms are substituted. The ketone substitution will not occur to an aryl group. The term "optionally substituted" refers to that it can optionally be substituted or not substituted. Unless specified otherwise, the type and number of the substituting group are random on a chemically-achievable basis.

In the event that any variable (e.g. R) appears more than once in the composition or structure of a compound, its definition is independent on a case by case basis. For example, if one group is substituted by 0-2 R, said group can be optionally substituted by up to two R and each of them can be selected independently in each case. Moreover, any combination of substituting groups and/or their variants can only be acceptable if such combination can give a stable compound.

If one variable of them is selected from those with single bond, it means that the two groups it bonds to are connected directly, e.g. the single bond of "L" in "A-L-Z" denotes an actual structure of A-Z.

If the bond of a substituting group can be cross-linked to two atoms of one ring, such substituting group can bond with any atom on the ring. If the enumerated substituting group is not specified as for the atom to bond a compound covered by the general formula of a chemical structure but not mentioned specifically, such substituting group can bond with any atom of it. Any combination of the substituting groups and/or their variants can only be acceptable if such combination can give a stable compound. e.g., the structural unit 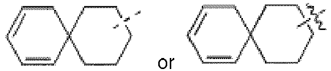 indicates that it can substitute any position of the cyclohexyl group or cyclo-diene.

The substituting groups of alkyl and heteroalkyl radical are usually referred to as "alkayl substituents." They can be selected from but not limited to one or more from the following groups: -R', -OR', =O, = NR', = N-OR', -NR'R" , -SR', halogen, -SiR'R" R''', OC(O)R', -C(O)R', -CO₂R', -CONR'R", -OC(O)NR'R", -NR"C(O)R', NR'C(O)NR"R''', -NR"C(O)₂R', -NR''''' -C(NR'R"R''')=NR'''', NR''''C(NR'R") =NR"', -S(O)R', -S(O)₂R', -S(O)₂NR'R", NR"SO₂R', -CN, -NO₂, -N₃, -CH(Ph)₂ and fluoro(C₁-C₄) alkayl groups. The number of substituting groups is 0 ∼ (2m'+1), where m' is the total number of carbon atoms in these atom radicals. The R', R", R''', R'''' and P''''' are independently selected preferentially from hydrogen, the substituted or unsubstituted heteroalkyl groups, substituted or unsubstituted aryl groups (e.g., those substituted by 1 ∼ 3 halogen atoms), the substituted or unsubstituted alkyl groups, alkoxy groups, thioalkoxy radicals or aralkyl groups. If a compound according to the present invention contains more than one R group, e.g., each R group is independently selected as if it is any from more than one R', R", R''', R'''' and R'''''. If R' and R" bond with the same N atom, they can from a 5-, 6- or 7-membered ring with this N atom, e.g., -NR'R" refers to include but not limited to 1-pyrrolidinyl and 4-morpholinyl. As per the aforementioned discussion of substituting group, those skilled in the art can understand the term "alkyl group" refers to include the groups formed by the carbon atoms bonding with non-hydrogen groups, for example halogenated alkyls (e.g. -CF₃, -CH₂CF₃) and acyl groups (e.g. -C(O)CH₃, -C(O)CF₃, -C(O)CH₂OCH₃, etc).

Similar to said substituting groups of alkyl groups, the substituting groups of aryl groups and heteroaryl groups are generally referred to as "aryl substituents" and are selected from, e.g. -R', -OR', -NR'R", -SR', -halogen, -SiR'R" R''', OC(O)R', -C(O)R, -CO2R', -CONR'R", -OC(O)NR'R", -NR"C(O)R', NR'C(O)NR"R''', -NR"C(O)2R', -NR'''''-C(NR'R"R''')=NR'''', NR''''C(NR'R")=NR''', -S(O)R', -S(O)₂R', -S(O)₂NR'R", NR"SO₂R', -CN, -NO₂, -N₃, -CH(Ph)₂, fluoro(C₁-C₄) alkoxy and fluoro-(C₁-C₄) alkyl groups.The number of substituting groups is in the range of 0 to the total number of open valence of the aromatic ring, where R', R", R'", R'''' and R''''' are independently selected respectively from hydrogen, the substituted or unsubstituted alkyl groups, the substituted or unsubstituted hetero alkyl groups, the substituted or unsubstituted aryl groups and the substituted or unsubstituted hetero aryl groups. If a compound according to the present invention contains more than one R group, e.g., each R group is independently selected as if it is any from more than one R', R", R''', R'''' and R'''''.

The two substituting groups on two adjacent atoms of aryl group or hetero aryl group can be substituted by a group with the general formula as -T-C(O)-(CRR')q-U-, where T and U are independently selected respectively from -NR-, -O-, CRR'- or single bond and q is an integer of 0 and 3. As an alternative, the two substituting groups on two adjacent atoms of aryl group or hetero aryl group can be replaced by a substituting group with a general formula of -A(CH2)r B-, where A and B are independently selected respectively from -CRR'-, -O-, -NR-, -S-, -S(O)-, S(O)₂-, -S(O)₂NR'- or single bond and r is an integer of 1∼4. Optionally, one single bond on the new ring formed thereby can be replaced by double bond. As an alternative, the two substituting groups on two adjacent atoms of aryl group or hetero aryl group can be replaced by a substituting group with a general formula of -A(CH2)r B-, where s and d are integers of 0 ∼ 3 respectively selected independently, X is -O-, -NR', -S-, -S(O)-, -S(O)₂- or -S(O)₂NR'-. The substituting group R, R', R" and R"' are respectively selected independently from the preferential hydrogen and the substituted or unsubstituted (C₁-C₆) alkyl groups.

Unless specified otherwise, the term "halogenating element" or "halogen" denotes fluorine, chlorine, bromine or iodine atom by itself or as a part of other substituting group. In addition, the term "halogenated alkyl group" refers to the monohalogenated and polyhalogenated alkyl groups. For example, the term "halo(C₁-C₄)alkyl group" means to include but be not limited to trifluoromethyl, 2,2,2-trifluoroethyl, 4-chlorobutyl and 3-bromopropyl, etc.

The examples of halogenated alkyl groups include but are not limited to trifluoromethyl group, trichloromethyl group, pentafluoroethyl group and pentachlorophenol ethyl group. The "alkoxy group" denotes the aforementioned alkyl groups with specific number of carbon atoms linked by oxygen bridge. The C₁₋₆ alkoxy groups include those of C₁, C₂, C₃, C₄, C₅ and C₆. The examples of alkoxy groups include but are not limited to methoxy, ethoxy, n-butoxy, isobutoxy, n-butoxy, sec-butoxy, tert-butoxy, n-pentyloxy and S-pentyloxy group. The "cycloalkyl group" includes saturated ring group, e.g., cyclopropyl, cyclobutyl or cyclopentyl group. The 3-7 cycloalkyl group includes those of C₃, C₄, C₅, C₆ and C₇. The "alkenyl group" includes the linear or branched hydrocarbon chains, where any stable point on the chain has one or more carbon-carbon double bond, e.g. vinyl and allyl groups.

The term "halogen" or "halogen element" refers to fluorine, chlorine, bromine, and iodine.

Unless specified otherwise, the term "hetero" refers to a hetero atom or hetero atom radical (i.e., atom radical containing hetero atom), including the atoms except carbon (C) and hydrogen (H) and their atom radicals, e.g. O, N, S, Si, Ge, Al, B, -O-, -S-, =O, =S, -C(=O)O-, -C(=O)-, -C(=S)-, -S(= O), -S(=O)₂- and any one selected from the substituted -C(=O)N(H)-, -N(H)-, -C(=NH)-, -S(= O)₂N(H)- or -S(=O)N(H)-.

Unless specified otherwise, the "ring" denotes a substituted or unsubstituted cycloalkyl group, heterocycloalkyl group, cycloalkenyl group, heterocycloalkenyl group, cycloalkynyl group, heterocyclyl alkynyl group, aryl, or heteroaryl group. The so-called ring includes monocycle, linked ring, spiro ring, bi-ring or bridged ring. The number of atoms in the ring is generally defined as the member number of ring, e.g., the "5∼7 membered ring" means that 5 ∼ 7 atoms are contained to form a ring. Unless specified otherwise, this ring optionally contains 1 ∼ 3 hetero atoms. Thus, the "5∼7 membered ring" includes, e.g. phenyl pyridine and piperidinyl group, on the other hand, the term "5∼7 membered heterocycloalkyl ring" includes pyridyl group and piperidinyl group rather than phenyl group. The term "ring" includes the ring system containing at least one ring, where each "ring" independently conforms to the aforementioned definition.

Unless specified otherwise, the term "heterocycle" or "heterocyclic group" refers to a monocycle, bi-cycle and tricycle containing hetero atoms or hetero atom radical. They can be saturated, partially saturated or unsaturated (aromatic) and consist of carbon atoms and 1, 2, 3 or 4 hetero atoms independently selected from N, O, and S, where any of the aforementioned heterocycles can be condensed together with benzene ring to form a bi-cycle. The N and S hetero atoms can be optionally oxidized (i.e., NO and S(O)p). The N atom can be substituted or unsubstituted (i.e., N or NR where R is H or other substituting group already defined in the patent). The hetero ring can bond to the side group of any hetero atom or carbon atom to form stable structure. If the generated compound is stable, said heterocycle according to the present invention can undergo substitution on C atom or N atom and N atom in the heterocycle can be optionally quaternized. One preferential scheme is that the atoms will not be adjacent if the total number of S and O atoms in the heterocycle is over 1. Another preferential scheme is that the total number of S and O in the heterocycle will not exceed 1. As employed in the present invention, the term "aromatic heterocyclic group" or "heteroaryl group" refers to the aromatic rings with stable 5,6 or 7-membered monocycles or bi-cycles or 7, 8, 9 or 10-membered bicyclic heterocyclic group. It can contain carbons atoms and 1, 2, 3, or 4 hetero atoms of N, O and S independently selected from N, O and S. N atom can be substituted or unsubstituted (i.e., N or NR, where R is H or other substituting group already defined in the patent). N and S hetero atoms can be optionally oxidized (i.e., NO and S(O)p). It is noteworthy that the total number of S and O atoms in the aromatic heterocycle will not exceed 1. The bridged rings are also covered by the definition of heterocycle. One or more atoms (i.e., C, O, N or S) will form a bridged ring when two non-adjacent C atoms or N atoms are linked. The preferential bridged rings include but are not limited to: one C atom, two C atoms, one N atom, two N atoms and one C-N group. It is noteworthy that one bridge always converts a monocycle ring into a tricycle. The substituting group in the ring can also be located on the bridge.

The examples of heterocyclic compounds include but are not limited to acridinyl group, azocine group, benzimidazolyl group, benzofuranyl group, mercaptobenzofuranyl group, mercaptobenzophenyl group, benzoxazolyl group, benzoxazolyl group, benzothiazolyl group, benzotriazolyl group, benzotetrazolyl group, benzoisoxazolyl group, benzisothiazol group, benzimidazolyl group, carbazolyl group, 4a *H-* carbazolyl group, carboline group, chromanyl group, chromene, cinnolinyl decahydroquinolyl group, 2*H*,6*H*-1,5,2-dithiazine group, dihydro-furo [2,3-b] tetrahydrofuran, furyl group, furazanyl group, imidazolidinyl group, imidazolinyl group, imidazolyl group, 1*H*-indazolyl group, indolenyl group, indolinyl group, indolizinyl group, indolyl group, 3*H*- indolyl group, isatino group, isobenzofuran group, pyranyl, isoindolyl group, isoindoline group, isoindolyl group, indolyl group, isoquinolinyl group, isothiazolyl group, isoxazolyl group, methylenedioxy phenyl group, morpholino group, naphthyridinyl group, octahydro-isoquinolinyl group, oxadiazolyl group, 1,2,3-oxadiazolyl group, 1,2,4-oxadiazolyl group, 1,2,5-oxadiazolyl group, 1,3,4-oxadiazolyl group, oxazolidinyl group, oxazolyl group, isoxazolyl group, oxindole group, pyrimidinyl group, phenanthridine group, phenanthroline group, phenazine, phenothiazine, xanthine benzo group, phenoxazine group, phthalazinyl group, piperazinyl group, piperidinyl group, piperidonyl group, 4-piperidonyl group, piperonyl group, pteridinyl group, purinyl group, pyranyl group, pyrazolyl group, pyrazolidinyl group, pyrazolinyl group, pyrazolyl group, pyridazinyl group, pyridine-oxazole, pyridine-imidazole, pyridine-thiazole, pyridinyl group, pyrimidinyl group, pyrrolidinyl group, pyrrolinyl group, 2*H*-pyrrolyl group, pyrrolyl group, pyrazolyl group, quinazolinyl group, quinolinyl group, 4*H*-quinolizinyl group, quinoxalinyl group, quinuclidinyl group, tetrahydrofuranyl group, tetrahydro-isoquinolinyl group, tetrahydroquinolinyl group, tetrazolyl group, 6*H*-1,2,5- thiadiazolyl group, 1,2,3-thiadiazolyl group, 1,2,4-thiadiazolyl group, 1,2,5-thiadiazolyl group, 1,3,4-thiadiazolyl group, thianthrenyl group, thiazolyl group, isothiazolyl thienyl group, thienyl group, thieno oxazolyl group, thienyl- thiazolyl group, thienyl-imidazolyl group, thienyl group, triazinyl group, 1,2,3-triazolyl group, 1,2,4-triazolyl group, 1,2,5-triazolyl group, 1,3,4-triazolyl group and xanthenyl group. The condensed ring compounds and spiro-compounds are also included.

Unless specified otherwise, the term "hydrocarbyl group" or its inferior concept (e.g., alkyl, alkenyl, alkynyl and phenyl, etc) denotes a linear, branched, or cyclic hydrocarbon radical or its combination by itself or as part of other substituting group. It can be fully saturated, mono-unsaturated or poly-unsaturated, can be mono-substituted, di-substituted or poly-substituted, can be univalent (e.g. methyl group), divalent (e.g. methylene group) or multivalent (e.g. methine), can be divalent or multivalent atom radical with specified number of carbon atoms (for example C₁-C₁₀ indicating 1 to 10 carbon atoms). The "hydrocarbyl group" includes but is not limited to aliphatic group and aromatic group. Said aliphatic group includes chains and rings, specifically including but not limited to alkyl group, alkenyl group and alkynyl group. Said aromatic group includes but is not limited to 6-12 membered aromatic group, e.g. benzene and naphthalene, etc.

In some embodiments, the term "alkyl group" denotes a linear or branched atom radical or their combination. It can be fully saturated, mono-unsaturated or poly-unsaturated and can be divalent and multivalent atom radical. The examples of saturated atom radical include but are not limited to the homologs or isomers of radicals such as methyl group, ethyl group, n-propyl group, isopropyl group, n-butyl group, tert-butyl group, isobutyl group, sec-butyl group, isobutyl group, cyclohexyl group, (cyclohexyl) methyl group, cyclopropylmethyl group and n-pentyl group, n-hexyl group, n-heptyl group and n-octyl, etc. The unsaturated alkyl group comprises one or more double bonds or triple bonds and its examples include but are not limited to ethenyl group, 2-propenyl group, butenyl group, crotyl group, 2-isopentenyl group, 2- (butadienyl) group, 2,4-pentadienyl group, 3- (1,4-pentadienyl) group, ethynyl group, 1- and 3-propynyl group, 3-butyny group and their higher homologs and isomers.

Unless specified otherwise, the term "heterohydrocarbyl group" or its inferior concept (e.g.heteroalkyl group, heteroalkenyl group, heteroalkynyl group and heteroaryl group, etc.) denotes a stable linear, branched or cyclic hydrocarbon radical or its combination by itself or in combination with other term and it consists of a number of carbon atoms and at least one hetero atom. In some embodiments, the term "heteroalkyl group" denotes a stable linear, branched hydrocarbon radical or its combination by itself or in combination with other term and it consists of a number of carbon atoms and at least one hetero atom. In a typical embodiment, the hetero atom is selected from B, O, N and S, where the N and S atoms are optionally oxidized and the N hetero atom is optionally quaternized. The hetero atoms B, O, N and S can be located at any internal position of heterohydrocarbyl group (including the positions except that where the hydrocarbon group is bonded). The examples include but are not limited to -CH₂-CH₂-O-CH₃, -CH₂-CH₂-NH-CH₃, -CH₂-CH₂-N(CH₃)-CH₃, -CH₂-S-CH₂-CH₃, -CH₂-CH₂, -S(O)-CH₃, -CH₂-CH₂-S(O)₂-CH₃, -CH=CH-O-CH₃, -CH₂-CH=N-OCH₃ and -CH=CH-N(CH₃)-CH₃. No more than two hetero atoms are consecutive, e.g. CH₂-NH-OCH₃.

The term "alkoxy group", "alkylamino group" and "alkylthio group" (thioalkoxy group) is idiomatic expression and refers to those alkyl groups bonded to other parts of the molecule via O atom, amino group or S atom.

Unless specified otherwise, the terms "cyclic hydrocarbon group ", "heterocyclic hydrocarbon group" or their inferior concepts (e.g. aryl group, heteroaryl group, cycloalkyl group, heterocycloalkyl group, cycloalkenyl group, heterocycloalkenyl group, cycloalkynyl group and heterocyclyl alkynyl group, etc) denote respectively the cyclic "hydrocarbon group" and "hetero hydrocarbon group" by itself or in combination with other term. In addition, as for the hetero hydrocarbon groups or heterocyclic hydrocarbon groups (e.g. hetero alkyl group and heterocyclic alkyl group), the hetero atom can occupy other positions except that the hetero ring bonds to the molecule. The examples of cycloalkyl group include but are not limited to cyclopentyl group, cyclohexyl group, 1-cyclohexenyl group, 3-cyclohexenyl group and cycloheptyl group, etc. The non-restrictive examples of heterocyclic group include 1- (1,2,5,6-tetrahydropyridyl) group, 1-piperidinyl group, 2-piperidinyl group, 3-piperidinyl group, 4-morpholinyl group, 3-morpholinyl group, tetrahydrofuran-2-yl group, tetrahydrofuran-3-yl group, tetrahydro-thiophen-2-yl group, tetrahydro-thiophen-3-yl group, 1-piperazinyl group and 2-piperazinyl group.

Unless specified otherwise, the term "aryl group" denotes poly-unsaturated aromatic substituent and can be monosubstituted, disubstituted or polysubstituted. It can be monocyclic or polycyclic (preferably 1 to 3 rings) and can be condensed together or linked covalently. The term "heteroaryl group" refers to aryl group (or ring) containing one to four hetero atoms. In a demonstrative embodiment, the hetero atom is selected from B, O, N and S, where the N and S atoms are optionally oxidized and the N hetero atom is optionally quaternized. The heteroaryl group can bond to other part of the molecule via hetero atom. The non-restrictive embodiments of aryl group or heteroaryl group include phenyl group, 1-naphthyl group, 2-naphthyl group, 4-biphenyl group, 1-pyrrolyl group, 2-pyrrolyl group, 3-pyrrolyl group, 3-pyrazolyl group, 2-imidazolyl group, 4-imidazolyl group, pyrazinyl group, 2-oxazolyl group, 4-oxazolyl group, 2-phenyl-4-oxazolyl group, 5-oxazolyl group, 3-isoxazolyl group, 4-isoxazolyl group, 5-isobutyl oxazolyl group, 2-thiazolyl group, 4-thiazolyl group, 5-thiazolyl group, 2-furyl group, 3-furyl group, 2-thienyl group, 3-thienyl group, 2-pyridyl group, 3-pyridyl group, 4-pyridyl group, 2-pyrimidinyl group, 4-pyrimidinyl group, 5-benzothiazolyl group, purinyl group, 2-benzimidazolyl group, 5-indolyl group, 1-isoquinolyl group, 5-isoquinolinyl group, 2-quinoxalinyl group, 5-quinoxalinyl group, 3-quinolyl group and 6-quinolyl group. Any aforementioned substituting group of aryl and heteroaryl ring is selected from the acceptable substituting group described as follows.

For simplicity, the aryl group to be used in combination with other term (e.g. aryloxy group, arylthio group and aralkyl group) includes the aryl group and heteroaryl ring as defined above. Thus, the term "aralkyl group" means to include those radicals via which the aryl group bonds to the alkyl group (e.g. benzyl, phenethyl, pyridylmethyl etc), including those alkyl groups that any C atom (e.g. methylene) is replaced by O atom, e.g., phenoxymethyl group, 2- pyridyloxy methyl-3-(1-naphthyloxy) propyl group, etc.

The term "leaving group" refers to a functional group or atom that can be replaced by another functional group or atom via substitution reaction (e.g., nucleophilic substitution reaction). For example, the representative leaving groups include triflate;chloro, bromo, iodo; sulfonate group such as mesylate, tosylate, brosylate, p-toluenesulfonate, etc; acyloxy group such as acetoxy group and trifluoroacetyl group, etc.

The term "protecting group" includes but is not limited to "amino protecting group", "hydroxy protecting group" or "mercapto-protecting group". The term "amino protecting group" refers to the protecting groups suitable to prevent secondary reaction on the position of amino nitrogen. The representative amino protecting groups include but are not limited to: formyl group; acyl groups such as alkanoyl group (e.g. acetyl group, trichloroacetyl group or trifluoroacetyl group); alkoxycarbonyl group such as tert-butoxycarbonyl (Boc); arylmethoxycarbonyl groups such as benzyloxycarbonyl (Cbz) and 9-fluorenyl methoxycarbonyl (Fmoc); arylmethyl group such as benzyl group (Bn), trityl group (Tr), 1,1-di- (4'-methoxyphenyl) methyl group; silyl groups such as trimethylsilyl group (TMS) and tert-butyldimethylsilyl (TBS), etc. The term "hydroxy protecting group" refers to the protecting groups suitable to prevent secondary reaction of hydroxy group. The representative hydroxy groups includes but are not limited to: alkyl groups such as methyl group, ethyl group and t-butyl group; acyl groups such as alkanoyl groups (e.g. acetyl group); arylmethyl groups such as benzyl (Bn), p-methoxybenzyl group (PMB), 9- fluorenyl methyl (Fm) and diphenylmethyl group (benzhydryl, DPM); silyl groups such as trimethylsilyl group (TMS) and tert-butyldimethylsilyl (TBS), etc.

The compound(s) according to the present invention can be prepared via many synthesis methods known by those skilled in the art, which include the following embodiments, the implementation ways formed by them with other chemical synthesis methods and the equivalent replacements known by those skilled in the art. The preferred embodiments include but are not limited to the examples according to the present invention.

All the solvents used in the present invention are commercially available and can be used directly without further purification. The present invention employs the following abbreviations: aq denoting water; HATU denoting O-(7-aza-benzotriazol-1-yl)-N,N,N',N'-tetramethyluronium hexafluorophosphate; EDC denoting N-(3-dimethylaminopropyl) -N'-ethylcarbodiimide hydrochloride; m-CPBA denoting 3-chloroperoxybenzoic acid; eq denoting equivalent and equal amount; CDI denoting carbonyl diimidazole; DCM denoting dichloromethane; PE denoting petroleum ether; DIAD denoting diisopropyl azodicarboxylate; DMF denoting N, N- dimethylformamide; DMSO denoting dimethylsulfoxide; EtOAc denoting ethyl acetate; EtOH denoting ethanol; MeOH denoting methanol; CBz denoting benzyloxycarbonyl that is an amine-protecing group; BOC denoting tert-carbonyl that is an amine-protecting group; HOAc denoting acetic acid; NaCNBH₃ denoting sodium cyanoborohydride; r.t. denoting room temperature; O/N denoting over night; THF denoting tetrahydrofuran; Boc₂O denoting di-tert-butyl dicarbonate; TFA denoting trifluoroacetate; DIPEA denoting diisopropylethyl amine; SOCl₂ denoting thionyl chloride; CS₂ denoting carbon disulfide; TsOH denoting p-toluene sulfonic acid; NFSI denoting N-fluoro-N-(phenylsulfonyl) benzenesulfonamide; NCS denoting 1-chlorine pyrrolidine-2,5-diketone; *n*-Bu₄NF denoting tetrabutylammonium fluoride; iPrOH denoting 2-propanol; mp denoting melting point; LDA denoting lithium diisopropylamide; Xantphos denoting 4,5-bisdiphenylphosphino-methyl-9,9-xanthene; Pd₂(dba)₃ denoting tris(dibenzylideneacetone) dipalladium; DAST denoting diethylaminosulphur trifluoride; Pd(dppf)Cl2 denoting 1,1'-bis(diphenylphosphino) ferrocene; PCC denoting pyridinium chlorochromate; TsCI denoting toluenesulfonyl chloride; Et₃N denoting triethylamine.

The compound(s) are named manually or via ChemDraw® software. The commercially-available compounds are used with the names from supplier directory.

Compared with the existing technologies, the compound(s) according to the present invention is efficient with low toxicity and realizes obvious or even unexpected progress in the factors such as activity, half time, solubility and pharmacokinetics and hence more suitable to prepare pharmaceuticals.

### DESCRIPTION OF THE EMBODIMENTS

In order to describe the present invention in more detail, the following examples are provided although the scope of the present invention is not limited to them.

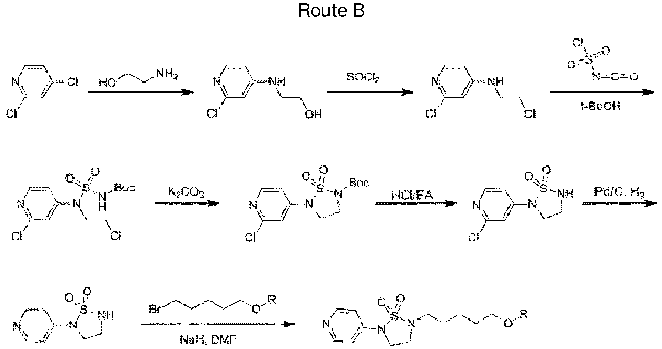

### Embodiment 4

### 2-(5-((4-fluorophenoxy)pentyl)-5-(pyridin-4-yl)-1,2,5-thiadiazole-1,1-dioxide

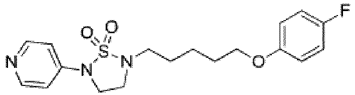

### Embodiment 4A

### 2-((2-chloropyridine-4-yl) amino) ethanol

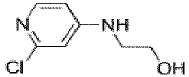

Agitate the solution of 2,4-dichloropyridine (100g, 675.7mmol), triethylamine (136.7g, 1.35 mol) and 2-aminoethanol (41.3g, 675.7mmol)) in ethanol (500ml) 72 hours at 100°C and then allow it to undergo rotary evaporation to remove the solvents. Then purify the residue through the chromatography column and yield the title compound (yellow oil, 46g, yield of 40%). ¹H-NMR(CDCl₃,400MHz)δ7.90(d,*J* = 5.8Hz,1H),6.49(d,*J* = 2.0Hz,1H),6.41(dd,*J* = 5.8,2.0Hz,1H),5.17(brs,1H),3.87(t,*J*=5.1Hz,2H),3.31(q,*J*=5.0Hz,2H); LCMS(ESI)m/z:173(M+1).

### Embodiment 4B

### 2-chloro-N-(2-chloroethyl) pyridin-4-amine

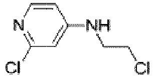

Dissolve Embodiment 4A (5g, 28.9mmol) in the mixture solvent of dichloromethane and N, N-dimethylformamide (50ml/5ml) and then drop the thionyl chloride (60ml) into this mixture solution at 15°C before heat to 40°C to react for 16 hours. Then cool the system to room temperature and pour ice water (200ml) into it. Then add 1 mol/L aqueous NaOH solution to adjust pH value to 10. Extract the aqueous layer with ethyl acetate (100ml x 6) and wash the combined organic layers with saline (50ml) before dry with Na₂SO₄. Then filter and evaporate to get the crude product. Then purify the crude product via the chromatography column to obtain the title compound (white solid, 1.7g, yield of 45%) and recycle the raw materials (white solid, 2.0g). LCMS (ESI) m/z: 191 (M+1).¹H-NMR (CDCl₃, 400MHz) δ 7.99 (d, *J*=6.0Hz,1H), 6.38-6.55 (m,2H), 4.75 (brs,1H), 3.67-3.78 (m,2H), 3.55(q, *J*= 5.5Hz, 2H).

### Embodiment 4C

### N-(2-chloroethyl)-N-(2- chloropyridin-4-yl) aminosulfonyl tert-Butyl carbamate

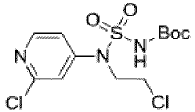

Under the protection of N₂ gas at 0°C, drop the chlorosulfonyl isocyanate (2.7ml) into the solution of tert-butanol (3.4ml) in dichloromethane (27ml). After the dropping is completed, heat the solution gradually to room temperature and then continue to agitate 30 minutes to clear up. Transfer the solution at vacuum into a constant pressure funnel to mix with triethylamine (11.6ml). Then under the protection at 0°C, drop the aforementioned mixture solution into the solution of Embodiment 4B (1.7g, 8.9mmol) in dichloromethane (20ml). After the dropping is completed, agitate the solution 48 hours at 12°C before quench with ice water. Then extract the aqueous phase with dichloromethane (50ml x 4). Dry the combined organic layers with Na₂SO₄ before filtering and evaporation to obtain the crude product (7g) of the title compound that can be directly used without necessity to purify any more. ¹H-NMR (CDCl₃, 400MHz) δ 8.45 (d, *J* = 5.5Hz,1H), 7.42 (d, *J* = 1.5Hz,1H), 7.34 (dd, *J* = 5.3,1.8Hz,1H), 4.32 (t, *J*=6.4Hz,2H), 3.67 (t, *J*=6.3Hz,2H), 1.49 (s,9H); LCMS (ESI)m/z:370 (M+1).

### Embodiment 4D

### 5-(2-chloropyridine-4-yl)-1,2,5-thiadiazolidine-2-tert-Butyl carboxylate-1,1-dioxide

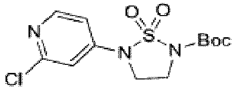

At 15°C, add K₂CO₃ powder (3.4g) into the solution of Embodiment 4C (7g, crude product) in dimethylsulfoxide (30ml) and agitate 3 hours. Then pour it into 1 L water and filter to obtain the white solid. Dissolve the solid in dichloromethane and then dry with Na₂SO₄ before filter and evaporate to obtain the title compound (2g, yield of 69%). ¹H-NMR(CDCl₃, 400MHz) δ 8.32 (d, *J*=6.0Hz, 1H), 7.06-7.21 (m, 2H), 3.98-4.12 (m, 2H), 3.79-3.92 (m, 2H), 1.59 (s,10H). LCMS(ESI) m/z: 334(M+1).

### Embodiment 4E

### 2-(2-chloropyridine-4-yl)-1,2,5-thiadiazolidine-1,1-dioxide

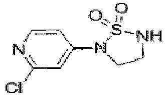

Add ethyl acetate hydrochloride (4mol/L, 40ml) into the mixture solution of Embodiment 4D (1.9g, 5.69mmol) in ethyl acetate / methanol (40ml/4ml) and agitate it 5 hours at 60°C. After removing the solution at vacuum, adjust the pH value of the residue with saturated sodium bicarbonate solution to 8. Extract the aqueous layer with ethyl acetate (250ml x 3) and dry the combined organic layers with Na₂SO₄ before filter and evaporate to obtain the title compound (white solid, 1.2g, 92%). ¹H-NMR (CDCl₃, 400MHz) δ 8.29 (d, *J*=5.8Hz, 1H), 7.05-7.09 (m, 1H), 7.00 (d, *J*=2.0Hz, 1H), 3.95-3.99 (m, 2H), 3.78-3.85 (m, 2H); LCMS (ESI) m/z: 234(M+1).

### Embodiment 4F

### 2- (pyridin-4-yl)-1,2,5-thiadiazolidine-1,1-dioxide

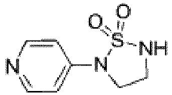

Mix Embodiment 4E (1.3g, 5.5mmol), Pd/C (100mg) and triethylamine (2ml) into methanol (30ml). Agitate this mixture 12 hours at 25°C before filter. Then remove the solvent at vacuum to obtain the title compound (3.8g, yield of 100%) that can be directly used without necessity to purify any more. ¹H-NMR (DMSO-d₆, 400MHz) 8.75(s, br, 1H), 8.68 (d, *J*=7.0Hz, 2H), 7.45 (d, *J*=7.0Hz, 2H), 4.08 (t, *J*=6.3 Hz, 2H), 3.64 (d, *J*=6.3 Hz, 2H); LCMS (ESI) m/z: 200(M+1).

### Embodiment 4G

### 1-(5-bromopentyl) oxy-4-fluorobenzene

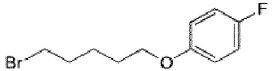

Add K₂CO₃ (14.79g, 107mmol), KI (0.59g, 3.6mmol) and 1,5-dibromopentane (24.61g, 107mmol) into the solution of p-fluorophenol (4g, 35.68mmol) in acetone (40ml) . Agitate the mixture solution 12 hours at 80°C. Then extract it with ethyl acetate. Dry the organic phase with anhydrous Na₂SO₄ before filter and evaporate. Then purify the residue with chromatography column (petroleum ether: ethyl acetate =10:1) to obtain the title compound (white solid, 6.6g, yield of 70.8%). ¹H-NMR (CDCl₃, 400MHz) δ 6.93-7.01 (m, 2H) 6.79-6.86 (m, 2H) 3.90-3.97 (m, 2H) 3.45 (t, *J*=6.8Hz, 2H) 1.94 (q,*J*= 7.2Hz, 2H) 1.76-1.86 (m, 2H) 1.60-1.68 (m, 2H). LCMS (ESI) m/z: 262 (M+1).

### Embodiment 4H

### 2-(5-((4-fluorophenoxy)pentyl)-5-(pyridin-4-yl)-1,2,5-thiadiazolidine-1,1-dioxide

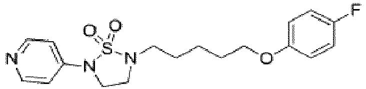

At 0°C, add sodium hydride (6mg, 0.16mmol, 60% content) into the solution of Embodiment 4F (30mg, 0.15mmol) in N, N-dimethylformamide (2ml). Keep it still 30 minutes at 0°C. Then add the solution of Embodiment 4G (39.3mg, 0.15mmol) in N, N- dimethylformamide (1ml) into the aforementioned mixture solution. Agitate it 6 hours at 15°C. Then add water to quench the reaction and extract the aqueous phase with ethyl acetate. Dry the combined organic phases with anhydrous Na₂SO₄ before filter and evaporate. Then separate it with HPLC to obtain the title compound (white solid, 28mg, yield of 49%). ¹H-NMR (400MHz, CDCl₃) δ 8.51 (d, *J*=6.0 Hz, 2H), 7.05 (d, *J*=5.5Hz, 2H), 6.91-7.01 (m, 2H), 6.82 (dd, *J*=9.0, 4.0Hz, 2H), 3.93 (t, *J*=6.3 Hz, 2H), 3.84 (t, *J*=6.5Hz, 2H), 3.54 (t, *J*=6.3Hz, 2H), 3.18 (t, *J*=7.0Hz, 2H), 1.72-1.87 (m, 5H), 1.56-1.61 (m, 1H). LCMS (ESI), m/z: 380 (M+1).

### Embodiment 5

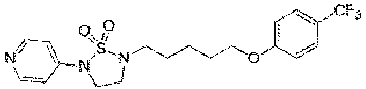

### Embodiment 5A

### 1- (5-bromo-pentyl) oxy-4-trifluoromethyl benzene

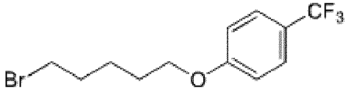

Please refer to the preparation method of Embodiment 4G for this embodiment. ¹H-NMR (400MHz, CDCl₃) δ 7.54 (d, *J*=8.53 Hz, 2H), 6.95 (d, *J*=8.53 Hz, 2H), 4.01 (t, *J*=6.27 Hz, 2H), 3.42-3.51 (m, 2H), 1.95 (quin, *J*=7.15 Hz, 2H), 1.76-1.89 (m, 2H), 1.61-1.68 (m, 2H).

### Embodiment 5B

### 2-(pyridin-4-yl)-5-(5-(4-trifluoromethyl-phenoxy)pentyl)-1,2,5-thiadiazolidine-1,1-dioxide

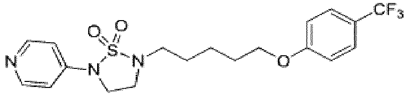

Please refer to the preparation method of Embodiment 4H for this embodiment. ¹H-NMR (400MHz, CDCl₃) δ 8.53 (d, *J*=6.27 Hz, 2H), 7.50-7.60 (m, 2H), 7.07 (d, *J*=6.27 Hz, 2H), 6.96 (d,*J* =8.78 Hz, 2H), 4.04 (t, *J*=6.27 Hz, 2H), 3.87 (t, *J*=6.40 Hz, 2H), 3.57 (t, *J*=6.40 Hz, 2H), 3.21 (t, *J*=7.15 Hz, 2H), 1.86-1.94 (m, 2H), 1.77-1.85 (m, 2H), 1.60-1.68 (m, 2H).

### Embodiment 6

### 2-(5-((4'-chloro-[1,1'-biphenyl]-4-yl)oxy)pentyl)-5-(pyridin-4-yl)-1,2,5-thiadiazolidine-1,1-dioxide

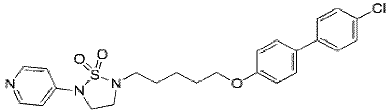

### Embodiment 6A

### 4'-chloro-[1,1'-biphenyl]-4-ol

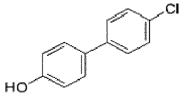

Mix 4-bromophenol (62g, 360mmol), 4-chlorophenyl boronic acid (56g, 360mmol), Pd(dppf)Cl₂ (10g, 10mmol) and Na₂CO₃ (76g, 720mmol) into THF/H₂O (600ml/100ml) and heat this mixture 6 hours at 70°C under the protection of N₂. Pour the reaction mixture into water and extract with ethyl acetate. Dry the combined organic phases with anhydrous Na₂SO₄ before filter and evaporate. Then purify the residue with chromatography column (petroleum ether: ethyl acetate =20:1) to obtain the title compound (60g, yield of 80%). LCMS(ESI)m/z:205(M+1).

### Embodiment 6B

### 4-((5-bromopentyl)oxy) -4'-chloro-1,1'-biphenyl

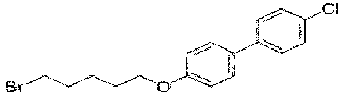

Add 1,5-dibromopentane (67g, 293.2mmol), K₂CO₃ (27g, 195.4mmol) and KI (1.62g, 9.77mmol) into the solution of Embodiment 6A (20g, 97.72mmol) in acetone (200ml) . After the substances are added, heat the mixture solution 12 hours at 80°C. After the reaction is completed, filter the reaction solution and then concentrate the filtrate. Then add petroleum ether (200ml) into the residue and agitate the system 2 hours at 0°C before filter to obtain the solid as the title compound (white solid, 25g, yield of 75%). ¹H-NMR (400MHz, CDCl₃) 7.48 (dd, *J*=2.01, 8.53 Hz, 4H), 7.35-7.41 (m, 2H), 6.91-7.00 (m, 2H), 3.96-4.06 (m, 2H), 3.41-3.50 (m, 2H), 1.96 (q, *J*=7.15 Hz, 2H), 1.80-1.90 (m, 2H), 1.60-1.71 (m, 2H).

### Embodiment 6C

### 2-(5-((4'-chloro-[1,1'-biphenyl]-4-yl)oxy)pentyl)-5-(pyridin-4-yl)-1,2,5-thiadiazolidine-1,1-dioxide

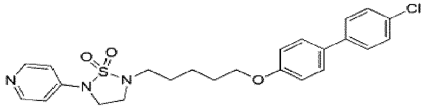

At 0°C, add sodium hydride (10mg, 0.4mmol, 60% content) into the solution of Embodiment 4F (40mg, 0.2mmol) in N, N- dimethylformamide (10ml). Then agitate it 0.5 hour at 0°C before add the solution of Embodiment 6B (71mg, 0.2mmol) in N, N- dimethylformamide (2ml). Continue to agitate it to react 2 hours at 15°C. Then add water to quench the reaction and extract the aqueous phase with ethyl acetate. Dry the combined organic phases with anhydrous Na₂SO₄ before evaporate. Then purify the residue with HPLC to obtain the title compound (white solid, 10mg, yield of 11%). ¹H-NMR (CDCl₃, 400 MHz) δ 8.51 (d, *J*=6.3 Hz, 2H), 7.50-7.44 (m, 4H), 7.41-7.34 (m, 2H), 7.10-7.04 (m, 2H), 6.95(d, *J*=8.8 Hz, 2H), 4.02(t, *J*=6.3 Hz, 2H), 3.89-3.81 (m, 2H), 3.55 (t, *J*=6.4 Hz, 2H), 3.19 (t, *J*=7.2 Hz, 2H), 1.90-1.84 (m,2H), 1.82-1.78 (m, 2H), 1.67-1.58 (m, 2H), LCMS (ESI) m/z: 472 (M+1).

### Embodiment 7

### (E)-4-((5-(1,1-dioxo-5-(pyridin-4-yl)-1,2,5-thiadiazol-2-yl)pentyl)oxy)benzaldehyde-O-ethyl oxime

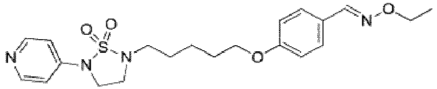

### Embodiment 7A

### (E)-4-hydroxybenzaldehyde-oxo-ethyl oxime

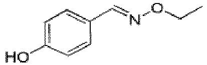

Add sodium acetate (3.36g, 40.94mmol) into the solution of p-hydroxy benzaldehyde (2.5g, 20.47mmol) and ethoxycarbonyl amino hydrochloride (3.91g, 40.94mmol) in water (50ml) and agitate the mixture 4 hours at 80°C. Add ethyl acetate (50ml) into the reaction system. Extract the aqueous layer with ethyl acetate (50ml x 3) and then dry the combined organic layers with Na₂SO₄ before filter and evaporate to obtain the title compound (brown solid, 3g, yield of 89%). ¹H-NMR (CDCl₃, 400MHz) 8.03 (s, 1H), 7.46 (d, *J*=8.5 Hz, 2H), 6.78-6.85 (m, 2H), 4.20 (q, *J*=7.0 Hz, 2H), 1.31 (t, *J*=7.0 Hz, 3H).

### Embodiment 7B

### (E) -4-((5-bromo-pentyl)-oxy) benzaldehyde-oxo-ethyloxime

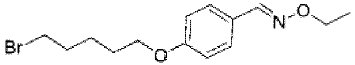

Mix Embodiment 7A (1.0g, 6.05mmol), 1,5-dibromopentane (1.39g, 6.05mmol), K₂CO₃ (1.67g, 12.11mmol) and KI (0.1g, 0.605mmol) into acetone (100ml). Then heat to reflux 10 hours. Filter and then dry the filtrate through rotation before purify with chromatography column to obtain the title compound (white crystal, 1.2g, 63%). ¹H-NMR(CDCl₃,400MHz)δ8.01-8.05(m,1H),7.49-7.55(m,2H),6.86-6.91(m,2H),4.17-4.24(m,2H),3.96-4.01(m,2H),3.41-3.47(m,2H),1.89-1.99(m,2H),1.78-1.86(m,2H),1.60-1.68(m,2H),1.29-1.34(m,3H).

### Embodiment 7C

### (E)-4-((5-(1,1-dioxo-5-(pyridin-4-yl)-1,2,5-thiadiazolidine-2-yl)-pentyl)-oxy)-benzaldehyde-O-ethyloxime

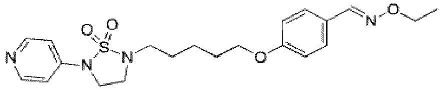

At 0°C, add sodium hydroxide (10mg, 250mmol, 60% content) in batch into the solution of Embodiment 4F (60ml, 301.2mmol) in N, N- dimethylformamide (1ml) and then agitate 1.5 hours. Then drop the solution of Embodiment 7B (114mg, 361.4mmol) in N, N- dimethylformamide (0.5ml) and heat it to room temperature before agitating for 2 hours. Then pour it into water (10ml) and extract with ethyl acetate (3ml x 3). Then dry the combined organic phases with anhydrous Na₂SO₄ and dry through rotation. Then purify with preparative HPLC to obtain the title compound (white solid, 40mg, yield of 30.7%). ¹H-NMR(CDCl₃,400MHz)δ8.53(d,*J*=6.02Hz,1H),8.04(s,1H),7.53(d,*J*=8.78Hz,2H),7.07(d,*J*=6.27Hz,2 H),6.89(d,*J*=8.78Hz,2H),4.22(q,*J*=7.03Hz,2H),4.02(t,*J*=6.27Hz,2H),3.87(t,*J*=6.40Hz,2H),3.56(t,*J*=6.4 0Hz,2H),3.20(t,*J*=7.15Hz,2H),1.85-1.93(m,2H),1.76-1.84(m,2H),1.67(br.s.,2H),1.34(t,*J*=7.03Hz,3H). LCMS(ESI)m/z:433(M+1).

### Embodiment 8

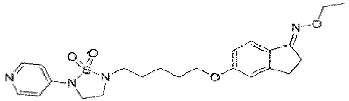

### Embodiment 8A

### 5-hydroxy-2,3-dihydro-1H-inden-1-one

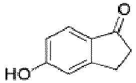

Under the protection of N₂ gas, mix 5-bromo-1-indanone (1.0g, 4.7mmol), N,N,N',N'-tetramethylethylenediamine (210mg, 2.37mmol), K₃PO₄ (1.0mg, 4.74mmol) and Cul (90mg, 0.47mmol) into water (5.0ml). Heat it with microwave for 2 hours at 120°C. After the reactions end, extract the solution mixture with ethyl acetate (10ml x 3). Dry the combined organic phases with anhydrous Na₂SO₄ before filter and concentrate. Then purify it with TLC to obtain the title compound (white solid, 120mg, yield of 17%). ¹H-NMR(CDCl₃,400MHz)δ7.71-7.69(m,1H),6.92-6.87(m,2H),3.11-3.09(m,2H),2.72-2.70(m,2H).

### Embodiment 8B

### (E)-5-hydroxy-2,3-dihydro-1H-inden-1-one-O-ethyloxime

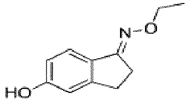

Add ethoxyamino hydrochloride (65mg, 0.67mmol) and sodium acetate (55mg, 0.67mmol) into the mixture solution of Embodiment 8A (50mg, 0.3mmol) and water (5ml). Heat this mixture solution 1 hour at 80°C. Then extract the aqueous phase with ethyl acetate (5.0ml × 3). Dry the combined organic phases with anhydrous Na₂SO₄ before filter and concentrate to obtain the crude product that will be used directly in the following reaction.
¹H-NMR(CDCl₃,400MHz)δ7.52-7.66(m,1H),6.68-6.87(m,2H),4.12-4.30(m,2H),2.78-3.07(m,4H),1.27-1.41(m,3H).

### Embodiment 8C

### (E)-5-((5-bromo-pentyl)-oxy-)-2,3-dihydro-1H-inden-1-one-O-ethyloxime

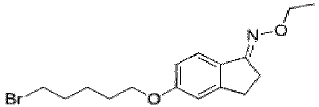

Add 1,5-dibromopentane (288mg, 1.26mmol, 3.0 equivalent), K₂CO₃ (578mg, 4.18mmol, 10 equivalent) and KI (7mg, 0.04mmol, 0.1 equivalent) into the mixture solution of Embodiment 8B (80mg, 0.4mmol) and acetone (5.0ml). Then heat this mixture solution 5 hours at 80°C. Filter the reaction solution before concentrate the filtrate. Then purify the residue obtained from concentrating with TLC to obtain the title compound (yellow solid, 140mg, yield of 98%). LCMS(ESI)m/z:340(M+1).

### Embodiment 8D

### (E)-2-(5-((1-(ethoxy-imino)-2,3-dihydro-1H-inden-5-yl)-oxy)-pentyl)-5-(pyridin-4-yl)-1,2,5-thiadiazole-1,1-dioxide

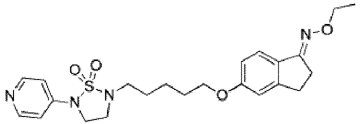

Under the protection of N₂ gas, add sodium hydride (8mg, 0.2mmol) into the solution of Embodiment 4F (20mg, 0.10mmol) in N, N-dimethylformamide (1.0ml). Agitate it 0.5 hour at 0°C, then add slowly the solution of Embodiment 8C (34mg, 0.1 mmol) in N, N-dimethylformamide (1.0ml) into the solution above. Agitate it at 15°C to react overnight. Then add water (0.5ml) to quench the reaction and then extract the aqueous phase with dichloromethane. Then dry the combined organic phases with anhydrous Na₂SO₄ before filter and evaporate. Then purify the residue with the preparative HPLC to obtain the title compound (17mg, yield of 37%).
¹H-NMR(CDCl₃,400MHz)δ8.50(d,*J*=5.0Hz,2H),7.59(d,*J*=9.5Hz,1H),7.05(d,*J*=5.5Hz,2H),6.74-6.82(m, 2H),4.19(q,*J*=7.0Hz,2H),3.96-4.08(m,2H),3.84(t,*J*=5.8Hz,2H),3.50-3.58(m,2H),3.17(t,*J*=7.0Hz,2H),2. 94-3.11(m,2H),2.81-2.92(m,2H),1.72-1.94(m,4H),1.56-1.68(m,2H),1.22-1.40(m,3H). LCMS(ESI)m/z:459(M+1).

### Embodiment 9

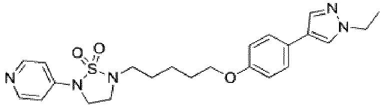

### Embodiment 9A

### 1-bromo-4-((5-bromo-pentyl)-oxy)-benzene

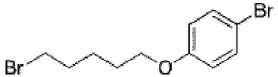

Add 1,5-dibromopentane (19.95g, 86.7mmol), K₂CO₃ (8g, 57.8mmol) and KI (0.4g, 2.89 mmol) into the solution of 4-bromophenol (5g, 28.9mmol) in acetone (50ml) . After the substances are added, agitate the mixture solution 12 hours at 80°C. Filter before concentrate the filtrate. Then add the petroleum ether (100ml) into the concentrated residue and then agitate 1 hour at 0°C before filter to obtain the title compound (yellow solid, 4g, yield of 43%). ¹H-NMR(CDCl₃,400MHz)δ7.37-7.39(m,1H),7.34-7.36(m,1H),6.77-6.79(m,1H),6.75-6.77(m,1H),3.93(t ,*J*=6.27Hz,2H),3.44(t,*J*=6.78Hz,2H),1.90-1.99(m,2H),1.75-1.84(m,2H),1.58-1.67(m,2H).
LCMS(ESI)m/z:323(M+1).

### Embodiment 9B

### 4-(4-((5-bromo-pentyl)-oxy)-phenyl)-1-ethyl-1H-pyrazole

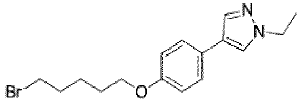

Under the protection of N₂ gas at 25°C, add the catalyst Pd(dppf)Cl₂ (9mg, 12.4mmol) into the mixture solution of Embodiment 9A (80mg, 0.25mmol), 1-ethyl-4-boronic acid pinacol ester (55mg, 0.25mmol) and Na₂CO₃ (53mg, 0.5mmol) in 1,4-dioxane (3ml) and water (0.5ml). Then agitate it 16 hours at 80°C and add water (10ml). Extract the aqueous layer with dichloromethane (10ml x 3). Then dry the combined organic layer with Na₂SO₄ before filter and evaporate. Then purify the residue with the preparative TLC to obtain the title compound (white solid, 26mg, yield of 31%). ¹H-NMR(CDCl₃,400MHz)δ7.70(s,1H),7.52-7.60(m,1H),7.38(d,*J*=8.8Hz,2H),6.89(d,*J*=8.8Hz,2H),4.20( q,*J*=7.3Hz,2H),3.98(t,*J*=6.4Hz,2H),3.45(t,*J*=6.8Hz,2H),1.95(q,*J*=7.2Hz,2H),1.77-1.86(m,2H),1.60-1.7 1(m,2H),1.49-1.54(m,3H).

### Embodiment 9C

### 2-(5-(4-(1-ethyl-1H- pyrazol-4-yl)phenoxy)pentyl)-5-(pyridin-4-yl)-1,2,5-thiadiazolidine-1,1-dioxide

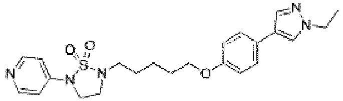

Please refer to the preparation method of Embodiment 8D for this embodiment.
¹H-NMR(CDCl₃,400MHz)δ8.51(d,*J*=6.5Hz,2H),7.70(s,1H),7.56(s,1H),7.38(d,*J*=8.5Hz,2H),7.05(d,*J*=6 .3Hz,2H),6.89(d,*J*=8.8Hz,2H),4.17-4.23(m,2H),3.99(t,*J*=6.1Hz,2H),3.83-3.86(m,2H),3.53-3.56(m,2H) ,3.18(t,*J*=7.2Hz,2H),1.70-1.99(m,4H),1.64(s,br.,2H),1.57-1.58(m,3H).LCMS(ESI)m/z:456(M+1).

### Embodiment 10

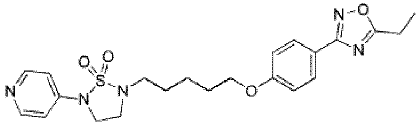

### Embodiment 10A

### N',4-dihydroxyphenyl formamidine

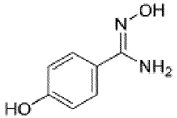

Add DIPEA (42g, 419mmol) and NH₂OH·HCl (29.1mg, 1.15mmol) into the 4-hydroxy-nitrile (5g, 42mmol) in isopropanol (90ml). Heat this mixture solution 4 hours at 80°C. After cool it to room temperature, filter the solution to obtain the solid as the title compound (4g, yield of 62.6%). ¹H-NMR(CDCl₃,400MHz)δ7.50(d,*J*=8.5Hz,2H),6.82(d,*J*=8.5Hz,2H). LCMS(ESI)m/z:153(M+1).

### Embodiment 10B

### 4-(5-ethyl-1,2,4-oxadiazol-3-yl)-phenol

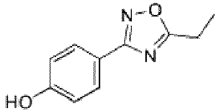

At 0°C, add propionic anhydride (2.57g, 19.57mmol) into the solution of Embodiment 10A (3g, 19.57mmol) in pyridine (10ml). After the substances are added, heat the mixture solution 1 hour at 60°C. Cool the reaction solution to room temperature and then concentrate it under vacuum before purity with column chromatography (petroleum ether: ethyl acetate = 10:1) to obtain the title compound (3g, yield of 80%). ¹H-NMR(CDCl₃,400MHz)δ7.97(d,*J*=8.5Hz,2H),6.94(d,*J*=8.5Hz,2H),2.99(q,*J*=7.6Hz,2H),1.46(t,*J*=7.5H z,3H). LCMS(ESI)m/z:191(M+1).

### Embodiment 10C

### 3-(4-((5-bromo-pentyl)oxy) phenyl) -5-ethyl-1,2,4-oxadiazole

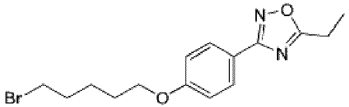

Add cesium carbonate (34.26g, 105.15mmol) and 1,5-dibromopentane (36.27g, 157.73mmol) into the solution of Embodiment 10B (10g, 52.58mmol) in N, N- dimethylformamide (100ml). Agitate the mixture solution 12 hours at 10°C. Then extract it with ethyl acetate. Dry the organic phase with anhydrous Na₂SO₄ before filter and evaporate. Then purify the residue with column chromatography (petroleum ether: ethyl acetate =10:1) to obtain the title compound (white solid, 10g, yield of 56%). ¹H-NMR(CDCl₃,400MHz)δ8.02(d,*J*=8.5Hz,2H),6.95-7.03(m,2H),4.00-4.08(m,2H),3.41-3.51(m,2H),2. 97(q,*J*=7.6Hz,2H),1.92-2.01(m,2H),1.82-1.91(m,2H),1.64-1.71(m,2H),1.46(t,*J*=7.5Hz,3H). LCMS(ESI)m/z:339(M+1).

### Embodiment 10D

### 2-(5-(4-(5-ethyl-1,2,4-oxadiazol-3-yl)-phenoxy)-pentyl)-5-(pyridin-4-yl)-1,2,5-thiadiazolidine-1,1-dioxide

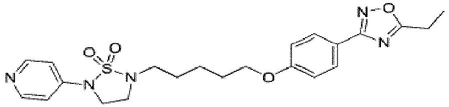

At 0°C, add sodium hydride (3.6mg, 0.15mmol) into the solution of Embodiment 4F (20mg, 0.1mmol) in N, N- dimethylformamide (5ml). Keep it still 30 minutes at 0°C. Then add the solution of Embodiment 10C (34mg, 0.1mmol) in N, N- dimethylformamide (1ml) into the aforementioned mixture solution. Agitate it 4 hours at 15°C. Then add water to quench the reaction and extract the aqueous phase with ethyl acetate. Dry the combined organic phases with anhydrous Na₂SO₄ before filter and evaporate to obtain the title compound (white solid, 3mg, yield of 7%). ¹H-NMR(400MHz,CDCl₃)δ8.51(d,*J*=5.0Hz,2H),8.00(d,*J*=9.0Hz,2H),7.05(d,*J*=6.3Hz,2H),6.96(d,*J*=8.8 Hz,2H),4.04(t,*J*=6.1Hz,2H),3.85(t,*J*=6.4Hz,2H),3.54(t,*J*=6.4Hz,2H),3.19(t,*J*=7.3Hz,2H),2.96(q,*J*=7.5H z,2H),1.91-1.76(m,4H),1.68-1.62(m,2H),1.44(t,*J*=7.7Hz,3H). LCMS(ESI)m/z:458(M+1).

### Embodiment 11

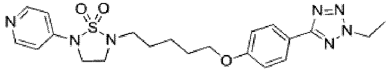

### Embodiment 11A

### 5-(4-methoxyphenyl)-1H-tetrazole

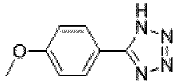

Add sodium azide (3.2g, 496mmol) and NH₄Cl (600mg, 11.2mmol) into the solution of 4-methoxybenzonitrile (6g, 450mmol) in N, N- dimethylformamide (60ml). Heat the mixture solution 24 hours at 110 °C. Cool it to room temperature before add water and then extract with dichloromethane. Then add 1mol/L HCl into the aqueous phase to adjust the pH value to ∼8 and filter the aqueous phase to obtain the solid as the title compound (white solid, 7g, yield of 88%). ¹H-NMR(CDCl₃,400MHz)δ7.96(d,*J*=8.8Hz,2H)7.13(d,*J*=8.8Hz,2H)3.90(s,3H).
LCMS(ESI)m/z:177(M+1).

### Embodiment 11B

### 2-ethyl-5-(4-methoxyphenyl)-2H-tetrazolium

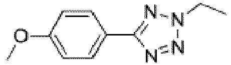

At 0°C, add K₂CO₃ (4.71g, 34.06mmol) and iodoethane (2.92g, 18.73mmol) into the solution of Embodiment 11A (3g, 17.07mmol) in acetonitrile (30ml). After the substances are added, heat the mixture solution 5 hours at 100°C. Cool the reaction solution to room temperature and then concentrate under vacuum before purity with column chromatography (petroleum ether: ethyl acetate = 10:1) to obtain the title compound (white solid, 2.1g, yield of 60%). ¹H-NMR(CDCl₃,400MHz)δ8.08(d,*J*=8.5Hz,2H)7.00(d,*J*=8.5Hz,2H)4.68(q,*J*=7.2Hz,2H)3.87(s,3H)1.68 (t,*J*=7.5Hz,3H). LCMS(ESI)m/z:205(M+1).

### Embodiment 11C

### 4- (2-ethyl-2H-tetrazol-5-yl)-phenol

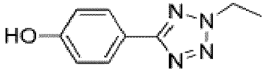

Add 10ml hydrogen bromide in acetic acid into Embodiment 11B (1g, 450mmol). Heat this mixture solution 24 hours at 110°C. Then cool the reaction solution to room temperature and add it into ice water. Filter the aqueous phase to obtain the solid as the title compound (white solid, 0.7g, yield of 75.2%).
¹H-NMR(CDCl₃,400MHz)δ8.08(d,*J*=8.5Hz,2H),7.00(d,*J*=8.5Hz,2H),4.68(q,*J*=7.2Hz,2H),3.87(s,2H),1. 68(t,*J*=7.5Hz,3H). LCMS(ESI)m/z:191(M+1).

### Embodiment 11D

### 5-(4-((5-bromo-pentyl)-oxy)-phenyl)-2-ethyl-2H-tetrazolium

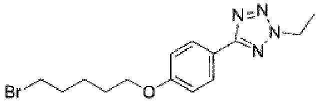

Add K₂CO₃ (0.653g, 4.73mmol) and 1,5-dibromopentane (1.09g, 4.73mmol) into the solution of Embodiment 11C (0.3g, 1.58mmol) in acetone (5ml). Agitate the mixture solution 12 hours at 80°C. Then extract it with ethyl acetate. Dry the organic phase with anhydrous Na₂SO₄ before filter and evaporate. Then purify the residue with column chromatography (petroleum ether: ethyl acetate =10:1) to obtain the title compound (white solid, 0.3g, yield of 56%). ¹H-NMR(CDCl₃,400MHz)δ8.08(d,*J*=8.8Hz,2H),7.01(d,*J*=8.8Hz,2H),4.70(q,*J*=7.4Hz,2H),4.05(t,*J*=6.4H z,2H),3.44-3.52(m,2H),1.93-2.03(m,2H),1.83-1.91(m,2H),1.64-1.73(m,5H),LCMS(ESI)m/z:340(M+1).

### Embodiment 11E

### 2-(5-(4-(2-ethyl--2H-tetrazol-5-yl)-phenoxy)pentyl)-5-(pyridin-4-yl)-1,2,5-thiadiazolidine-1,1-dioxide

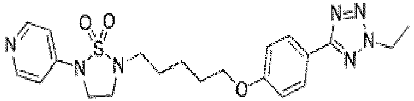

At 0°C, add sodium hydride (7mg, 0.17mmol, 60% content) into the solution of Embodiment 4F (30mg, 0.15mmol) in N, N- dimethylformamide (2ml). Keep it still 30 minutes at 0°C. Then add the solution of Embodiment 11D (51mg, 0.15mmol) in N,N-dimethylformamide (1ml) into the mixture solution above. Agitate it 6 hours at 15°C. Then add water to quench the reaction and extract the aqueous phase with ethyl acetate. Dry the combined organic phases with anhydrous Na₂SO₄ before filter and evaporate to obtain the title compound (white solid, 5mg, yield of 7.26%). ¹H-NMR(400MHz,CDCl₃):δ8.51(d,*J*=6.5Hz,2H),8.06(d,*J*=8.5Hz,2H),7.05(d,*J*=6.0Hz,2H),6.99(d,*J*=9.0 Hz,2H),4.68(q,*J*=7.5Hz,2H),4.04(t,*J*=6.0Hz,2H),3.85(t,*J*=6.3Hz,2H),3.55(t,*J*=6.3Hz,2H),3.19(t,*J*=7.3H z,2H),1.85-1.93(m,2H),1.75-1.82(m,2H),1.68(t,*J*=7.5Hz,5H). LCMS(ESI)m/z:458(M+1).

### Embodiment 12

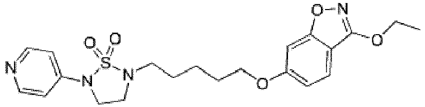

### Embodiment 12A

### N, 2- dihydroxy-4-methoxybenzamide

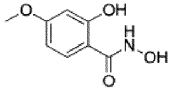

With magnetic stirring, drop slowly the solution of NaOH (71g, 1.78mol) in water (250ml) into the solution of hydroxylamine hydrochloride (53g, 766mmol) in water (500ml). Under the protection of N₂ gas, immediately drop slowly the solution of 4-methoxy salicylate (93g, 510mmol) in dioxane (250ml) into the solution above to react 12 hours. After the reaction ends, add slowly the reaction solution into ice water before drop the concentrated hydrochloric acid until the pH value becomes 2.
Then filter to obtain the title compound (84g, yield of 90%). ¹H-NMR(400MHz,MeOD)δ7.57(d,*J*=9.04Hz,1H),6.41-6.54(m,2H),3.75-3.87(m,3H). LCMS(ESI)m/z:184(M+1).

### Embodiment 12B

### 6-methoxy-benzo[d]isoxazol-3(2H)-one

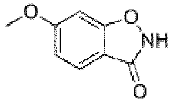

Drop Embodiment 12A (40g, 0.22mol) into the solution of carbonyl diimidazole (50g, 0.31mol) in anhydrous THF (600ml). After the dropping is completed, heat to reflux 8 hours. After the reaction ends, dry the reaction solution through rotation. Then add 400ml water and adjust it with citric acid to pH = 6. Then filter to obtain the title compound (23g, yield of 63%). ¹H-NMR(400MHz,MeOD)δ7.57(d,*J*=8.53Hz,1H),6.97(d,*J*=1.51Hz,1H),6.91(dd,*J*=1.76,8.78Hz,1H),3. 84-3.93(m,3H). LCMS(ESI)m/z:166(M+1).

### Embodiment 12C

### 3-ethoxy-6-methoxy-benzo[d]isoxazole

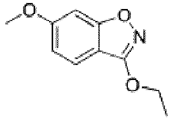

In ice bath, add sequentially anhydrous ethanol (3.5g, 76mmol) and triphenylphosphine (24g, 90mmol) into the solution of Embodiment 12B (10g, 60mmol) in anhydrous THF (200ml). Then add slowly DIAD (18ml, 90mmol) into the solution above and agitate it to react 15 minutes at 0°C. Heat slowly it to room temperature and agitate overnight. After the reaction ends, add water (200ml) to quench the reaction. Then extract the aqueous layer with ethyl acetate (200ml x 2). Then wash the combined organic layers with water and saline water sequentially. Then dry it with anhydrous Na₂SO₄ before filter and evaporate. Then purify the residue with the silica gel column chromatography to obtain the title compound (5.6g, yield of 48%). LCMS(ESI)m/z:194(M+1).

### Embodiment 12D

### 3-ethoxy-benzo[d]isoxazole-6-ol

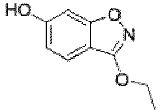

Under the protection of N₂ gas at -78°C, drop slowly boron tribromide (4.2g, 16.8 mmol) into the solution of Embodiment 12C (810mg, 4.2mmol) in dichloromethane (10ml). After the dropping is completed, heat the reaction solution slowly to room temperature and agitate overnight. After the reaction is completed, drop slowly the reaction solution into ice water (100ml) to quench the reaction. Then extract the aqueous layer with ethyl acetate (100ml x 2). Then wash the combined organic layers with water and saline water. Then dry it with anhydrous Na₂SO₄ before filter and evaporate to obtain the title compound (650mg, yield of 86.7%). ¹H-NMR(CDCl₃,400MHz)δ7.48(d,*J*=8.53Hz,1H),6.90-7.08(m,2H),6.85(d,*J*=8.03Hz,1H),4.47(q,*J*=7.03 Hz,2H),1.51(t,*J*=7.03Hz,3H).

### Embodiment 12E

### 6-((5-bromo-pentyl)oxy)-3-ethoxypropionate[d]isoxazole

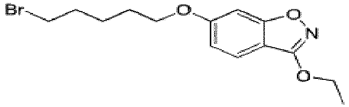

Under the protection of N₂ gas at 0°C, add sodium hydride (54mg, 1.34mmol, 60% content) in batch into the solution of Embodiment 12D (0.2g, 1.12mmol) in N, N- dimethylformamide (20ml). After the substances are added, agitate the mixture solution 1 hour at room temperature, then add the solution of 1,5-dibromopentane (770ml, 2.33mmol) in N, N- dimethylformamide (10ml) into the solution above. Then agitate it to react 10 hours at room temperature. Pour the reaction solution into water (100ml) and then extract the aqueous phase with ethyl acetate (50ml x 3). Then dry the combined organic phases with anhydrous Na₂SO₄ before filter and concentrate to obtain the title compound (yellow liquid, 0.2g, yield of 54.6%). LCMS(ESI)m/z:328(M+1).

### Embodiment 12F

### 2-(5-((3-ethoxy-benzo[d]isoxazole-6-yl)-oxy)pentyl)-5-(pyridin-4-yl)-1,2,5-thiadiazolidine-1,1-dioxide

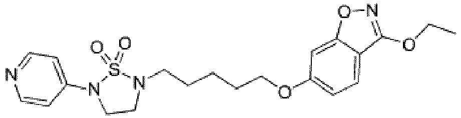

Under the protection of N₂ gas at 0°C, add sodium hydride (24mg, 0.6mmol, 60% content) into the solution of Embodiment 4F (60mg, 0.3mmol) in N, N- dimethylformamide (3ml). Agitate it 0.5 hour at 0°C. Then drop the solution of Embodiment 12E (100mg, 0.3mmol) in N, N- dimethylformamide (1ml) dropwise into it. Agitate the reaction mixture 1.5 hours at 0°C. Quench the reaction mixture with the saturated NH₄Cl aqueous solution (5ml). Then dilute it with water (50ml) and extract with ethyl acetate (15ml x 4). Dry the combined organic phases with the saturated salt water. Then dry with anhydrous Na₂SO₄ before filter and dry through rotation. Then purify the residue with preparative chromatography to obtain the title compound (white solid, 60mg, yield of 45%). ¹H-NMR (400MHz, CDCl₃) δ 8.53 (br.s.,1H), 7.47 (d, *J*=8.5 Hz, 1H), 7.27 (s, 1H), 7.07 (br.s.,2H), 6.78-6.89 (m, 2H), 4.46 (q, *J*=7.0 Hz, 2H), 4.03 (t, *J*=6.2 Hz, 2H), 3.85 (t, *J*=6.2 Hz, 2H), 3.55 (t, *J*=6.27 Hz, 2H), 3.19 (t, *J*=7.28 Hz, 2H), 1.85-1.94 (m, 2H), 1.80 (q, *J*=7.4 Hz, 2H), 1.64-1.68 (m, 2H), 1.50 (t, *J*=7.0 Hz, 3H). LCMS (ESI) m/z: 447 (M+1).

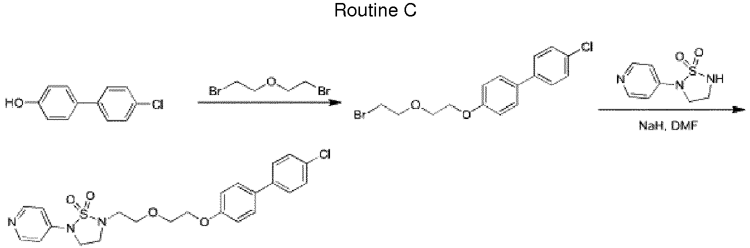

### Embodiment 13

### 2-(2-(2-((4'-chloro-[1,1'-biphenyl]-4-yl)oxy)ethoxy)ethyl)-5-(pyridin-4-yl)-1,2,5-thiadiazolidine-1,1-dioxide

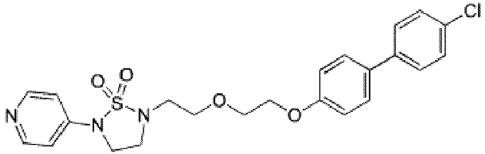

### Embodiment 13A

### 4-(2-(2-bromoethoxy) ethoxy)-4'-chloro-1,1'-biphenyl

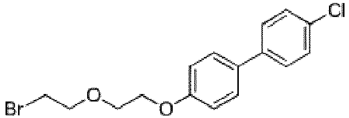

Add 1-bromo-2- (2-bromoethoxy)-ethane (3.4g, 14.66mmol) into the mixture of Embodiment 6A (1g, 4.89mmol), K₂CO₃ (1.35g, 9.77mmol) and KI (810mg, 4.89mmol) in acetone (15ml). Agitate the mixture 3 hours at 70°C before dry through rotation. Then add 20ml petroleum ether into the system and filter it to obtain the title compound (1.5g, yield of 86%). ¹H-NMR(CDCl₃,400MHz)7.47(dd,*J*=8.5,4.0Hz,4H),7.34-7.41(m,2H),6.99(d,*J*=8.5Hz,2H),4.16-4.22(m, 2H),3.85-3.96(m,4H),3.51(t,*J*=6.3Hz,2H).

### Embodiment 13B

### 2-(2-(2-((4'-chloro-[1,1'-biphenyl]-4-yl)-oxy)-ethoxy)-ethyl)-5-(pyridin-4-yl)-1,2,5-thiadiazolidine-1,1-dioxide

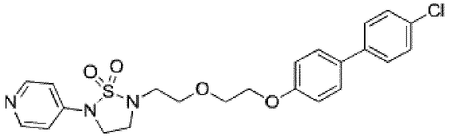

Please refer to the preparation method of Embodiment 12F for this embodiment. ¹H-NMR(CDCl₃,400MHz)8.48(d,*J*=6.0Hz,2H),7.45-7.51(m,4H),7.36-7.41(m,2H),7.02(d,*J*=6.3Hz,2H), 6.98(d,*J*=8.8Hz,2H),4.16-4.20(m,2H),3.88(q,*J*=4.9Hz,4H),3.76(dd,*J*=11.9,5.1Hz,4H),3.40(t,*J*=4.9Hz,2 H). LCMS(ESI)m/z:474(M+1).

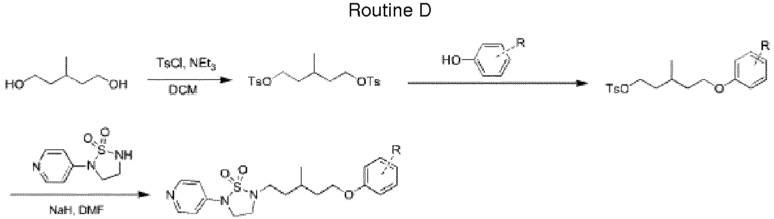

### Embodiment 14

### (E)-4-((5-(1,1-dioxo-5-(pyridin-4-yl) -1,2,5-thiadiazolidine-2-yl)-3-methyl-pentyl)-oxy)-benzaldehyde-O-ethyloxime

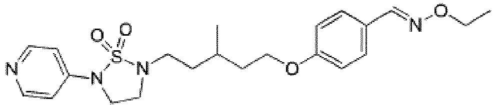

### Embodiment 14A

### 3-methylpentane-1,5-bis (4-methyl-benzenesulfonyl)

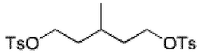

Add triethylamine (160ml) and p-toluensulfonyl chloride (258.1g, 1.35mol) into the solution of 3-methyl-1,5-pentanediol (40g, 338.5mmol) in dichloromethane (400ml). Agitate the mixture solution 12 hours at 20°C. Then extract it with ethyl acetate. Dry the combined organic phases with anhydrous Na₂SO₄ before filter and evaporate. Then purify the residue with column chromatography (petroleum ether: ethyl acetate =20:1) to obtain the title compound (colorless liquid, 80g, yield of 55%). ¹H-NMR(CDCl₃,400MHz)δ7.78(d,*J*=8.0Hz,4H),7.37(d,*J*=8.0Hz,4H),4.01(q,*J*=6.0Hz,4H),2.46(s,6H)1. 57-1.69(m,3H)1.43(dq,*J*=13.4,6.7Hz,2H),0.78(d,*J*=6.3Hz,3H). LCMS(ESI)m/z:299(M+1).

### Embodiment 14B

### (E)-5-(4-((ethoxyimino)methyl)-phenoxy)-3-methylpentyl-4-methylbenzenesulfonyl

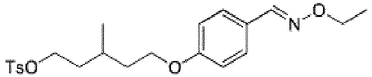

Allow the mixture of Embodiment 7A (4.98g, 30.17mmol), Embodiment 14A(11.7g, 27.43mmol), K₂CO₃ (7.58g, 54.86mmol) and acetonitrile (110ml) to react 16 hours at 80°C. Add ethyl acetate (50ml) and water (50ml) into the reaction system. Extract the aqueous layer with ethyl acetate (100ml x 3) and then dry the combined organic layers with Na₂SO₄ before filter and evaporate. Then purify the residue with column chromatography to obtain the title compound (colorless solid, 3.7g, yield of 32%). ¹H-NMR(CDCl₃,400MHz)8.03(s,1H),7.46(d,*J*=8.5Hz,2H),6.78-6.85(m,2H),4.20(q,*J*=7.0Hz,2H),1.31(t, *J*=7.0Hz,3H).

### Embodiment 14C

### (E)-4-((5-(1,1-dioxo-5-(pyridin-4-yl)-1,2,5-thiadiazolidine-2-yl)-3-methyl-pentyl)-oxy)-benzaldehyde-O-ethyloxime

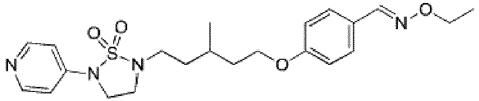

Please refer to the preparation method of Embodiment 12F for this embodiment.
¹H-NMR(CDCl₃,400MHz)δ8.51(d,*J*=5.5Hz,2H),8.02(s,1H),7.50(d,*J*=8.5Hz,2H),7.04(d,*J*=6.0Hz,2H),6. 87(d,*J*=8.5Hz,2H),4.17-4.24(m,2H),3.98-4.07(m,2H),3.82(t,*J*=6.5Hz,2H),3.48-3.55(m,2H),3.20(t,*J*=7. 5Hz,2H),1.79-1.93(m,3H),1.66-1.73(m,1H),1.56-1.61(m,1H),1.31(t,*J*=7.0Hz,3H),1.03(d,*J*=6.5Hz,3H). LCMS(ESI)m/z:447(M+1).

### Embodiment 15

### 2-(5-((3-ethoxy-benzo[d]isoxazole-6-yl)-oxy)-3-methyl-pentyl)-5-(pyridin-4-yl)-1,2,5-thiadiazolidine-1,1-dioxide

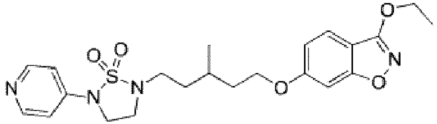

### Embodiment 15A

### 5-((3-ethoxy-benzo[d]isoxazole-6-yl)-oxy)-3-methyl-pentyl-4-methylbenzenesulfonate

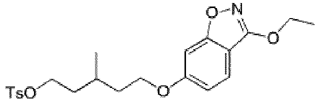

Add K₂CO₃ (0.69g, 5.0mmol) and Embodiment 14A (2.14g, 5.0mmol) into the solution of Embodiment 12D (0.3g, 1.67mmol) in N,N-dimethylformamide (5ml). Agitate the mixture solution 12 hours at 80°C. Then extract it with ethyl acetate. Dry the organic phase with anhydrous Na₂SO₄ before filter and evaporate. Then purify the residue with column chromatography (petroleum ether: ethyl acetate =10:1) to obtain the title compound (white solid, 0.3g, yield of 41.3%). ¹H-NMR(CDCl₃,400MHz)δ7.79(d,*J*=8.0Hz,2H)7.46(d,*J*=8.5Hz,1H)7.33(d,*J*=8.0Hz,2H)6.78-6.84(m,2 H)4.47(q,*J*=7.0Hz,2H)4.07-4.15(m,2H)3.93-4.01(m,2H)2.43(s,3H)1.74-1.89(m,3H)1.60-1.66(m,1 H)1. 54-1.58(m,1H)1.51(t,*J*=7.0Hz,3H)0.92(d,*J*=6.5Hz,3H).LCMS(ESI)m/z:434(M+1).

### Embodiment 15B

### 2-(5-((3-ethoxy-benzo[d]isoxazole-6-yl)oxy)-3-methyl-pentyl)-5-(pyridin-4-yl)-1,2,5-thiadiazolidine-1,1-dioxide

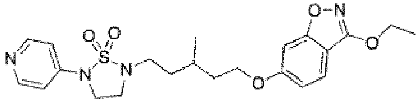

At 0°C, add sodium hydride (6.62mg, 0.165mmol) into the solution of Embodiment 4F (30mg, 0.15mmol) in N, N- dimethylformamide (2ml). Agitate 30 minutes at 0°C. Then add the solution of Embodiment 15A (65mg, 0.15mmol) in N, N- dimethylformamide (1ml) into the mixture solution above. Agitate it 6 hours at 15°C. Then add water to quench the reaction and extract the aqueous phase with ethyl acetate. Dry the combined organic phases with anhydrous Na₂SO₄ before filter and evaporate to obtain the title compound (white solid, 18mg, yield of 25.8%). ¹H-NMR(400MHz,CDCl₃)δ8.53(d,*J*=6.0Hz,2H)7.46-7.51(m,1H)7.06(d,*J*=6.3Hz,2H)6.86(dd,*J*=4.3,2.5 Hz,2H)4.48(q,*J*=7.0Hz,2H)4.08(q,*J*=5.7Hz,2H)3.86(t,*J*=6.4Hz,2H)3.50-3.60(m,2H)3.24(t,*J*=7.4Hz,2H )1.88-1.99(m,2H)1.77-1.87(m,1H)1.61-1.75(m,3H)1.52(t,*J*=7.2Hz,3H)1.07(d,*J*=6.3Hz,3H). LCMS(ESI)m/z:461(M+1).

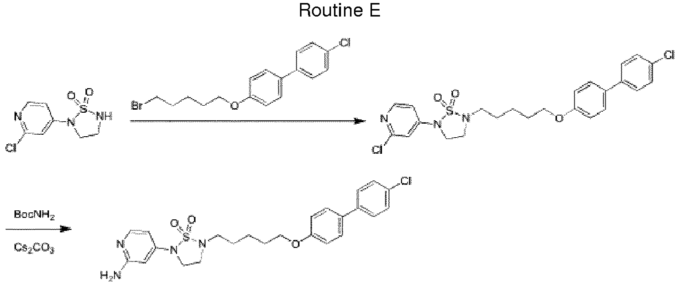

### Embodiment 16

### 2-(2-aminopyridine-4-yl)-5-(5-((4'-chloro-[1,1'-biphenyl]-4-yl)-oxy)-pentyl)-1,2,5-thiadiazolidine-1,1-dioxide

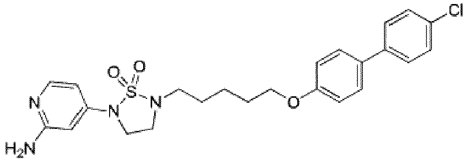

### Embodiment 16A

### 2-(5-((4'-chloro-[1,1'-biphenyl]-4-yl)-oxy)-pentyl)-5-(2-chloro-pyridin-4-yl)-1,2,5-thiadiazolidine-1,1-dioxane

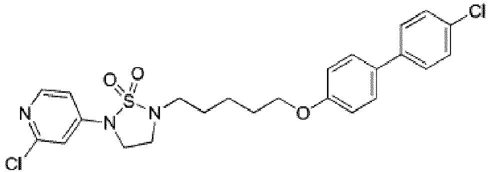

Under the protection of N₂ gas at 0°C, add sodium hydride (68mg, 1.72mmol, 60% content) in batch into the solution of Embodiment 4E (200mg, 0.86mmol) in N, N- dimethylformamide (3ml). Agitate the reaction solution 0.5 hour at room temperature. Then add the solution of Embodiment 6B (303mg, 0.86mmol) in N, N- dimethylformamide (5ml). Then agitate it 8 hours at room temperature. Add water to quench the reaction. Then, extract it with ethyl acetate (50ml x 3) and dry the combined organic layer with Na₂SO₄ before filter and evaporate. Then separate and purify the residue with TLC plate to obtain the title compound (80mg, yield of 22%). ¹H-NMR(CDCl₃,400MHz)8.29(d,*J*=5.8Hz,1H),7.48-7.52(m,4H),7.38-7.42(m,2H),7.09(dd,*J*=5.8,2.3Hz, 1H),7.01(d,*J*=2.0Hz,1H),6.98(d,*J*=8.8Hz,2H),4.05(t,*J*=6.3Hz,2H),3.86(t,*J*=6.4Hz,2H),3.58(t,*J*=6.4Hz, 2H),3.21(t,*J*=7.2Hz,2H),1.76-1.95(m,4H),1.63-1.70(m,2H). LCMS(ESI)m/z:506(M+1).

### Embodiment 16B

### 2-(2-aminopyridine-4-yl)-5-(5-((4'-chloro-[1,1'-biphenyl]-4-yl)-oxy)-pentyl)-1,2,5-thiadiazolidine-1,1-dioxide

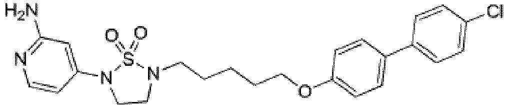

Under the protection of N₂ gas, add Pd₂(dba)₃ (20mg, 0.03mmol) and Xant-Phos (10mg, 0.03mmol) into the mixture solution of Embodiment 16A (80mg, 0.16mmol), tert-butyl carbamate (74mg, 0.63mmol) and cesium carbonate (103mg, 0.32mmol) in dioxane / N, N-dimethylformamide (1.5ml/0.5ml). After the substances are added, agitate the mixture solution 16 hours at 110°C. After remove the solvent under vacuum, extract the residue with ethyl acetate (250ml x 3) and then dry the combined organic layers with Na₂SO₄ before filter and evaporate. Then purify the residue with the preparative HPLC to obtain the title compound (30mg, yield of 31%).
¹H-NMR(CDCl₃,400MHz):7.80(sbr,1H),7.48(dd,*J*=8.8,3.3Hz,4H),7.35-7.41(m,2H),6.96(d,*J*=8.5Hz,2H ),6.58(s,br,1H),6.33(s,br,1H),4.02(t,*J*=6.0Hz,2H),3.87(s,br,2H),3.55(t,*J*=6.3Hz,2H),3.19(t,*J*=7.3Hz,2H ),1.85-1.90(m,2H),1.76-1.82(m,2H),1.63(d,*J*=6.5Hz,2H). LCMS(ESI)m/z:487(M+1).

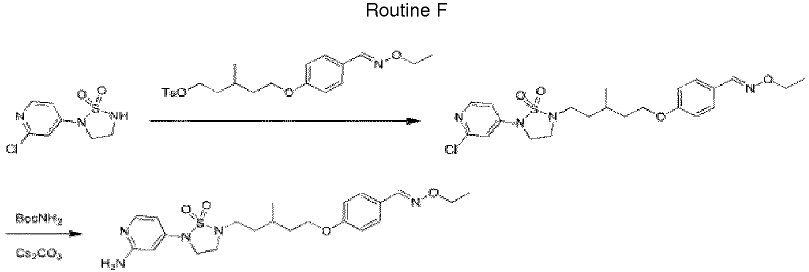

### Embodiment 17

### (E)-4-((5-(5-(2-amino-pyridin-4-yl)-1,1-dioxo-1,2,5-thiadiazolidine-2-yl)-3-methyl-pentyl)-oxy)-benzaldehyde-O-ethylketoxime

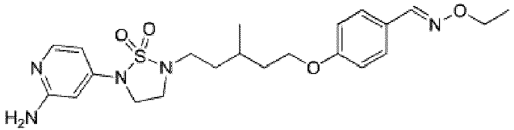

### Embodiment 17A

### (E) -4-((5-(5-(2-chloropyridin-4-yl)-1,1-dioxo-1,2,5-thiadiazolidine-2-yl) -3-methyl-pentyl)-oxy)-benzaldehyde-O-ethylketoxime

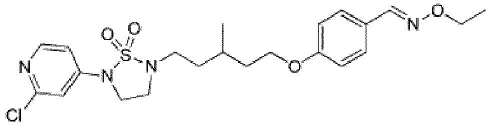

This embodiment is prepared with the method as described in Embodiment 16A.
¹H-NMR(CDCl₃,400MHz):8.29(d,*J*=5.5Hz,1H),8.04(s,1H),7.53(d,*J*=8.8Hz,2H),7.08(dd,*J*=5.8,2.0Hz,1 H),7.01(s,1H),6.90(d,*J*=8.5Hz,2H),4.22(q,*J*=7.0Hz,2H),4.01-4.12(m,2H),3.80-3.88(m,2H),3.50-3.61( m,2H),3.23(t,*J*=7.5Hz,2H),1.60-1.98(m,5H),1.34(t,*J*=7.0Hz,3H),1.06(d,*J*=6.5Hz,3H). LCMS(ESI)m/z:481(M+1).

### Embodiment 17B

### (E)-4-((5-(5-(2-amino-pyridin-4-yl)-1,1-dioxo-1,2,5--thiadiazolidine2-yl)-3-methyl-pentyl)-oxy)-benzaldehyde-O-ethylketoxime

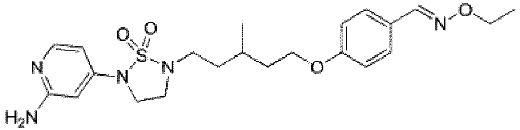

Please refer to the preparation method of Embodiment 16 for this embodiment.
¹H-NMR(CDCl₃,400MHz)8.03(s,1H),7.98(d,*J*=5.5Hz,1H),7.51(d,*J*=8.5Hz,2H),6.88(d,*J*=8.5Hz,2H),6.4 1(d,*J*=6.0Hz,1H),6.29(s,1H),4.20(q,*J*=7.2Hz,2H),4.01-4.09(m,2H),3.77(t,*J*=6.3Hz,2H),3.43-3.55(m,2 H),3.19(t,*J*=7.5Hz,2H),1.63-1.95(m,5H),1.32(t,*J*=7.0Hz,3H),1.03(d,*J*=6.5Hz,3H). LCMS(ESI)m/z:462(M+1).

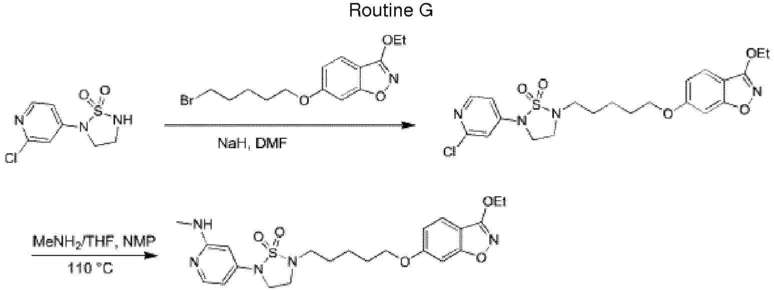

### Embodiment 18

### 2-(5-((3-ethoxy-benzo[d]isoxazol-6)-oxo)-pentyl)-5-(2-(methylamino)-pyridine-4)-1,2, 5-thiadiazoline-1,1-dioxide

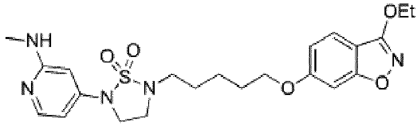

### Embodiment 18A

### 2-(2-chloro-pyridine-4-)-5-(5-((3-ethoxy-benzo[d]isoxazol-6-)oxo)-pentyl)-1,2,5-thiadiazoline-1,1-dioxide

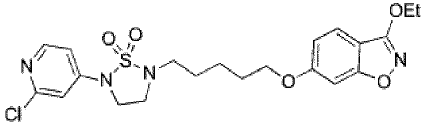

Under the protection of N₂ gas at 0°C, add sodium hydride (93mg, 2.32mmol) into the solution of Embodiment 4E (271 mg, 1.16mmol) in N, N- dimethylformamide. Agitate it 0.5 hour at 0°C. Then add the solution of Embodiment 12E (400mg, 1.22mmol) in N, N- dimethylformamide (5ml). Agitate the reaction mixture 1 hour at 0°C and then agitate 12 hours at room temperature. Quench the reaction mixture with the saturated NH₄Cl aqueous solution (5ml). Then dilute it with a large amount of water (120ml) before extract with ethyl acetate (30ml x 3). Then wash the combined organic phases with the saturated salt water. Then dry with anhydrous Na₂SO₄ before filter and dry through rotation. Then purify the residue with column chromatography (dichloromethane: methanol = 20:1) to obtain the title compound (white solid, 210mg, yield of 38%).
¹H-NMR(400MHz,CDCl₃)δ8.27(d,*J*=6.0Hz,1H),7.47(d,*J*=9.0Hz,1H),7.06(dd,*J*=6.0,2.0Hz,1H),6.99(d,*J* =2.0Hz,1H),6.81-6.87(m,2H),4.46(q,*J*=7.0Hz,2H),4.02(t,*J*=6.2Hz,2H),3.84(t,*J*=6.2Hz,2H),3.56(t,*J*=6. 2Hz,2H),3.19(t,*J*=7.0Hz,2H),1.85-1.93(m,2H),1.80(q,*J*=7.4Hz,2H),1.50(t,*J*=7.0Hz,3H),0.80-0.92(m,2 H). LCMS(ESI)m/z:481(M+1).

### Embodiment 18B

### 2-(5-((3-ethoxy-benzo[d]isoxazol-6)-oxo)-pentane) 5-(2-(methylamino)-pyridine-4)-1,2, 5-thiadiazoline-1,1-dioxide

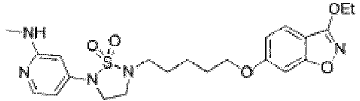

Add the solution of methylamine in THF (5ml, 2mol/L, 12.5mmol) into the solution of Embodiment 18A (100mg, 0.2mmol) in N-methylpyrrolidone (8ml). Place the mixture into a sealed pot and agitate it to react 72 hours at 110°C. Then remove the solvent of the reaction solution at vacuum. Then separate and purify the residue with the preparative chromatography to obtain the title compound (yellow powder, 10mg, yield of 10%). ¹H-NMR(400MHz,CDCl₃)δ8.02(br s,1H),7.48(br s,1H),6.85(br s,2H),6.38(br s,1H),6.16(br s,1H),4.49(br s,2H),4.04(br s,2H),3.84(br s,2H),3.52(br s,2H),3.19(br s,2H),2.94(br s,3H),1.91(br s,2H),1.81(br s,2H),1.69-1.75(m,2H),1.52(br s,3H). LCMS(ESI)m/z:476(M+1).

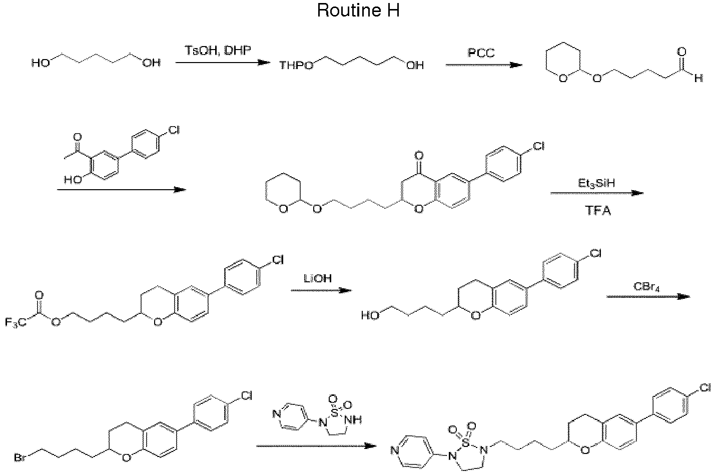

### Embodiment 19

### 2-(4-(6-(4-chlorophenyl)-chroman-2-yl)-butyl)-5-(pyridin-4-yl)-1,2,5-thiadiazolidine-1,1-dioxide

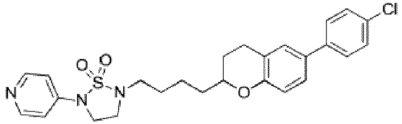

### Embodiment 19A

### 5-((tetrahydro-2H-pyran-2-yl)-oxy)-pentan-1-ol

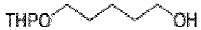

Agitate 1,5-pentanediol (60g, 0.57mol), 3,4-dihydropyran (48.5g, 0.57mol) and p-toluenesulfonic acid (50mg) 3 hours at 40°C. After the reaction ends, add K₂CO₃ solution (200ml) before extract the aqueous phase with ethyl acetate (150ml x 3). Dry the combined organic phases with anhydrous Na₂SO₄. Then filter and dry through evaporation to obtain the residue to directly use further. ¹H-NMR(CDCl₃,400MHz)δ4.54(d,*J*=3.5Hz,1H),3.78-3.89(m,1H),3.68-3.77(m,1H),3.60(br.s.,2H),3.43-3.52(m,1H),3.32-3.41(m,1H),2.02(br.s.,1H),1.80(m,1H),1.65-1.68(m,1H),1.39-1.70(m,10H).

### Embodiment 19B

### 5-((tetrahydro-2H-pyran-2-yl)-oxy)-pentanal

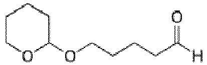

Add PCC (90g) in batch into the solution of Embodiment 19A (60g, 0.30mol) in dichloromethane (400ml) and agitate the reaction mixture 5 hours at room temperature. Filter and then concentrate the filtrate. Then purify it with column chromatography to obtain the title compound.
¹H-NMR(400MHz,CDCl₃)δ9.62-9.79(m,1H),4.50(br.s.,1H),3.58-3.91(m,2H),3.25-3.53(m,2H),2.50-2.6 8(m,2H),2.42(dd,*J*=1.25,3.51Hz,2H),1.48-1.79(m,8H).

### Embodiment 19C

### 6-(4-chlorophenyl)-2-(4-((tetrahydro-2H-pyran-2-yl)-oxy)-butyl)-chroman-4-one

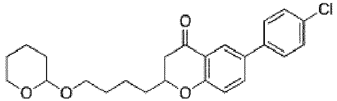

Agitate the mixture of Embodiment 19B (7.5g, 40.27mmol), Embodiment 1F (9.93g, 40.27mmol) and piperidine (3.43g, 40.27mmol) in ethanol (100ml) 3 hours at 85°C. Add ethyl acetate (50ml) and water (50ml) into the reaction system. Then adjust the pH value of the reaction system with dilute aqueous hydrochloric acid to 6. Extract the aqueous layer with dichloromethane (100ml x 3) and then dry the combined organic layers with Na₂SO₄ before filter and evaporate. Then purify the residue with column chromatography to obtain the title compound (yellow liquid, 7g, yield of 42%). ¹H-NMR(400MHz,CDCl₃)δ8.05(d,*J*=2.3Hz,1H),7.67(dd,*J*=8.7,2.4Hz,1H),7.48(d,*J*=8.5Hz,2H),7.38(d,*J* =8.5Hz,2H),7.04(d,*J*=8.5Hz,1H),4.58(d,*J*=4.3Hz,1H),4.43-4.52(m,1H),3.86(td,*J*=7.3,3.9Hz,1H),3.74-3.82(m,1H),3.47-3.54(m,1H),3.39-3.45(m,1H),2.67-2.76(m,2H),1.89-1.99(m,1 H),1.76-1.86(m,2H),1.6 3-1.71(m,4H),1.49-1.59(m,5H).

### Embodiment 19D

### 4-(6-(4-chlorophenyl)-chroman-2-yl)-butyl-2,2,2-trifluoroacetate

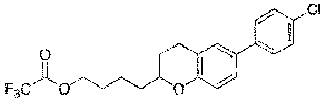

Add the triethylsilyl hydride (10ml) into the solution of Embodiment 19C (2.5g, 6.03mmol) in trifluoroacetic acid (20ml). Allow the mixture to react 3 hours at 50°C. Evaporate it to dry the solvent. Then add ethyl acetate (20ml) and water (20ml) into the reaction system. Extract the aqueous layer with ethyl acetate (20ml x 3) and then dry the combined organic layers with Na₂SO₄ before filter and evaporate to obtain the title compound (yellow liquid, 2g, yield of 80%).

### Embodiment 19E

### 4-(6-(4-chlorophenyl)-chroman-2-yl)-butan-1-ol

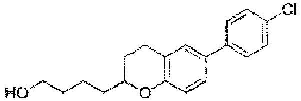

Add LiOH (29mg, 1.21 mmol) into the mixture of Embodiment 19D (500mg, 1.21 mmol) in 1,4-dioxane (5ml) and water (2ml). Then allow the mixture to react 3 hours at 25°C. Add ethyl acetate (10ml) and water (10ml) into the reaction system. Extract the aqueous layer with ethyl acetate (10ml x 3) and then dry the combined organic layers with Na₂SO₄ before filter and evaporate. Then purify the residue with column chromatography to obtain the title compound (colorless solid, 300mg, yield of 78%). ¹H-NMR(400MHz,CDCl₃)δ7.43-7.47(m,2H),7.33-7.38(m,2H),7.26-7.30(m,2H),6.86(d,*J*=8.3Hz,1H),4. 03(br.s.,1 H),3.70(t,*J*=5.9Hz,2H),2.76-2.95(m,2H),1.98-2.08(m,1 H),1.71-1.85(m,2H),1.58-1.70(m,5H).

### Embodiment 19F

### 2-(4-bromobutyl)-6-(4-chlorophenyl)-chroman

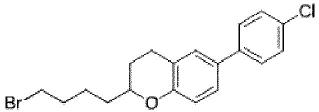

At 0°C, add slowly the triphenylphosphine (420mg, 1.6mmol) into the mixture of Embodiment 19E (500mg, 1.6mmol), CBr₄ (3.4g, 10.3mmol) in THF (5ml). Allow the mixture to react 1 hour at 60°C. Concentrate the reaction solution under reduced pressure. Purify the residue with column chromatography to obtain the title compound (yellow solid, 658mg, yield of 83%). ¹H-NMR(400MHz,CDCl₃)δ7.43-7.48(m,2H),7.34-7.38(m,2H),7.24-7.29(m,2H),6.86(d,*J*=8.5Hz,1H),4. 00-4.07(m,1H),3.45-3.49(m,2H),2.77-2.96(m,2H),1.90-2.02(m,3H),1.62-1.80(m,5H).

### Embodiment 19G

### 2-(4-(6-(4-chlorophenyl)-chroman-2-yl)-butyl)-5-(pyridin-4-yl)-1,2,5-thiadiazolidine-1,1-dioxide

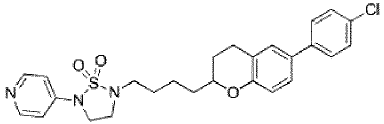

This embodiment is prepared with the method as described in Embodiment 18A.
¹H-NMR(400MHz,CDCl₃)δ8.51(d,*J*=6.0Hz,2H),7.42-7.48(m,2H),7.33-7.40(m,2H),7.29(s,3H),7.05(d,*J* =6.0Hz,2H),6.85(d,*J*=8.5Hz,1H),4.03(br.s.,1H),3.86(t,*J*=6.5Hz,2H),3.52-3.59(m,2H),3.20(t,*J*=7.0Hz,2 H),2.77-2.95(m,2H),2.03(d,*J*=13.1Hz,1H),1.64-1.85(m,7H). LCMS(ESI)m/z:498(M+1).

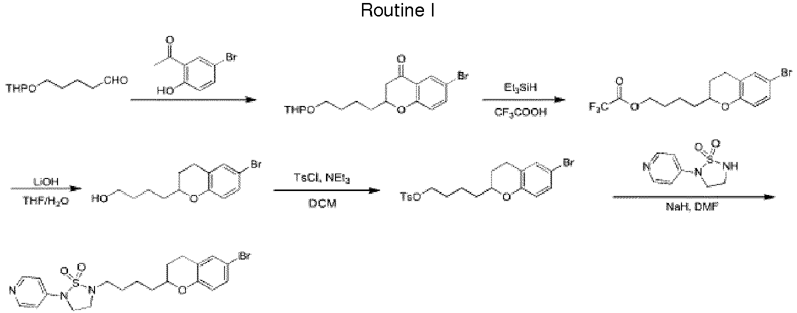

### Embodiment 20

### 2-(4-(6-bromo-chroman-2-yl)-butyl)-5-(pyridin-4-yl)-1,2,5-thiadiazolidine-1,1-dioxide

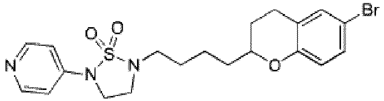

### Embodiment 20A

### 6-bromo-2-(4-((tetrahydro-2H-pyran-2-yl)-oxy)-butyl)-chroman-4-one

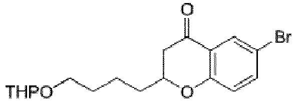

Mix Embodiment 19B (5.0g, 29mmol), 5'-bromo-2'-hydroxyacetophenone (6.37g, 29mmol) and piperidine (2.0ml) into ethanol (150ml) and reflux 4 hours. Then dry it under reduced pressure and purify the yielded residue with column chromatography to obtain the title compound (yellow solid, 6.5g, yield of 57%). ¹H-NMR(CDCl₃,400MHz)δ8.00(d,*J*=2.3Hz,1H),7.56(dd,*J*=8.8,2.3Hz,1H),6.90(d,*J*=8.8Hz,1H),4.60(br. s.,1H),4.40-4.52(m,1H),3.72-3.95(m2H),3.37-3.59(m,2H),2.66-2.80(m,2H),1.48-2.01(m,12H).

### Embodiment 20B

### 4-(6-bromo-chroman-2-yl)-2,2,2-trifluoroacetate

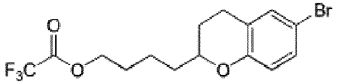

Mix Embodiment 20A (1.0g, 2.57mmol) and triethylsilane hydrogen (7.3g, 62.8mmol) into trifluoroacetate (20ml). Then heat it 5 hours at 60°C. After concentrate it under reduced pressure, use directly the crude product (5.8g, yield of crude product is 97%). ¹H-NMR(CDCl₃,400MHz)δ7.17(d,*J*=2.0Hz,2H),6.60-6.70(m,1H),4.30-4.47(m,2H),3.96(td,*J*=7.8,2.0Hz ,1H),2.67-2.90(m,2H),1.97(ddt,*J*=13.5,5.6,2.8Hz,1H),1.48-1.89(m,8H).

### Embodiment 20C

### 4-(6-bromo-chroman-2-yl)-butan-1-ol

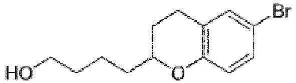

Please refer to the preparation method of Embodiment 19E for this embodiment.
¹H-NMR(CDCl₃,400MHz)δ7.11-7.24(m,1H),6.64-6.75(m,1H),3.99(d,*J*=5.0Hz,1H),3.61-3.80(m,2H),2. 66-2.91(m,2H),1.93-2.06(m,1 H),1.47-1.84(m,10H).

### Embodiment 20D

### 4-(6-bromo-chroman-2-yl)-butyl-4-methyl-benzenesulfonate

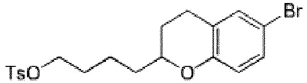

Add triethylamine (76mg, 0.75mmol) and p-toluensulfonyl chloride (95mg, 0.5mmol) into the solution of Embodiment 20C (71mg, 0.25mmol) in dichloromethane (5.0ml). Agitate the reaction mixture 4 hours at 12°C. After concentrate it under reduced pressure, purify the crude product with TLC to obtain the title compound (colorless liquid, 60mg, yield of 55%). ¹H-NMR(CDCl₃,400MHz)δ7.73-7.85(m,2H),7.36(d,*J*=8.0Hz,2H),7.17(d,*J*=4.8Hz,2H),6.61-6.69(m,1H) , 4.03-4.11(m,2H),3.84-3.97(m,1H),2.66-2.89(m,2H),2.45(s,3H),1.88-2.01(m,1H),1.42-1.83(m,7H).

### Embodiment 20E

### 2-(4-(6-bromo-chroman-2-yl)-butyl)-5-(pyridin-4-yl)-1,2,5-thiadiazolidine-1,1-dioxide

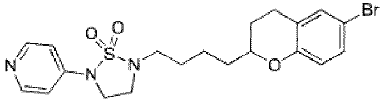

Please refer to the preparation method of Embodiment 18A for this embodiment. ¹H-NMR(CDCl₃,400MHz)δ8.52(d,*J*=6.0Hz,2H),7.14-7.19(m,2H),7.06(d,*J*=6.0Hz,2H),6.65-6.69(m,1H) ,3.97(s.,1H),3.86(t,*J*=6.3Hz,2H),3.56(t,*J*=6.5Hz,2H),3.19(t,*J*=7.0Hz,2H),2.67-2.89(m,2H),1.94-2.04(m ,1H),1.53-1.84(m,7H). LCMS(ESI)m/z:466(M+1).

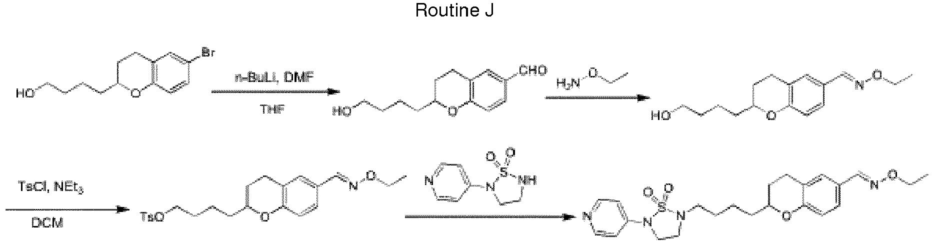

### Embodiment 21

### (E)-2-(4-(1,1-dioxo-5-(pyridin-4-yl)-1,2,5-thiadiazolidine-2-yl)-butyl)-chroman-6-formaldehyde-O-ethyloxime

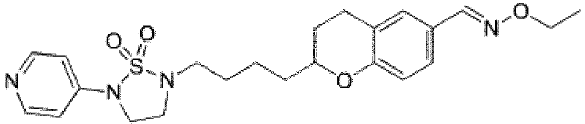

### Embodiment 21A

### 2-(4-hydroxybutyl)-chroman-6-carbaldehyde

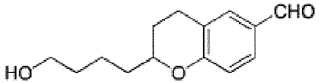

Under the protection of N₂ gas at -78°C, add slowly N-butyllithium (2.5mol/L, 1.8ml, 4.6mmol) into the solution of Embodiment 20C (600mg, 2.1mmol) in anhydrous THF (10.0ml). After the substances are added, continue to agitate 2 hours. Then lower down the temperature of the reaction solution again to -78°C and then drop slowly N, N- dimethylformamide (380mg, 5.2mmol). After the dropping is completed, heat the reaction solution slowly to room temperature and agitate overnight. Add water (5ml) to quench the reaction. Then extract the aqueous phase with ethyl acetate (10.0ml x 2). Dry the combined organic phases with anhydrous Na₂SO₄ before filter and evaporate. Then purify the residue with TLC to obtain the title compound (colorless liquid, 70.0mg, yield of 15%). ¹H-NMR(CDCl₃,400MHz)δ9.83(s,1H),7.55-7.68(m,2H),6.83-6.92(m,1 H),4.03-4.20(m,1 H),3.71(t,J=6. 0Hz,2H),2.72-2.92(m,2H),2.00-2.13(m,1H),1.46-1.87(m,7H).

### Embodiment 21B

### (E)-2-(4-hydroxybutyl)-chroman-6-carbaldehyde-O-ethyloxime

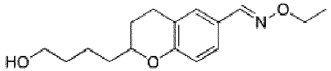

Add ethoxyamino hydrochloride (97mg, 1.6mmol) and sodium acetate (130mg, 1.59mmol) into the solution of Embodiment 21A (70mg, 0.32mmol) in water (2.0ml). Then heat it 3 hours at 60°C before stop the reaction. Extract the aqueous phase with ethyl acetate (5.0ml x 3). Dry the combined organic phases with anhydrous Na₂SO₄. Then filter and concentrate to obtain the crude product that can be directly used further (70mg, yield of 79%).

### Embodiment 21C

### (E)-4-(6-((ethoxyiminomethyl)-methyl)-chroman-2-yl)-butyl-4-methylbenzenesulfonate

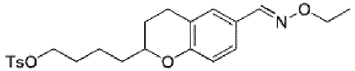

Add triethylamine (76ml, 0.75mmol) and p-toluensulfonyl chloride (95mg, 0.5mmol) into the solution of Embodiment 21B (70mg, 0.25mmol) in dichloromethane (5.0ml). Agitate the reaction mixture 4 hours at 12°C. After concentrate it under reduced pressure, purify the crude product with TLC to obtain the title compound (colorless liquid, 60mg, yield of 55%). ¹H-NMR(CDCl₃,400MHz)δ7.98(s,1H),7.79(d,*J*=8.0Hz,2H),7.24-7.39(m,2H),6.69-6.77(m,1H),4.12-4.2 3(m,2H),4.06(t,*J*=6.3Hz,2H),3.92(br
s,1H),2.68-2.87(m,2H),2.43(s,3H),1.89-1.99(m,1 H),1.43-1.78(m,7H),1.26-1.37(m,3H).

### Embodiment 21D

### (E)-2-(4-(1,1-dioxo-5-(pyridin-4-yl)-1,2,5-thiadiazolidine-2-yl)-butyl)-chroman-6-carbaidehyde-O-ethyioxime

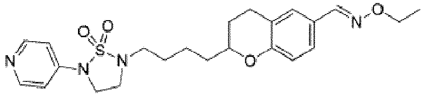

Please refer to the preparation method of Embodiment 18A for this embodiment.
1H-NMR(CDCl3,400MHz)8.52(brs,2H),7.98(s,1H),7.21-7.35(m,2H),7.07(d,J=5.0Hz,2H),6.77(d,J=9.0 Hz,1H),4.15-4.28(m,2H),4.02(brs,1H),3.86(t,J=6.5Hz,2H),3.52-3.61(m,2H),3.19(t,J=7.0Hz,2H),2.72-2.90(m,2H),1.95-2.04(m,1H),1.57-1.84(m,8H),1.31(t,J=7.0Hz,3H). LCMS(ESI)m/z:459(M+1).

### Embodiment 22

### (R)-1-(4-(6-(4-chlorophenyl)-chroman-2-yl)-butyl)-3-(pyridin-4-yl)-imidazolidinone

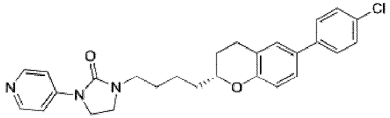

### (S)-1-(4-(6-(4-chlorophenyl)-chroman-2-yl)-butyl)-3-(pyridin-4-yl)-imidazolidinone

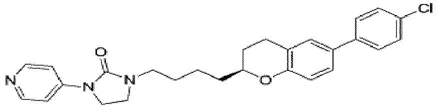

Use SFC to split the products of Embodiment 2 to obtain two chiral isomers. The conditions used to split are as follows:
Method: AS-H_S_5_40_3ML_8MIN_15CM
Chromatographic column: Chiralpak AS-H 150*4.6mm I.D.,5um
Mobile phase: 40% ethanol (0.05%DEA)-CO2
Flow rate: 3mL/min
Wavelength: 220nm;

Embodiment 22a as the first isomer has a retention time of 2.80 minutes; Embodiment 22b as the second isomer has a retention time of 4.04 minutes.

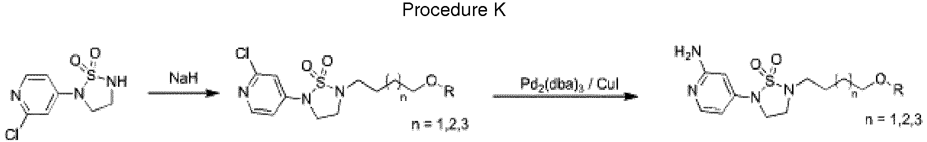

### Embodiment 23

### (E)-4-((5(5(2-amino-pyridin-4-yl)-1,1-dioxide-1,2,5-thiadiazolidine-2-yl)-pentyl)-oxy)-benzaldehyde-O-ethyl ketoxime

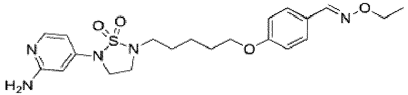

### Embodiment 23A

### (E)-4-hydroxybenzaldehyde-O-ethyloxime

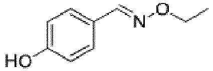

Add sodium acetate (3.4g, 40.9mmol) into the solution of p-hydroxy benzaldehyde (2.5g, 20.5mmol) and ethoxyamino hydrochloride (3.9g, 40.9mmol) in water (50ml) and then agitate the mixture 4 hours at 80°C. Add ethyl acetate (50ml) into the reaction system. Extract the aqueous layer with ethyl acetate (50ml x 3) and then dry the combined organic layer with Na₂SO₄ before filter and evaporate to obtain the title compound (brown solid, 3g, yield of 89%). ¹H NMR(CDCl₃,400MHz)8.03(s,1H),7.46(d,*J*=8.5Hz,2H),6.78-6.85
(m,2H),4.20(q,*J*=7.0Hz,2H),1.31(t,*J*=7.0Hz,3H).

### Embodiment 23B

### (E)-4-((6-bromohexyl)-oxy)-benzaldehyde-O-ethylketoxime

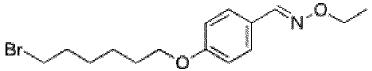

Heat and reflux the solution of Embodiment 23A (1.0g, 6.1mmol), 1,5-dibromopentane (1.4g, 6.1mmol), K₂CO₃ (1.67g, 12.1mmol) and KI (0.1g, 0.61mmol) in acetone (100ml) for 10 hours. After the reaction solution cools to room temperature, filter and then dry the filtrate through rotation. Then purify it with column chromatography to obtain the title compound (white crystal, 1.2g, 63%). ¹H NMR(CDCl₃,400MHz)δ8.01-8.05(m,1H),7.49-7.55(m,2H),6.86-6.91(m,2H),4.17-4.24(m,2H),3.96-4.0 1(m,2H),3.41-3.47(m,2H),1.89-1.99(m,2H),1.78-1.86(m,2H),1.60-1.68(m,2H),1.29-1.34(m,3H).

### Embodiment 23C

### (E)-4-((5-(5-(2-chloropyridin-4-yl)-1,1-dioxide-1,2,5-thiadiazolidine-2-yl)-pentyl)-oxy)-benzaldehyde-O-ethyl ketoxime

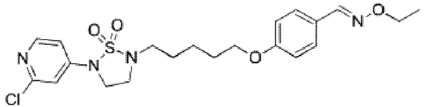

Under the protection of N₂ gas at 10°C, add the sodium hydroxide (51.4mg, 1.3mmol) into the solution of Embodiment 4E (100mg, 428µmol) in N, N- dimethylformamide (3ml). After agitate the reaction solution 2 hours, add the solution of Embodiment 23B (134.5mg, 428µmol) in N, N-dimethylformamide (1ml) into it. Then agitate the system to react 8 hours at 40°C. After the TLC verifies that reaction is completed, add water (10ml) to quench the reaction. Then extract the aqueous layer with ethyl acetate (20ml x 3). Then combine the organic layers and dry anhydrous Na₂SO₄ before filter and evaporate. Then separate the crude product with TLC plate (petroleum ether / ethyl acetate = 1:1) to obtain the title compound (white solid, 140mg, yield of 70%). ¹H NMR(400MHz,CHLOROFORM-d)8.29(d,*J*=5.77Hz,1H),8.05(s,1H),7.53(d,*J*=9.03Hz,2H),7.09(dd,*J*=2 .01,5.77Hz,1H),7.01(d,*J*=2.01Hz,1H),6.89(d,*J*=8.78Hz,2H),4.22(q,*J*=7.03Hz,2H),4.02(t,*J*=6.15Hz,2H ),3.86(t,*J*=6.40Hz,2H),3.54-3.60(m,2H),3.20(t,*J*=7.28Hz,2H),1.60-1.91(m,6H),1.34(t,*J*=7.15Hz,3H). LCMS(M+1):467.

### Embodiment 23D

### (E)-4-((5-(5-(2-aminopyridin-4-yl)-1,1-dioxide-1,2,5-thiadiazolidine-2-yl)-pentyl)-oxy)-benzaldehyde-O-ethyl ketoxime

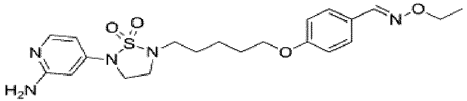

Under the protection of N₂ gas, add Pd₂(dba)₃ (30mg, 343µmol) and Xant-Phos (15mg, 38µmol) into the mixture solution of Embodiment 23C (80mg, 171.3µmol), tert-butyl carbamate (120mg, 1.0mmol) and Cs₂CO₃(112mg, 343µmol) in dioxane / N, N-dimethylformamide (3ml) / (1ml). After agitate the mixture solution evenly, heat it to 110°C to react 10 hours. After the TLC (petroleum ether / ethyl acetate = 2:1 ∼ dichloromethane: methanol = 20:1), verify that the reaction is complete, add water (10ml) to quench. Then extract it with ethyl acetate (30ml x 3). Then combine the organic layers and dry with Na₂SO₄ before filter and evaporate. Separate the crude product with the preparative HPLC (trifluoroacetate) to obtain the title compound (white solid, 20mg, yield of 26%). ¹H NMR(400MHz,CHLOROFORM-d)7.95-8.07(m,2H),7.51(d,*J*=8.53Hz,2H),6.88(d,*J*=8.53Hz,2H),6.42(d d,*J*=2.01,5.52Hz,1H),6.29
(d,*J*=2.01Hz,1H),4.52(br.s.,1H),4.20(q,*J*=7.19Hz,2H),4.00(t,*J*=6.27Hz,2H),3.80(t,*J*=6.53Hz,2H),3.50(t ,*J*=6.27Hz,2H),3.16(t,*J*=7.28Hz,2H),1.73-1.90(m,4H),1.62-1.65(m,2H),1.32(t,*J*=7.03Hz,3H). LCMS(M+1):448.

### Embodiment 24

### 4-((5-(5-(2-aminopyridin-4-yl)-1,1-dioxide-1,2,5-thiadiazolidine-2-yl)-pentyl)-oxy)-benzaldehyde

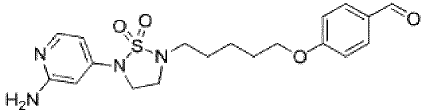

Agitate the reaction solution of Embodiment 23 (30mg, 67µmol) and trifluoroacetate (0.5ml) in water (0.5ml) and methanol (0.5ml) 16 hours at 40°C. Dry it through rotation and then separate the residue with the preparative HPLC to obtain the title compound (2mg, yield of 7%). ¹H NMR(CDCl₃,400MHz):7.75(d,*J*=8.5Hz,2H),7.68(s,1H),6.96(d,*J*=8.8Hz,2H),4.45(q,*J*=7.4Hz,2H),3.84( s,3H),1.59(d,*J*=3.8Hz,3H). LCMS(ESI)m/z:405(M+1).

### Embodiment 25

### (E)-4-((6-(5-(2-aminopyridin-4-yl)-1,1-dioxide-1,2,5-thiadiazolidine-2-yl)-hexyl)-oxy)-benzaldehyde-O-ethyl ketoxime

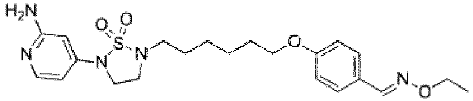

### Embodiment 25A

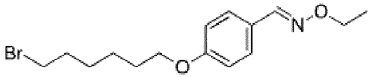

### (E)-4-((6-bromohexyl)-oxy)-benzaldehyde-O-ethyl ketoxime

Please refer to the preparation method of Embodiment 23B for this embodiment. ¹H NMR(400MHz,CHLOROFORM-d)8.04(d,*J*=4.52Hz,1H),7.51(d,*J*=4.52Hz,2H),6.88(d,*J*=5.77Hz,2H),4. 15-4.29(m,2H),3.99(d,*J*=5.77Hz,2H),3.37-3.50(m,2H),1.74-2.00(m,4H),1.52(br.s.,4H), 1.23-1.37(m,3 H).

### Embodiment 25B

### 4-((6-(5-(2-chloropyridin-4-yl)-1,1-dioxide-1,2,5-thiadiazolidine-2-yl)-hexyl)-oxy)-benzaldehyde-O-ethyl ketoxime

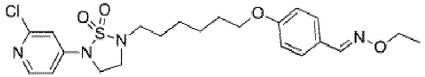

Please refer to the preparation method of Embodiment 23C for this embodiment. ¹H NMR(400MHz,CHLOROFORM-d)8.28(d,*J*=6.02Hz,1H),8.04(s,1H),7.52(d,*J*=8.78Hz,2H),7.07(dd,*J*=2 .13,5.90Hz,1H),7.01(d,*J*=2.01Hz,1H),6.89(d,*J*=8.78Hz,2H),4.21(q,*J*=7.03Hz,2H),4.00(t,*J*=6.27Hz,2H ),3.84(t,*J*=6.40Hz,2H),3.55(t,*J*=6.27Hz,2H),3.17(t,*J*=7.28Hz,2H),1.79-1.87(m,2H),1.70-1.78(m,2H),1. 49-1.58(m,4H),1.33(t,*J*=7.03Hz,4H). LCMS(ESI)m/z:480(M+1).

### Embodiment 25C

### (E)-4-((6-(5-(2-aminopyridin-4-yl)-1,1-dioxide-1,2,5-thiadiazolidine-2-yl)-hexyl)-oxy)-benzaldehyde-O-ethyl ketoxime

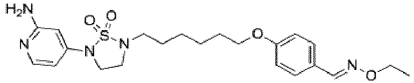

Please refer to the preparation method of Embodiment 23 for this embodiment. ¹H NMR(400MHz,CHLOROFORM-d)8.00-8.06(m,1H),7.93(d,*J*=6.02Hz,1H),7.51(d,*J*=9.03Hz,3H),6.87(d ,*J*=8.53Hz,3H),6.46(d,*J*=4.52Hz,1H),6.28(s,1H),4.12-4.24(m,3H),3.98(t,*J*=6.27Hz,3H),3.79(t,*J*=6.27 Hz,2H),3.49(t,*J*=6.27Hz,2H),3.13(t,*J*=7.03Hz,3H),1.77-1.85(m,2H),1.67-1.76(m,3H),1.51(t,*J*=10.54H z,5H),1.32(t,*J*=7.03Hz,4H). LCMS(ESI)m/z:462(M+1).

### Embodiment 26

### (E)-4-(4-(5-(2-aminopyridin-4-yl)-1,1-dioxide-1,2,5-thiadiazolidine-2-yl)-butyl)-oxy)-benzaldehyde-O-ethyl ketoxime

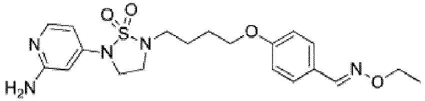

Please refer to the preparation method of Embodiment 25 for this embodiment. ¹H NMR(400MHz,CHLOROFORM-d)8.05(s,1H),7.99(d,*J*=5.77Hz,1H),7.53(d,*J*=8.78Hz,2H),6.90(d,*J*=8. 78Hz,2H),6.43(d,*J*=4.52Hz,1H),6.30(s,1H),4.56(br.s.,1H),4.22(q,*J*=7.03Hz,2H),4.06(t,*J*=5.14Hz,2H), 3.77(t,*J*=6.27Hz,2H),3.51 (t,*J*=6.27Hz,2H),3.22(t,*J*=6.40Hz,2H),1.93(br.s.,5H),1.34(t,*J*=7.15Hz,4H). LCMS(ESI)m/z:434(M+1).

### Embodiment 27

### 4-(5-(5-(4-(2-ethyl-tetrazol-5-yl)-phenoxy)-pentyl)-1,1-dioxide-1,2,5-thiadiazolidine-2-yl)-pyridin-2-amine

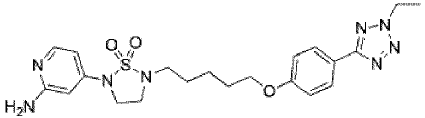

### Embodiment 27A

### 2-(5-(4-(2-ethyl-tetrazol-5-yl)-phenoxy)-pentyl)-5-(pyridin-4-yl)-1,2,5-thiadiazolidine-1,1-dioxide

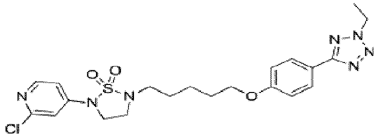

At 0°C, add sodium hydride (20mg, 0.85mmol) into the solution of Embodiment 4E (100mg, 0.43mmol) in N,N-dimethylformamide (3ml). Keep it still 30 minutes at 0°C. Then add the solution of Embodiment 11D (51mg, 0.15mmol) in N,N-dimethylformamide (1ml) into the mixture solution above. Agitate it 6 hours at 15°C. Then add water to quench the reaction and extract the aqueous phase with ethyl acetate. Dry the combined organic phases with anhydrous Na₂SO₄.

Then filter and evaporate it before separate it with column (petroleum ether: ethyl acetate=1:1) to obtain the title compound (white solid, 120mg, yield of 57%). ¹H NMR(CDCl₃400MHz,)δ8.27(d,*J*=6.0Hz,1H),8.07(d,*J*=8.5Hz,2H),7.07(dd,*J*=5.8,2.3Hz,1H),6.99-7.00( m,2H),4.69(q,*J*=7.2Hz,2H),4.05(t,*J*=6.3Hz,2H),3.84(t,*J*=6.3Hz,2H),3.56(t,*J*=6.3Hz,2H),3.19(t,*J*=7.0H z,2H),1.86-1.93(m,2H),1.76-1.83(m,2H),1.64-1.71(m,5H). LCMS(ESI)m/z:492(M+1).

### Embodiment 27B

### 4-(5-(5-(4-(2-tetrazol-5-yl)-phenoxy)-pentyl)-1,1-oxide-1,2,5-thiadiazolidine-2-yl)-pyridin-2-amine

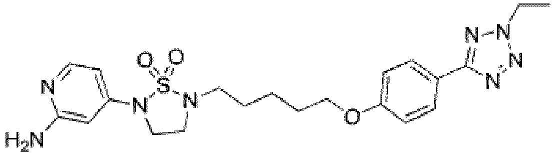

At 0°C, add cesium carbonate (159mg, 0.49mmol) and tert-butyl carbamate (171mg, 1.5mmol) into the solution of Embodiment 27A (120mg, 0.24mmol) in N,N-dimethylformamide (1ml). Under the protection of N₂ gas, add Pd₂(dba)₃ (8.6mg, 20mmol) and Xantphos (11.7mg, 20mmol). Agitate it 15 hours at 110°C. Then add water to quench the reaction and extract the aqueous phase with ethyl acetate. Dry the combined organic phases with anhydrous Na₂SO₄ before filter and evaporate to obtain the title compound (white solid, 15mg, yield of 12%). ¹H NMR(CDCl₃400MHz,)δ8.51(d,*J*=6.5Hz,2H)8.06(d,*J*=8.5Hz,2H)7.05(d,*J*=6.0Hz,2H)6.99(d,*J*=9.0Hz,2 H)4.68(q,*J*=7.5Hz,2H)4.04(t,*J*=6.0Hz,2H)3.85(t,*J*=6.3Hz,2H)3.55(t,*J*=6.3Hz,2H)3.19(t,*J*=7.3Hz,2H)1. 85-1.93(m,2H)1.76-1.83(m,2H)1.68(t,*J*=7.5Hz,5H). LCMS(ESI)m/z:473(M+1).

### Embodiment 28

### 4-(5-(5-((3-ethoxy-1,2-benzoxazol-6-yl)-oxy)-pentyl)-1,1-dioxide-1,2,5-thiadiazolidine-2-yl)-pyridin-2-amine

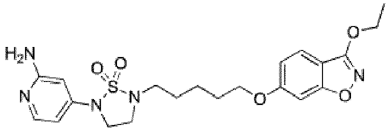

### Embodiment 28A

### 2-(2-chloro-4-pyridyl)-5-(5-((3-ethoxy-1,2-benzoxazol-6-yl)-oxy)-pentyl)-1,2,5-thiadiazolidine-1,1-dioxide

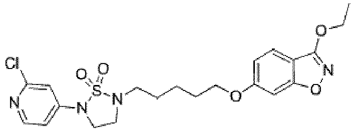

Under the protection of N₂ gas at 0°C, add the sodium hydroxide (53.3mg, 2.2mmol) in batch into the solution of Embodiment 4E (500mg, 1.8mmol) in N,N-dimethylformamide (1ml). After allow it to react 1 hour at 0°C, drop the solution of Embodiment 12E (911mg, 2.8mmol) in N,N-dimethylformamide (1ml) into the system above. Then heat it to room temperature to react 2 hours. Then pour the reaction solution into water (10ml). Then extract with ethyl acetate (10ml x 3). Then dry the combined organic phases with anhydrous Na₂SO₄ before filter. Then remove the solvent with rotary evaporator to obtain the title compound (white solid, 50.00mg, yield of 5.6%). LCMS(ESI)m/z:481(M+1).

### Embodiment 28B

### 4-(5-(5-((3-ethoxy-1,2-benzoxazol-6-yl)-oxy)-pentyl)-1,1-dioxide-1,2,5-thiadiazolidine-2-yl)-pyridin-2-amine

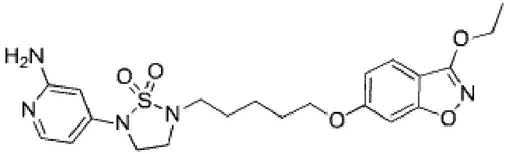

Under the protection of N₂ gas, add cesium carbonate (339mg, 1.0mmol), Pd₂(dba)₃ (47.6mg, 52µmol) and Xantphos (60.2mg, 0.1mmol) into the solution of Embodiment 28A (250mg, 0.5mmol) and tert-butyl carbamate (61mg, 0.5mmol) in dioxane (2ml) and N,N-dimethylformamide (1ml). Then heat the reaction solution to 110°C to react 10 hours. Then pour the reaction solution into water (150ml) and extract with ethyl acetate (100ml x 3). Combine the organic phases and wash it with the saturated salt water (100ml x 2). Then dry it with anhydrous Na₂SO₄ before filter and evaporate. Then purify the residue with the preparative HPLC to obtain the title compound (white solid, 25mg, yield of 10.4%). ¹H NMR(CDCl₃,400MHz)δ7.98(d,*J*=6.02Hz,1H),7.47(d,*J*=9.54Hz,1H),6.81-6.87(m,2H),6.42(dd,*J*=1.76,5 .77Hz,1H),6.30(d,*J*=1.51Hz,1H),4.53(br.s.,1H),4.47(q,*J*=7.03Hz,2H),4.03(t,*J*=6.02Hz,2H),3.81(t,*J*=6. 53Hz,2H),3.51(t,*J*=6.53Hz,2H),3.17(t,*J*=7.28Hz,2H),1.85-1.95(m,2H),1.79(quin,*J*=7.40Hz,2H),1.65-1. 68(m,2H),1.51(t,*J*=7.28Hz,3H). LCMS(ESI)m/z:462(M+1).

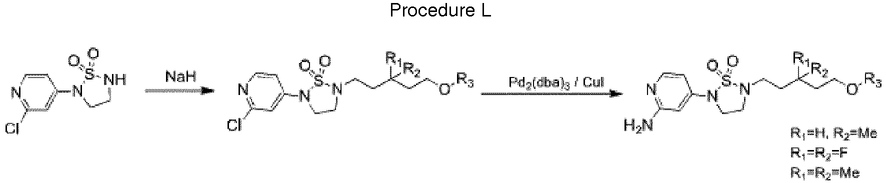

### Embodiment 29

### (S, E)-2-(2-aminopyridin-4-yl)-5-(5-((1-(ethoxy-imino)-2,3-dihydro-1H-inden-5-yl)-oxy)-3-methylpentyl)-1,2,5-thiadiazolidine-1,1-dioxide

### and (R, E)-2-(2-aminopyridin-4-yl)-5- (5 - ((1-(ethoxy-imino)-2,3-dihydro-1H-inden-5-yl)-oxy)-3-methylpentyl)-1,2,5-thiadiazolidine-1,1-dioxide

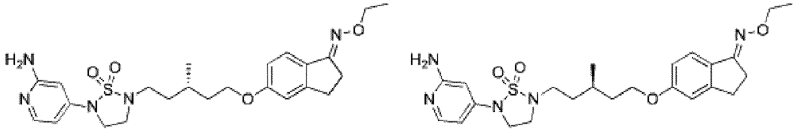

### Embodiment 29A

### (E)-5-hydroxy-2,3-dihydro-1H-inden-1-O-ethyl ketoxime

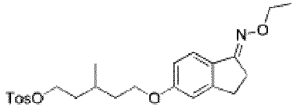

Allow the mixture solution of Embodiment 8B (2.5g, 13.1mmol), 14A (6.1g, 14.4mmol) and K₂CO₃ (3.6g, 26.1 mmol) in acetonitrile (50ml) to react 16 hours at 80°C. Add ethyl acetate (50ml) and water (50ml) into the reaction system. Extract the aqueous layer with ethyl acetate (50ml x 3) and then dry the combined organic layers with Na₂SO₄ before filter and evaporate. Then purify the residue with column chromatography (petroleum ether: ethyl acetate = 20:1∼5:1) to obtain the title compound (colorless solid, 2.5g, yield of 39%).
¹H-NMR(CDCl₃,400MHz)7.79(d,*J*=8.0Hz,2H),7.60(d,*J*=9.0Hz,1H),7.34(d,*J*=8.0Hz,2H),6.74-6.80(m,2 H),4.20(q,*J*=7.0Hz,2H),4.08-4.13(m,2H),3.91-4.01 (m,2H),2.95-3.03(m,2H),2.85-2.93(m,2H),2.44(s,3 H),1.72-1.87(m,3H),1.50-1.61(m,2H),1.33(t,*J*=7.0Hz,3H),0.91(d,*J*=6.5Hz,3H).

### Embodiment 29B

### (E)-2-(2-chloropyridin-4-yl)-5-(5-((1-(ethoxy-imino)-2,3-dihydro-1H-inden-5-yl)-oxy) -3-methylpentyl) -1,2,5-thiadiazolidine-1,1-dioxide

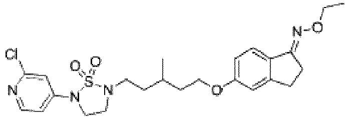

At 0°C, add slowly sodium hydroxide (673mg, 16.8mmol) into the mixture solution of Embodiment 4E (1.3g, 4.8mmol) in N,N-dimethylformamide (40ml). Thirty minutes later, drop the mixture solution of Embodiment 29A (2.5g, 5.6mmol) in N,N-dimethylformamide (40ml). Then allow the mixture to react 12 hours at 25°C. Add water (30ml) into the reaction system. Extract the aqueous layer with dichloromethane (40ml x 3) and then dry the combined organic layers with Na₂SO₄ before filter and evaporate. Then purify the residue with column chromatography (petroleum ether: ethyl acetate = 5:1∼1:1) to obtain the title compound (yellow liquid, 2.1g, yield of 66%).
¹H-NMR(CDCl₃,400MHz)8.25(d,*J*=6.0Hz,1H),7.55-7.61(m,1H),7.04(dd,*J*=5.5,2.0Hz,1H),6.98(d,*J*=2.0 Hz,1H),6.75-6.80(m,2H),4.18(q,*J*=7.0Hz,2H),4.11(q,*J*=7.4Hz,1H),3.97-4.05(m,2H),3.80(t,*J*=6.5Hz,2H ),3.47-3.55(m,2H),3.19(t,*J*=7.3Hz,2H),2.94-2.98(m,2H),2.86-2.90(m,2H),1.78-1.87(m,2H),1.53-1.71( m,2H),1.31 (t,*J*=7.0Hz,3H),1.02(d,*J*=6.5Hz,3H). LCMS(ESI)m/z:507(M+1).

### Embodiment 29C

### (E)-2-(2-amino-pyridin-4-yl)-5-(5-((1-(ethoxy-imino)-2,3-dihydro-1 H-inden-5-yl)-oxy) -3-methylpentyl)-1,2,5-thiadiazolidine-1,1-dioxide

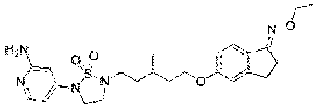

Please refer to the preparation method of Embodiment 28 for this embodiment.
¹H-NMR(CDCl₃,400MHz)7.97(d,*J*=5.8Hz,1H),7.53-7.68(m,1H),6.76-6.80(m,2H),6.41(dd,*J*=5.8,2.0Hz, 1H),6.27(d,*J*=2.0Hz,1H),4.49(br.s.,2H),4.19(q,*J*=7.0Hz,2H),4.03(q,*J*=6.0Hz,2H),3.76(t,*J*=6.4Hz,2H),3 .44-3.50(m,2H),3.18(t,*J*=7.5Hz,2H),2.95-3.00(m,2H),2.86-2.91(m,2H),1.68-1.92(m,5H),1.32(t,*J*=7.0H z,3H),1.03(d,*J*=6.3Hz,3H). LCMS(ESI)m/z:488(M+1).

### Embodiment 29D

### (S, E) -2-(2-amino-pyridin-4-yl) -5-(5-((1-(ethoxy-imino)-2,3-dihydro-1H-inden-5-yl)-oxy)-3-methylpentyl)-1,2,5-thiadiazolidine-1,1-dioxide

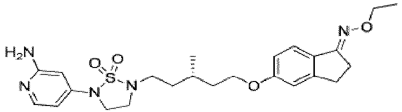

### (R, E) -2-(2-amino-pyridin-4-yl) -5-(5-((1-(ethoxy-imino)-2,3-dihydro-1H-inden-5-yl)-oxy)-3-methylpentyl)-1,2,5-thiadiazolidine-1,1-dioxide

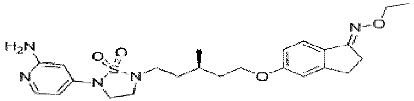

Embodiment 29C undergoes chiral separation (AD-H_3UM_3_60_3ml_5MIN, Column: Chiralpak AD-350^{∗}4.6mm I.D., 3um mobile phase: 60% methanol (0.05%DEA)-CO₂ flow rate: 3ml/M; wavelength: 220nm; peak appearance time: 1.334min, 1.823min) to obtain the title compound. ¹H-NMR(CDCl₃,400MHz)7.97(d,*J*=6.0Hz,1H),7.56-7.63(m,1H),6.79(br.s.,2H),6.41(dd,*J*=6.0,2.0Hz,1H ),6.27(d,*J*=1.8Hz,1H),4.51(br.s.,2H),4.17-4.23(m,2H),3.99-4.08(m,2H),3.76(t,*J*=6.4Hz,2H),3.45-3.51( m,2H),3.18(t,*J*=7.5Hz,2H),2.95-3.00(m,2H),2.86-2.91(m,2H),1.69-1.93(m,5H),1.32(t,*J*=7.0Hz,3H),1.0 3(d,*J*=6.5Hz,3H). LCMS(ESI)m/z:488(M+1).

¹H-NMR(CDCl₃,400MHz)7.97(d,*J*=5.8Hz,1H),7.53-7.68(m,1H),6.76-6.80(m,2H),6.41(dd,*J*=5.8,2.0Hz, 1H),6.27(d,*J*=2.0Hz,1H),4.49(br.s.,2H),4.19(q,*J*=7.0Hz,2H),4.03(q,*J*=6.0Hz,2H),3.76(t,*J*=6.4Hz,2H),3 .44-3.50(m,2H),3.18(t,*J*=7.5Hz,2H),2.95-3.00(m,2H),2.86-2.91(m,2H),1.68-1.92(m,5H),1.32(t,*J*=7.0H z,3H),1.03(d,*J*=6.3Hz,3H). LCMS(ESI)m/z:488(M+1).

### Embodiment 30

### (E)-2-(2-amino-pyridin-4-yl)-5-(5-((1-(ethoxy-imino)-2,3-dihydro-1H-inden-5-yl)-oxy) -3,3-dimethylpentyl)-1,2,5-thiadiazolidine-1,1-dioxide

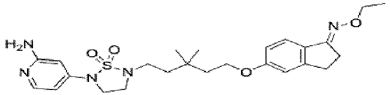

### Embodiment 30A

### 3,3-dimethyl pentane-1,5-diol

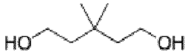

Under the protection of N₂ gas at 0°C, add LiAlH₄ (534mg, 14.1 mmol) into the solution of 4,4-dimethyl glutaric anhydride (500mg, 3.5mmol) in THF (20ml). After the substances are added, agitate the reaction mixture 12 hours at 80°C. Cool the reaction solution to room temperature and then pour it into ice water (50ml) and agitate 10 minutes. Extract the aqueous phase with ethyl acetate (30ml x 3). Then dry the combined organic phases with anhydrous Na₂SO₄ before filter and concentrate to obtain the title compound (yellow liquid, 300mg, yield of 64%). 1H NMR(400MHz,CHLOROFORM-d)3.69(t,*J*=7.15Hz,4H),3.25(br.s.,2H), 1.49-1.63(m,4H),0.89-0.95(m,6 H).

### Embodiment 30B

### 3,3-dimethylpentane-1,5-bis(4-methylbenzenesulfonate)

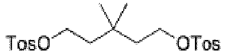

At 0°C, add p-toluensulfonyl chloride (43g, 227mmol) and triethylamine (23g, 227mmol) into the solution of Embodiment 30A (10g, 75.6mmol) in dichloromethane (200ml). After the substances are added, agitate the mixture solution above 12 hours at 25°C. After the raw materials disappear, pour the reaction solution into ice water (300ml) and then agitate 20 minutes. Extract the aqueous phase with dichloromethane (50ml x 4). Wash the combined organic phases with the saturated salt water (50ml) and then dry with anhydrous Na₂SO₄ before filter and concentrate. Then purify the crude product with column chromatography (petroleum ether:
ethyl acetate = 3:1) to obtain the title compound (yellow liquid, 20g, yield of 60%). 1H NMR(400MHz,CHLOROFORM-d)7.77(d,*J*=8.53Hz,2H),7.35(d,*J*=8.03Hz,2H),4.02(t,*J*=7.03Hz,2H),2. 45(s,3H),1.55(t,*J*=7.03Hz,2H),0.84(s,3H).

### Embodiment 30C

### (E)-5-((1-(ethoxy-imino)-2,3-dihydro-1H-inden-5-yl)-oxy)-3,3-dimethylpentyl-4-methly-benzenesulfonic acid

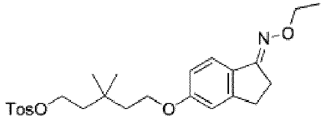

Add K₂CO₃ (627mg, 4.54mmol) into the solution of Embodiment 30C (1.0g, 2.3mmol) and Embodiment 8B (173.6mg, 908µmol) in acetone (20ml). Agitate the reaction solution 12 hours at 70°C and cool it to room temperature before concentrate under reduced pressure. Add water (50ml) into the residue and then extract with ethyl acetate (30ml x 3). Dry the combined organic phases with anhydrous Na₂SO₄. Then filter and concentrate it before purifying it with column (petroleum ether: ethyl acetate=3:1) to obtain the title compound (yellow solid, 300mg, yield of 72%). 1H NMR(400MHz,CHLOROFORM-d)7.77-7.82(m,2H),7.59(d,*J*=8.53Hz,1H),7.33(d,*J*=8.53Hz,2H),6.75(b r.s.,2H),4.11-4.24(m,4H),3.97(t,*J*=6.78Hz,2H),2.81-3.02(m,4H),2.38-2.46(m,4H),1.70(dt,*J*=2.51,7.03 Hz,4H),1.32(t,*J*=7.03Hz,3H),0.96(s,6H).

### Embodiment 30D

### (E)-2-(2-chloropyridin-4-yl)-5-(5-((1-(ethoxy-imino)-2,3-dihydro-1H-inden-5-yl)-oxy) -3,3-dimethylpentyl)-1,2-thiadiazolidine-1,1-dioxide

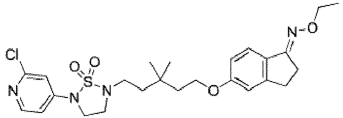

Under the protection of N₂ gas at 0°C, add sodium hydride (52mg, 1.3mmol) into the solution of Embodiment 4E (153mg, 653µmol) in N,N-dimethylformamide (5ml). After the substances are added, agitate the solution at room temperature to react 30 minutes. Add Embodiment 30C (300mg, 653µmol) into the solution above. Then continue to agitate 11 hours at room temperature. Pour the reaction solution into ice water (30ml) and extract the aqueous phase with ethyl acetate (20ml x 3). Dry the combined organic phases with anhydrous Na₂SO₄.
Then filter and concentrate it before purify the crude product with column (petroleum ether: ethyl acetate=3:1) to obtain the title compound (yellow solid, 150mg, yield of 44%). 1H NMR(400MHz,CHLOROFORM-d)8.27(d,*J*=6.02Hz,1H),8.02(s,2H),7.60(d,*J*=9.03Hz,1H),7.06(dd,*J*=2 .26,5.77Hz,1H),6.99(d,*J*=2.01Hz,1H),6.77-6.82(m,2H),4.20(q,*J*=7.03Hz,2H),4.05(t,*J*=6.78Hz,2H),3.8 2(t,*J*=6.27Hz,2H),3.54(t,*J*=6.27Hz,2H),3.18-3.26(m,2H),2.97-3.03(m,2H),1.79(t,*J*=6.53Hz,2H), 1.68-1 .76(m,2H),1.32(t,*J*=7.03Hz,4H),1.06(s,6H). LCMS(ESI)m/z:521(M+1).

### Embodiment 30E

### (E)-2-(2-amino-pyridin-4-yl)-5-(5-((1-(ethoxy-imino)-2,3-dihydro-1H-inden-5-yl)-oxy)-3,3-dimethylpentyl) -1,2-thiadiazolidine-1,1-dioxide

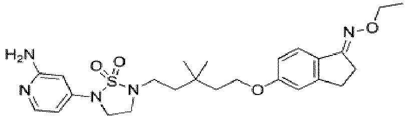

Please refer to the preparation method of Embodiment 29 for this embodiment. ¹H NMR(400MHz,CHLOROFORM-d)7.82(br.s.,1H),7.61(d,*J*=8.53Hz,1H),6.81(br.s.,2H),6.58(d,*J*=4.02H z,1H),6.36(br.s.,1H),4.21(d,*J*=6.78Hz,2H),4.06(br.s.,2H),3.84(br.s.,2H),3.53(br.s.,2H),3.21(br.s.,2H),2 .74-3.07(m,4H),1.58-1.85(m,4H),1.33(t,*J*=6.53Hz,4H),1.06(br.s.,6H). LCMS(ESI)m/z:502(M+1).

### Embodiment 31

### (E)-2- (2-amino-pyridin-4-yl) -5- (5- ((1- (ethoxy-imino)-2,3-dihydro-1H-inden-5-yl)-oxy) -3,3-difluoropentyl)-1,2,5-thiadiazolidine-1,1-dioxide

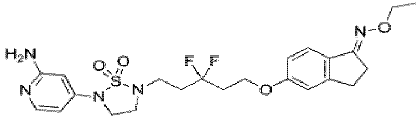

### Embodiment 31A

### Diethyl-3,3-difluoro-glutarate

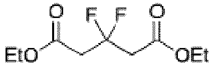

At -65°C, add slowly the solution of diethyl- 2,2 '- (1,3-dithiolane-2,2-diyl)diacetate (501 mg, 1.8mmol) in dichloromethane into the solution of dibromohydantoin (2.16g, 7.5mmol) and pyridine hydrofluoride solution (2.2ml, 1.8mmol) in dichloromethane (15ml). Continue to agitate the mixture solution 5 hours at -65°C before heat to 25°C to agitate 3 hours. Add water (10ml) into the reaction system. Adjust the pH value of the system with Na₂CO₃ aqueous solution to 3-4. Then extract the aqueous layer with dichloromethane (20ml x 3) before filter and dry through rotation. Then purify the residue with column chromatography (petroleum ether: ethyl acetate = 10:1) to obtain the title compound (white solid, 210mg, yield of 38%) and the title compound (yellow liquid, 300mg, yield of 74%). ¹H NMR(CDCl₃,400MHz)δ4.18(q,*J*=7.0Hz,4H),3.24(t,*J*=15.1Hz,4H),1.26-1.30(m,6H).

### Embodiment 31B

### 3,3-difluoro pentane-1,5-diol

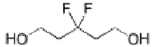

At 0°C, drop Embodiment 31A (300mg, 1.34mmol) into the solution of LiAlH₄ (102mg, 2.68mmol) in THF (3ml). After it is added, agitate the mixture solution 3 hours at 25°C. Add water (0.5ml) and 10% NaOH solution (0.5ml) into the reaction system. Dry the system with Na₂SO₄ before filter and evaporate to obtain the title compound (160mg, yield of 72%). ¹H NMR(CDCl₃,400MHz):3.77-3.90(m,4H),2.21(tt,*J*=16.8,5.8Hz,4H).

### Embodiment 31C

### 1,5-dibromo-3,3-difluoro-pentane

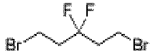

At 0°C, drop CBr4 (1.5g, 4.6mmol) into the solution of Embodiment 31B (160mg, 1.1mmol), triphenyl phosphine (1.79g, 6.8mmol) in THF (5ml). After the substances are added, agitate the mixture solution 3 hours at 60°C. Filter the solution before wash the filter cake with ethyl acetate (300ml x 3). Then dry the filter through rotation and purify the residue with column chromatography (petroleum ether: ethyl estate = 10:1) to obtain the title compound (200mg, yield of 53%). ¹H NMR(CDCl₃,400MHz):3.45-3.51(m,4H),2.48(tt,*J*=16.2,7.9Hz,4H).

### Embodiment 31D

### (E)-5-((5-bromo-3,3-difluoro-pentyl)-oxy)-2,3-dihydro-1H-inden-1-one-O-ethyl ketoxime

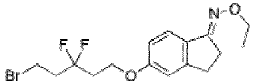

Please refer to the preparation method of Embodiment 30C for this embodiment. ¹H NMR(CDCl₃),400MHz):7.56-7.62(m,1H),6.76-6.83(m,2H),4.19(q,*J*=6.9Hz,2H),4.01(t,*J*=6.0Hz,2H),3.4 3-3.52(m,2H),2.95-3.02(m,2H),2.84-2.92(m,2H),2.03-2.12(m,2H), 1.91-2.00(m,2H), 1.32(t,*J*=7.0Hz,3H ).

### Embodiment 31E

### (E)-2-(2-chloropyridin-4-yl)-5-(5-((1-(ethoxy-imino)-2,3-dihydro-1H-inden-5-yl)-oxy)-3,3-difluoro-pentyl)-1,2-thiadiazolidine-1,1 -dioxide

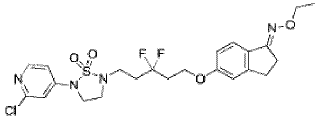

Please refer to the preparation method of Embodiment 30D for this embodiment. ¹H NMR(CDCl₃,400MHz):8.26(d,*J*=5.8Hz,1H),7.60(d,*J*=9.3Hz,1H),7.04(dd,*J*=5.8,2.3Hz,1H),6.98(d,*J*=2. 0Hz,1H),6.73-6.86(m,3H),4.19(d,*J*=7.0Hz,2H),4.00-4.05(m,2H),3.76-3.84(m,2H),3.55(t,*J*=6.4Hz,2H), 3.23(t,*J*=6.1Hz,2H),2.96(d,*J*=7.3Hz,2H),2.86-2.90(m,2H),1.86-1.96(m,4H),1.32(s,3H). LCMS(ESI)m/z:529(M+1).

### Embodiment 31F

### (E)-2-(2-amino-pyridin-4-yl)-5-(5-((1-(ethoxy-imino)-2,3-dihydro-1H-inden-5-yl)-oxy) -3,3-difluoropentyl)-1,2,5-thiadiazolidine-1,1-dioxide

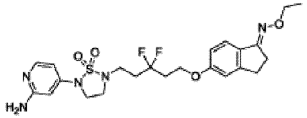

Please refer to the preparation method of Embodiment 29 for this embodiment. ¹H NMR(COCl₃,400MHz):7.96(d,*J*=5.8Hz,1H),7.51-7.64(m,1H),6.71-6.84(m,2H),6.40(dd,*J*=5.8,1.8Hz,1 H),6.24(d,*J*=1.5Hz,1H),4.55(br.s.,2H),4.19(q,*J*=7.0Hz,2H),4.04(t,*J*=5.4Hz,2H),3.72(t,*J*=6.4Hz,2H),3.4 2-3.55(m,2H),3.20(t,*J*=6.5Hz,2H),2.92-3.00(m,2H),2.83-2.92(m,2H),1.86-1.95(m,4H),1.32(t,*J*=7.0Hz, 3H). LCMS(ESI)m/z:510(M+1).

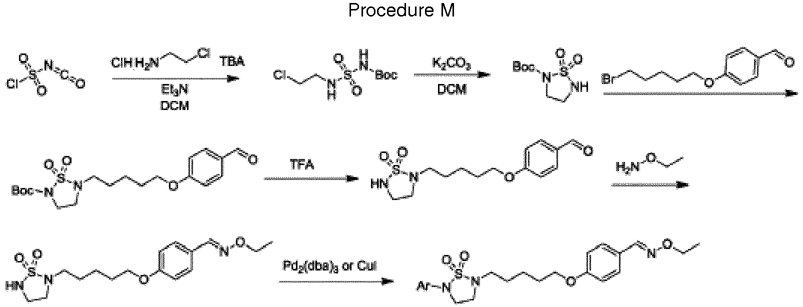

### Embodiment 32

### (E)-4 - ((5-(5-(2-methylpyridin-4-yl)-1,1-dioxide-1,2,5-thiadiazol-2-yl)-pentyl)-oxy)-benzaldehyde-O-ethyl ketoxime

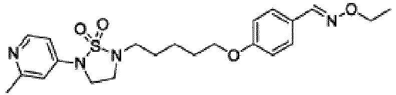

### Embodiment 32A

### N-t-butyl-N-(2-chloroethyl)-sulfonylurea

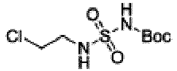

Under the protection of N₂ gas at 0°C, drop the solution of N-(oxymethylene)-sulfamoyl chloride (244g, 1.73mol) in dichloromethane (1.0L) into t-butanol (453g, 2.1mol) in 1 hour. After the substances are added, agitate the mixture solution 1 hour at 25°C. Add the mixture of this mixture solution and triethylamine (872g, 8.6mol) into the solution of 2-chloro-ethylamine (200g, 1.73mol) in chloromethane (2L) in 2 hours. Agitate the mixture solution 10 hours at 25°C before filter. Then concentrate the filtrate before dissolve it in 2L water. Then adjust it with 4N salt to pH = 5-6. A large amount of solids will precipitate out. Then filter the solids and wash them with petroleum ether (1L) to obtain the title compound (white solid, 350g, yield of 78.5%) that can be directly used without necessity to purify further.

### Embodiment 32B

### t-butyl-1,2,5-thiadiazolidine-2-carboxylate-1,1-dioxide

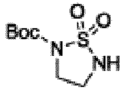

At room temperature, add the mixture solution of K₂CO₃ (280g, 2.03mol) into the solution of Embodiment 32A (350g, 1.35mol) in dimethylsulfoxide (3L) and agitate 10 hours at room temperature before filter. Pour the filtrate into 5L water and adjust it with 4N hydrochloric acid to pH = 5-6. Then filter the solids and wash the solids with petroleum ether (1L). Then recrystallize the solids with (petroleum ether / ethyl acetate = 1 / 1) (1L) to obtain the title compound (white solid, 250g, yield of 84%).

### Embodiment 32C

### 4-((5-bromophenyl)-pentyl)-benzaldehyde

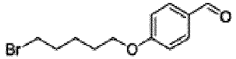

Please refer to the preparation method of Embodiment 4G for this embodiment. ¹H NMR(400MHz,CHLOROFORM-d)9.81-9.89(m,1H),7.81(d,*J*=8.80Hz,2H),6.97-7.02(m,2H),4.04(t,*J*=6. 40Hz,2H),3.39-3.46(m,2H),1.94(t,*J*=7.60Hz,2H),1.84(t,*J*=7.20Hz,2H),1.61-1.65(m,2H).

### Embodiment 32D

### tert-butyl-5-(5-(4-formyl-phenoxy)-pentyl)-1,2,5-thiadiazolidine-2-carboxylate-1,1-dioxide

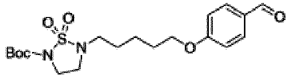

Under the protection of N₂ gas at 0°C, add sodium hydroxide (81mg, 2.0mmol) into the solution of Embodiment 32C (550mg, 2.5mmol) in N,N-dimethylformamide (8ml). After agitate it 0.5 hour at 0°C, drop the solution of 4-((5-bromophenyl)-pentyl)-benzaldehydein (671mg, 2.5mmol) in N,N-dimethylformamide (19ml) into it. Agitate it 0.5 hour at 0°C and then the TLC verifies the reaction ends. Add water (10ml) to quench the reaction. Then extract the aqueous layer with ethyl acetate (10ml x 3). Then combine the organic layers and dry with anhydrous Na₂SO₄ before filter and evaporate. Then separate the crude product with TLC plate (petroleum ether / ethyl acetate = 3:1) to obtain the title compound (white solid, 400mg, yield of 60%). ¹H NMR(400MHz,CHLOROFORM-d)9.88(s,1H),7.82(d,*J*=8.53Hz,2H),6.98(d,*J*=8.53Hz,2H),4.05(t,*J*=6.2 7Hz,2H),3.79(t,*J*=6.53Hz,2H),3.32(t,*J*=6.27Hz,2H),3.08(t,*J*=7.28Hz,2H),1.82-1.91(m,2H),1.72-1.79( m,2H),1.57-1.64(m,2H),1.54(s,10H).

### Embodiment 32E

### 4-((5-(1,1-dioxide-1,2,5-thiadiazolidine-2-yl)-pentyl)-oxy)-benzaldehyde

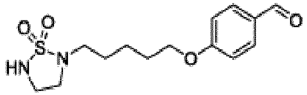

At 0 °C, add trifluoroacetate (5ml) into the solution of Embodiment 32D (4g, 9.7mmol) in dichloromethane (40ml) and agitate it 4 hours at 15°C. After TLC verifies the reaction ends, adjust it with the saturated NaHCO₃ solution to pH = 7 at 0 °C and extract it with dichloromethane (30ml x 2). Then combine the organic layers with Na₂SO₄ before filter and evaporate to obtain the crude product of the title compound (colorless oily form, 2.66g, yield of 88%) that can be used directly further.

### Embodiment 32F

### (E)-4-((5-(1,1-dioxide-1,2,5-thiadiazolidine-2-yl)-pentyl)-oxy)-benzaldehyde-O-ethyl ketoxime

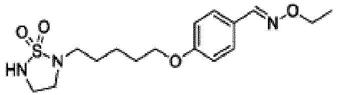

Under the protection of N₂ gas, add ethoxyamino hydrochloride (1.56g, 16mmol) and sodium acetate (1.31g, 16mmol) into the solution of Embodiment 32E (1.0g, 3.2mmol) in ethanol (50ml) and heat it to 60°C to react 1 hour. After TLC verifies that the reaction ends, concentrate and evaporate the solvent before add water (20ml). Then extract it with ethyl acetate (20ml x 3). Then combine the organic layers and dry it with Na₂SO₄ before filter and evaporate. Purify the crude product with column chromatogratphy (petroleum ether: ethyl acetate = 2:1) to obtain the title compound (colorless oily form, 1.1g, yield of 97%) that can be directly used further.

### Embodiment 32G

### (E)-4-((5-(5-2-methylpyridin-4-yl)-1,1-dioxide-1,2,5-thiadiazolidine-2-yl)-pentyl)-oxy)-benzaidehyde-O-ethyl ketoxime

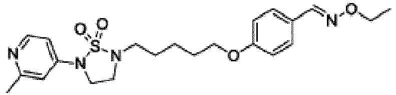

Under the protection of N₂ gas, heat the solution of Embodiment 32F (130mg, 366µmol), 4-bromo-2-methylpyridine (189mg, 1.1 mmol), Cs₂CO₃ (179mg, 549 µmol), Pd₂(dba)₃ (34mg, 37µmol) and Xant-phos (21mg, 37µmol) in dioxane (2ml) to 100°C for 6 hours. TLC indicates that the raw materials are consumed completely. Cool the reaction solution to room temperature. Then add ethyl acetate (20ml). Then wash the organic phase with saturated salt water (30ml) and dry the organic phase with Na₂SO₄. Then filter and concentrate before purify it with the preparative TLC (dichloromethane: methanol = 30:1) to obtain the title compound (white solid, 100mg, yield of 61%). ¹H
NMR(400MHz,CHLOROFORM-d)8.39(d,*J*=5.52Hz,1H),8.02(s,1H),7.51(d,*J*=8.53Hz,2H),6.95(s,1H), 6.87(d,*J*=8.53Hz,3H),4.20(q,*J*=7.36Hz,2H),4.00(t,*J*=6.27Hz,2H),3.84(t,*J*=6.27Hz,2H),3.53(t,*J*=6.27H z,2H),3.18(t,*J*=7.28Hz,2H),2.56(s,3H),1.82-1.90(m,2H),1.74-1.81 (m,2H),1.62(br.s.,2H),1.25-1.36(m,3 H). LCMS(ESI)m/z:447(M+1).

### Embodiment 33

### (E)-4-((5-(5-(6-methyl-pyridazin-3-yl)-1,1-dioxide-1,2,5-thiadiazolidine-2-yl)-pentyl)-oxy)-benzaldehyde-O-ethy I ketoxime

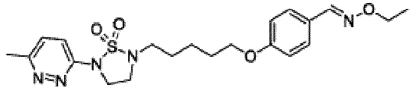

Please refer to the preparation method of Embodiment 32 for this embodiment. ¹H NMR(400MHz,CHLOROFORM-d)8.02(s,1H),7.61(d,*J*=9.03Hz,1H),7.51(d,*J*=8.53Hz,2H),7.32(d,*J*=9. 54Hz,1H),6.87(d,*J*=8.53Hz,2H),4.16-4.30(m,4H),4.00(t,*J*=6.27Hz,2H),3.56(t,*J*=6.27Hz,2H),3.19(t,*J*= 7.28Hz,2H),2.66(s,3H),1.75-1.91 (m,4H),1.60-1.66(m,2H),1.32(t,J=7.03Hz,3H). LCMS(ESI)m/z:447(M+1).

### Embodiment 34

### (E)-4-((5-(5-(6-chloro-pyridazin-3-yl)-1,1-dioxide-1,2,5-thiadiazoiidine-2-yl)-pentyl)-oxy)-benzaldehyde-O-ethyl ketoxime

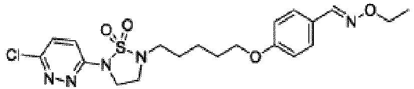

Please refer to the preparation method of Embodiment 32 for this embodiment. 1H NMR(400MHz,CHLOROFORM-d)d
8.02(s,1H),7.68(d,*J*=9.03Hz,1H),7.45-7.54(m,1H),6.87(d,*J*=8.53Hz,1H),4.15-4.29(m,5H),4.00(t,*J*=6.0 2Hz,2H),3.57(t,*J*=6.53Hz,2H),3.20(t,*J*=7.28Hz,2H),1.75-1.91 (m,4H),1.60-1.67(m,2H),1.32(t,*J*=7.03H z,3H). LCMS(ESI)m/z:468(M+1).

### Embodiment 35

### (E)-4-((5-(1,1-dioxide-5-(thiazol-2-yl)-1,2,5-thiadiazolidine-2-yl)-pentyl)-oxy)-benzaldehyde-O-ethyl ketoxime

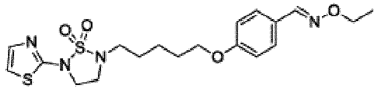

Please refer to the preparation method of Embodiment 32 for this embodiment. 1H NMR(400MHz,CHLOROFORM-d)d
8.02(s,1H),7.51(d,*J*=8.53Hz,2H),7.43(d,*J*=3.51Hz,1H),6.98(d,*J*=3.51Hz,1H),6.87(d,*J*=9.03Hz,2H),4.2 0(q,*J*=7.03Hz,2H),4.12(t,*J*=6.53Hz,2H),4.00(t,*J*=6.27Hz,2H),3.56(t,*J*=6.53Hz,2H),3.19(t,*J*=7.28Hz,2H ),1.82-1.89(m,2H),1.74-1.80(m,2H),1.58-1.65(m,2H),1.32(t,*J*=7.03Hz,3H). LCMS(ESI)m/z:439(M+1).

### Embodiment 36

### (E)-N-(4-(5-(5-(4-((ethoxy--imino)-methyl)-phenoxy)-pentyl)-1,1-dioxide-1,2,5-thiadiazolidine-2-yl)-pyridin-2-yl) -acetamide

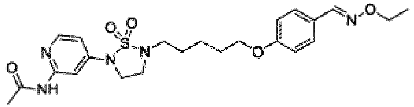

### Embodiment 36A

### N-(4-bromo-2-yl)-acetam ide

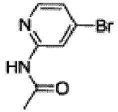

Under the protection of N₂ gas at 0°C, add acetyl chloride (454mg, 5.8mmol) into the solution of 4-bromo-2-aminopyridine (1.0g, 5.8mmol) and triethylamine (1.75g, 17mmol) in dichloromethane (50ml). Allow the solution to react 1 hour at room temperature. Then pour it into ice water (50ml) to quench before extract with dichloromethane (20ml). Combine the organic layers and dry it with anhydrous Na₂SO₄ before filter and evaporate. Separate and purify the crude product with column chromatography (petroleum ether: ethyl acetate = 5 :1) to obtain the title compound (white solid, 400mg, 33%). ¹H NMR(400MHz,CHLOROFORM-d)8.38-8.53(m,1H),8.08(d,J=5.02Hz,1H),7.93(br.s.,1H),7.21(d,J=4.5 2Hz,1H),2.22(s,3H).

### Embodiment 36B

### (E)-N-(4-(5-(5-(4-((ethoxy-imino)-methyl)-phenoxy)-pentyl)-1,1-dioxide-1,2,5-thiadiazolidine-2-yl)-pyridin-2-yl)-acetamide

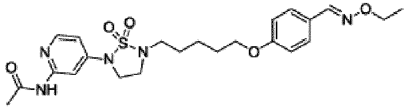

Please refer to the preparation method of Embodiment 32 for this embodiment. ¹H NMR(400MHz,CHLOROFORM-d)8.13(d,*J*=5.52Hz,1H),8.02(s,1H),7.92(br.s.,1H),7.79(s,1H),7.51(d,*J* =8.53Hz,2H),7.17(dd,*J*=2.01,6.02Hz,1H),6.87(d,*J*=8.53Hz,2H),4.13-4.28(m,2H),4.00(t,*J*=6.27Hz,2H) ,3.90(t,*J*=6.53Hz,2H),3.53(t,*J*=6.53Hz,2H),3.18(t,*J*=7.28Hz,2H),2.21(s,3H),1.73-1.92(m,4H),1.60-1.6 5(m,2H),1.28-1.40(m,3H). LCMS(ESI)m/z:490(M+1).

### Embodiment 37

### (E)-ethyl -4-(5-(5-(4-((ethoxycarbonyl)-methyl)-phenoxy)-pentyl)-dioxide-1,2,5-thiadiazolidine-2-yl)-pyridin-2-carboxylate

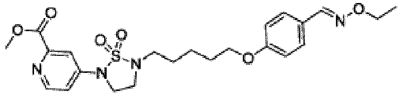

Please refer to the preparation method of Embodiment 32 for this embodiment. ¹H NMR(400MHz,CHLOROFORM-d)8.63(d,J=5.77Hz,1H),8.04(s,1H),7.75(d,J=2.26Hz,1H),7.53(d,J=8. 78Hz,2H),7.40(dd,J=2.38,5.65Hz,1H),6.89(d,J=8.78Hz,2H),4.22(q,J=7.03Hz,2H),4.00-4.06(m,5H),3. 93(t,J=6.40Hz,2H),3.59(t,J=6.40Hz,2H),3.21 (t,J=7.28Hz,2H),1.77-1.93(m,4H),1.61-1.70(m,5H),1.33( t,J=7.03Hz,3H). LCMS(ESI)m/z:491 (M+1).

### Embodiment 38

### (E)-4-((5-(5-(6-amino-pyrimidin-4-yl)--1,1-dioxide-1,2,5-thiadiazolidine-2-yl)-pentyl)-oxy)-benzaldehyde-O-ethy l ketoxime

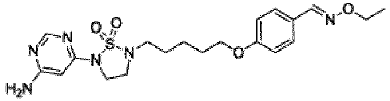

### Embodiment 38A

### (E)-4-(5-(5-(6-chloro-pyrimidin-4-yl)-1,1-dioxide-1,2,5-thiadiazolidine-2-yl)-pentyl)-oxy)-benzaldehyde-O-ethyl ketoxime

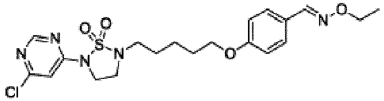

Please refer to the preparation method of Embodiment 32 for this embodiment.
LCMS(ESI)m/z:468(M+1).

### Embodiment 38B

### (E)-4-((5-(5-(6-amino-pyrimidin-4-yl)--1,1-dioxide-1,2,5-thiadiazolidine-2-yl)-pentyl)-oxy)-benzaldehyde-O-ethyl ketoxime

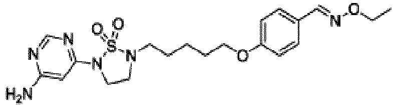

Please refer to the preparation method of Embodiment 28 for this embodiment. ¹H NMR(CHLOROFORM-d,400MHz):8.33(s,1H),8.02(s,1H),7.51(d,*J*=8.5Hz,2H),6.87(d,*J*=8.5Hz,2H),6. 34(s,1H),4.93(br.s.,2H),4.16-4.25(m,2H),4.05(t,*J*=6.5Hz,2H),4.00(t,*J*=6.3Hz,2H),3.44-3.50(m,2H),3.1 6(t,*J*=7.3Hz,2H),1.82-1.90(m,2H),1.74-1.79(m,2H),1.55-1.60(m,2H),1.32(t,*J*=7.0Hz,3H). LCMS(ESI)m/z:449(M+1).

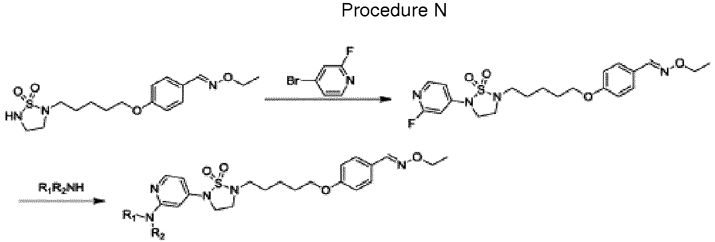

### Embodiment 39

### (E)-4-((5-(5-(2-Fluoro-4-yl)-1,1-dioxide-1,2,5-thiadiazolidine-2-yl)-pentyl)-oxy)-benzaldehyde-O-ethyl ketoxime

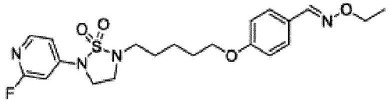

Under the protection of N₂ gas, add Pd₂(dba)₃ (15mg, 17 µmol) and Xant-Phos (10mg, 17µmol) into the solution of Embodiment 32F (60mg, 169µmol), 4-bromo-2-fluoro-pyridine (30mg, 169µmol) and Cs₂CO₃ (83mg, 253µmol) in dioxane (2ml). Then agitate it evenly and heat it to 100°C to react for 4 hours. After TLC verifies that the reaction ends, concentrate and evaporate the solvent. Add water (20ml) and then extract with ethyl acetate (20ml x 3). Then combine the organic layers and dry with anhydrous Na₂SO₄ before filter and evaporate. Then separate and purify the crude product with TLC plate (petroleum ether / ethyl acetate = 2:1) to obtain the title compound (white solid, 70mg, yield of 92%). ¹H
NMR(400MHz,CHLOROFORM-d)8.12(d,*J*=6.02Hz,1H),8.03(s,1H),7.51(d,*J*=8.53Hz,2H),7.02(d,*J*=5. 52Hz,1H),6.82-6.93(m,2H),6.58(d,*J*=2.01Hz,1H),4.20(q,*J*=7.03Hz,2H),4.00(t,*J*=6.02Hz,2H),3.85(t,*J*= 6.53Hz,2H),3.52-3.59(m,2H),3.19(t,*J*=7.28Hz,2H),1.74-1.91(m,4H),1.59-1.67(m,2H),1.29-1.39(m,3H). LCMS(M+1):451.

### Embodiment 40

### (E)-4-((5-(5-(2-((2-hydroxyethyl)-amino)-pyridin-4-yl)-1,1-dioxide -1,2,5-thiadiazolidine-2-yl)-pentyl)-oxy)-benzaldehyde-O-ethyl ketoxime

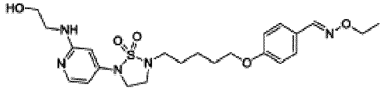

Under the protection of N₂ gas, add triethylamine (27mg, 266µmol) into the solution of Embodiment 39 (20mg, 44µmol) and 2-aminoethanol (27mg, 444µmol) in dimethylsulfoxide (0.5ml). Allow the mixture solution to react for 2 hours at 100°C. After TLC verifies that the reaction ends, add water (5ml) to quench and extract with ethyl acetate (10ml). Combine the organic layers and dry it with anhydrous Na₂SO₄ before filter and evaporate. Separate and purify the crude product with TLC (dichloromethane: methnol = 20:1) to obtain the title compound (colorless oily liquid, 12mg, 55%). ¹H NMR(400MHz,CHLOROFORM-d)8.02(s,1H),7.84-7.95(m,1H),7.51(d,*J*=8.53Hz,2H),6.85-6.93(m,2H) ,6.41(dd,*J*=1.76,6.27Hz,1H),6.25(d,*J*=1.51Hz,1H),5.47(br.s.,1H),4.15-4.29(m,2H),4.00(t,*J*=6.27Hz,2 H),3.81(t,*J*=4.77Hz,4H),3.45-3.56(m,4H),3.16(t,*J*=7.03Hz,2H),1.81-1.90(m,2H),1.72-1.81(m,2H),1.56 -1.67(m,2H), 1.27-1.40(m,3H).LCMS(ESI)m/z:492(M+1).

### Embodiment 41

### (E)-4-((5-(5-(2-(methylamino)-pyridin-4-yl)-1,1-dioxide-1,2,5-thiadiazolidine-2-yl)-pentyl)-oxy)-benzaldehyde-O -ethyl ketoxime

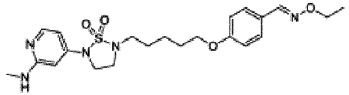

Please refer to the preparation method of Embodiment 40 for this embodiment. ¹H NMR(400MHz,CHLOROFORM-d)7.97-8.05(m,2H),7.51(d,*J*=8.53Hz,2H),6.87(d,*J*=8.53Hz,2H),6.36(d d,*J*=2.01,6.02Hz,1H),6.14(d,*J*=2.01Hz,1H),4.64(br.s.,1H),4.20(q,*J*=7.03Hz,2H),4.00(t,*J*=6.27Hz,2H), 3.82(t,*J*=6.27Hz,2H),3.49(t,*J*=6.27Hz,2H),3.16(t,*J*=7.28Hz,2H),2.92(d,*J*=5.02Hz,3H),1.73-1.91(m,4H ),1.62-1.66(m,2H),1.28-1.39(m,3H). LCMS(ESI)m/z:462(M+1).

### Embodiment 42

### (E)-4-((5-(5-(2-(azetidin-1-yl)-pyridin-4-yl)-1,1-dioxide-1,2,5-thiadiazolidine-2-yl)-pentyl)-oxy)-benzaldehyde-O-ethyl ketoxime

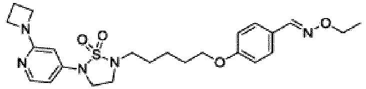

Please refer to the preparation method of Embodiment 40 for this embodiment. ¹H NMR(400MHz,CHLOROFORM-d)8.00-8.06(m,1H),7.50(d,J=8.78Hz,1H),6.87(d,J=8.78Hz,1H),6.40(d d,J=2.01,5.77Hz,1H),5.92(d,J=1.76Hz,1H),4.19(q,J=7.03Hz,2H),4.05(t,J=7.40Hz,4H),3.99(t,J=6.27H z,2H),3.80(t,J=6.40Hz,2H),3.43-3.51 (m,3H),3.15(t,J=7.15Hz,2H),2.38(quin,J=7.34Hz,2H),1.81-1.89( m,2H),1.72-1.79(m,2H),1.57-1.65(m,2H),1.31 (t,J=7.03Hz,3H). LCMS(ESI)m/z:488(M+1).

### Embodiment 43

### (E)-4-((5-(5-(2-(dimethylamino)-pyridin-4-yl)-1,1-dioxide-1,2,5-thiadiazolidine-2-yl)-pentyl)-oxy)-benzaldehyde-O-ethyl ketoxime

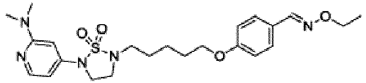

Please refer to the preparation method of Embodiment 40 for this embodiment. 1H NMR(400MHz,CHLOROFORM-d)8.09(d,J=5.77Hz,1H),8.03(s,1H),7.52(d,J=8.53Hz,2H),6.88(d,J=8. 78Hz,2H),6.38(dd,J=1.88,5.90Hz,1H),6.26(d,J=1.76Hz,1H),4.21(q,J=7.03Hz,2H),3.97-4.04(m,2H),3. 85(t,J=6.27Hz,2H),3.51 (t,J=6.27Hz,2H),3.17(t,J=7.15Hz,2H),3.12(s,5H),1.73-1.92(m,4H),1.58-1.67( m,2H),1.33(t,J=7.03Hz,3H). LCMS(ESI)m/z:476(M+1).

### Embodiment 44

### (E)-4-(5-(5- (4-((ethoxy)-methyl)-phenoxy)-pentyl)-1,1-dioxide-1,2,5-thiadiazolidine-2-yl)-benzonitrile

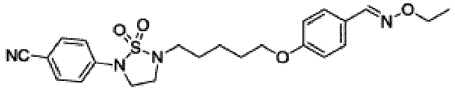

Under the protection of N₂ gas at 0°C, add K₂CO₃ (39mg, 281 µmol) into the solution of Embodiment 32F (50mg, 141 µmol) and 4-fluorobenzonitrile (17mg, 141 µmol) in N,N-dicarboximide (1ml). Allow the mixture solution to react for 3 hours at 80°C. After the reaction ends, pour the solution into water (40ml) and extract with ethyl acetate (20ml). Combine the organic layers and dry it with anhydrous Na₂SO₄ before filter and evaporate. Separate and purify the crude product with TLC (petroleum ether: ethyl acetate = 2 :1) to obtain the title compound (white solid, 30mg, 66%). ¹H NMR(400MHz,CHLOROFORM-d)8.02(s,1H),7.66(d,*J*=8.53Hz,2H),7.51(d,*J*=8.53Hz,2H),7.25(s,2H), 6.82-6.93(m,2H),4.16-4.29(m,2H),4.00(t,*J*=6.27Hz,2H),3.86(t,*J*=6.53Hz,2H),3.54(t,*J*=6.27Hz,2H),3.1 8(t,J=7.28Hz,2H),1.74-1.90(m,4H),1.60-1.67(m,2H),1.29-1.40(m,3H). LCMS(ESI)m/z:457(M+1).

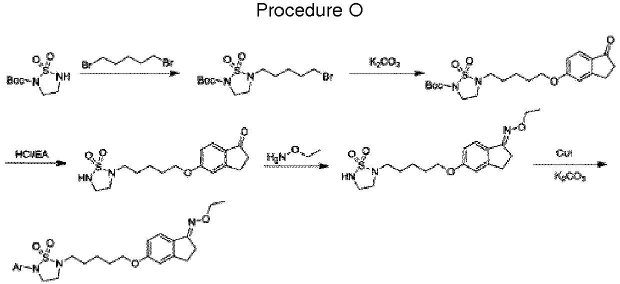

### Embodiment 45

### (E) -2-(2-amino-pyridin-4-yl)-5-(5-((1-(ethoxy-imino)-2,3-dihydro-1H-inden-5-yl)-oxy)-pentyl)-1,2,5-thiadiazolidine-1,1-dioxide

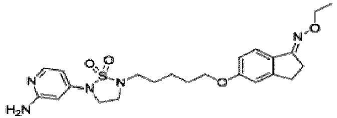

### Embodiment 45A

### tert-butyl-5-(5-bromo-pentyl)-1,2,5-thiadiazolidine-2-t-butyl formate-1,1-dioxide

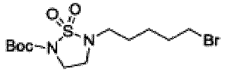

Under the protection of N₂ gas at 10°C, add sodium hydride (17mg, 428µmol) into the solution of Embodiment 32B (100mg, 428µ mol) in DMF (8ml). One hour later, add this mixture solution into the solution of 1,5-dibromopentane (98mg, 428µmol) in DMF (4ml). Then agitate it react 10 hours at 10°C before add water to quench the reaction. Then extract the aqueous phase with ethyl acetate (20ml x 3) and wash the combined organic phase with saturated salt water (10ml x 2). Then dry with anhydrous Na₂SO₄ before filter and concentrate. Then purify the crude product with the preparative TLC (dichloromethane: methanol = 20:1) to obtain the title compound (yellow liquid, 50mg, yield of 38%). ¹H NMR(400MHz,CHLOROFORM-d)3.82(t , *J*=6.53Hz,2H),3.44(t,*J*=6.65Hz,2H),3.34(t,*J*=6.53Hz,2H),3.08(t,*J*=7.15Hz,2H), 1.92(quin,*J*=7.09Hz,2H ),1.66-1.79(m,2H), 1.56-1.62(m, 12H).

### Embodiment 45B

### (E)-tert-butyl-5-(5-((1-(ethoxy-imino)-2,3-dihydro-1H-inden-5-yl)-oxy)-pentyl)-1,2,5-thiadiazolidine-2-tert-butyl formate-1,1-dioxide

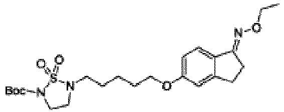

Under the protection of N₂ gas, add K₂CO₃ (112mg, 808µmol) into the solution of Embodiment 45A (100mg, 269µmol) and Embodiment 8B (52mg, 269µmol) in N,N-dimethylformamide (1ml). Allow the reaction solution to react for 10 hours at 80°C. After TLC shows the reaction ends, add water (50ml) to quench the reaction and extract the aqueous phase with ethyl acetate (20ml). Dry the organic layer with anhydrous Na₂SO₄ before filter and evaporate. Separate and purify the crude product with TLC (petroleum ether: ethyl acetate = 2 :1) to obtain the title compound (white solid, 70mg, 54%). ¹H NMR(400MHz,CHLOROFORM-d)7.54-7.64(m,1H),6.74-6.83(m,2H),4.20(q,*J*=7.03Hz,2H),3.98(t,*J*=6. 27Hz,2H),3.80(t,*J*=6.53Hz,2H),3.32(t,*J*=6.53Hz,2H),3.08(t,*J*=7.28Hz,2H),2.95-3.02(m,2H),2.85-2.93( m,2H),1.67-1,87(m,4H),1.51-1,59(m,11H),1,32(t,*J*=7.03Hz,3H).

### Embodiment 45C

### (E)-2-(5-((1-(ethoxy-imino)-2,3-dihydro-1H-inden-5-yl)-oxy)-pentyl)-1,2,5-thiadiazolidine-1,1-dioxide

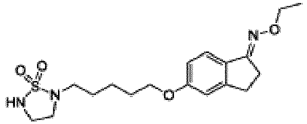

Under the protection of N₂ gas, add K₂CO₃ (57mg, 415µmol) into the solution of Embodiment 45B (100mg, 208µmol) in methanol (1ml). Allow the reaction solution to react for 72 hours at 50°C. After TLC shows the reaction ends, concentrate, and evaporate the solvent out. Then add water (10ml) before extract with ethyl acetate (20ml). Combine the organic layers and dry it with anhydrous Na₂SO₄ before filter and evaporate. Separate and purify the crude product with TLC (petroleum ether: ethyl acetate = 2 :1) to obtain the title compound (white solid, 50mg, 63%). ¹H NMR(400MHz,CHLOROFORM-d)8.09(d,*J*=8.78Hz,2H),7.03(d,*J*=8.78Hz,2H),4.40(s,3H),3.89(s,3H).

### Embodiment 45D

### (E)-2-(2-amino-pyridin-4-yl)-5-(5-((1-(ethoxy-imino) -2,3-dihydro-1H-inden-5-yl)-oxy)-pentyl)-1,2,5-thiadiazolidine-1,1-dioxide

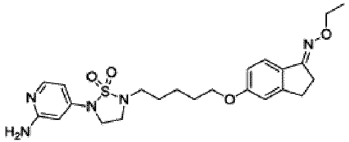

Under the protection of N₂ gas, heat the solution of Embodiment 45C (550mg, 1.4mmol), 4-bromo-2-aminopyridine (499mg, 2.9mmol), K₂CO₃ (498mg, 3.6mmol), 8-hydroxyquinoline (209mg, 1.4mmol), (1R, 2R) - cyclohexanediamine (165mg, 1.4mmol) and Cul (275mg, 1.4mmol) in DMF (10ml) to 120°C to react for 10 hours. After the reaction ends, add water to quench and extract the aqueous phase with ethyl acetate (10ml x 3). Wash the organic phase with saturated salt water (5ml). Then dry and concentrate with anhydrous Na₂SO₄. Then purify the crude product with the preparative TLC (dichloromethane: methanol = 30:1) to obtain the title compound (white solid, 350mg, yield of 51%). ¹H
NMR(400MHz,DMSO-d₆)8.09(d,*J*=8.78Hz,1H),7.44(d,*J*=8.53Hz,1H),6.92(s,1H),6.82(d,*J*=8.53Hz,1H) ,6.34(br.s.,1H),6.15(br.s.,1H),4.09(q,*J*=7.03Hz,2H),3.99(t,*J*=6.40Hz,2H),3.79(t,*J*=6.40Hz,2H),3.49(t,*J*=
6.40Hz,2H),3.04(t,*J*=7.03Hz,2H),2.89-2.98(m,2H),2.69-2.82(m,2H),1.59-1.81(m,5H),1.40-1.54(m,2H) ,1.22(t,*J*=7.03Hz,3H). LCMS(ESI)m/z:474(M+1).

### Embodiment 46

### (E)-2- (5-((1-(ethoxy-imino)-2,3-dihydro-1H-inden-5-yl)-oxy)-pentyl)-5-(thiophen-3-yl)-1,2,5-thiadiazolidine-1,1-dioxide

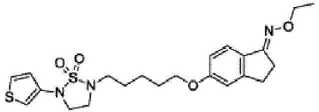

Please refer to the preparation method of Embodiment 44 for this embodiment. ¹H NMR(CDCl3 400MHz,)δ7.60(d,J=9.5Hz,1H)7.35(dd,*J*=5.0,3.0Hz,1H)7.18(dd,*J*=5.5,1.0Hz,1H)6.76-6.85(m,3H)4.2 0(q,*J*=7.0Hz,2H)3.99(t,*J*=6.3Hz,2H)3.79(t,*J*=6.3Hz,2H)3.49(t,*J*=6.5Hz,2H)3.16(t,*J*=7.3Hz,2H)2.95-3. 02(m,2H)2.86-2.92(m,2H)1.81-1.89(m,2H)1.70-1.79(m,2H)1.64(br.s.,2H)1.32(t,*J*=7.0Hz,3H).
LCMS(ESI)m/z:464(M+1).

### Embodiment 47

### (E)-2-(6-amino-pyridin-3-yl)-5-(5-((1-(ethoxy-imino)-2,3-dihydro-1H-inden-5-yl)-oxy)-pentyl)-1,2,5-thiadiazolidine-1,1-dioxide

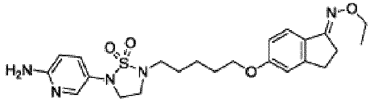

Please refer to the preparation method of Embodiment 44 for this embodiment. ¹H NMR(400MHz,CHLOROFORM-d)8.01(br.s.,1H),7.59(dt,*J*=3.26,6.02Hz,2H),6.76-6.85(m,2H),6.56(d, *J*=9.03Hz,1H),4.20(q,*J*=7.19Hz,2H),3.99(t,*J*=6.27Hz,2H),3.74(t,*J*=6.27Hz,2H),3.46(t,*J*=6.40Hz,2H),3. 15(t,*J*=7.28Hz,2H),2.95-3.01(m,2H),2.86-2.92(m,2H),1.72-1.89(m,4H),1.56-1.60(m,2H),1.28-1.38(m, 3H).

### Embodiment 48

### (E)-2-(2-amino-3-fluoropyridin-4-yl)-5-(5-((1-(ethoxy-imino)-2,3-dihydro-1 H-indene-5-yl)-oxy)-pentyl) -1,2,5-thiadiazolidine-1,1-dioxide

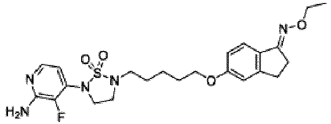

### Embodiment 48A

### (E)-2-(2-chloro-3-fluoropyridin-4-yl) -5- (5 - ((1- (ethoxy-imino)-2,3-dihydro -1H-indene-5-yl)-oxy)-pentyl)-1,2,5-thiadiazolidine-1,1-dioxide

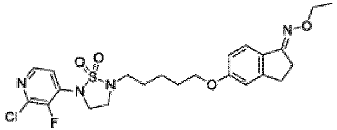

Under the protection of N₂ gas, add 2-chloro-5-fluoro-4-iodopyridine (101mg, 393µmol), K₂CO₃ (54mg, 393µmol), 8-hydroxyquinoline (38mg, 262µmol) and Cul (25mg, 131µmol) into the solution of Embodiment 45C (100mg, 262µmol) in N,N-dimethylformamide (3ml). Allow the mixture solution to react for 10 hours at 120°C. After TLC verifies that the reaction ends, add water (10ml) to quench and extract with ethyl acetate (10ml x 2). Combine the organic layers and dry it with anhydrous Na₂SO₄ before filter and evaporate. Separate and purify the crude product with TLC plate (petroleum ether: ethyl acetate = 2 :1) to obtain the title compound (white solid, 50mg, 37%). ¹H NMR(400MHz,CHLOROFORM-d)8.21(d,*J*=3.51Hz,1H),7.60(d,*J*=9.03Hz,1H),7.46(d,*J*=6.02Hz,1H),6. 74-6.84(m,2H),4.20(q,*J*=7.03Hz,2H),4.07(dt,*J*=3.01,6.27Hz,2H),4.00(t,*J*=6.27Hz,2H),3.54(t,*J*=6.27H z,2H),3.18(t,*J*=7.28Hz,2H),2.95-3.02(m,2H),2.86-2.93(m,2H),1.73-1.91 (m,4H),1.61-1.66(m,2H),1.32( t,*J*=7.03Hz,3H).

### Embodiment 48B

### (E)-2-(2-amino-3-fluoropyridin-4-yl)-5-(5-((1-(ethoxy-imino)-2,3-dihydro-1 H-indene-5-yl)-oxy)-pentyl) -1,2,5-thiadiazolidine-1,1-dioxide

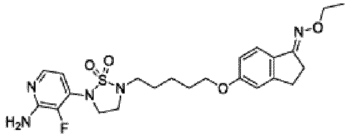

Under the protection of N₂ gas, add tert-butyl carbamate (69mg, 587µmol), Cs₂CO₃ (64mg, 196µmol), Pd₂(dba)₃ (9mg, 9.8µmol) and Xant-phos (6mg, 10µmol) into the solution of Embodiment 48A (50mg, 98µmol) in dioxane (1ml). Allow the mixture solution to react for 10 hours at 110°C. After TLC verifies that the reaction ends, add water (10ml) to quench and extract with ethyl acetate (10ml x 3). Combine the organic layers and dry it with anhydrous Na₂SO₄ before filter and evaporate. Separate and purify the crude product with TLC plate (dichloromethane: methnol = 20:1) to obtain the title compound as white solid (5mg, 10%). ¹H NMR(400MHz,METHANOL-d₄)7.97(d,*J*=6.53Hz,1H),7.56(d,*J*=8.53Hz,1H),6.91(s,1H),6.79-6.88(m,2 H),4.05(t,*J*=6.27Hz,2H),3.65(t,*J*=6.27Hz,2H),3.22(t,*J*=7.03Hz,2H),2.99-3.05(m,2H),2.86-2.92(m,2H), 1.73-1.93(m,5H),1.58-1.70(m,2H),1.31(t,*J*=7.03Hz,4H). LCMS(ESI)m/z:492(M+1).

### Embodiment 49

### (E)-2-(2-amino-5-fluoro-pyridin-4-yl)-5-(5-((1-(ethoxy-imino)-2,3-dihydro-1H-indene-5-yl)-oxy)-pentyl) -1,2,5-thiadiazolidine-1,1-dioxide

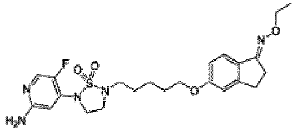

### Embodiment 49A

### (E)-2-(2-chloro-5-fluoro-pyridin-4-yl)-5-(5-((1-(ethoxy-imino)-2,3-dihydro-1H-indene-5-yl)-oxy)-pentyl)-1,2,5-thiadiazolidine-1,1-dioxide

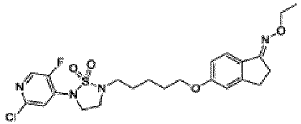

Under the protection of N₂ gas, add 2-chloro-5-fluoro-4-iodopyridine (101mg, 393µmol), K₂CO₃ (54mg, 393µmol), 8-hydroxyquinoline (38mg, 262µmol) and Cul (25mg, 131µmol) into the solution of Embodiment 45C (100mg, 262µmol) in N,N-dimethylformamide (3ml). Allow the mixture solution to react for 10 hours at 120°C. After TLC verifies that the reaction ends, add water (10ml) to quench and extract with ethyl acetate (10ml x 2). Combine the organic layers and dry it with anhydrous Na₂SO₄ before filter and evaporate. Separate and purify the crude product with TLC plate (petroleum ether: ethyl acetate = 2 :1) to obtain the title compound (white solid, 50mg, 37%). ¹H NMR(400MHz,CHLOROFORM-d)8.21(d,*J*=3.51Hz,1H),7.60(d,*J*=9.03Hz,1H),7.46(d,*J*=6.02Hz,1H),6. 74-6.84(m,2H),4.20(q,*J*=7.03Hz,2H),4.07(dt,*J*=3.01,6.27Hz,2H),4.00(t,*J*=6.27Hz,2H),3.54(t,*J*=6.27H z,2H),3.18(t,*J*=7.28Hz,2H),2.95-3.02(m,2H),2.86-2.93(m,2H),1.73-1.91(m,4H),1.61-1.66(m,2H),1.32( t,*J*=7.03Hz,3H).

### Embodiment 49B

### (E)-2-(2-amino-5-fluoro-pyridin-4-yl)-5-(5-((1- (ethoxy-imino)-2,3-dihydro-1H-indene-5-yl)-oxy)-pentyl) -1,2,5-thiadiazolidine-1,1-dioxide

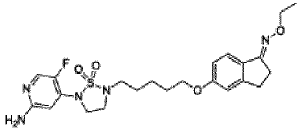

Under the protection of N₂ gas, add tert-butyl carbamate (69mg, 587µmol), Cs₂CO₃ (64mg, 196µmol), Pd₂(dba)₃ (9mg, 9.8µmol) and Xant-phos (5.6mg, 9.8µmol) into the solution of Embodiment 49A (50mg, 98µmol) in dioxane (1ml). Allow the mixture solution to react for 10 hours at 110°C. After TLC verifies that the reaction ends, add water (10ml) to quench and extract with ethyl acetate (10ml x 3). Combine the organic layers and dry it with anhydrous Na₂SO₄ before filter and evaporate. Separate and purify the crude product with TLC (dichloromethane: methnol = 20:1) to obtain the title compound as white solid (5mg, 10%). ¹H NMR(400MHz,METHANOL-d₄)7.97(d,*J*=6.53Hz, 1H),7.56(d,*J*=8.53Hz,1H),6.91(s,1H),6.79-6.88(m,2 H),4.05(t,*J*=6.27Hz,2H),3.65(t,*J*=6.27Hz,2H),3.22(t,*J*=7.03Hz,2H),2.99-3.05(m,2H),2.86-2.92(m,2H), 1.73-1.93(m,5H),1.58-1.70(m,2H),1.31 (t,*J*=7.03Hz,4H). LCMS(ESI)m/z:492(M+1).

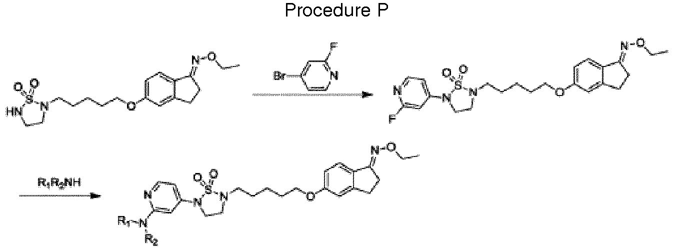

### Embodiment 50

### (E)-2-(5-((1-(ethoxy-imino)-2,3-dihydro-1 H-inden-5-yl)-oxy)-pentyl)-5-(2-(methylamino)-pyridin-4-yl) -1,2-thiadiazolidine-1,1-dioxide

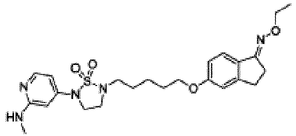

### Embodiment 50A

### (E)-2-(5-((1-(ethoxy-imino)-2,3-hydrogen-1 H-inden-5-yl)-oxy)-pentyl)-5-(2-fluoro-pyridin-4-yl) -1,2,5-thiadiazolidine-1,1-dioxide

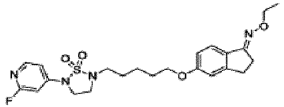

This embodiment is prepared with the method as described in Embodiment 50A. ¹H NMR(400MHz,CHLOROFORM-d)7.80(d,J=5.5Hz,1H)7.62(d,J=9.3Hz,1H)6.77-6.85(m,3H)4.65(br.s., 2H)4.22(q,J=7.0Hz,2H)3.99-4.05(m,4H)3.52(t,J=6.4Hz,2H)3.18(t,J=7.2Hz,2H)2.97-3.04(m,2H)2.87-2 .94(m,2H)1.75-1.91(m,4H)1.63-1.68(m,2H)1.34(t,J=7.0Hz,3H). LCMS(ESI)m/z:474(M+1).

### Embodiment 50B

### (E)-2-(5-((1-(ethoxy-imino)-2,3-dihydro-1 H-inden-5-yl)-oxy)-pentyl)-5-(2-methylamino)-pyridin-4-yl) -1,2-thiadiazolidine-1,1-dioxide

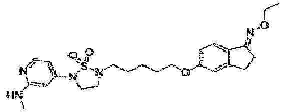

Please refer to the preparation method of Embodiment 40 for this embodiment. ¹H NMR(400MHz,METHANOL-d₄)7.80(d,*J*=7.53Hz,1H),7.56
(d,*J*=8.53Hz,1H),6.81-6.93(m,3H),6.39(d,*J*=2.01 Hz,1H),4.17(q,*J*=7.03Hz,2H),4.05(t,*J*=6.15Hz,2H),3. 99(t,*J*=6.53Hz,2H),3.66(t,*J*=6.53Hz,2H),3.22(t,*J*=7.03Hz,2H),2.96-3.05(m,5H),2.84-2.92(m,2H),1.76-1.91 (m,4H),1.58-1.71 (m,2H),1.31 (t,*J*=7.03Hz,3H. LCMS(ESI)m/z:488(M+1).

### Embodiment 51

### (E)-2-(5-((1-(ethoxy-imino)-2,3-dihydro-1H-inden-5-yl)-oxy)-pentyl)-5-(2-((2-hydroxyethyl)-amino)-pyridin-4-yl)-1,2,5-thiadiazolidine-1,1-dioxide

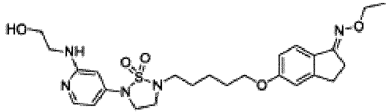

Please refer to the preparation method of Embodiment 40 for this embodiment. ¹H NMR(400MHz,CHLOROFORM-d)7.84(d,*J*=6.3Hz,1H),7.61(d,*J*=9.0Hz,1H),6.74-6.85(m,2H),6.52(d,*J* =5.3Hz,1H),6.27(s,1H),4.22(q,*J*=7.0Hz,2H),4.01(t,*J*=6.1Hz,2H),3.87(d,*J*=4.8Hz,4H),2.86-3.62(m,10H ),1.85-1.89(m,2H),1.76-1.82(m,2H),1.63(d,*J*=7.0Hz,2H),1.34(t,*J*=7.0Hz,3H).
LCMS(ESI)m/z:518(M+1).

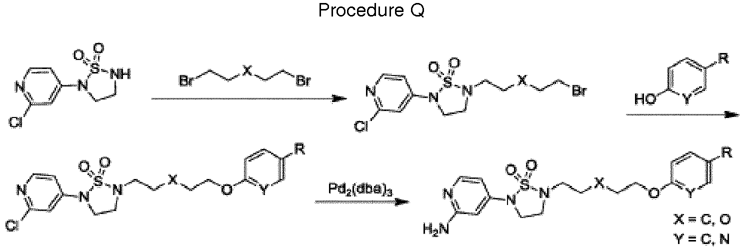

### Embodiment 52

### 4-(5-(2-(2-(4-((E)-ethoxy-imino-methyl)-phenoxy)-ethoxy)-ethyl)-1,1-dioxide-1,2,5-thiadiazolidine-2-yl)-pyridin-2-amine

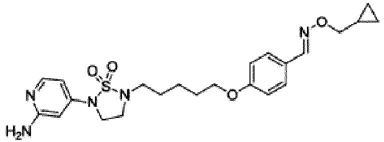

### Embodiment 52A

### 2-(5-bromo-pentyl)-5-(2-chloro-4-yl)-1,2,5-thiadiazolidine-1,1-dioxide

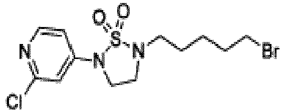

At 10°C, add sodium hydroxide (1.0g, 27mmol) into the solution of Embodiment 4E (4.0g, 17mmol) in N,N-dimethylformamide (140ml). After the substance is added, agitate at 10°C to react 1 hour. Keep this temperature and drop the solution of 1,5-dibromopentane (3.9g, 17mmol) in N,N-dimethylformamide (40ml) into the reaction solution. Then continue to react 1 hour.

After TLC verifies the reaction ends, pour the reaction solution into ice water (100ml) and extract with ethyl acetate (100ml x 2). Take and dry the supernatant liquid. Concentrate it and purify with column chromatography (petroleum ether: ethyl acetate = 2:1) to obtain the title compound (white solid, 2.7g, yield of 41%). ¹H NMR(400MHz,CHLOROFORM-d)8.27(d,*J*=5.5Hz,1H),7.07(dd,*J*=6.0,2.0Hz,1H),7.00(d,*J*=1.5Hz,1H), 3.85(t,*J*=6.5Hz,2H),3.56(t,*J*=6.3Hz,2H),3.44(t,*J*=6.5Hz,2H),3.17(t,*J*=7.0Hz,2H),1.88-1.98(m,2H),1.74 (q,*J*=7.4Hz,2H),1.58-1.64(m,2H).

### Embodiment 52B

### (E)-1-(4-(2-(2-(5-(2-chloro-4-pyridyl)-1,1-dioxo-1,2,5-thiadiazolidine-2-yl)-ethoxy)-ethoxy)-phenyl)-N-ethoxy formimidate

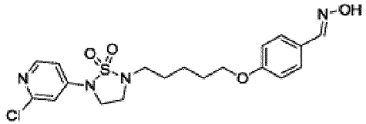

Add K₂CO₃ (36mg, 0.26mmol), KI (43mg, 0.26mmol) and 4-ammonia hydroxy phenol (43mg, 0.26mmol) into the solution of Embodiment 52A (0.1g, 0.26mmol) in N,N-dimethylformamide (5ml). Agitate the mixture solution for 15 hours at 80°C. Then extract it with ethyl acetate. Dry the organic phase with anhydrous Na₂SO₄ before filter and evaporate. Then purify the residue with column chromatography (petroleum ether: ethyl acetate =2:1) to obtain the title compound (white solid, 60mg, yield of 49%). LCMS(ESI)m/z:439(M+1).

### Embodiment 52C

### (E)-1-(4-(5-(5-(2-chloro-4-pyridyl)-1,1-dioxide-1,2,5-thiadiazolidine-2-yl)-pentyloxy)-phenyl)-N-(cyclopropyl)-methylamine

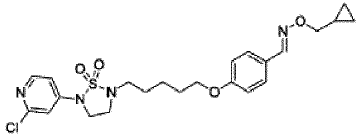

At 0°C, add cyclopropyl bromide (34mg, 0.25mmol) and K₂CO₃ (35mg, 0.15mmol) into the solution of Embodiment 52B (110mg, 0.25mmol) in N,N-dimethylformamide (2ml). Agitate it to react for 15 hours at 50°C. Then add water to quench the reaction and extract the aqueous phase with ethyl acetate. Dry the combined organic phases with anhydrous Na₂SO₄. Then filter and evaporate it before separate it with column chromatography (petroleum ether: ethyl acetate=1:1) to obtain the title compound (yellow solid, 50mg, yield of 40%). LCMS(ESI)m/z:450(M+1). ¹H NMR(400MHz,CDCl₃)8.04(s,1H),7.63(d,*J*=7.0Hz,1H),7.53(d,*J*=8.5Hz,2H),6.90(d,*J*=8.5Hz,2H),6.62(d ,*J*=7.5Hz,1H),6.10(s,1H),4.16-4.25(m,4H),3.82-3.88(m,4H),3.72(dd,*J*=11.5,5.0Hz,4H),3.40(t,*J*=4.8Hz ,2H),1.32(t,*J*=7.0Hz,3H).

### Embodiment 52D

### 4-(5-(2-(2-(4-((E)-ethoxy-imino-methyl)-phenoxy)-ethoxy)-ethyl)-1,1-dioxide-1,2,5-thiadiazolidine-2-yl)-pyridin-2-amine

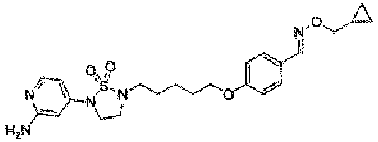

At 0°C, add cesium carbonate (66mg, 0.2mmol) and tert-butyl carbamate (23mg, 0.2mmol) into the solution of Embodiment 52C (50mg, 0.1 mmol) in N,N-dimethylformamide (1.5ml). After replace with N₂ gas, under the protection of N₂ gas, add Pd₂(dba)₃ (19mg, 20µmol) and Xantphos (12mg, 20µmol). Agitate it to react for 15 hours at 110°C. Then add water to quench the reaction and extract the aqueous phase with ethyl acetate. Dry the combined organic phase with anhydrous Na₂SO₄ before filter and evaporate. Then purify it with HPLC to obtain the title compound (white solid, 5mg, yield of 10%). ¹H
NMR(400MHz,CDCl₃)8.08(s,1H),7.65(d,*J*=7.0Hz,1H),7.53(d*,J*=8.8Hz,2H),6.89(d,*J*=8.5Hz,2H),6.70(d ,*J*=6.8Hz,1H),6.21(br.s.,1H),3.95-4.06(m,4H),3.83-3.90(m,2H),3.59(t,*J*=6.1Hz,2H),3.21(t,*J*=7.2Hz,2H ),1.75-1.92(m,3H),1.57-1.69(m,3H),1.16-1.28(m,1H),0.57-0.64(m,2H),0.34(q,*J*=4.9Hz,2H). LCMS(ESI)m/z:474(M+1).

### Embodiment 53

### (E)-4-((5-(5-(2-amino-pyridin-4-yl)-1,1-dioxide-1,2,5-thiadiazolidine-2-yl)-pentyl)-oxy)-benzaldehyde-O-isopropyl oxime

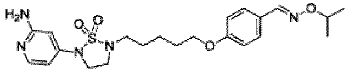

Please refer to the preparation method of Embodiment 52 for this embodiment. ¹H NMR(400MHz,CHLOROFORM-d)8.00(s,1H),7.95(d,*J*=6.0Hz,1H),7.50(d,*J*=8.5Hz,2H),6.86(d,*J*=9.0H z,2H),6.43(d,*J*=5.0Hz,1H),6.28(br.s.,1H),4.42(dt,*J*=12.3,6.4Hz,1H),3.99(t,*J*=6.3Hz,2H),3.80(t,*J*=6.3H z,2H),3.47-3.53(m,2H),3.15(t,*J*=7.3Hz,2H),1.82-1.89(m,2H),1.73-1.80(m,4H),1.29(d,*J*=6.0Hz,6H). LCMS(ESI)m/z:462(M+1).

### Embodiment 54

### (E)-2-(2-amino-pyridin-4-yl)-5-(5-(4-(1-(ethoxyimino)-propyl)-phenoxy)-pentyl)-1,2, 5-thiadiazolidine-1,1-dioxide

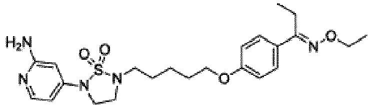

### Embodiment 54A

### (E) -1-(4-hydroxyphenyl)-propan-1-one-O-ethyl ketoxime

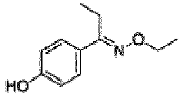

Agitate the mixture solution of 4-hydroxyphenyl acetone (1g, 6.7mmol), sodium acetate (1.1g, 3.3mmol), ethoxyamine hydrochloride (814mg, 13.3mmol) in ethanol (10ml) 2 hours at 80°C. Then add water (10ml) and ethyl acetate (20ml) into the reaction system. Then extract the aqueous layer with ethyl acetate (20ml x 3) before filter and evaporate to obtain the title compound (brown solid, 1.28g, yield of 99%). ¹H NMR(400MHz,CDCl₃)7.48(d,*J*=8.5Hz,2H),6.74-6.79(m,2H),4.20(q,*J*=7.0Hz,2H),2.72(q,*J*=7.7Hz,2H), 1.30(t,*J*=7.0Hz,3H),1.11(t,*J*=7.5Hz,3H).

### Embodiment 54B

### (E)-2-(2-chloropyridin-4-yl-5-(5-(4-(1-(ethoxyimino)-propyl)-phenoxy)-pentyl)-1,2,5-thiadiazolidine-1,1-dioxide

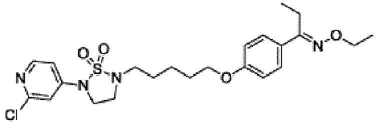

Please refer to the preparation method of Embodiment 52C for this embodiment. ¹H NMR(400MHz,CDCl₃)8.26(d,*J*=5.8Hz,1H),7.57(d,*J*=8.5Hz,2H),7.06(dd,*J*=5.8,2.0Hz,1H),6.99(d,*J*=1.8 Hz,1H),6.86(d,*J*=8.8Hz,2H),4.20(q,*J*=7.0Hz,2H),3.99(t,*J*=6.1Hz,2H),3.83(t,*J*=6.4Hz,2H),3.54(t,*J*=6.4 Hz,2H),3.17(t,*J*=7.3Hz,2H),2.72(q,*J*=7.5Hz,2H),1.74-1.88(m,4H),1.61(d,*J*=7.0Hz,2H),1.31(t,*J*=7.0Hz, 3H),1.12(t,*J*=7.5Hz,3H). LCMS(ESI)m/z:495(M+1).

### Embodiment 54C

### (E)-2-(2-amino-pyridin-4-yl)-5-(5-(4-(1-(ethoxyimino)-propyl)-phenoxy)-pentyl)-1,2, 5-thiadiazolidine-1,1-dioxide

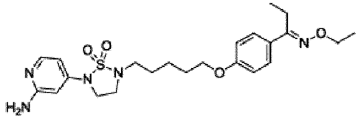

Please refer to the preparation method of Embodiment 52 for this embodiment. ¹H NMR(400MHz,CDCl₃)7.85(d,*J*=6.0Hz,1H),7.57(d,*J*=8.5Hz,2H),6.86(d,*J*=9.0Hz,2H),6.52(d,*J*=6.5Hz,1 H),6.29(s,1H),5.58(br.s.,2H),4.20(q,*J*=7.0Hz,2H),3.99(t,*J*=6.0Hz,2H),3.80-3.85(m,2H),3.52(t,*J*=6.3Hz ,2H),3.17(t,*J*=7.3Hz,2H),2.69-2.75(m,2H),1.85(d,*J*=7.0Hz,2H),1.75(br.s.,2H),1.61 (d,*J*=7.0Hz,2H),1.3 1(t,*J*=7.0Hz,3H),1.12(t,*J*=7.5Hz,3H). LCMS(ESI)m/z:477(M+1).

### Embodiment 55

### (E)-4-(2-2-(5-(2-amino-pyridin-4-yl)-1,1-dioxide-1,2,5-thiadiazolidine-2-yl)-ethoxy)-ethoxy) -benzaldehyde-O- ethyl ketoxime

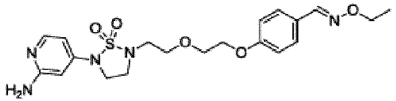

### Embodiment 55A

### 2-2-2(-bromo-ethoxy)-ethyl)-5-2-chloro-pyridin-4-yl)-1,2,5-thiadiazolidine-1,1-dioxide

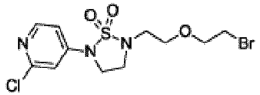

Please refer to the preparation method of Embodiment 52A for this embodiment. ¹H NMR(400MHz,CDCl₃)8.29(d,*J*=5.77Hz,1H),7.08(dd,*J*=2.26,5.77Hz,1H),7.02(d,*J*=2.26Hz,1H),3.78-3. 92(m,8H),3.53(t,*J*=5.52Hz,2H),3.42(t,*J*=4.89Hz,2H).

### Embodiment 55B

### (E)-1-(4-(2-(2-(5-(2-chloro-4-pyridyl)-1,1-dioxide-1,2,5-thiadiazolidine-2-yl)-ethoxy)-ethoxy)-phenyl)-N-ethoxy formimidate

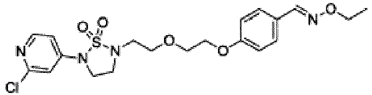

Add K₂CO₃ (72mg, 0.52mmol), KI (8.6mg, 0.05mmol) and Embodiment 7A (47mg, 0.29mmol) into the solution of Embodiment 55A (0.1g, 0.26mmol) in N,N-dimethylformamide (2ml). Agitate the mixture solution 12 hours at 80°C. Then extract it with ethyl acetate. Dry the organic phase with anhydrous Na₁SO₄ before filter and evaporate. Then purify the residue with column chromatography (petroleum ether: ethyl acetate =1:1) to obtain the title compound (white solid, 60mg, yield of 49%). LCMS(ESI)m/z:469(M+1).

### Embodiment 55C

### 4-(5-(2-(2-(4-((E)-ethoxy-imino-methyl)-phenoxy)-ethoxy)-ethyl)-1,1-dioxo-1,2,5-thiadiazolidine-2-yl)-pyridin-2-amine

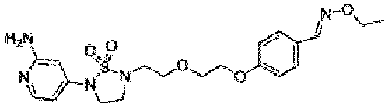

At 0°C, add cesium carbonate (84mg, 0.26mmol) and tert-butyl carbamate (18mg, 0.15mmol) into the solution of Embodiment 55B (60mg, 0.13mmol) in N,N-dimethylformamide (1ml). After replace with N₂ gas, under the protection of N₂ gas, add Pd₂(dba)₃ (22mg, 24µmol) and Xantphos (14mg, 24µmol). Agitate it to react for 15 hours at 110°C. Then add water to quench the reaction and extract the aqueous phase with ethyl acetate. Dry the combined organic phases with anhydrous Na₂SO₄ before filter and evaporate to obtain the title compound (white solid, 5mg, yield of 8.7%). ¹H NMR(400MHz,CDCl₃)8.04(s,1H),7.63(d,*J*=7.0Hz,1H),7.53(d,*J*=8.5Hz,2H),6.90(d,*J*=8.5Hz,2H),6.62(d ,*J*=7.5Hz,1H),6.10(s,1H),4.16-4.25(m,4H),3.82-3.88(m,4H),3.72(dd,*J*=11.5,5.0Hz,4H),3.40(t,*J*=4.8Hz ,2H),1.32(t,*J*=7.0Hz,3H). LCMS(ESI)m/z:450(M+1).

### Embodiment 56

### (E)-5-((5-(5-(2-amino-pyridin-4-yl)-1,1-dioxide-1,2,5-thiadiazolidine-2-yl)-pentyl)-oxy)-picolinaldehyde-O-ethyl ketoxime

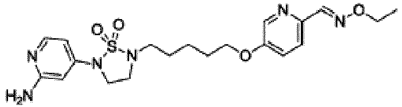

### Embodiment 56A

### (E)-5-hydroxy-pyridinecarboxaldehyde-O-ethyl ketoxime

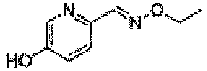

Heat the aqueous solution (5ml) of ethoxyamino hydrochloride (244mg, 2.5mmol), 5-hydroxy-pyridine-2-aldehyde (308mg, 2.5mmol), sodium acetate (410mg, 5.0mmol) to 80°C to agitate 2 hours. After TLC indicates the raw materials disappear, extract the reaction mixture with ethyl acetate (10ml x 3). Mix the organic phases and dry with anhydrous Na₂SO₄ before filter and concentrate to obtain the title compound (white solid, 410mg, yield of 99%) that is directly used without necessity to purify further.

### Embodiment 56B

### (E)-5-((5-(5-(2-chloropyridin-4-yl)-1,1-dioxide-1,2,5-thiadiazolidine-2-yl)-pentyl)-oxy)-picolinaldehyde-O-ethyl ketoxime

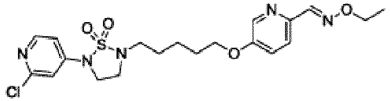

Heat the solution of Embodiment 52A (346mg, 998µmol), Embodiment 56A (163mg, 978µmol) and K₂CO₃ (276mg, 2.0mmol) and KI (17mg, 100µmol) in acetone solution (5ml) to 70°C to react for 3 hours. TLC indicates that the raw materials are consumed completely. Cool the reaction solution to room temperature before filter and concentrate to obtain the crude product. Purify the crude product with TLC plate (petroleum ether: ethyl acetate = 3 :1) to obtain the title compound (white solid, 150mg, yield of 35%). ¹H NMR(400MHz,CHLOROFORM-d) 8.30-8.24(m,2H),8.12(s,1H),7.71(d,*J*=9.0Hz,1H),7.18(dd,*J*=2.8,8.8Hz,1H),7.05(dd,*J*=2.0,5.5Hz,1H),6 .99(d,*J*=2.0Hz,1H),4.25(q,*J*=7.0Hz,2H),4.04(t,*J*=6.3Hz,2H),3.84(t,*J*=6.5Hz,2H),3.55(t,*J*=6.5Hz,2H),3. 22-3.14(m,2H),1.92-1.83(m,2H),1.79(quin,*J*=7.4Hz,2H),1.70-1.56(m,2H),1.33(t,*J*=7.3Hz,3H).

### Embodiment 56C

### (E)-5-((5-(5-(2-amino-pyridin-4-yl)-1,1-dioxide-1,2,5-thiadiazolidine-2-yl)-pentyl)-oxy)-picolinaldehyde-O-ethyl ketoxime

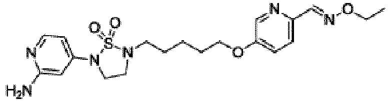

Under the protection of N₂ gas, heat the solution of tert-butyl carbamate (163mg, 1.4mmol) and Embodiment 56B (120mg, 278µml), Pd₂(dba)₃(25mg,28µmol), Xantphos(32mg, 56µmol), Cs₂CO₃ (181mg, 556µmol) in dioxane (3ml) to 100°C to react 6 hours. Cool the reaction heat to room temperature before filter and concentrate. Then purify the obtained crude product with the preparative HPLC (neutral separation method) to obtain the title compound (white solid, 30mg, yield of 26%). ¹H NMR(400MHz,CHLOROFORM-d)8.27(d,*J*=2.0Hz,1H),8.11(s,1H),7.97(d,*J*=5.5Hz,1H),7.70(d,*J*=8.8H z,1H),7.18(dd,*J*=2.3,8.5Hz,1H),6.41(d,*J*=4.5Hz,1H),6.28(s,1H),4.49(br.s.,2H),4.25(q,*J*=6.9Hz,2H),4.0 4(t,*J*=6.0Hz,2H),3.79(t,*J*=6.1Hz,2H),3.49(t,*J*=6.3Hz,2H),3.15(t,*J*=7.2Hz,2H),1.93-1.82(m,2H),1.82-1. 67(m,2H),1.67-1.55(m,2H),1.33(t,J=6.9Hz,3H). LCMS(ESI)m/z:449(M+1).

### Embodiment 57

### (E)-2-(2-amino-pyridin-4-yl)-5-(5-((1-(ethoxy-imino) -2,3-dihydro-1H-inden-5-yl)-oxy)-pentyl)-1,2,5-thiadiazolidine-1,1-dioxide

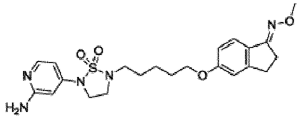

### Embodiment 57A

### 5-((5-(5-(2-chloro-4-yl)-1,1-dioxide-1,2,5-thiadiazolidine-2-yl)-pentyl)-oxy)-2,3-dihydro-1H-inden-1-one

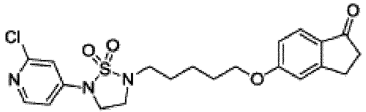

At room temperature, add K₂CO₃ (1.45g, 10.5mmol) into the solution of Embodiment 52A (2.0g, 5.2mmol) and 5-hydroxy-1-indanone (774mg, 5.2mmol) in acetone (30ml). Then heat the reaction solution to 70°C to react for 2 hours to consume the raw materials completely. Cool the reaction liquid to room temperature before concentrate. Then pour the residue into water (50ml). Then extract the aqueous phase with ethyl acetate (30ml x 3) and dry the organic phase with anhydrous Na₂SO₄. Then filter and concentrate before purify the crude product with column chromatography (petroleum ether: ethyl acetate = 2:1) to obtain the title compound (yellow solid, 1.3g, yield of 55%). ¹H NMP(400MHz,CDCl₃)8.28(d,*J*=5.77Hz,1H),7.67-7.73(m,1H),7.08(dd,*J*=2.01,5.77Hz,1H),7.01(d,*J*=1. 76Hz,1H),6.88-6.93(m,2H),4.07(t,*J*=6.15Hz,2H),3.80-3.91(m,2H),3.58(t,*J*=6.27Hz,2H),3.21(t,*J*=7.28 Hz,2H),3.04-3.13(m,3H),2.65-2.73(m,3H),1.87-1.96(m,2H),1.76-1.85(m,2H),1.57-1.68(m,2H).

### Embodiment 57B

### (E)-2-(2-chloropyridin-4-yl)-5-(5-((1-(hydroxyimino)-2,3-dihydro-1 H-inden-5-yl)-oxy)-pentyl) -1,2,5-thiadiazolidine-1,1-dioxide

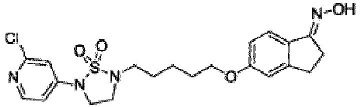

Under the protection of N₂ gas at room temperature, add sodium acetate (711mg, 8.7mmol) into the solution of Embodiment 57A (1.3g, 2.9mmol) and hydroxylamine hydrochloride (603mg, 8.7mmol) in ethanol (30ml). Then heat the reaction solution to 70°C to react for 2 hours to consume the raw materials completely. Concentrate the reaction solution and pour it into water (50ml) and then extract it with ethyl acetate (30ml x 3). Then dry the organic phase with anhydrous Na₂SO₄ before filter and concentrate to obtain the title compound (yellow solid, 1.30g, yield of 97%). ¹H NMP(400MHz,CDCl₃)8.29(d,*J*=5.77Hz,1H),7.53-7.58(m,1H),7.08(dd,*J*=2.26,5.77Hz,1H),7.01(d,*J*=2. 01Hz,1H),6.82(d,*J*=5.02Hz,2H),3.98-4.07(m,2H),3.86(t,*J*=6.40Hz,2H),3.57(t,*J*=6.40Hz,2H),3.20(t,*J*= 7.15Hz,2H),2.94-3.10(m,5H),1.75-1.94(m,4H),1.56-1.71(m,2H).

### Embodiment 57C

### (E)-2-(2-amino-pyridin-4-yl)-5-(5-((1-(ethoxy-imino) -2,3-dihydro-1H-inden-5-yl)-oxy)-pentyl)-1,2,5-thiadiazolidine-1,1-dioxide

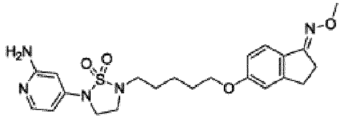

Please refer to the preparation method of Embodiment 56 for this embodiment.
¹H-NMR(400MHz,CDCl₃)7.97(d,J=5.8Hz,1H),7.59(d,*J*=9.3Hz,1H),6.77-6.81(m,2H),6.42(dd,*J*=5.9,1.9 Hz,1H),6.29(d,*J*=1.8Hz,1H),4.52(br.s.,2H),3.99(t,*J*=6.3Hz,2H),3.96(s,3H),3.79(t,*J*=6.4Hz,2H),3.47-3. 52(m,2H),3.16(t,*J*=7.2Hz,2H),2.96-3.01(m,2H),2.85-2.90(m,2H),1.65-1.93(m,4H),1.61-1.64(m,2H). LCMS(ESI)m/z:460(M+1).

### Embodiment 58

### (E)-2-(2-amino-pyridin-4-yl)-5-(5-(1-(isopropoxy)-2,3-dihydro-1H-inden-5-yl)-oxy)-pentyl) -1,2,5-thiadiazolidine-1,1-dioxide

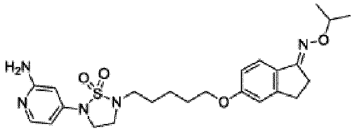

Please refer to the preparation method of Embodiment 56 for this embodiment.
¹H-NMR(400MHz,CDCl₃)7.80(br.s.,1H),7.58(d,*J*=9.0Hz,1H),6.77(br.s.,2H),6.57(br.s.,1H),6.36(br.s.,1 H),4.36-4.41(m,1H),3.96-4.00(m,2H),3.84(br.s.,2H),3.51(br.s.,2H),3.14(t,*J*=6.9Hz,2H),2.93-2.98(m,2 H),2.83-2.88(m,2H),1.80-1.85(m,2H),1.71-1.77(m,2H),1.59(d,*J*=6.8Hz,2H),1.32(d,*J*=6.0Hz,2H),1.24-1.28(m,6H). LCMS(ESI)m/z:488(M+1).

### Embodiment 59

### (E)-2-(2-amino-pyridin-4-yl)-5-(5-((1-ethoxy-imino)-7-fluoro-2,3-dihydro-1H-5-yl)-oxy)-pentyl)-1,2,5-thiadiazolid ine-1,1-dioxide

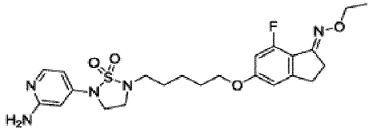

### Embodiment 59A

### 5-bromo-7-fluoro-indan-1 keto-O-ethyl ketoxime

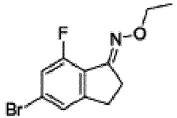

Heat the solution of 5-bromo-7-fluoro-inden-1-one (1.8g, 7.9mmol) and ethoxyamino hydrochloride (1.53g, 15.7mmol) and sodium acetate (1.29g, 15.7mmol) in ethanol (10ml) to 70°C to react for 3 hours. After the raw materials disappear, add water (20ml). Then extract the aqueous phase with ethyl acetate (30ml x 3). Then dry the combined organic phases with anhydrous Na₂SO₄ before filter and concentrate to obtain the title compound (yellow solid, 2.0g, yield of 94%) that is directly further.

### Embodiment 59B

### (E)-7-fluoro-5-(4,4,5,5-tetramethyl-1,3,2-dioxaborolan-2-yl)-2,3-dihydro-1H-inden-1-one-O-ethyl ketoxime

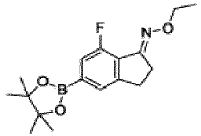

Under the protection of N₂ gas, heat the solution of Embodiment 59A (2.00g, 7.35mmol), bis(pinacolato)diboron (2.24g, 8.82mmol), potassium acetate (2.16g, 22.05mmol) and Pd(dppf)Cl₂ (537.80mg, 735.00µmol) in dioxane (5ml) to 50°C to react for 10 hours. Cool the reaction solution to room temperature before filter out the crude product that can be directly used further.

### Embodiment 59C

### (E)-7-fluoro-5-hydroxy-2,3-dihydro-1 H-inden-1-one-oxo-ethyl ketoxime

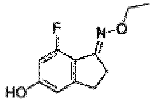

Add hydrogen peroxide (2.3g, 65.5mmol) into the mixture solution of Embodiment 59B (2.0g, 6.6mmol) in NaOH (2ml, 2N) and THF (8ml). Mix the mixture solution 10 minutes at 25°C before add water (5.0ml). Extract this mixture solution with ethyl acetate (10ml x 3). Dry the organic phase with anhydrous Na₂SO₄. Then filter and concentrate it before purify the crude product with the preparative TLC (petroleum ether: ethyl acetate=2:1) to obtain the title compound (yellow solid, 1.20g, yield of 88%). ¹H
NMR(400MHz,CDCl₃)6.56(m,1H),6.48-6.46(m,1H),4.24-4.18(m,2H),4.14-4.09(m,2H),2.96-2.89(m,2 H),1.31-1.23(m,3H).

### Embodiment 59D

### (E)-2-(2-chloropyridin-4-yl)-5-(5-((1-(ethoxy-imino)-7-fluoro-2,3-dihydro-1 H-indene-5-yl)-oxy)-pentyl) -1,2,5-thiadiazolidine-1,1-dioxide

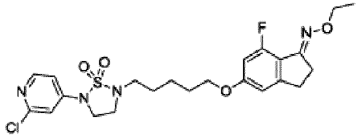

Add KI (8mg, 50µmol) and K₂CO₃ (139mg, 1.0mmol) into the solution of Embodiment 52A (192mg, 502µmol) and Embodiment 59C (105mg, 502µmol) in acetone solution (5ml). Agitate the mixture solution for 15 hours at 70°C to allow the raw materials to react completely. Add water (5.0ml) and extract with ethyl acetate (10ml x 3). Dry the organic phase with anhydrous Na₂SO₄ before filter and evaporate to obtain the title compound (yellow solid, 220mg, yield of 86%). LCMS(ESI)m/z:511(M+1).

### Embodiment 59E

### (E)-2- (2-amino-pyridin-4-yl)-5- (5-((1-(ethoxy-imino)-7-fluoro-2,3-dihydro-1H-indene-5-yl)-oxy)-pentyl)-1,2,5-thiadiazolidine-1,1-dioxide

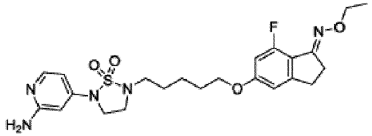

Add Xantphos (23mg, 40µmol) and K₂CO₃ (83mg, 599µmol) into the solution of Embodiment 59D (102mg, 199µmol), tert-butyl carbamate (222mg, 1.9mmol), Pd₂(dba)₃ (18mg, 20µmol) in dioxane (5.0ml). Agitate the reaction solution 16 hours at 100°C. Then filter and concentrate the reaction solution to purify with the preparative HPLC (neutral) to obtain the title compound (white solid, 52mg, yield of 53%). ¹H NMR(400MHz,CHLOROFORM-d)7.97(d,*J*=6.0Hz,1H),6.60(s,1H),6.51(d,*J*=11.5Hz,1H),6.42(d,*J*=4.0 Hz,1H),6.29(s,1H),4.56(br.s.,2H),4.23(q,*J*=7.0Hz,2H),3.50(t,*J*=6.5Hz,2H),3.16(t,*J*=7.3Hz,2H),2.99(d, *J*=8.0Hz,2H),2.93(d,*J*=8.5Hz,2H),1.88-1.80(m,2H),1.80-1.72(m,2H),1.32(t,*J*=7.0Hz,3H). LCMS(ESI)m/z:492(M+1).

### Embodiment 60

### 2-(2-chloro-4-pyridyl)-5-(5-(4-(2-isopropyl-tetrazol-5-yl)-phenoxy)-pentyl)-1,2,5-thiadiazolidine-1,1-dioxide

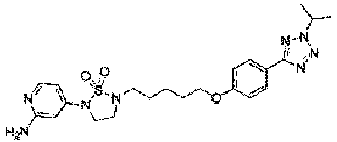

### Embodiment 60A

### 2-isopropyl-5-(4-methoxyphenyl)-tetrazole

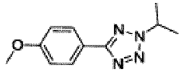

At 0°C, add K₂CO₃ (4.7g, 34mmol) and isopropyl iodide (2.92g, 17mmol) into the solution of Embodiment 11A (3g, 17mmol) in acetonitrile (30ml). After the substances are added, heat the mixture solution for 5 hours at 100°C. Cool the reaction solution to room temperature and then concentrate under vacuum before purity with column chromatography (petroleum ether: ethyl acetate = 10:1) to obtain the title compound (white solid, 2.1g, yield of 60%). ¹H NMR(400MHz,CDCl₃)8.08(d,*J*=8.5Hz,2H),7.00(d,*J*=8.5Hz,2H),4.68(q,*J*=7.2Hz,2H),3.87(s,3H),1.68(t, *J*=7.5Hz,3H). LCMS(ESI)m/z:205(M+1).

### Embodiment 60B

### 4-(2-isopropyl-2H-tetrazol-5-yl)-phenol

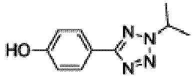

Add the solution of HBr+CH3COOH (20ml) into Embodiment 60A (1g, 4.6mmol). Heat this mixture solution for 24 hours at 110°C. Then cool the reaction solution and add it into ice water. Filter the aqueous phase out to obtain the solid as the title compound (white solid, 0.7g, yield of 75%). ¹H NMR(400MHz,CDCl₃)8.08(d,*J*=8.5Hz,2H),7.00(d,*J*=8.5Hz,2H),4.68(q,*J*=7.2Hz,2H),3.87(s,2H),1.68(t, *J*=7.5Hz,3H). LCMS(ESI)m/z:191(M+1).

### Embodiment 60C

### 2-(2-chloro-4-pyridyl)-5-(5-(4-(2-isopropyl-tetrazol-5-yl)-phenoxy)-pentyl)-1,2,5-thiadiazolidine-1,1-dioxide

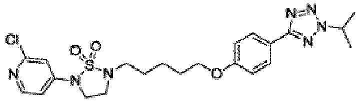

At 0°C, add K₂CO₃ (270mg, 2mmol) and KI (81 mg, 0.49mmol) into the solution of Embodiment 52A (200mg, 0.52mmol) in acetone (3ml). Then add Embodiment 60B (449mg, 2.2mmol) into the mixture solution above. Agitate it to react for 15 hours at 80°C. Then add water to quench the reaction and extract the aqueous phase with ethyl acetate. Dry the combined organic phases with anhydrous Na₂SO₄ before filter and evaporate to obtain the title compound (white solid, 200mg, yield of 74%). ¹H NMR(400MHz,CDCl3)8.28(d,*J*=6.0Hz,1H),8.09(d,*J*=8.8Hz,2H),7.08(dd,*J*=5.8,2.0Hz,1H),6.96-7.02(m ,3H),5.10(dt,*J*=13.5,6.7Hz,1H),4.06(t,*J*=6.1Hz,2H),3.86(t,*J*=6.3Hz,2H),3.57(t,*J*=6.3Hz,2H),3.51(s,2H) ,3.21(t,*J*=7.2Hz,2H),1.86-1.93(m,2H),1.78-1.84(m,2H),1.72(s,3H),1.70(s,3H).
LCMS(ESI)m/z:506(M+1).

### Embodiment 60D

### 2-(2-chloro-4-pyridyl)-5-(5-(4-(2-isopropyl-tetrazol-5-yl)-phenoxy)-pentyl)-1,2,5-thiadiazolidine-1,1-dioxide

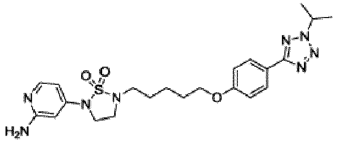

At 0°C, add cesium carbonate (386mg, 1.2mmol) and tert-butyl carbamate (93mg, 0.79mmol) into the solution of Embodiment 60C (200mg, 0.40mmol) in N,N-dimethylformamide (1ml). Under the protection of N₂ gas, add Pd₂(dba)₃ (19mg, 20µmol) and Xantphos (12mg, 20µmol). Agitate it to react for 15 hours at 110°C. Then add water to quench the reaction and extract the aqueous phase with ethyl acetate. Dry the combined organic phase with anhydrous Na₂SO₄ before filter and evaporate. Then purify it with the preparative HPLC to obtain the title compound (white solid, 40mg, yield of 20%). ¹H
NMR(400MHz,CDCl₃)8.08(d,*J*=8.5Hz,2H),7.99(d,*J*=5.3Hz,1H),7.00(d,*J*=8.8Hz,2H),6.44(d,*J*=4.0Hz,1 H),6.30(s,1H),5.10(dt,*J*=13.4,6.7Hz,1H),4.54(br.s.,2H),4.05(t,*J*=6.1Hz,2H),3.80(t,*J*=6.3Hz,2H),3.47-3 .54(m,2H),3.18(t,*J*=7.2Hz,2H),1.88(d,*J*=7.5Hz,2H),1.76-1.82(m,2H),1.71(d,*J*=6.8Hz,6H),1.61-1.68(m ,2H). LCMS(ESI)m/z:487(M+1).

### Embodiment 61

### 2-(2-amino-pyridin-4-yl)-5-(5-(4-(2-methyl--2H-tetrazol-5-yl)-phenoxy)-pentyl)-1,2,5-thiadiazolidine-1,1-dioxide

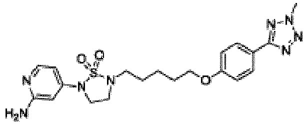

Please refer to the preparation method of Embodiment 60 for this embodiment. ¹H NMR(400MHz,DMSO-d₆)7.95(d,*J*=8.78Hz,2H),7.80-7.89(m,1H),7.09(d,*J*=8.78Hz,2H),6.59(br.s.,2H), 6.41(d,*J*=4.27Hz,1H),6.25(s,1H),4.38(s,3H),4.04(t,*J*=6.27Hz,2H),3.84(t,*J*=6.40Hz,2H),3.52(t,*J*=6.27H z,2H),3.07(t,*J*=7.03Hz,2H),1.62-1.83(m,4H),1.44-1.56(m,2H). LCMS(ESI)m/z:459(M+1).

### Embodiment 62

### 2-(2-amino-pyridin-4-yl)-5-(5-((3-ethyl-benzo[d]6-yl)-oxy)-pentyl)-1,2,5-thiadiazolidine-1,1-dioxide

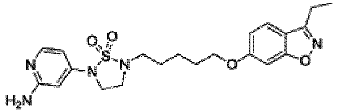

### Embodiment 62A

### 3-ethyl-benzo[d]isoxazole-6-ol

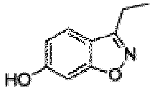

Please refer to the preparation method of Tetrahedron.Lett.2006, 8247-8250 for this embodiment. ¹H NMR(400MHz,CDCl₃)7.50-7.48(m,1H),7.00(m,1H),6.85-6.82(m,1H),2.94(m,2H),1.45(t,J=8.0Hz,3H).

### Embodiment 62B

### 2-(2-chloro-4-yl)-5-(5-((3-ethyl-benzo[d]6-yl)-oxy)-pentyl)-1,2,5-thiadiazolidine-1,1-dioxide

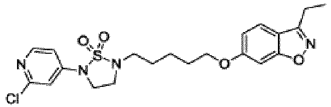

Add K₂CO₃ (36mg, 0.26mmol) and KI (4.3mg, 0.026mmol) into the mixture solution of Embodiment 52A (100mg, 0.26mmol) and Embodiment 62A (42.6mg, 0.26mmol) in acetone (10ml). Then allow it to react for 10 hours at 50°C. Filter and evaporate the reaction solution. Then purify the residue with HPLC to obtain the title compound (white solid, 100mg, yield of 82%). ¹H NMR(400MHz,CDCl₃)δ8.28(d,*J*=5.77Hz,1H),7.54(d,*J*=8.53Hz,1H),7.08(dd,*J*=2.26,5.77Hz,1H),7.01(d ,*J*=2.01Hz,2H),6.89(dd,*J*=2.38,8.66Hz,1H),4.02(t,*J*=6.15Hz,2H),3.86(t,*J*=6.40Hz,2H),3.57(t,*J*=6.40H z,2H),3.15-3.24(m,2H),2.91-2.97(m,2H),1.85-1.98(m,2H),1.74-1.84(m,2H),1.58-1.69(m,2H),1.45(t,*J*= 7.65Hz,3H).

### Embodiment 62C

### 2- (2-amino-pyridin-4-yl)-5-(5-((3-ethyl-benzo[d] isoxazol-6-yl)-oxy)-pentyl)-1,2,5-thiadiazolidine-1,1-dioxide

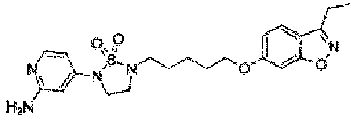

Under the protection of N₂ gas, add Cs₂CO₃ (189mg, 0.58mmol), Xantphos (22mg, 0.038mmol) and Pd₂(dba)₃ (18mg, 0.019mmol) into the solution of Embodiment 62B (90mg, 0.193mmol) and tert-butyl carbamate (136mg, 0.116mmol) in dioxane (2ml). Then allow it to react for 10 hours at 110°C. Then pour the reaction solution into water and extract with ethyl acetate (100ml x 3). Then mix the organic phases and dry with Na₂SO₄. Then filter and evaporate before purify the residue with the preparative HPLC to obtain the title compound (white solid, 20mg, yield of 23%). ¹H NMR(400MHz,CDCl₃)7.98(d,*J*=5.52Hz,1H),7.50-7.56(m,1H),7.00(d,*J*=2.01Hz,1H),6.85-6.90(m,1H),6 .42(d,*J*=6.02Hz,1H),6.30(s,1H),4.01(t,*J*=6.27Hz,2H),3.76-3.84(m,2H),3.51(t,*J*=6.27Hz,2H),3.17(t,*J*=7 .28Hz,2H),2.93(d,*J*=7.53Hz,2H),1.84-1.97(m,2H),1.79(t,*J*=7.53Hz,2H),1.64(d,*J*=7.03Hz,2H),1.43(t,*J* =7.78Hz,3H). LCMS(ESI)m/z:446(M+1).

### Embodiment 63

### 4-(5- (5- (4- (5-ethyl-1,3,4-oxadiazol-2-yl)-phenoxy)-pentyl) -1,1-dioxo-1,2,5-thiadiazolidine-2-yl)-pyridin-2-amine

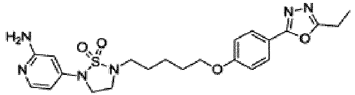

### Embodiment 63A

### 4-(5-ethyl-1,3,4-oxadiazol-3-yl)-phenol

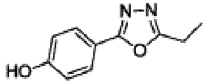

Add methanesulfonic acid (63mg, 658µmol) into 4-hydroxy benzoyl hydrazine (500mg, 3.3mmol) and triethyl orthoformate (579mg, 3.3mmol) in dioxane (10ml). Then allow it to react 1 hour at 110°C. Filter the reaction system before filter and evaporate to obtain the title compound (yellow solid, 150.00mg, yield of 24%). ¹H
NMR(400MHz,CDCl₃)7.79(d,*J*=9.03Hz,2H),6.87(d,*J*=8.53Hz,2H),2.87(q,*J*=7.53Hz,2H),1.36(t,*J*=7.53 Hz,3H).

### Embodiment 63B

### 2-(2-chloro-4-pyridyl)-5- (5- (4-(5-ethyl-1,3,4-oxadiazol-2-yl)-phenoxy)-pentyl)-1,2,5-thiadiazolidine-1,1-dioxide

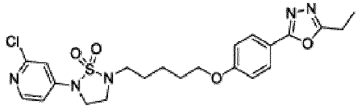

Add K₂CO₃ (29mg, 0.02mmol) and KI (3.5mg, 2µmol) into the mixture solution of Embodiment 52A (80mg, 0.02mmol) and Embodiment 63A (47.71mg, 0.25mmol) in N,N-dimethylformamide (2ml). Allow it to react for 10 hours at 80°C and then pour the reaction solution into water (100ml). Extract it with ethyl acetate (100ml x 3). Mix the organic phases and dry it with anhydrous Na₂SO₄ before filter and evaporate. Then pass it through column to obtain the title compound (yellow solid, 100mg, yield of 97%). ¹H NMR(400MHz,CDCl₃)8.22(d,*J*=5.77Hz,1H),7.91(d,*J*=8.78Hz,2H),7.02(dd,*J*=2.26,5.77Hz,1H),6.98(d, *J*=1.76Hz,1H),6.94(d,*J*=9.03Hz,2H),3.95-4.04(m,2H),3.83(t,*J*=6.40Hz,3H),3.54(t,*J*=6.40Hz,3H),3.11-3.22(m,3H),2.87-2.90(m,2H),1.79-1.90(m,2H),1.69-1.79(m,2H),1.52-1.64(m,2H),1.39(t,*J*=7.65Hz,3H)

### Embodiment 63C

### 4-(5- (5- (4- (5-ethyl-1,3,4-oxadiazol-2-yl)-phenoxy)-pentyl) -1,1-dioxo-1,2,5-thiadiazolidine-2-yl)-pyridin-2-amine

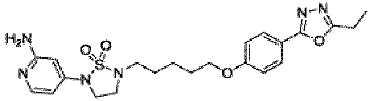

Under the protection of N₂ gas, add Pd₂(dba)₃ (15mg, 0.016mmol), cesium carbonate (106mg, 0.032mol), and Xantphos (19mg, 3µmol) into the solution of Embodiment 63B (80mg, 0.16mmol) and tert-butyl carbamate (114mg, 0.40mmol) in dioxane (1ml). Then allow it to react for 10 hours at 110°C. After the reaction ends, pour the reaction solution into water (20ml) and extract with ethyl acetate (10ml x 3). Then dry the organic phase with anhydrous Na₂SO₄ before filter and evaporate. Then purify the residue with the preparative HPLC to obtain the title compound (white solid, 25mg, yield of 33%). ¹H NMR(400MHz,CDCl₃)7.92-8.02(m,3H),6.98(d,*J*=9.03Hz,2H),6.43(dd,*J*=1.76,5.77Hz,1H),6.30(d,*J*=1. 51Hz,1H),4.57(br.s.,1H),4.05(t,*J*=6.02Hz,2H),3.73-3.87(m,2H),3.51(t,*J*=6.53Hz,2H),3.18(t,*J*=7.28Hz, 2H),2.95(q,*J*=7.53Hz,2H),1.84-1.94(m,2H),1.75-1.83(m,2H),1.65-1.69(m,2H),1.44(t,*J*=7.53Hz,3H). LCMS(ESI)m/z:473(M+1).

### Embodiment 64

### 2-(2-amino-pyridin-4-yl)-5-(5- (4-(2-ethyl-2H-1,2,3-triazol-4-yl)-phenoxy)-pentyl)-1,2,5-thiadiazolidine-1,1-dioxide

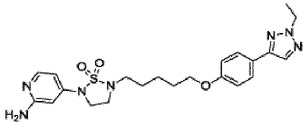

### Embodiment 64A

### 4-(4-methoxyphenyl)-1,2,3-triazole

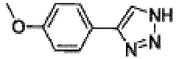

Under the protection of N₂ gas, add trimethylsilyl azide (2.62g, 22.7mmol) into the solution of CuI (144g, 757mmol), 4-methoxyphenyl acetylene (2g, 15.1mmol) in N,N-dimethylformamide (5.4ml) and methanol (0.6ml). Agitate the mixture solution 16 hours at 100°C. Then filter and evaporate before purify the residue with column chromatography (petroleum ether: ethyl acetate =10:1, petroleum ether: ethyl acetate =2:1) to obtain the title compound (yellow solid, 2g, yield of 75%). ¹H NMR(400MHz,CDCl₃)7.88(s,1H),7.75(d,*J*=8.5Hz,2H),6.98(d,*J*=8.5Hz,2H),3.85(s,3H).

### Embodiment 64B

### 2-ethyl-4 (4-methoxyphenyl)-1,2,3-triazole

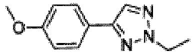

Allow the mixture of Embodiment 64A (1g, 5.71 mmol), ethyl iodide (2.67g, 17.13mmol) and K₂CO₃ (1.58g, 11.42mmol) in acetonitrile (10ml) to react 16 hours at 70°C. Add water (10ml) into the reaction system. Extract the aqueous layer with ethyl acetate (10ml x 3) and then dry the combined organic layers with Na₂SO₄ before filter and evaporate. Then purify the residue with column chromatography (petroleum ether: ethyl acetate = 5:1) to obtain the title compound (yellow liquid, 1g, yield of 86%). ¹H NMP(400MHz,CDCl₃)7.62-7.73(m,3H),6.92(d,*J*=8.5Hz,2H),4.45(q,*J*=7.5Hz,2H),3.81(s,3H),1.56(t,*J*= 7.3Hz,3H).

### Embodiment 64C

### 4-(2-ethyl-2H-1,2,3-triazol-4-yl)-phenol

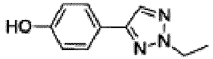

At -78°C, add BBr₃ (986mg, 3.94mmol) into the solution of Embodiment 64B (200mg, 984µmol) in dichloromethane (5ml). Allow the mixture to react 1 hour at 25°C. Then add water (5ml) into the reaction system and extract the aqueous layer with dichloromethane (10ml x 3). Then filter and evaporate to obtain the title compound (yellow solid, 160mg, yield of 86%). ¹H NMR(400MHz,CDCl₃)δ7.74(s,1H),7.66(d,*J*=8.5Hz,2H),6.89(d,*J*=8.5Hz,2H),4.46-4.52(m,2H),1.59(t,*J* =7.3Hz,3H).

### Embodiment 64D

### 2-(2-chloropyridin-4-yl)-5-(5-(4-(2-ethyl-2H-1,2,3-triazol-4-yl)-phenoxy)-pentyl)-1,2,5-thiadiazolidine-1,1-dioxide

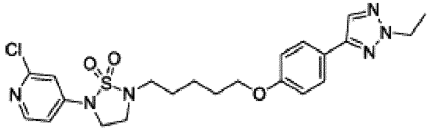

Please refer to the preparation method of Embodiment 63 for this embodiment. ¹H NMP(400MHz,CDCl₃)8.26(d,*J*=6.0Hz,1H),7.74(s,1H),7.69(d,*J*=9.0Hz,2H),7.06(dd,*J*=5.5,2.0Hz,1H),6 .99(d,*J*=2.0Hz,1H),6.93(d,*J*=8.5Hz,2H),4.49(q,*J*=7.4Hz,2H),4.01(t,*J*=6.3Hz,2H),3.83(t,*J*=6.5Hz,2H),3 .55(t,*J*=6.5Hz,2H),3.18(t,*J*=7.0Hz,2H),1.83-1.90(m,2H),1.75-1.82(m,2H),1.62-1.67(m,2H),1.61(d,*J*=4. 0Hz,3H). LCMS(ESI)m/z:492(M+1).

### Embodiment 64E

### 2-(2-amino-pyridin-4-yl)-5-(5-(4-(2-ethyl--2H-[1,2,3] triazol-4-yl)-phenoxy)-pentyl)-1,2,5-thiadiazolidine-1,1-dioxide

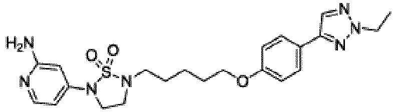

Under the protection of N₂ gas, add Xantphos (8mg, 13.8µmol) and Pd₂(dba)₃ (9mg, 9.8µmol) into the solution of Embodiment 64D (80mg, 163µmol), tert-butyl carbamate (115mg, 978µmol) and Cs₂CO₃ (106mg, 326µmol) in dioxane (3ml) and N,N-dimethylformamide (0.5ml). Allow the mixture to react 16 hours at 110°C. Then add water (10ml) into the reaction system and extract the aqueous layer with dichloromethane (10ml x 3). Then filter and evaporate before obtain the title compound from the residue through preparation and separation (brown solid, 30mg, yield of 39%). ¹H NMR(400MHz,CDCl₃)δ7.98(d,*J*=6.0Hz,1H),7.74(s,1H),7.69(d,*J*=8.5Hz,2H),6.94(d,*J*=9.0Hz,2H),6.38-6.44(m,1H),6.29(s,1H),4.49(q,*J*=7.5Hz,4H),4.01(t,*J*=6.0Hz,2H),3.80(t,*J*=6.5Hz,2H),3.47-3.53(m,2H), 3.16(t,*J*=7.3Hz,2H),1.84-1.91(m,2H),1.74-1.80(m,2H),1.64(br.s.,3H). LCMS(ESI)m/z:472(M+1).

### Embodiment 65

### (E)-2-(2-amino-pyridin-4-yl)-5-(5-((5(ethoxymethylene)-5,6,7,8-tetrahydronaphthalen-2-yl)-oxy)-pentyl) -1,2,5-thiadiazolidine-1,1-dioxide

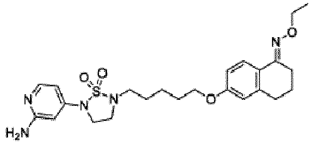

### Embodiment 65A

### (E)-6-hydroxy-3,4-dihydro-naphthalene-1(2H)-one-O-ethyl ketoxime

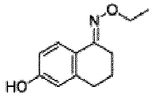

Add ethoxyamino hydrochloride (1.88g, 30.9mmol) and sodium acetate (2.5g, 30.9mmol) into the solution of 6-hydroxy-1-tetralone (1g, 6.2mmol) in water (10ml). Agitate the mixture solution 2 hours at 80°C. Then extract it with ethyl acetate. Dry the organic phase with anhydrous Na₂SO₄ before filter and evaporate to obtain the title compound (yellow solid, 0.8g, yield of 63%). LCMS(ESI)m/z:206(M+1).

### Embodiment 65B

### (E)-2-(2-chloropyridin-4-yl)-5-(5-((5-(ethoxymethylene)-5,6,7,8-tetrahydronaphthalen-2-yl)-oxy)-pentyl) -1,2,5-thiadiazolidine-1,1-dioxide

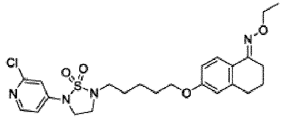

Add K₂CO₃ (72mg, 0.52mmol), KI (9mg, 0.05mmol) and Embodiment 64A (53.6mg, 0.26mmol) into the solution of Embodiment 52A (0.1g, 0.26mmol) in N,N-dimethylformamide (2ml). Agitate the mixture solution for 15 hours at 80°C. Then extract it with ethyl acetate. Dry the organic phase with anhydrous Na₁SO₄ before filter and evaporate. Then purify the residue with column chromatography (petroleum ether: ethyl acetate =1:1) to obtain the title compound (white solid, 60mg, yield of 45%). ¹H NMR(400MHz,CDCl₃)8.28
(d,*J*=5.8Hz,1H),7.92(d,*J*=8.8Hz,1H),7.07(dd,*J*=5.8,2.0Hz,1H),7.01(d,*J*=2.0Hz,1H),6.74(dd,*J*=8.8,2.5 Hz,1H),6.63(d,*J*=2.3Hz,1H),4.21(q,*J*=7.0Hz,2H),3.99(t,*J*=6.3Hz,2H),3.85(t,*J*=6.3Hz,2H),3.56(t,*J*=6.4 Hz,2H),3.15-3.22(m,2H),2.67-2.77(m,4H),1.84(dt,*J*=12.1,6.4Hz,6H),1.58-1.66(m,2H),1.33(t,*J*=7.0Hz, 3H). LCMS(ESI)m/z:507(M+1).

### Embodiment 65C

### (E)-2-(2-amino-pyridin-4-yl)-5-(5-((5-(ethoxymethylene)-5,6,7,8-tetrahydronaphthalen-2-yl)-oxy)-pentyl) -1,2,5-thiadiazolidine-1,1-dioxide

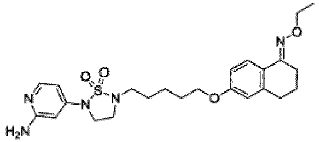

At 0°C, add Cs₂CO₃ (77mg, 0.24mmol), tert-butyl carbamate (15mg, 0.13mmol) into the solution of Embodiment 65B (60mg, 0.12mmol) in N,N-dimethylformamide (1ml). After replace with N₂ gas, under the protection of N₂ gas, add Pd₂(dba)₃ (22mg, 23.7mmol) and Xantphos (14mg, 23.7mmol). Agitate it to react for 15 hours at 110°C. Then add water to quench the reaction and extract the aqueous phase with ethyl acetate. Dry the combined organic phase with anhydrous Na₂SO₄ before filter and evaporate. Then purify it with the preparative HPLC to obtain the title compound (white solid, 15mg, yield of 26%). ¹H NMR(400MHz,CDCl₃)7.91(d,*J*=8.5Hz,1H),7.62(d,*J*=7.0Hz,1H),6.68-6.75(m,2H),6.62(d,*J*=2.0Hz,1H), 6.18(s,1H),4.20(q,*J*=7.0Hz,2H),3.98(t,*J*=6.3Hz,2H),3.84(t,*J*=6.3Hz,2H),3.57(t,*J*=6.3Hz,2H),3.19(t,*J*=7 .0Hz,2H),2.65-2.76(m,4H),1.81-1,87(m,4H),1,76(d,*J*=7.0Hz,2H),1.57-1,63(m,2H),1.32(t,*J*=7.0Hz,3H). LCMS(ESI)m/z:488(M+1).

### Embodiment 66

### 2-(2-amino-pyridin-4-yl)-5-(5-((2-propyl-benzo[d]oxazole-6-yl)-oxy)-pentyl)-1,2,5-thiadiazolidine-1,1-dioxide

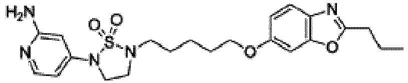

### Embodiment 66A

### 2-propyl-benzo[d]oxazole-6-ol

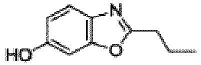

Please refer to the operation of Patent (WO 2005037814)LCMS(ESI)m/z:178(M+1) for this embodiment.

### Embodiment 66B

### 2-(2-chloro-4-yl)-5-(5-((2-propyl-benzo[d]oxazole-6-yl)-oxy)-pentyl)-1,2,5-thiadiazolidine-1,1-dioxide

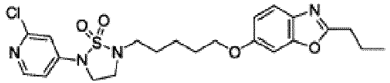

Add K₂CO₃ (72mg, 0.52mmol), KI (9mg, 0.05mmol) and 2-propyl-6-hydroxybenzoxazole (56ml, 0.3mmol) into the solution of Embodiment 52A (0.1g, 0.26mmol) in N,N-dimethylformamide (2ml). Agitate the mixture solution for 15 hours at 80°C. Then extract it with ethyl acetate. Dry the organic phase with anhydrous Na₁SO₄ before filter and evaporate. Then purify the residue with column chromatography (petroleum ether: ethyl acetate =1:1) to obtain the title compound (white solid, 50mg, yield of 40%). LCMS(ESI)m/z:479(M+1).

### Embodiment 66C

### 2-(2-amino-pyridin-4-yl)-5-(5-((2-propyl-benzo[d]oxazole-6-yl)-oxy)-pentyl)-1,2,5-thiadiazolidine-1,1-dioxide

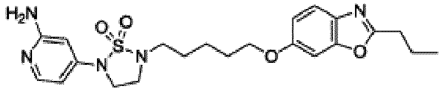

At 0°C, add Cs₂CO₃ (68mg, 0.21mmol), tert-butyl carbamate (15mg, 0.13mmol) into the solution of Embodiment 66B (50mg, 0.10mmol) in N,N-dimethylformamide (1ml). After replace with N₂ gas, under the protection of N₂ gas, add Pd₂(dba)₃ (19mg, 21 µmol) and Xantphos (12mg, 20µmol). Agitate it to react for 15 hours at 110°C. Then add water to quench the reaction and extract the aqueous phase with ethyl acetate. Dry the combined organic phases with anhydrous Na₂SO₄ before filter and evaporate. Then purify it with the preparative HPLC to obtain the title compound (white solid, 8mg, yield of 17%). ¹H NMR(400MHz,CDCl₃)7.92(d,*J*=6.3Hz,1H),7.54(d,*J*=8.5Hz,1H),7.02(d,*J*=2.3Hz,1H),6.90(dd,*J*=8.8,2.3 Hz,1H),6.50(d,*J*=5.8Hz,1H),6.33(s,1H),4.03(t,*J*=6.3Hz,2H),3.85(t,*J*=6.4Hz,2H),3.54(t,*J*=6.3Hz,2H),3. 19(t,*J*=7.2Hz,2H),2.89(t,*J*=7.5Hz,2H),1.87-1.94(m,4H),1.80(q,*J*=7.3Hz,2H),1.61-1.70(m,2H),1.06(t,*J* =7.4Hz,3H). LCMS(ESI)m/z:460(M+1).

### Embodiment 67

### 2-(2-amino-pyridin-4-yl)-5-(5-(4-(5-ethylisoxazole-3-yl)-phenoxy)-pentyl)-1,2,5-thiadiazolidine-1,1-dioxide

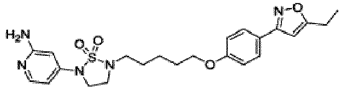

### Embodiment 67A

### (E)-4-hydroxybenzaldehyde oxime

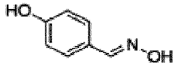

Add sodium acetate (20g, 246mmol) and hydroxylamine hydrochloride (17mg, 246mmol) into the solution of 4-hydroxybenzaldehyde (10g, 82mmol) in ethanol (100ml). Heat the mixture solution for 15 hours at 70°C. Cool it to room temperature before add water and then extract with dichloromethane. Then dry the organic phase through rotation and separate through column (petroleum ether: EA = 3:1) to obtain the solid as the title compound (white solid, 7g, yield of 62%). LCMS(ESI)m/z:138(M+1).

### Embodiment 67B

### 4- (5-ethyl-3-yl) phenol

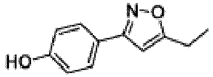

At 0°C, add chlorosuccinimide (2.9g, 22mmol) into the solution of Embodiment 67A (3g, 22mmol) in 1,2-dichloroethane (30ml). After allow it to react 1 hour at room temperature, add 1-butyne (1.2g, 22mmol) in ice bath. After the substance is added, heat the mixture solution 2 hours at 20°C. Also, add triethylamine (2.2g, 22mmol) into the reaction solution in ice bath. After the substance is added, heat the mixture solution for 15 hours at 20°C. Cool the reaction solution to room temperature and then concentrate under vacuum before purity with column chromatography (petroleum ether: ethyl acetate = 10:1) to obtain the title compound (white solid, 1g, yield of 24%). LCMS(ESI)m/z:190(M+1).

### Embodiment 67C

### 2-(2-chloropyridin-4-yl)-5-(5-(4-(5-ethyl isoxazol-3-yl)-phenoxy)-pentyl) -1,2,5-thiadiazolidine-1,1-dioxide

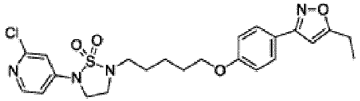

Add K₂CO₃ (72mg, 0.5mmol), KI (9mg, 0.05mmol) and Embodiment 67B (59mg, 0.3mmol) into the solution of Embodiment 52A (0.1g, 0.26mmol) in N,N-dimethylformamide (2ml). Agitate the mixture solution for 15 hours at 80°C. Then extract it with ethyl acetate. Dry the organic phase with anhydrous Na₂SO₄ before filter and evaporate. Then purify the residue with column chromatography (petroleum ether: ethyl acetate =2:1) to obtain the title compound (white solid, 60mg, yield of 49%). LCMS(ESI)m/z:492(M+1).

### Embodiment 67D

### 2-(2-amino-pyridin-4-yl)-5-(5-(4-(5-ethylisoxazole-3-yl)-phenoxy)-pentyl)-1,2,5-thiadiazolidine-1,1-dioxide

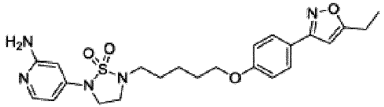

At 0°C, add cesium carbonate (66.36mg, 0.202mmol) and tert-butyl carbamate (23.26mg, 0.202mmol) into the solution of Embodiment 67C (50mg, 0.101 mmol) in N,N-dimethylformamide (1.5ml). After replace with N₂ gas, under the protection of N₂ gas, add Pd₂(dba)₃ (18.57mg, 20.28mmol) and Xantphos (11.74mg, 20.28mmol). Agitate it to react for 15 hours at 110°C. Then add water to quench the reaction and extract the aqueous phase with ethyl acetate. Dry the combined organic phases with anhydrous Na₂SO₄ before filter and evaporate to obtain the title compound (white solid, 3mg, yield of 6.25%). ¹H NMR(400MHz,CDCl₃)7.98(d,*J*=5.5Hz,1H),7.72(d,*J*=8.5Hz,2H),6.95(d,*J*=8.5Hz,2H),6.39-6.44(m,1H), 6.29(s,1H),6.23(s,1H),4.48(br.s.,2H),4.03(t,*J*=6.0Hz,2H),3.79(t,*J*=6.5Hz,2H),3.50(t,*J*=6.3Hz,2H),3.17 (t,*J*=7.3Hz,2H),2.81(q,*J*=7.5Hz,2H),1.82-1.94(m,2H),1.70-1.82(m,2H),1.64(br.s.,2H),1.34(t,*J*=7.8Hz,3 H). LCMS(ESI)m/z:472(M+1).

### Embodiment 68

### 2-(2-amino-pyridin-4-yl)-5-(5-(4-(5 (trifluoromethyl)-1,2,4-oxadiazol-3-yl)-phenoxy)-pentyl)-1,2,5-thiadiazolidine-1,1-dioxide

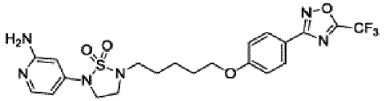

### Embodiment 68A

### 4-(5-(5-(2-chloro-4-pyridyl)-1,1-dioxo-1,2,5-thiadiazolidine-2-yl)-pentyloxy)benzonitrile

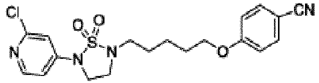

At 0°C, add 4-cyanophenol (187mg, 1.57mmol) into the solution of Embodiment 52A (500mg, 1.31mmol) in N,N-dimethylformamide (5ml). Add K₂CO₃ (362mg, 2.62mmol) into the mixture solution above. Agitate it to react for 15 hours at 80°C. Then add water to quench the reaction and extract the aqueous phase with ethyl acetate. Dry the combined organic phases with anhydrous Na₂SO₄. Then filter and evaporate it before separating it with column (petroleum ether: ethyl acetate=2:1) to obtain the title compound (white solid, 350mg, yield of 63%). LCMS(ESI)m/z:421(M+1).

### Embodiment 68B

### 4-(5-(5-(2-amino-4-pyridyl)-1,1-dioxo-1,2,5-thiadiazolidine-2-yl)-pentyloxy)benzonitrile

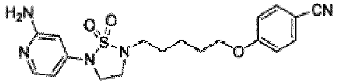

At 0°C, add cesium carbonate (154mg, 0.48mmol) and tert-butyl carbamate (42mg, 0.36mmol) into the solution of Embodiment 68A (100mg, 0.24mmol) in N, N- dimethylformamide (1.5ml). Under the protection of N₂ gas, add Pd₂(dba)₃ (18.57mg, 20.28µmol) and Xantphos (11.7mg, 20µmol). Agitate it to react for 15 hours at 110°C. Then add water to quench the reaction and extract the aqueous phase with ethyl acetate. Dry the combined organic phases with anhydrous Na₂SO₄. Then filter and evaporate it before separating it with column (dichloromethane: methanol=10:1) to obtain the title compound (white solid, 30mg, yield of 31%). LCMS(ESI)m/z:402(M+1).

### Embodiment 68C

### N-(4-(5-(5-(4-cyanophenoxy)-pentyl)-1,1-dioxo-1,2,5-thiadiazolidine-2-yl)-2-pyridyl)tert-butyl carbamate

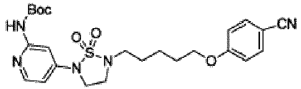

Add N,N-dimethylaminopyridine (45mg, 0.37mmol) and BOC anhydride (489mg, 2.2mmol) into the solution of Embodiment 68B (0.3g, 0.75mmol) in acetonitrile. Heat this mixture solution for 15 hours at 20°C. Then cool and add the reaction solution into ice water. Extract the aqueous phase with ethyl acetate. Dry the combined organic phases with anhydrous Na₂SO₄. Then filter and evaporate it before separating it with column (dichloromethane: methanol=10:1) to obtain the title compound (red oily form, 0.2g, yield of 53%). LCMS(ESI)m/z:502(M+1).

### Embodiment 68D

### N-(4-(5-(5-(4-((Z)-N'-hydroxy-amidino)-phenoxy)-pentyl)-1,1-dioxo-1,2,5-thiadiazolidine-2-yl) -2-pyridinyl) tert-butyl carbamate

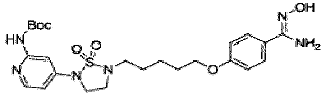

Add triethylamine (40g, 0.4mmol) and hydroxylamine hydrochloride (27.8mg, 0.4mmol) into the solution of Embodiment 68C (0.1g, 0.2mmol) in isopropanol (2ml). Agitate the mixture solution for 5 hours at 80°C. Then extract it with ethyl acetate. Dry the organic phase with anhydrous Na₂SO₄ before filter and evaporate to obtain the title compound (white solid, 70mg, yield of 66%). LCMS(ESI)m/z:535(M+1).

### Embodiment 68E

### 2- (2-amino-pyridin-4-yl)-5-(5- (4-(5 (trifluoromethyl)-1,2,4-oxadiazol-3-yl)-phenoxy)-pentyl)-1,2,5-thiadiazolidine-1,1-dioxide

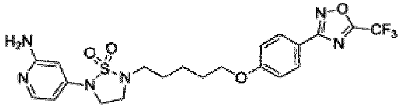

At 0°C, add TFAA (35mg, 0.17mmol) into the solution of Embodiment 68D (60mg, 0.11mmol) in pyridine (1ml). Agitate it to react for 2 hours at 80°C. Then add water to quench the reaction and extract the aqueous phase with ethyl acetate. Dry the combined organic phases with anhydrous Na₂SO₄ before filter and evaporate to obtain the title compound (white solid, 5mg, yield of 7%). ¹H NMR(400MHz,CDCl₃)8.06(d,*J*=8.8Hz,2H),8.00(d,*J*=6.0Hz,1H),7.02(d,*J*=8.8Hz,2H),6.44(dd,*J*=5.8,2.0 Hz,1H),6.31(d,*J*=2.0Hz,1H),4.50(br.s.,2H),4.08(t,*J*=6.3Hz,2H),3.83(t,*J*=6.3Hz,2H),3.53(t,*J*=6.4Hz,2H) ,3.19(t,*J*=7.2Hz,2H),1.87-1.96(m,2H),1.81(quin,*J*=7.4Hz,2H),1.65-1.70(m,2H).
LCMS(ESI)m/z:513(M+1).

### Embodiment 69

### 2-(2-amino-pyridin-4-yl)-5-(5-(4-(5-methyl-1,2,4-oxadiazol-3-yl)-phenoxy)-pentyl-1,2,5-thiadiazolidine-1,1-dioxi de

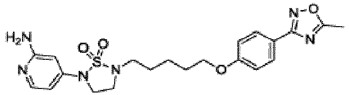

Please refer to the preparation method of Embodiment 68 for this embodiment. ¹H NMR(400MHz,CDCl3)7.96(d,*J*=8.78Hz,1H),7.79(d,*J*=7.28Hz,1H),7.06(d,*J*=8.78Hz,2H),6.80(dd,*J*=2. 51,7.28Hz,1H),6.47(d,*J*=2.26Hz,1H),4.10(t,*J*=6.15Hz,2H),3.97(t,*J*=6.40Hz,2H),3.60-3.71(m,2H),3.22 (t,*J*=7.03Hz,2H),2.65(s,3H),1.85-1.94(m,2H),1.76-1.84(m,2H),1.60-1.70(m,2H). LCMS(ESI)m/z:459(M+1).

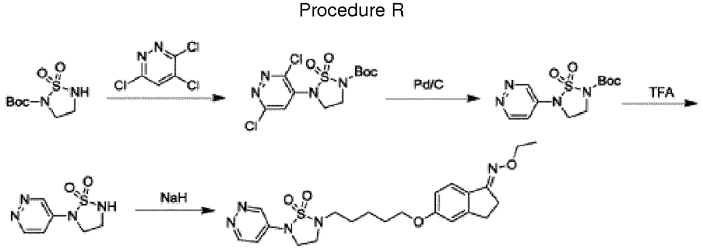

### Embodiment 70

### (E)-2-(5-((1-ethoxy-imino)-2,3-dihydro-1H-inden-5-yl)-oxy)-pentyl)-5-(pyridazin-4-yl)-1,2,5-thiadiazolidine-1,1-dioxide

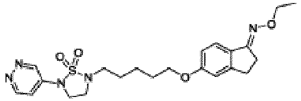

### Embodiment 70A

### 5-(3,6-dichloro-pyridazin-4-yl)-1,2,5-thiadiazolidine-2-tert-butyl carboxylate-1,1-dioxide

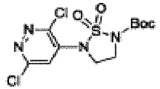

Please refer to the preparation method of Embodiment 32G for this embodiment. ¹H NMR(400MHz,CDCl₃)7.87(s,1H),4.02-4.12(m,4H),1.58(s,10H). LCMS(ESI)m/z:369(M+1).

### Embodiment 70B

### 5-(pyridazin-4-yl)-1,2,5-thiadiazolidine-2-tert-butyl carboxylate-1,1-dioxide

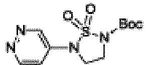

Under the protection of N₂ gas, add sodium acetate (396mg, 4.8mmol) and Pd/C (1g) into the solution of Embodiment 70A (890mg, 2.4mmol) in methanol (40ml). Replace N₂ gas with H₂ gas at 15°C and allow the mixture to react 5 hours at 15 Psi. After TLC indicates the reaction ends, filter the mixture and concentrate the filtrate to obtain the title compound that is yellow solid (520mg, 72%). ¹H NMR(400MHz,CDCl₃)9.13(d,*J*=3.01Hz,1H),9.08(d,*J*=6.02Hz,1H),7.35(dd,*J*=3.01,6.02Hz,1H),4.06-4. 12(m,2H),3.93-3.98(m,2H),1.59(s,9H). LCMS(ESI)m/z:301(M+1).

### Embodiment 70C

### 2-(pyridazin-4-yl)-1,2,5-thiadiazolidine-1,1-dioxide

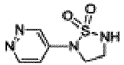

Please refer to the preparation method of Embodiment 32E for this embodiment. ¹H NMR(400MHz,DMSO-d₆)9.07(d,*J*=2.51Hz,1H),9.01(d,*J*=6.02Hz,1H),8.26(br.s.,1H),7.26(dd,*J*=3.01,6. 02Hz,1H),3.96-4.00(m,2H),3.62(br.s.,2H).

### Embodiment 70D

### (E)-2-(5-((1-ethoxy-imino)-2,3-dihydro-1H-inden-5-yl)-oxy)-pentyl)-5-(pyridazin-4-yl)-1,2,5-thiadiazolidine-1,1-dioxide

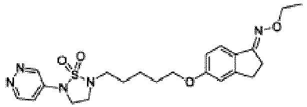

Please refer to the preparation method of Embodiment 32D for this embodiment. ¹H NMR(400MHz,CDCl₃)8.01(br.s.,1H),7.59(dt,*J*=3.26,6.02Hz,2H),6.76-6.85(m,2H),6.56(d,*J*=9.03Hz,1H ),4.20(q,*J*=7.19Hz,2H),3.99(t,*J*=6.27Hz,2H),3.74(t,*J*=6.27Hz,2H),3.46(t,*J*=6.40Hz,2H),3.15(t,*J*=7.28H z,2H),2.95-3.01(m,2H),2.86-2.92(m,2H),1.72-1.89(m,4H),1.56-1.60(m,2H),1.28-1.38(m,3H).
LCMS(ESI)m/z:474(M+1).

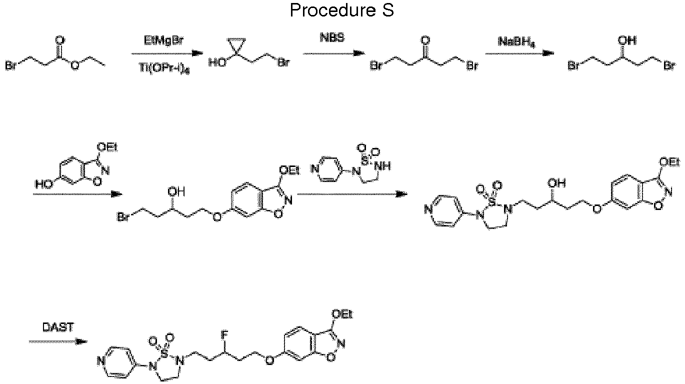

### Embodiment 71

### 2-(5-((3-ethoxy-benzo[d]isoxazole-6-yl)-oxy)-3-fluoro-pentyl)-5-(pyridin-4-yl)-1,2,5-thiadiazolidine-1,1-dioxide

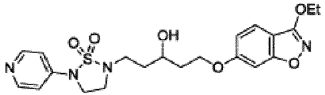

### Embodiment 71A

### 1-(2-bromoethyl)-cyclopropanol

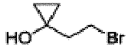

At 0°C, add titanium tetraisopropoxide (7.9g, 28mmol) into the mixture of ethyl 3-bromopropionate (10g, 55mmol) in THF (250ml). Then slowly add ethyl Grignard reagent (14.7g, 110mmol) at 0°C into the reaction system. Agitate it 1 hour and then heat to 25°C before agitate 2 hours. At 0°C, add saturated ammonium chloride aqueous solution into the system. Then extract with ethyl acetate. Then dry the combined organic phases with anhydrous Na₂SO₄. Then filter and evaporate before purify the residue with column chromatography (petroleum ether: ethyl acetate + 10:1 ∼ petroleum ether: ethyl acetate = 5:1) to obtain the title compound (colorless liquid, 4.9g, yield of 54%). ¹H NMR(400MHz,CDCl₃)3.61(t,*J*=7.3Hz,2H),2.26(br.
s.,1H),2.11(t,*J*=7.3Hz,2H),0.78-0.82(m,2H),0.50-0.56(m,2H).

### Embodiment 71B

### 1,5-dibromopentane-3-one

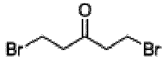

At 0°C, add N-bromosuccinimide (539mg, 3.0mmol) into the solution of Embodiment 71A (500mg, 3.0mmol) in CCl₄ (8ml). Then allow the mixture to react 16 hours at 25°C. Filter and evaporate before purify the residue with column chromatography (petroleum ether: ethyl acetate = 20:1 ∼ petroleum ether: ethyl acetate =10:1) to obtain the title compound (yellow liquid, 500mg, yield of 68%). ¹H NMR(400MHz,CDCl₃)3.56(t,*J*=6.8Hz,2H),3.04(t,*J*=6.8Hz,2H).

### Embodiment 71C

### 1,5-dibromopentane-3-ol

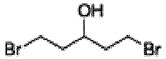

At 0°C, add NaHB₄ (155mg, 4.1mmol) into the solution of Embodiment 71B (500mg, 2.1mmol) in methanol (5ml). Then allow the mixture to react for 3 hours at 25°C. Add water (5ml) into the reaction system. Extract the aqueous layer with dichloromethane (10ml x 3) and then dry the combined organic layer with Na₂SO₄ before filter and evaporate to obtain the title compound (340mg, yield of 67%). ¹H NMR(400MHz,CDCl₃)4.07(d,*J*=4.6Hz,1H),3.51-3.57(m,4H),1.96-2.04(m,4H).

### Embodiment 71D

### 1-bromo-5-((3-ethoxy-benzo[d]isoxazole-6-yl)-oxy)-pentyl-3-ol

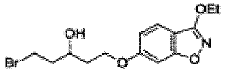

Allow the mixture of Embodiment 71C(329mg, 1.34mmol), Embodiment 12D(150mg, 837µmol) and KI (14mg, 84µmol) in acetone (5ml) to react 16 hours at 60°C. Add water (5ml) and dichloromethane (10ml) into the reaction system. Extract the aqueous layer with dichloromethane (10ml x 3) and then dry the combined organic layers with Na₂SO₄ before filter and evaporate. Then purify the residue with column chromatography (petroleum ether: ethyl acetate = 2:1) to obtain the title compound (colorless solid, 190mg, yield of 66%). ¹H NMR(400MHz,CDCl₃)7.45-7.50(m,1H),6.83-6.88(m,2H),4.43-4.48(m,2H),4.11-4.32(m,3H),3.55-3.65( m,2H),1.94-2.09(m,4H),1.50(t,*J*=7.2Hz,3H).

### Embodiment 71E

### 2-(5-((3-ethoxy-benzo[d]isoxazole-6-yl)-oxy)-3-hydroxy-pentyl)-5-(pyridin-4-yl)-1,2,5-thiadiazolidine-1,1-dioxide

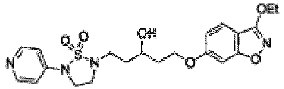

Allow the mixture of Embodiment 71 D (20mg, 58µmol), Embodiment 4F (12mg, 58µmol) and KI (1mg, 5.8µmol) in N,N-dimethylformamide (3ml) to react for 3 hours at 60°C. Add water (5ml) and dichloromethane (10ml) into the reaction system. Extract the aqueous layer with dichloromethane (10ml x 3) and then dry the combined organic layers with Na₂SO₄ before filter and evaporate. Then separate and purify the residue with the preparative column chromatography to obtain the title compound (colorless solid, 8mg, yield of 30%). ¹H NMR(400MHz,CDCl₃)8.64(d,*J*=6.5Hz,2H), 7.48(d,*J*=8.5Hz,1H),7.31(d,*J*=6.5Hz,2H),6.86(d,*J*=4.5Hz,2H),4.45-4.50(m,2H),4.11-4.27(m,2H),3.95-4.01(m,2H),3.71-3.75(m,4H),3.44(dt,*J*=15.6,7.8Hz,2H),2.03(br.s.,4H),1.49-1.53(m,3H). LCMS(ESI)m/z:463(M+1).

### Embodiment 72

### 2-(5-((3-ethoxy-benzo[d]isoxazole-6-yl)-oxy)-3-fluoro-pentyl)-5-(pyridin-4-yl)-1,2,5-thiadiazolidine-1,1-dioxide

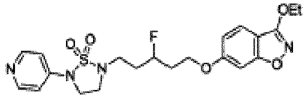

Under the protection of N₂ gas at -78 °C, drop DAST (37mg, 227mmol) into the solution of Embodiment 71E (35mg, 76µmol) in dichloromethane (2ml). Agitate the mixture solution 1 hour at 20°C. Then add water (5ml) into the reaction system. Extract the aqueous layer with dichloromethane (10ml x 3) and then dry the combined organic layers with Na₂SO₄ before filter and evaporate. Then separate and purify the residue with the preparative column chromatography to obtain the title compound (colorless solid, 35mg, yield of 95%). ¹H NMR(400MHz,CDCl₃)8.53(d,*J*=6.3Hz,2H),7.47(d,*J*=9.3Hz,1H),7.07(d,*J*=6.3Hz,2H),6.84-6.87(m,2H), 4.83-5.12(m,1H),4.42-4.49(m,2H),4.06-4.19(m,2H),3.83-3.89(m,2H),3.57-3.62(m,2H),3.31-3.42(m,2 H),1.98-2.29(m,4H),1.48-1.50(m,3H). LCMS(ESI)m/z:465(M+1).

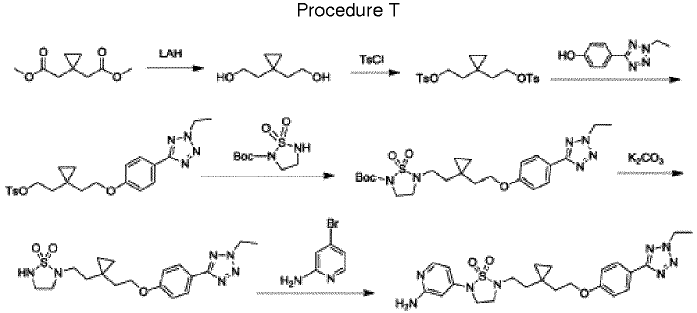

### Embodiment 73

### 2-(2-amino-pyridin-4-yl)-5-(2-(1-(2-(4-(2-ethyl--2H-tetrazol-5-yl)-phenoxy)-ethyl)-cyclic propyl)-ethyl)-1,2,5-thiadiazolidine-1,1-dioxide

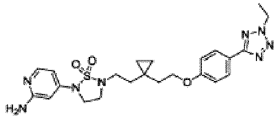

### Embodiment 73A

### Dimethyl 2,2'-(cyclopropane-1,1-diyl)-diacetate

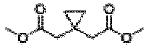

Please refer to the reference (Journal of Medicinal Chemistry,57(2),364-377; 2014) for the preparation method of Embodiment 8A.

### Embodiment 73B

### 2,2'-(cyclopropane-1,1-diyl)-diethanol

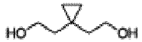

At 0°C, drop Embodiment 72A (249mg, 1.34mmol) into the solution of LiAlH₄ (102mg, 2.68mmol) in THF (3ml). After it is added, agitate the mixture solution 3 hours at 25°C. Add water (0.5ml) and 10% NaOH solution (0.5ml) into the reaction system. Dry the system with Na₂SO₄ before filter and evaporate to obtain the title compound (122mg, yield of 72%).

### Embodiment 73C

### 2,2'-(cyclopropane-1,1-diyl)-di-ethyl-4-methyl-benzenesulfonate

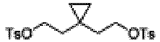

Agitate the mixture solution of Embodiment 73B (1g, 7.9mmol), p-toluensulfonyl chloride (5.9g, 30.7mmol), triethylamine (3.1g, 30.7mmol) in dichloromethane (80ml) for 16 hours at 25°C. Then agitate it 12 hours at room temperature. Add water (40ml) into the reaction system. Extract the aqueous layer with dichloromethane (40ml x 3) and then dry the combined organic layers with Na₂SO₄ before filter and evaporate. Then purify the residue with column chromatography (petroleum ether: ethyl acetate = 10:1∼petroleum ether: ethyl acetate =5:1) to obtain the title compound (yellow liquid, 730mg, yield of 20%). ¹H NMR(400MHz,CDCl₃)δ7.78(s,4H),7.34(br.s.,4H),4.03-4.07(m,4H),2.45(s,6H),1.48-1.52(m,3H),0.25-0 .34(m,4H).

### Embodiment 73D

### 2-(1-(2-(4-(2-ethyl-2H-tetrazol-5-yl)-phenoxy)-ethyl)-cyclopropyl)-ethyl-4-methylbenzenesulfonate

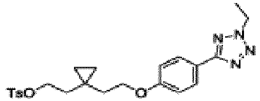

This embodiment is prepared with the method as described in Embodiment 29A. ¹H NMR(400MHz,CDCl₃)8.04-8.07(m,4H),7.72-7.77(m,4H),7.36(br.s.,4H),6.91-7.00(m,4H),2.42(s,3H),1. 49-1.55(m,3H),0.37(d,*J*=9.5Hz,2H),0.28(s,2H). LCMS(ESI)m/z:457(M+1).

### Embodiment 73E

### 5(2-(1-(2-(4-(2-ethyl-2H-tetrazol-5-yl)-phenoxy)-ethyl)-cyclopropylamino)-ethyl)-1,2-thiadiazolidine-2-tert-butyl formate-1,1-dioxide

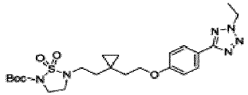

Please refer to the preparation method of Embodiment 32D for this embodiment.
LCMS(ESI)m/z:507(M+1).

### Embodiment 73F

### 2-(2-(1-(2-(4-(2-ethyl-2H-tetrazol-5-yl)-phenoxy)-ethyl)cyclopropyl)-ethyl)-1,2,5-thiadiazolidine-1,1-dioxide

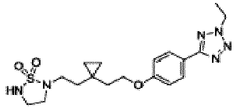

Agitate the solution of Embodiment 73E (60mg, 0.18mmol), K₂CO₃ (82mg, 0.6mmol) in methanol (2ml) to react for 4 hours at 60°C. Dry it through rotation. Then add water (5ml) and dichloromethane (10ml) into the reaction system. Extract the aqueous layer with dichloromethane (10ml x 3) and then dry the combined organic layers with Na₂SO₄ before filter and evaporate to obtain the title compound (colorless solid, 40mg, yield of 75%). LCMS(ESI)m/z:407(M+1).

### Embodiment 73G

### 2-(2-amino-pyridin-4-yl)-5-(2-(1-(2-(4-(2-ethyl-2H-tetrazol-5-yl)-phenoxy)-ethyl)-cyclic propyl)-ethyl)-1,2,5-thiadiazolidine-1,1-dioxide

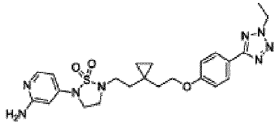

Under the protection of N₂ gas, add 8-hydroxyquinoline (14mg, 98µmol) and CuI (2mg, 9.8µmol) into the solution of Embodiment 73F (40mg, 988µmol), K₂CO₃ (27mg, 0.2mmol) and 2-amino-4-bromopyridine (26mg, 148µmol) in N,N-dimethylformamide (2ml). Allow the mixture solution to react 12 hours at 120°C. Dry it through rotation. Then add water (5ml) into the reaction system. Extract the aqueous layer with dichloromethane (10ml x 3) and then dry the combined organic layers with Na₂SO₄ before filter and evaporate. Then separate and purify the residue with the preparative chromatography (dichloromethane : methanol = 20:1, dichloromethane : ethyl acetate =1:2) to obtain the title compound (colorless solid, 20mg, yield of 39%). ¹H NMR(400MHz,CDCl₃)8.05-8.08(m,2H),7.91(d,*J*=6.0Hz,1H),6.99-7.02(m,2H),6.45-6.47(m,1H),6.28(br .s.,1H),5.00(br.s.,2H),4.66-4.69(m,2H),4.11-4.17(m,2H),3.76-3.80(m,2H),3.50(t,*J*=6.5Hz,2H),3.26-3.3 4(m,2H),1.80(t,*J*=6.3Hz,2H),1.71-1.76(m,2H),1.68(s,3H),0.25-0.62(m,4H). LCMS(ESI)m/z:499(M+1).

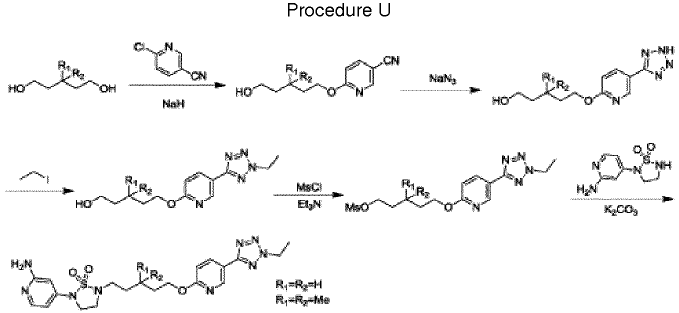

### Embodiment 74

### 4-(5-(5-((5-(2-ethyl-tetrazol-5-yl)-2-pyridinyl)-oxy)-pentyl)-1,1-dioxo-1,2,5-thiadiazolidine-2-yl)-pyridin-2-amine

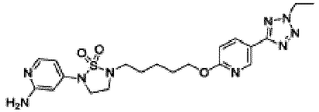

### Embodiment 74A

### 5-(2-amino-4-pyridyl)-1,1-dioxo-1,2,5-thiadiazolidine-2-tert-butyl formate

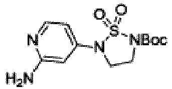

Dissolve 4-bromo-2-aminopyridine (10g, 57.8 mmol), Embodiment 32B (25.7g, 115.6mmol), (1S,2S)-N₁, N₂-dimethyl-cyclohexanediamine (1.64g, 11.6mmol), K₂CO₃ (24g, 173mmol) and CuI (16.5g, 86.7mmol) in N,N-dimethylformamide (150ml). Agitate the mixture 12 hours at 100°C under the protection of N₂ gas. Pour the reaction solution into the mixture solution stirred continuously of ice water (300ml), ammonia (100ml) and ethyl acetate (200ml). Extract it with ethyl acetate and dry the combined organic phases with anhydrous Na₂SO₄. Then filter and evaporate before recrystallize the residue with methanol. Then filter it to obtain the title compound (7.5g, yield of 70%). ¹H NMR(400MHz,DMSO-d6)7.87(d,*J*=5.0Hz,1H),6.40(d,*J*=4.0Hz,1H),6.30(s,1H),6.09(br.s.,2H),3.99-3.8 9(m,2H),3.87-3.75(m,2H),1.50(s,9H).

### Embodiment 74B

### 4-(1,1-dioxo-1,2,5-thiadiazolidine-2-yl)-pyridin-2-amine

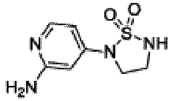

At 0°C, add the solution of hydrogen chloride in ethyl acetate (4mol/L) (50ml) into the solution of Embodiment 74A (5.0g, 15.9mmol) in ethyl acetate (20ml). Allow the mixture to react for 2 hours at 25°C. Then dry through rotation to obtain the title compound of hydrochloride (3.95g, yield of 97%). ¹H NMR(400MHz,DMSO-d6)8.44(t,*J*=7.8Hz,1H),7.91(d,*J*=7.3Hz,1H),7.82(br.s.,2H),6.63(dd,*J*=2.3,7.3Hz ,1H),6.41(d,*J*=2.3Hz,1H),3.94(t,*J*=6.3Hz,2H),3.59(q,*J*=6.5Hz,2H).

### Embodiment 74C

### 6-(5-hydroxypentyl-oxy)-pyridine-3-carbonitrile

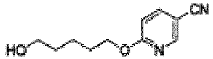

Under the protection of N₂ gas at 0°C, add sodium hydride (8.7g, 216.5mmol, 60%) in batch into the solution of Embodiment 1,5-pentanediol (11.3g, 108.3mmol) in N,N-dimethylformamide (50ml). Agitate the reaction solution 0.5 hour at 0°C. Then add the solution of 2-chloro-5-cyano-pyridine (5g, 36.1mmol) in N,N-dimethylformamide (20ml). Then agitate it 2 hours at 0°C. Add water to quench the reaction. Then extract it with ethyl acetate (100ml x 3) and dry the combined organic layer with Na₂SO₄ before filter and evaporate. Then purify the residue with column chromatography to obtain the title compound (4.7g, yield of 63%). ¹H NMR(400MHz,CDCl₃)δ8.49(d,*J*=2.0Hz,1H),7.78(dd,*J*=2.3,8.5Hz,1H),
6.81(d,*J*=8.5Hz,1H),4.46-4.33(m,2H),3.79-3.61(m,2H),1.84(quin,*J*=7.2Hz,2H),1.71-1.45(m,4H).
LCMS(ESI)m/z:207(M+1).

### Embodiment 74D

### 5-((5(2H-tetrazol-5-yl)-2-pyridinyl)-oxy)-pentan-1-ol

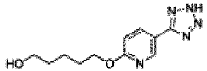

Add sodium azide (1.89g, 29.09mmol) and NH₄Cl (1.56g, 29.09mmol) into the solution of Embodiment 74C (2.00g, 9.70mmol) in N,N-dimethylformamide (20.00ml). Agitate the mixture solution 12 hours at 110°C. Then add water to quench the reaction and adjust its pH value to 3 with dilute hydrochloric acid. Then dry the organic phase with anhydrous Na₂SO₄ before filter and evaporate to obtain the title compound (white solid, 2.3g, yield of 90%). ¹H NMR(400MHz,CDCl₃)8.81(d,*J*=2.3Hz,1H),8.27(dd,*J*=2.4,8.7Hz,1H),6.99(d,*J*=8.8Hz,1H),4.52-4.29(m, 2H),3.68-3.50(m,2H),1.86(quin,*J*=7.0Hz,2H),1.75-1.47(m,4H). LCMS(ESI)m/z:250(M+1).

### Embodiment 74E

### 5-((5-(2-ethyl-tetrazol-5-yl)-2-pyridinyl)-oxy)-pentan-1-ol

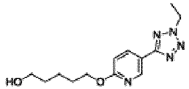

Under the protection of N₂ gas, add iodoethane (93mg, 2.32mmol) into the solution of Embodiment 74D (1g, 4.01mmol) and K₂CO₃ (2.77g, 20.05mmol) in acetonitrile (15ml). Agitate 4 hours at 25°C. Quench the reaction mixture with water (50ml). Then extract with ethyl acetate (30ml x 3). Then wash the combined organic phases with saturated salt water. Then dry with anhydrous Na₂SO₄ before filter and dry through rotation. Then purify the residue with column chromatography (petroleum ether: ethyl acetate = 3:1) to obtain the title compound (white solid, 700mg, yield of 60%). ¹H NMR(400MHz,CDCl₃)8.93(d,*J*=2.0Hz,1H),8.29(dd,*J*=2.3,8.8Hz,1H),6.85(d,*J*=8.5Hz,1H),4.72(q,*J*=7.3 Hz,2H),4.39(t,*J*=6.7Hz,2H),3.81-3.62(m,2H),1.86(quin,*J*=7.1Hz,2H),1.75-1.50(m,7H). LCMS(ESI)m/z:278(M+1).

### Embodiment 74F

### 5-((5-(2-ethyl-tetrazol-5-yl)-2-pyridinyl)-oxy)-pentyl methanesulfonate

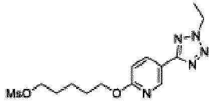

Under the protection of N₂ gas at 0°C, drop the solution of methanesulfonyl chloride (83mg, 0.72mmol) in THF (1ml) into the solution of Embodiment 74E (0.1g, 0.36mmol) and triethylamine (146mg, 1.44mmol) in THF (8ml). Agitate 2 hours at 0°C. Quench the reaction mixture with water (20ml). Then extract with ethyl acetate (20ml x 3). Then wash the combined organic phases with saturated salt water. Then dry with anhydrous Na₂SO₄ before filter and dry through rotation to obtain the title compound (white solid, 120mg, yield of 92.7%). ¹H NMR(400MHz,CDCl₃)δ8.93(d,*J*=2.3Hz,1H),8.30(dd,*J*=2.4,8.7Hz,1H),6.85(d,*J*=8.8Hz,1H),4.72(q,*J*=7. 3Hz,2H),4.40(t,*J*=6.5Hz,2H),4.33-4.23(m,2H),3.03(s,3H),1.98-1.67(m,9H). LCMS(ESI)m/z:356(M+1).

### Embodiment 74G

### 4-(5-(5-((5-(2-ethyl-tetrazol-5-yl)-2-pyridinyl)-oxy)-pentyl)-1,1-dioxo-1,2,5-thiadiazolidine-2-yl)-pyridin-2-amine

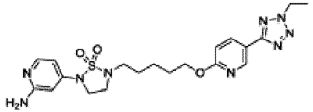

Under the protection of N₂ gas, add Embodiment 73F (422mg, 1.97mmol) into the solution of Embodiment 74B (0.7g, 1.97mmol) and K₂CO₃ (0.54g, 3.94mmol) in N,N-dimethylformamide (12ml). Agitate for 12 hours at 60°C. Quench the reaction mixture with water (50ml). Then extract with ethyl acetate (30ml x 3). Then wash the combined organic phases with saturated salt water. Then dry with anhydrous Na₂SO₄ before filter and dry through rotation. Then purify the residue with column chromatography (petroleum ether: ethyl acetate = 1:1) to obtain the title compound (white solid, 350mg, yield of 36%). ¹H NMR(400MHz,CDCl₃)8.86(d,*J*=2.0Hz,1H),8.32(dd,*J*=2.3,8.8Hz,1H),7.80(d,*J*=6.3Hz,1H),6.95(d,*J*=8.8 Hz,1H),6.52(dd,*J*=2.0,6.0Hz,1H),6.31(d,*J*=1.8Hz,1H),4.76(q,*J*=7.3Hz,2H),4.41(t,*J*=6.4Hz,2H),3.84(t, *J*=6.4Hz,2H),3.56(t,*J*=6.3Hz,2H),3.22-3.10(m,2H),1.97-1.76(m,4H),1.75-1.58(m,5H). LCMS(ESI)m/z:474(M+1).

### Embodiment 75

### 2-(2-amino-pyridin-4-yl)-5- (5-(4-(2-ethyl-2H-tetrazol-5-yl)-phenoxy)-3,3-dimethyl-pentyl) -1,2,5-thiadiazolidine-1,1-dioxide

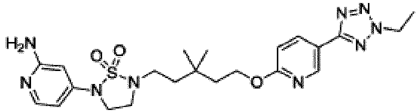

### Embodiment 75A

### 3,3-dimethyl pentanediol

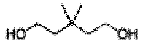

At 80°C, drop the solution of 3,3-dimethyl glutarate (50g, 312mmol) in THF (200ml) into the solution of LiAlH₄ (50g, 1.32mol) in THF (1.3L). After it is added, agitate the mixture solution 2 hours at 80°C. Add water (200ml) into the reaction system. Extract the aqueous layer with ethyl acetate (300ml x 3) and then dry the combined organic layers with Na₂SO₄ before filter and evaporate to obtain the title compound (38g, yield of 92%). ¹H NMR(400MHz,CDCl₃)3.67(t,*J*=7.03Hz,4H), 3.50(br.s.,1H),1.50-1.61(m,4H),0.92(s,6H).

### Embodiment 75B

### 6-((5-hydroxy-3,3-dimethyl-pentyl)-oxy)-nicotinonitrile

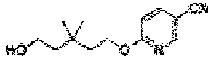

Please refer to the preparation method of Embodiment 74C for this embodiment. ¹H NMR(400MHz,CDCl₃)8.47(d,*J*=2.0Hz,1H),7.76(dd,*J*=8.5,2.3Hz,1H),6.78(d,*J*=8.8Hz,1H),4.44(t,*J*=7.4 Hz,2H),3.75(t,*J*=7.4Hz,2H),1.75(t,*J*=7.4Hz,2H),1.61(t,*J*=7.4Hz,2H),1.01(s,6H).

### Embodiment 75C

### 5-((5-(2H-tetrazol-5-yl)-pyridin-2-yl)-oxy)-3,3-dimethyl-1-ol

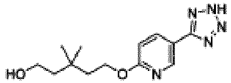

Please refer to the preparation method of Embodiment 74D for this embodiment. ¹H NMR(400MHz,CDCl₃)8.81(d,*J*=2.3Hz,1H),8.26(dd,*J*=8.8,2.3Hz,1H),7.00(d,*J*=8.8Hz,1H),4.39(t,*J*=7.4 Hz,2H),3.49(t,*J*=7.4Hz,2H),1.68(t,*J*=7.4Hz,2H),1.45(t,*J*=7.4Hz,2H),0.92-0.97(m,6H).

### Embodiment 75D

### 5-((4-(2-ethyl-tetrazol-5-yl)-phenoxy)-3,3-dimethyl-pentan-1-ol

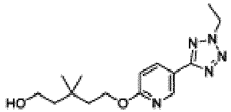

Please refer to the preparation method of Embodiment 74E for this embodiment. ¹H NMR(400MHz,CDCl₃)8.92(d,*J*=2.0Hz,1H),8.27(dd,*J*=8.5,2.3Hz,1H),6.82(d,*J*=8.8Hz,1H),4.70(q,*J*=7.4 Hz,2H),4.44(t,*J*=7.4Hz,2H),3.77(t,*J*=7.4Hz,2H),1.78(s,3H),1.64-1.71(m,4H),1.02(s,6H).

### Embodiment 75E

### 5-(4-(2-ethyl-2H-tetrazol-5-yl)-phenoxy)-3,3-dimethyl-pentyl-methanesulfonate

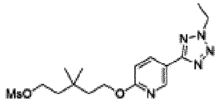

Please refer to the preparation method of Embodiment 74F for this embodiment. ¹H NMR(400MHz,CDCl₃)8.92(d,*J*=2.0Hz,1H),8.27(dd,*J*=8.5,2.0Hz,1H),6.83(d,*J*=9.0Hz,1H),4.66-4.73(m, 2H),4.44(t,*J*=7.0Hz,2H),4.37(t,*J*=7.3Hz,2H),3.02(s,3H),1.80(dt,*J*=14.2,7.2Hz,4H),1.69(t,*J*=7.5Hz,3H), 1.06(s,6H).

### Embodiment 75F

### 2-(2-amino-pyridin-4-yl)-5-(5-(4-(2-ethyl-2H-tetrazol-5-yl)-phenoxy)-3,3-dimethyl-pentyl) -1,2,5-thiadiazolidine-1,1-dioxide

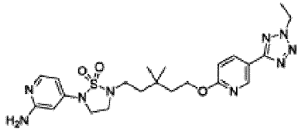

Please refer to the preparation method of Embodiment 74G for this embodiment. ¹H NMR(400MHz,CDCl₃)8.93(d,*J*=2.0Hz,1H),8.28(dd,*J*=8.8,2.3Hz,1H),7.97(d,*J*=6.0Hz,1H),6.85(d,*J*=8.5 Hz,1H),6.43(dd,*J*=5.8,2.3Hz,1H),6.30(d,*J*=2.0Hz,1H),4.71(q,*J*=7.4Hz,2H),4.58(br.s.,2H),4.45(t,*J*=7.0 Hz,2H),3.81(t,*J*=6.5Hz,2H),3.50-3.56(m,2H),3.24(d,*J*=8.5Hz,2H),1.81(t,*J*=7.0Hz,2H),1,74(d,*J*=8.5Hz, 2H),1.67-1.71(m,3H),1.08(s,6H). LCMS(ESI)m/z:501(M+1).

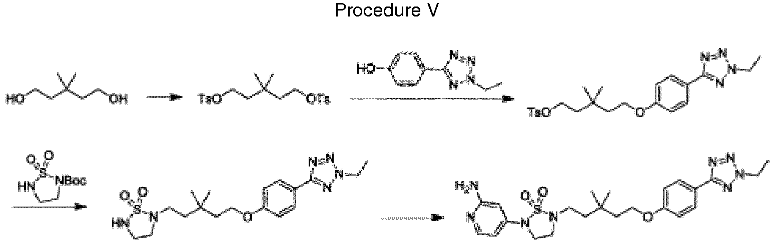

### Embodiment 76

### 4-(5-(5-(4-(2-ethyl-tetrazol-5-yl)-phenoxy)-3,3-dimethyl-pentyl)-1,1-dioxo-1, 2,5-thiadiazolidine-2-yl)-pyridin-2-amine

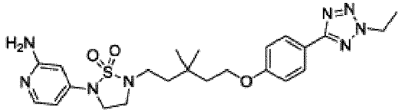

### Embodiment 76A

### 3,3-dimethylpentyl-1,5-bis(4-methylbenzenesulfonate)

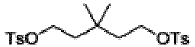

Please refer to the preparation method of Embodiment 14A for this embodiment. 1H NMR(400MHz,CDCl₃)7.77(d,*J*=8.53Hz,2H),7.35(d,*J*=8.03Hz,2H),4.02(t,*J*=7.03Hz,2H),2.45(s,3H),1.5 5(t,*J*=7.03Hz,2H),0.84(s,3H).

### Embodiment 76B

### 5-(4-(2-ethyl-2H--tetrazol-5-yl)-phenoxy)-3,3-dimethyl-pentyl-4-methylbenzenesuifonate

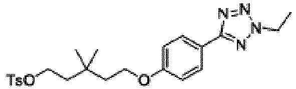

Add K₂CO₃ (8.72g, 63.29mmol) and KI (5.24g, 31.55mmol) into the solution of Embodiment 76A (6g, 31.55mmol) and Embodiment 11A (2.6g, 31.55mmol) in acetone solution (100ml). Agitate the mixture solution 12 hours at 80°C. Then extract it with ethyl acetate. Dry the organic phase with anhydrous Na₂SO₄ before filter and evaporate. Then purify the residue with column chromatography (petroleum ether: ethyl acetate =10:1) to obtain the title compound (white solid, 8.0g, yield of 56%). ¹H NMR(400MHz,CDCl₃)8.09(d,*J*=8.5Hz,2H),7.00(d,*J*=8.8Hz,2H),4.70(q,*J*=7.3Hz,2H),4.38(t,*J*=7.4Hz,2 H),4.11(t,*J*=6.8Hz,2H),3.0-3.04(m,3H),1.80-1.87(m,4H),1.70(t,*J*=7.4Hz,3H),1.08(s,6H). LCMS(ESI)m/z:459(M+1).

### Embodiment 76C

### 5-(4-(2-ethyl-2H-tetrazol-5-yl)-phenoxy)-3,3-dimethyl-pentyl-1,2,5-thiadiazolidine-1,1 dioxide

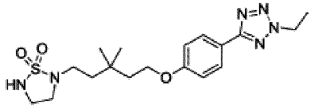

At 0°C, add sodium hydride (41.9mg, 1.7mmol) into the solution of Embodiment 76B (969mg, 4.4mmol) in N,N-dimethylformamide (5ml). Keep it still 30 minutes at 0°C. Then add the solution of Embodiment 32B (400mg, 0.87mmol) in N,N-dimethylformamide (1ml) into the mixture solution above. After agitate it at 20°C to react for 2 hours, add K₂CO₃ (0.24mg, 1.7mmol) into the reaction solution. Then add water to quench the reaction and extract the aqueous phase with ethyl acetate. Dry the combined organic phases with anhydrous Na₁SO₄. Then filter and evaporate it before separating it with column (petroleum ether: ethyl acetate=1:1) to obtain the title compound (yellow oily form, 100mg, yield of 28%). ¹H NMR(400MHz,CDCl₃)7.56(d,*J*=8.5Hz,2H),6.92(d,*J*=9.0Hz,2H),4.00(t,*J*=6.5Hz,2H),3.66-3.72(m,2H),3 .47-3.52(m,2H),3.46(s,6H),3.35-3.41(m,2H),3.04(t,*J*=7.3Hz,2H),1.79-1.88(m,2H),1.66-1.74(m,2H),1. 52-1.60(m,2H),1.23(t,*J*=7.0Hz,3H). LCMS(ESI)m/z:409(M+1).

### Embodiment 76D

### 4-(5-(5-(4-(2-ethyl-tetrazol-5-yl)-phenoxy)-3,3-dimethylpentyl)-1,1-dioxo-1,2,5-thiadiazolidine-2-yl)-pyridin-2-amine

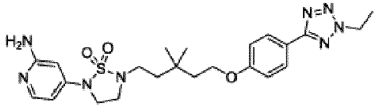

At 0 °C, add K₂CO₃ (67mg, 0.49mmol), 2-amino-4-bromo-pyridine (85mg, 0.489mmol), 8-hydroxyquinoline (35mg, 0.25mmol) and CuI (23mg, 0.12mmol) into the solution of Embodiment 76C (100mg, 0.24mmol) in N,N-dimethylformamide (2ml). After replace with N₂ gas, add cyclohexanediamine (14mg, 0.12mmol). Agitate it to react for 15 hours at 110°C. Then add water to quench the reaction and extract the aqueous phase with ethyl acetate. Dry the combined organic phases with anhydrous Na₂SO₄ before filter and evaporate to obtain the title compound (white solid, 35mg, yield of 29%). ¹H NMR(400MHz,CDCl₃)8.07(d,*J*=8.5Hz,1H),7.98(d,*J*=5.5Hz,1H),6.99(d,*J*=8.5Hz,2H),6.42(d,*J*=4.5Hz,1 H),6.29(s,1H),4.69(q,*J*=7.5Hz,2H),4.48(br.s.,2H),4.11(t,*J*=6.5Hz,2H),3.79(t,*J*=6.0Hz,2H),3.46-3.53(m ,2H),3.16-3.25(m,2H),1.83(t,*J*=6.5Hz,2H),1.68-1.76(m,5H),1.07(s,6H). LCMS(ESI)m/z:501(M+1).

### Experimental example 1: Test of EV71 In-Vitro Cytopathic Effect (CPE)

### Purpose of experiment:

To test the in-vitro antiviral activity and cytotoxicity of a compound to HFMD virus EV71 by means of cytopathic effect (CPE) test.

### Test materials:

1. Virus strain: Shenzhen/120F1/09
2. Cell line: RD cells of human rhabdomyoma
3. Cell culture media: DMEM medium added with 10% serum, Penicillin/Streptomycin and L-Glutamine(1×)
4. Test reagent: cellular activity test reagent CCK8

### Test method:

1. Cell inoculation: digest off the RD cells from the attached state, then dilute them with culture medium to a density of 80000/ml and inoculate 100µl into the micro wells of the 96-well plate.
2. Dilution of compound:
   Step 1: prepare the dry powder of a compound to test into a 10mM solution in DMSO. Then dilute the compound by three times to eight concentration points. Dilute the reference compounds with the same method.
   Step 2: further dilute the diluted solution of the compound in DMSO with cell culture medium. Add 10µl DMSO solution for each well into 240µl culture medium.
3. Add the diluted compound solution by 50µl each well into the 96-well plate inoculated with cells (double holes, final concentration of DMSO is 1%).
4. Dilution of virus: dilute EV71 virus solution by 10000 times to a concentration of 100TCID₅₀/50µl/. Add the diluted virus solution by 50µl each well into the 96-well plate. Prepare another 96-well plate to replace the virus with culture medium and similarly inoculate the cells. Add the compound to test the toxicity of compound on cells.
5. Cultivate the 96-well plate 3 days at the conditions of 37°C and 5% CO₂.
6. EC50 and CC50 tests: add the cellular activity test reagent CCK8 into the micro-wells by 20µl/well. Use ELISA to read the absorbancy at the wavelength of 450nm and 630nm.
7. Analysis of data: analyze the data with Prism5.0 and calculate the antiviral activity of EC50 and the cellular toxicity of C50 of the compound.

The result is as shown in Table 1:

**Table 1: Result of EC₅₀ by EC71 CPE test**

| **Test sample** (Title compound) | **EV71** |
|---|---|
| Embodiment 1 | D |
| Embodiment 2 | A |
| Embodiment 6 | B |
| Embodiment 7 | A |
| Embodiment 8 | B |
| Embodiment 10 | B |
| Embodiment 11 | B |
| Embodiment 12 | A |
| Embodiment 13 | C |
| Embodiment 14 | A |
| Embodiment 15 | A |
| Embodiment 16 | A |
| Embodiment 17 | A |
| Embodiment 18 | A |
| Embodiment 19 | C |
| Embodiment 21 | A |
| Embodiment 22a | A |
| Embodiment 22b | D |
| Embodiment 23 | A |
| Embodiment 24 | D |
| Embodiment 25 | A |
| Embodiment 26 | C |
| Embodiment 27 | A |
| Embodiment 28 | A |
| Embodiment 29 | A |
| Embodiment 30 | A |
| Embodiment 31 | C |
| Embodiment 32 | A |
| Embodiment 33 | C |
| Embodiment 34 | D |
| Embodiment 35 | C |
| Embodiment 36 | A |
| Embodiment 37 | D |
| Embodiment 38 | A |
| Embodiment 39 | B |
| Embodiment 40 | A |
| Embodiment 41 | A |
| Embodiment 42 | C |
| Embodiment 43 | C |
| Embodiment 44 | B |
| Embodiment 45 | A |
| Embodiment 46 | C |
| Embodiment 47 | D |
| Embodiment 48 | C |
| Embodiment 49 | C |
| Embodiment 50 | A |
| Embodiment 51 | A |
| Embodiment 52 | A |
| Embodiment 53 | A |
| Embodiment 54 | D |
| Embodiment 55 | C |
| Embodiment 56 | B |
| Embodiment 57 | A |
| Embodiment 58 | A |
| Embodiment 59 | A |
| Embodiment 60 | A |
| Embodiment 61 | A |
| Embodiment 62 | C |
| Embodiment 63 | C |
| Embodiment 64 | A |
| Embodiment 65 | C |
| Embodiment 66 | B |
| Embodiment 67 | A |
| Embodiment 68 | D |
| Embodiment 69 | A |
| Embodiment 70 | C |
| Embodiment 71 | C |
| Embodiment 72 | B |
| Embodiment 73 | A |
| Embodiment 74 | A |
| Embodiment 75 | A |
| Embodiment 76 | A |

| | |
|---|---|
| Note: A≤50nM; 50nM<B≤100nM; 100nM<C≤500nM; 500nM<D≤1000nM. Conclusion: the compounds according to the present invention have an obvious inhibitory effect to EV71 virus at a cell level. | |

## Claims

1. A compound(s) or its pharmaceutically-acceptable salt(s) as shown by Formula (II), 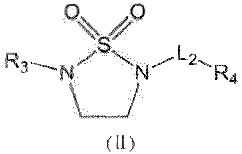
R₃ is any selected from 5-membered heterocycles , C_{6∼12} aryl groups, C_{6∼12} aralkyl groups, C_{5∼12} heteroaromatic rings and C_{5∼12} heteroaryl groups substituted with R₀₁;
L₂ is independently selected respectively from 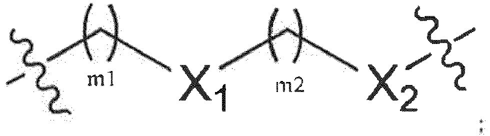
m₁ and m₂ are independently selected respectively from 1, 2, or 3;
X₁ and X₂ are independently selected from -C(R_{d1})(R_{d2})-, -C(=O)N(R_{d3})-, -N(R_{d4})-, -C(=NR_{d5})-, -S(=O)₂N(R_{d6})-, -S(=O)N(R_{d7})-, -O-, -S-, -C(=O)O-, -C(=O)-, -C(=S)-, -S(=O)- or -S(=O)₂-,
R₄ is selected from 5∼14-membered cyclic hydrocarbyl or heterocycloalkyl groups,
R_{d1} and R_{d2} are independently selected respectively from H, F, Cl, Br, I, CN, OH, SH, NH₂, CHO, COOH or any selected from C₁₋₁₀ alkyl or heteroalkyl groups and C₃₋₁₀ cyclic hydrocarbyl or heterocycloalkyl groups substituted with R₀₁, C₁₋₁₀ alkyl or heteroalkyl groups substituted with C₃₋₁₀ hydrocarbyl or heterocycloalkyl groups;
R₀₁ is selected from F, Cl, Br, I, CN, OH, SH, NH2, CHO, COOH, R₀₂;
R₀₂ is selected from C₁₋₁₀ alkaly groups, C₁₋₁₀ alkylamino groups, N,N-(C₁₋₁₀ alkayl) amino groups, C₁₋₁₀ alkoxy groups, C₁₋₁₀ alkanoyl groups, C₁₋₁₀ alkoxycarbonyl groups, C₁₋₁₀ alkylsulfonyl groups, C₁₋₁₀ alkylsulfinyl groups, C3-10 cycloalkyl groups, C₃₋₁₀ cycloalkylamino groups, C₃₋₁₀ heterocyclic alkyl amino groups, C₃₋₁₀ cycloalkoxy groups, C₃₋₁₀ cycloalkyl acyl groups, C₃₋₁₀ cycloalkoxy-carbonyl groups, C₃₋₁₀ cycloalkylsulfonyl groups and C₃₋₁₀ cycloalkylsulfinyl groups;
the "hetero" represents a hetero atom or hetero atom radical selected from -C(=O)N(R_{d3})-, -N(R_{d4})-, -C(=NR_{d5})-, -S(=O)₂N(R_{d6})-, -S(=O)N(R_{d7})-, -O-, -S-, =O, =S, -C(=O)O-, -C(=O)-, -C(=S)-, -S(=O)- and/or -S(=O)₂;
R_{d3-d7} is independently selected from H, R₀₃;
R₀₃ is selected from C₁₋₁₀ alkyl groups, C₁₋₁₀ alkanoyl groups, C₁₋₁₀ alkoxycarbonyl groups, C₁₋₁₀ alkylsulfonyl groups, C₁₋₁₀ alkylsulfinyl groups, C₃₋₁₀ cycloalkyl groups, C₃₋₁₀ ycloalkyl acyl groups, C₃₋₁₀ cycloalkoxy-carbonyl groups, C₃₋₁₀ cycloalkylsulfonyl groups and C₃₋₁₀ cycloalkylsulfinyl groups;
R₀₂ and R₀₃ are any substituted necessarily by R₀₀₁;
R₀₀₁ is selected from F, Cl, Br, I, CN, OH, N(CH₃)₂, NH(CH₃), NH₂, CHO, COOH, trifluoromethyl group, aminomethyl group, hydroxymethyl group, methyl group, methoxy group, formyl group, methoxycarbonyl group, methylsulfonyl group and methylsulfinyl group;
the number of R₀₁, R₀₀₁ and hetero atom or hetero atom radical is independently selected respectively from 0, 1, 2 or 3;
optionally, R_{d1} and R_{d2} interconnect together to form a 3 or 4-membered carbocyclic or heterocyclic ring.

2. Said compound(s) or its pharmaceutically-acceptable salt(s) according to claim 1, where: R₃ is any selected from substituted 5 ∼ 6 membered aryl and substituted heteroaryl group;
preferentially, R₃ is any selected optionally from pyridyl group, phenyl group, furanyl group, pyrazolyl group, pyrrolyl group, thiazolyl group, pyridazinyl group, pyrimidinyl group and thienyl group substituted by R₀₁ and the definition of R₀₁ is as claim 1 and the number of R₀₁ is selected from 0, 1 2 or 3;
preferentially, R₀₁ is selected from F, Cl, Br, I, CN, OH, SH, NH₂, CHO, COOH, Me,

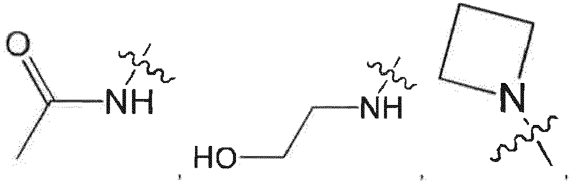

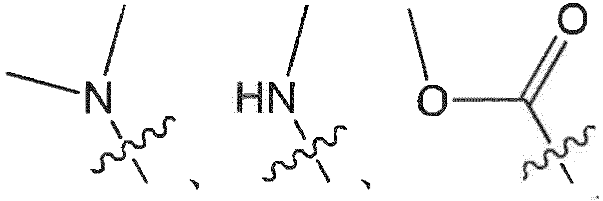

3. Said compound(s) or its pharmaceutically-acceptable salt(s) according to claim 2, where: R₃ is selected from:

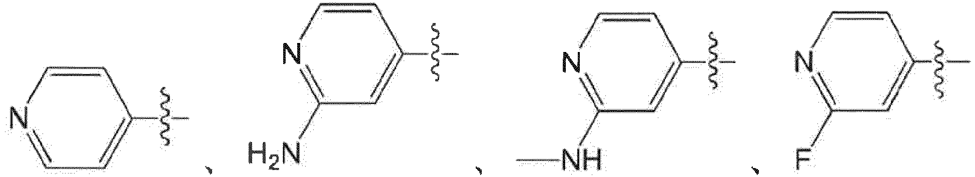

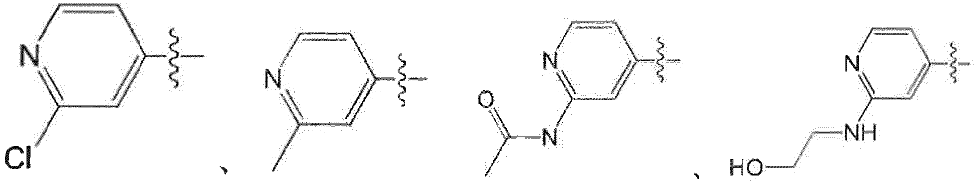

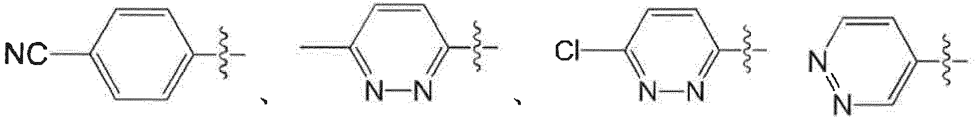

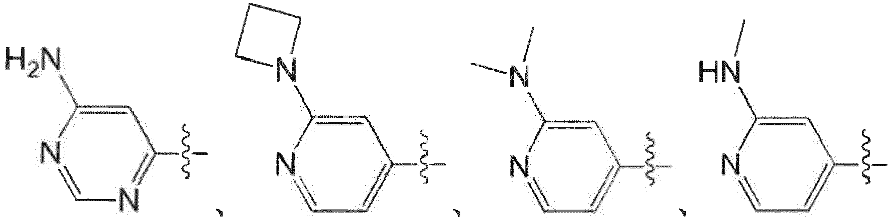

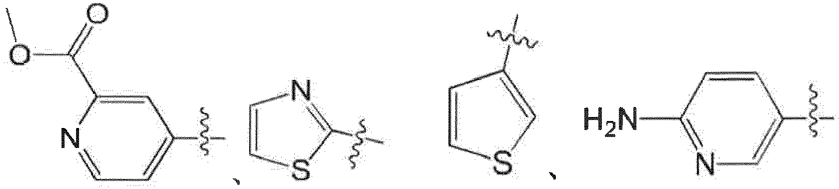

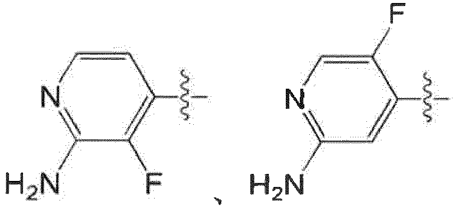

4. Said compound(s) or its pharmaceutically-acceptable salt(s) according to claim 1, wherein:,
X₁, X₂ are independently selected respectively from -O-, -C(=O)-, -CH(CH₃)-, -C(CH₃)₂-, -CF₂-,
-CH(F) 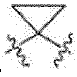 -CH(OH), -CH₂-, -NH- and -N(CH₃)-;
preferentially, L₂ is selected from: 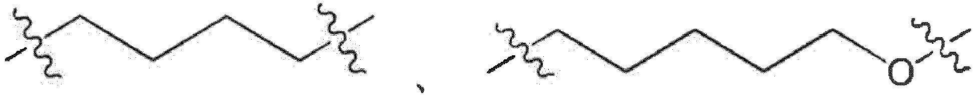 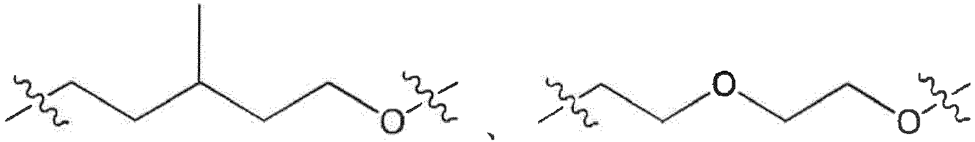 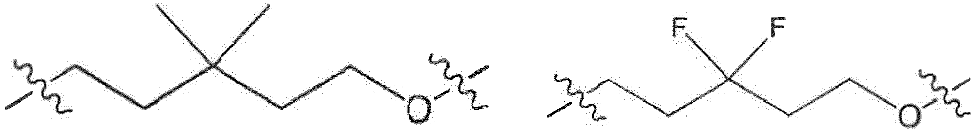 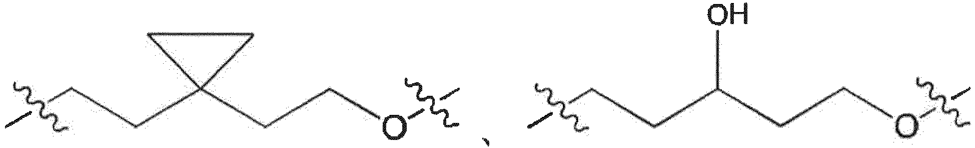 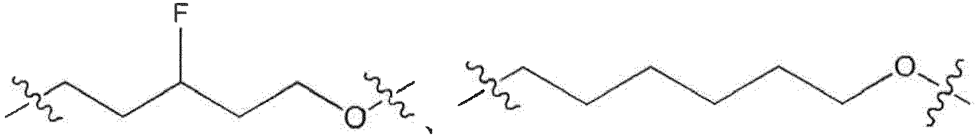 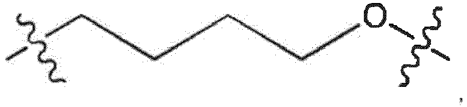 other variables are defined as claim 1,

5. Said compound(s) or its pharmaceutically-acceptable salt(s) according to claim 1, where: R₄ is selected from the following: 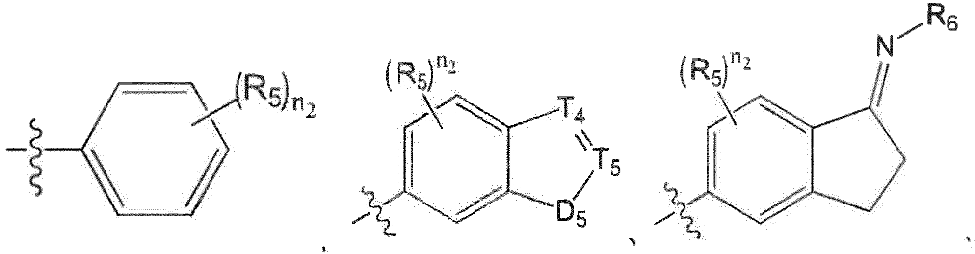 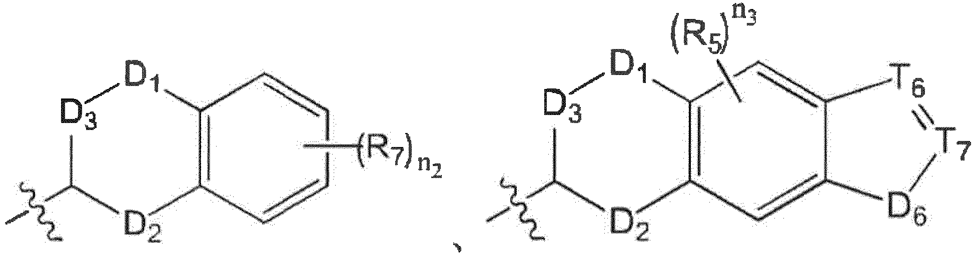 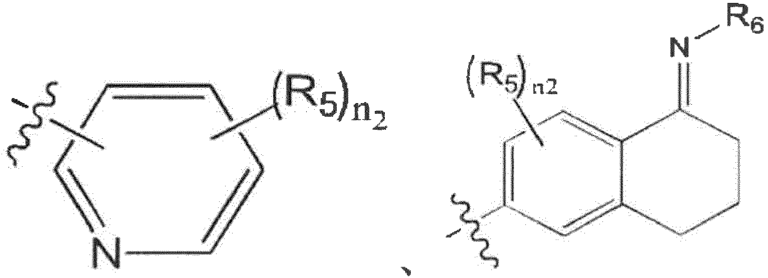
T₄₋₇ is independently selected respectively from N or C(Rₜ);
D₁₋₃, D₅ and D₆ are independently selected respectively from single bond, -C(R_{d1})(R_{d2})-, -C(=O)N(R_{d3})-, -N(R_{d4})-, -C(=NR_{d5})-, -S(=O)₂N(R_{d6})-, -S(=O)N(R_{d7})-, -O-, -S-, -C(=O)O-, -C(=O)-, -C(=S)-, -S(=O)- or -S(=O)₂;
n₂ is selected from 0, 1, 2 or 3;
n₃ is selected from 0, 1 or 2;
R₅, R₆, R₇, Rₜ, R_{d1} and R_{d2} are independently selected respectively from H, F, Cl, Br, I, CN, OH, SH, NH₂, CHO and COOH or any selected from C₁₋₁₀ alkyl or heteroalkyl groups and C₃₋₁₀ cyclic hydrocarbyl or heterocycloalkyl groups substituted with R₀₁, C₁₋₁₀ alkyl or heteroalkyl groups substituted with C₃₋₁₀ hydrocarbyl or heterocycloalkyl groups, other variables are defined as in claim 1.

6. Said compound(s) or its pharmaceutically-acceptable salt(s) according to claim 5, where: R₄ is selected from
the following: 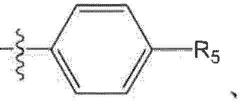 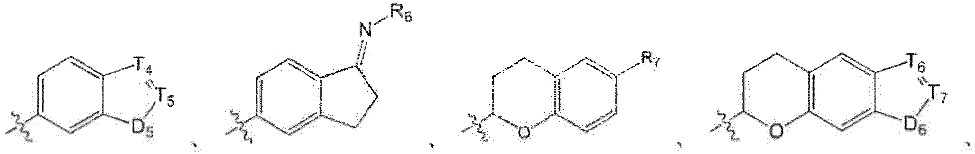 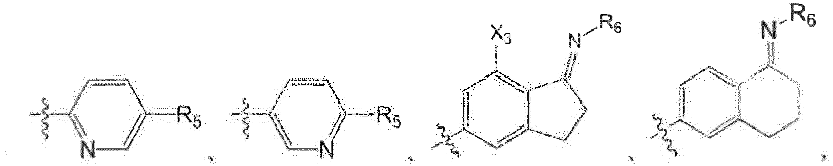 X₃ is selected from F, Cl, Br, I, CN, OH, SH, NH₂, CHO, COOH or any selected from C₁₋₁₀ alkyl or heteroalkyl groups and C₃₋₁₀ cyclic hydrocarbyl or heterocycloalkyl groups substituted with R₀₁, C₁₋₁₀ alkyl or heteroalkyl groups substituted with C₃₋₁₀ hydrocarbyl or heterocycloalkyl groups.

7. Said compound(s) or its pharmaceutically-acceptable salt(s) according to claim 5 or claim 6, wherein: R₅₋₇ are independently selected respectively from: 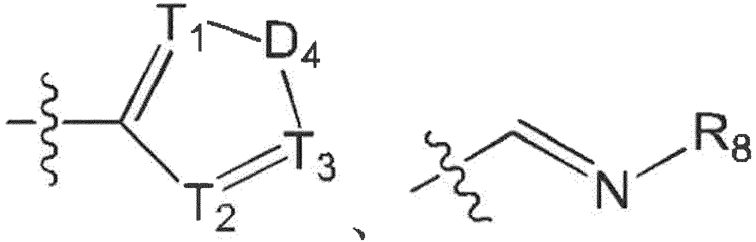 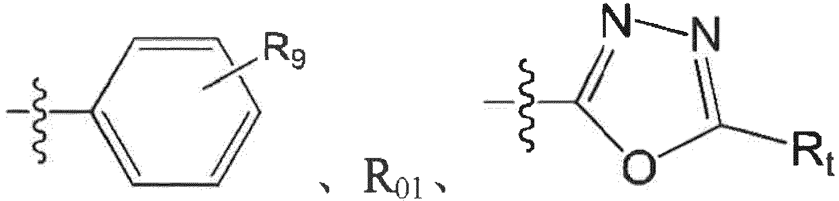 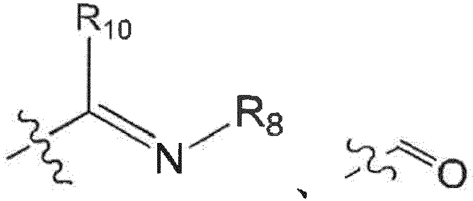 , wherein:
T₁₋₃ is independently selected respectively from N or C(Rₜ);
D₄ is selected from -C(R_{d1})(R_{d2})-, -C(=O)N(R_{d3})-, -N(R_{d4})-, -C(=NR_{d5})-, -S(=O)2N(R_{d6})-, -S(=O)N(R_{d7})-, -O-, -S-, -C(=O)O-, -C(=O)-, -C(=S)-, -S(=O)- or -S(=O)₂-;
R₈, R₉ and Rₜ are independently selected respectively from H, F, Cl, Br, I, CN, OH, SH, NH₂, CHO, COOH or any selected from C₁₋₁₀ alkyl or heteroalkyl groups and C₃₋₁₀ cyclic hydrocarbyl or heterocycloalkyl groups substituted with R₀₁, C₁₋₁₀ alkyl or heteroalkyl groups substituted with C₃₋₁₀ hydrocarbyl or heterocycloalkyl groups;
R₁₀ is selected from F, Cl, Br, I, CN, OH, SH, NH₂, CHO, COOH or any selected from C₁₋₁₀ alkyl or heteroalkyl groups and C₃₋₁₀ cyclic hydrocarbyl or heterocycloalkyl groups substituted with R₀₁, C₁₋₁₀ alkyl or heteroalkyl groups substituted with C₃₋₁₀ hydrocarbyl or heterocycloalkyl groups.

8. Said compound(s) or its pharmaceutically-acceptable salt(s) according to claim 7, wherein: R₅₋₉, R₀₁ and Rₜ are independently selected respectively from the following: 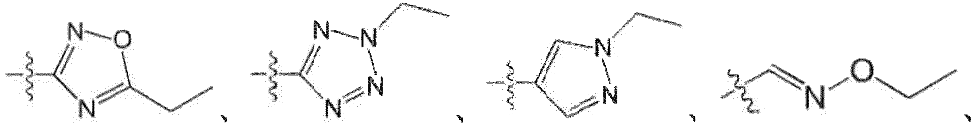 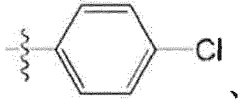 F, Cl, Br, CF₃, -C(C₂H₅)-, -C(OC₂H₅)-, methyl group, ethyl group, propyl group, methoxy group, ethoxy group, propoxy group,

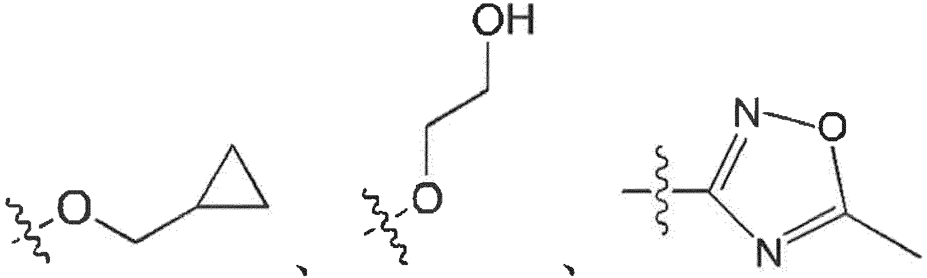

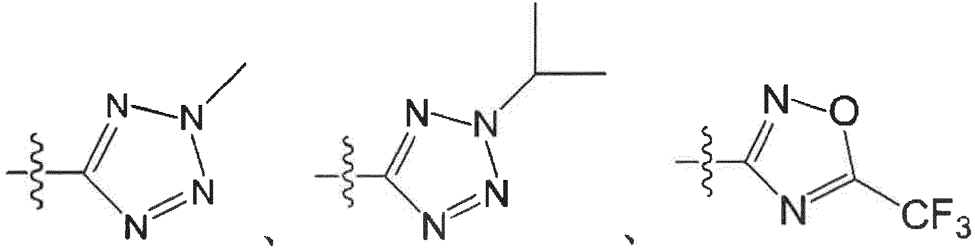

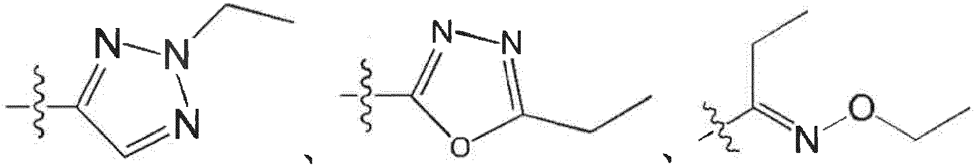

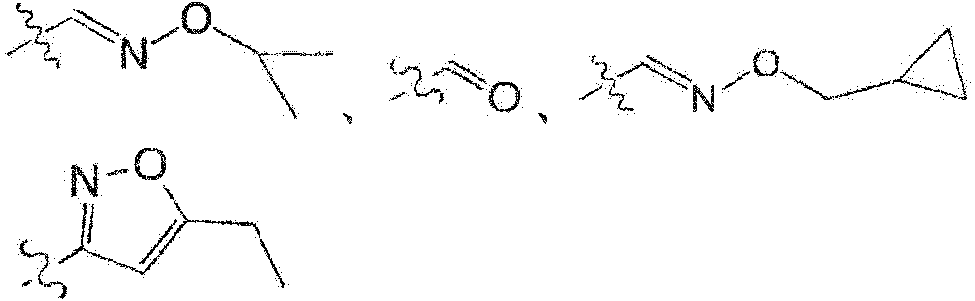

9. Said compound(s) or its pharmaceutically-acceptable salt(s) according to claim 8, wherein: said R₄ is selected from the following :

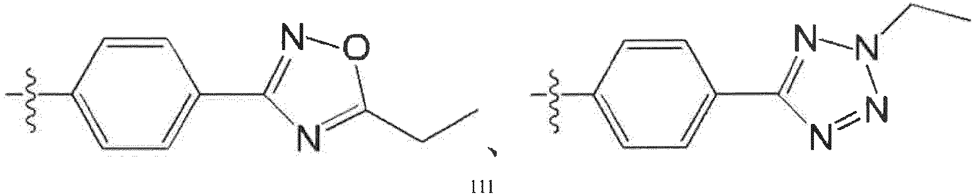

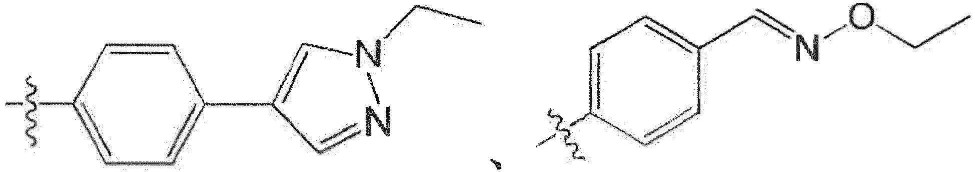

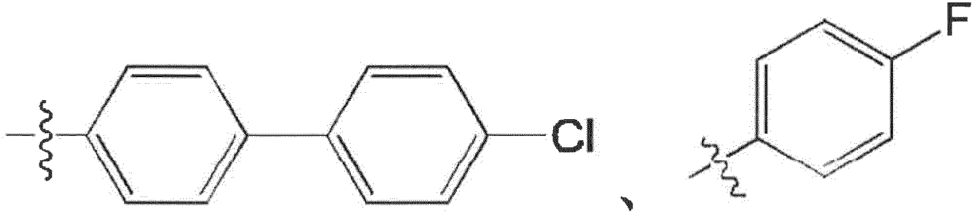

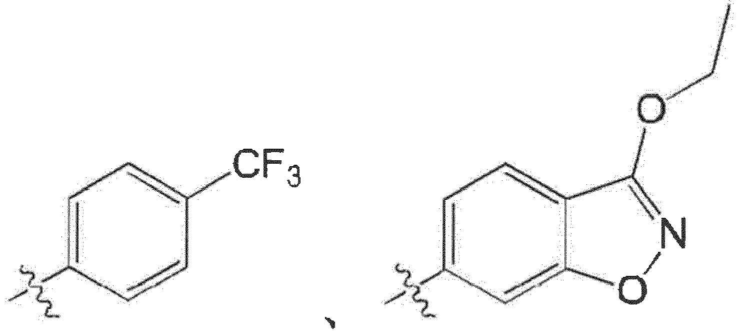

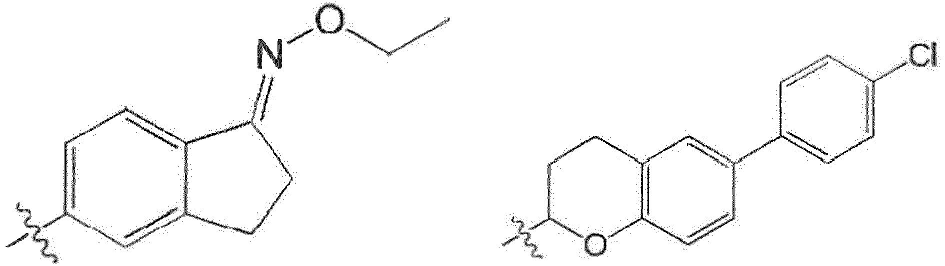

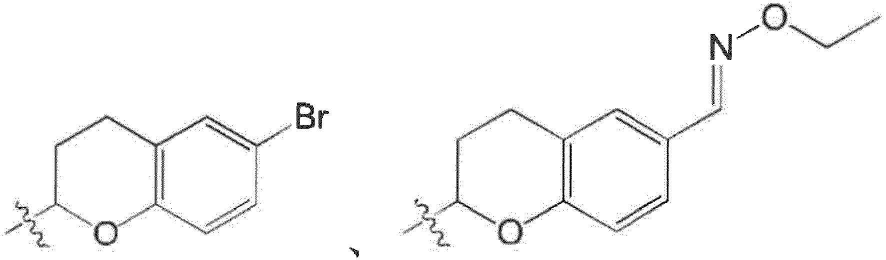

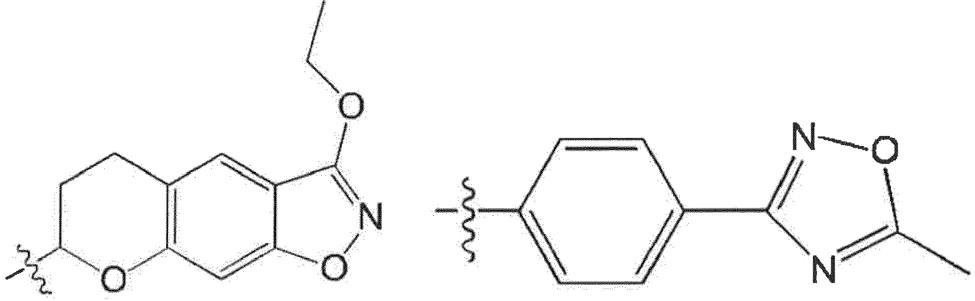

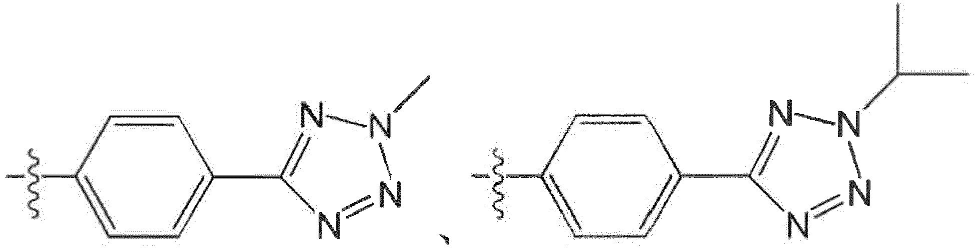

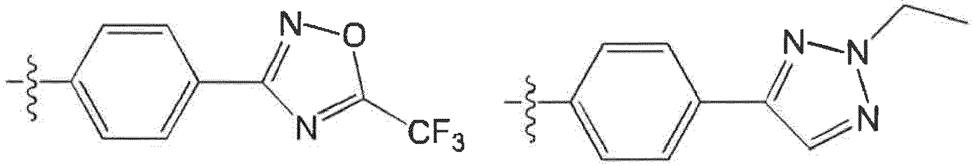

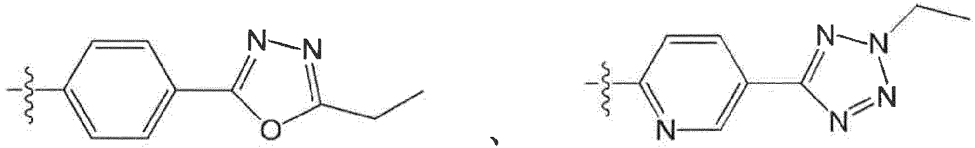

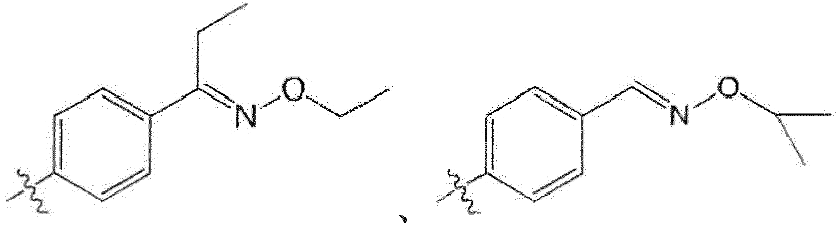

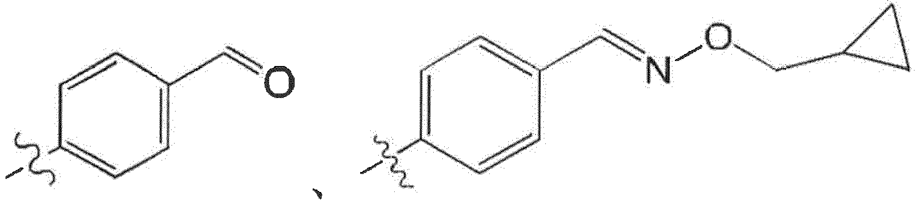

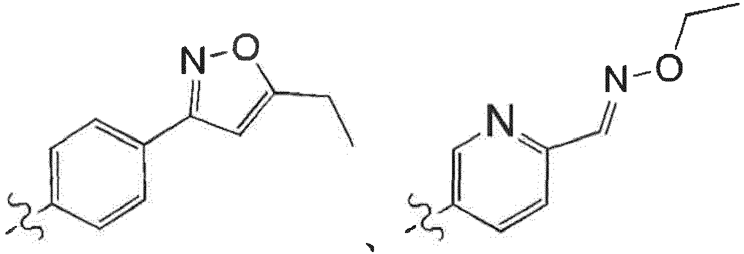

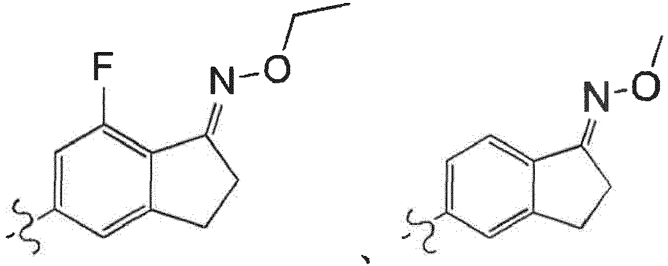

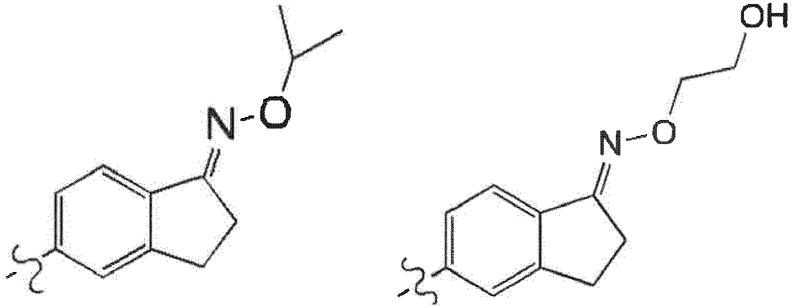

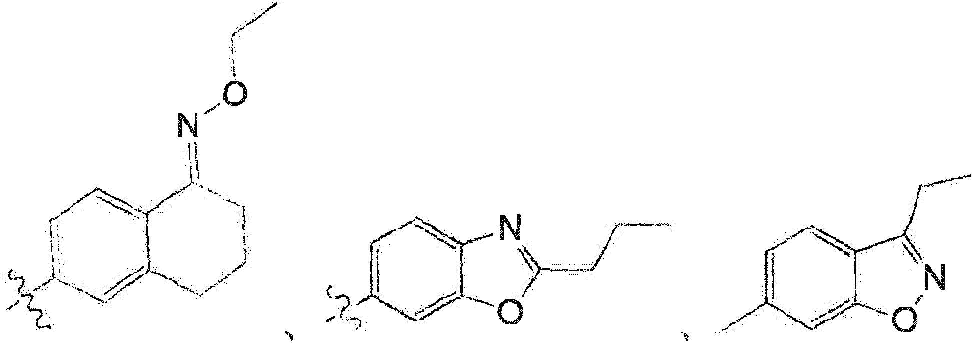

10. Said compound(s) or its pharmaceutically-acceptable salt(s) according to claim 1, which is selected from the following

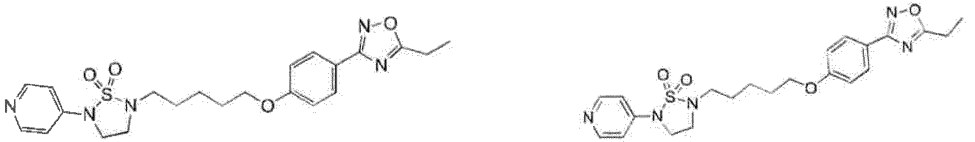

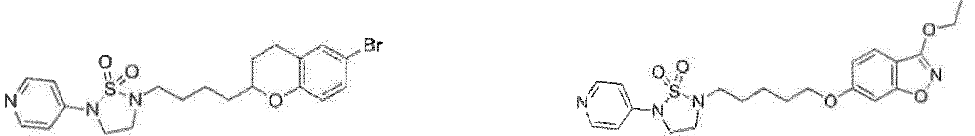

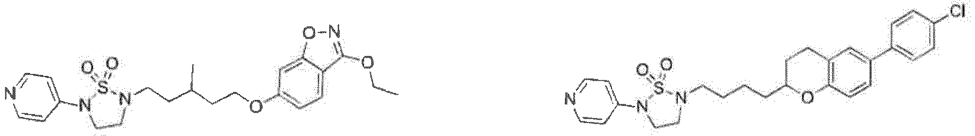

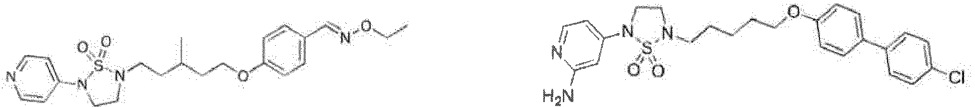

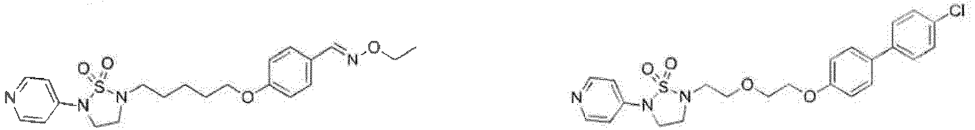

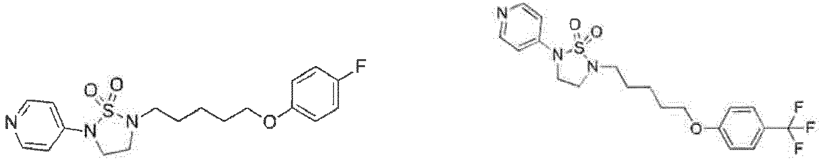

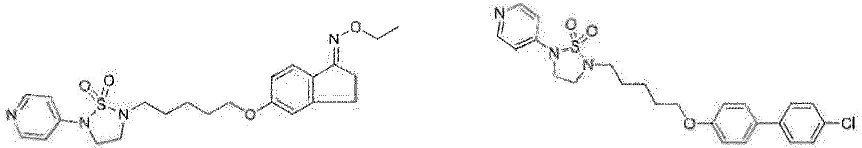

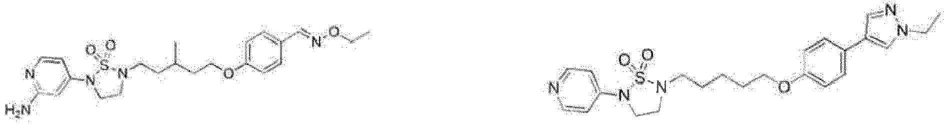

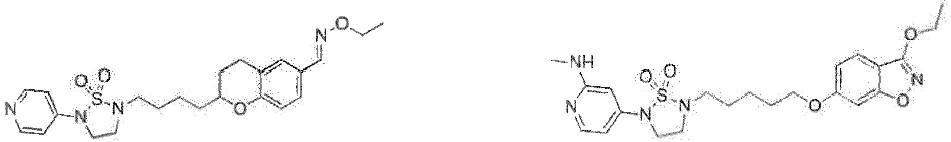

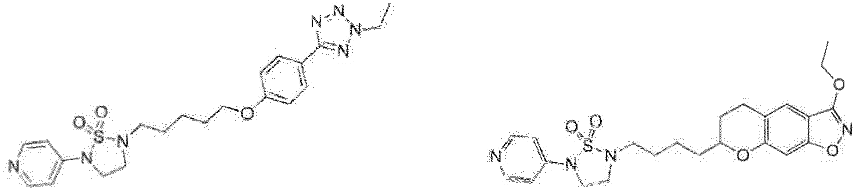

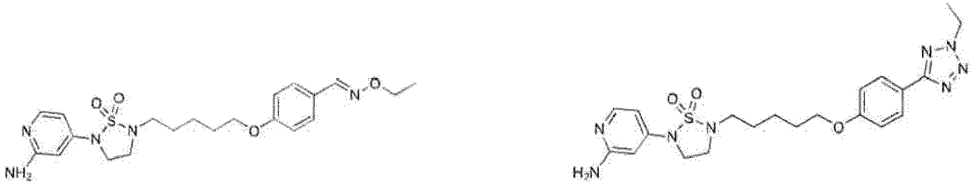

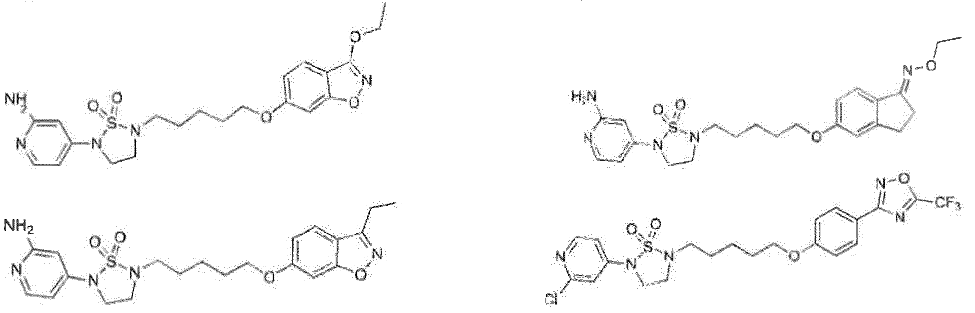

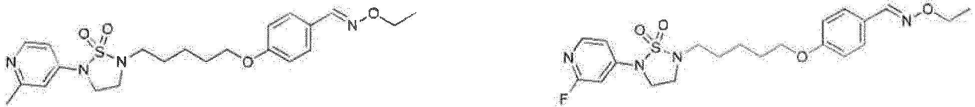

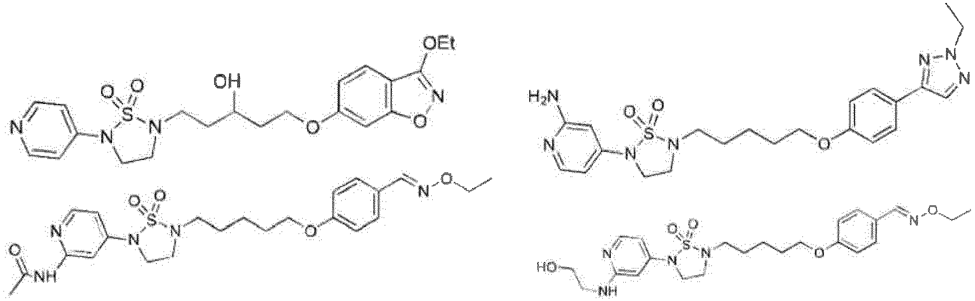

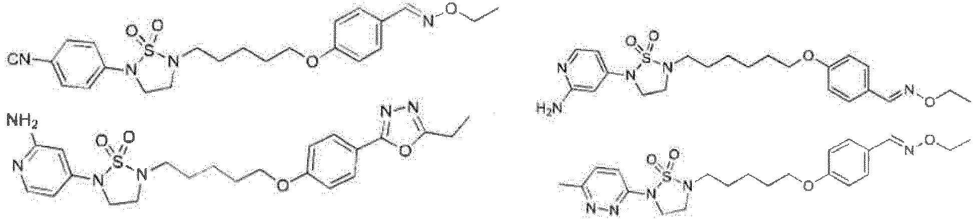

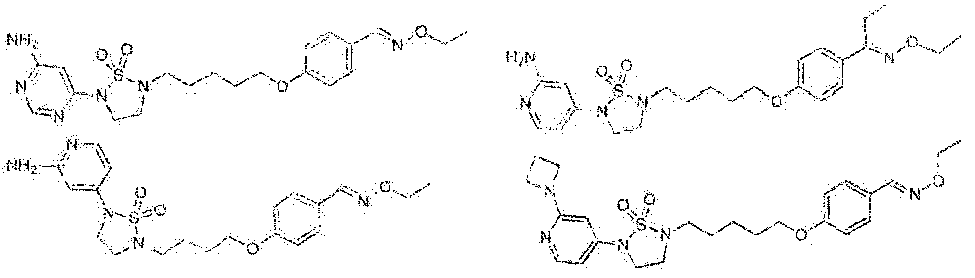

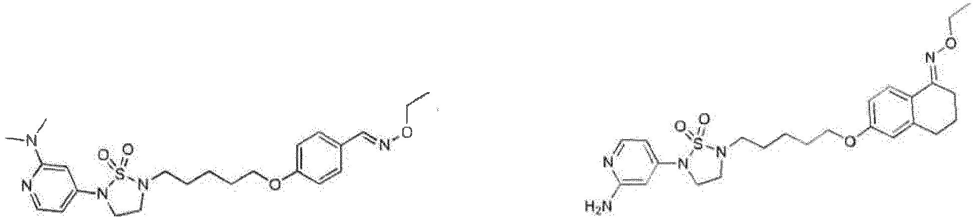

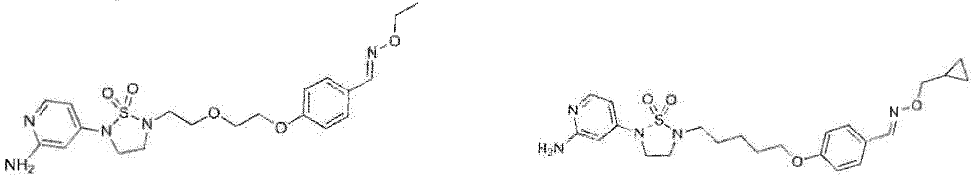

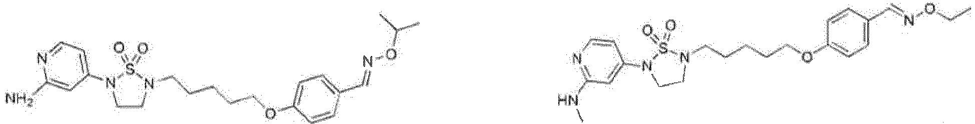

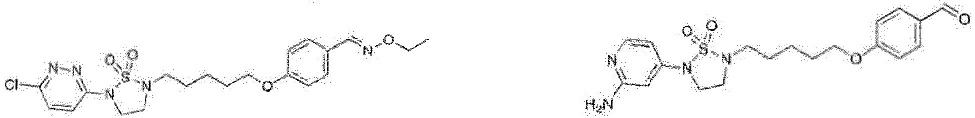

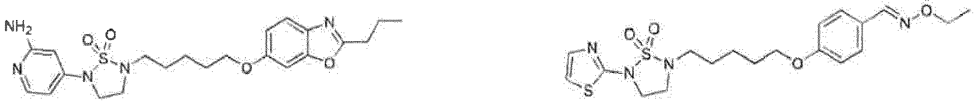

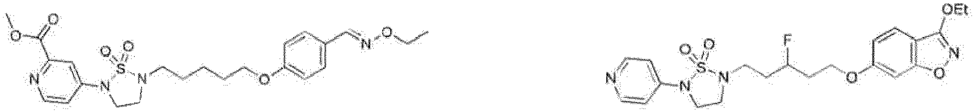

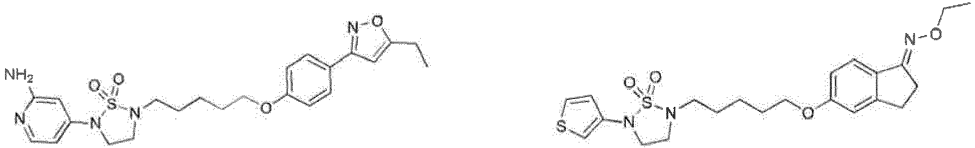

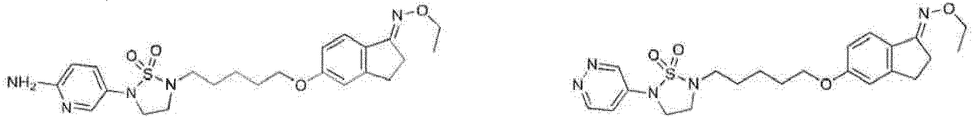

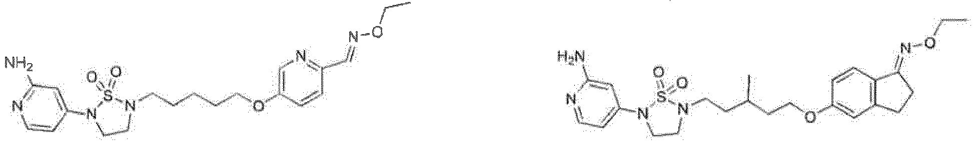

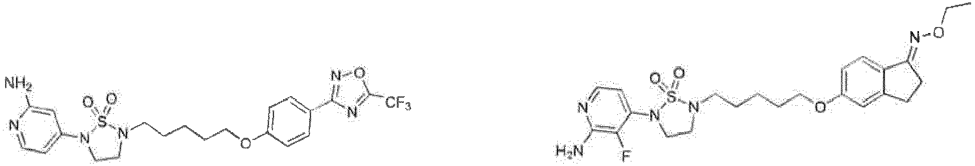

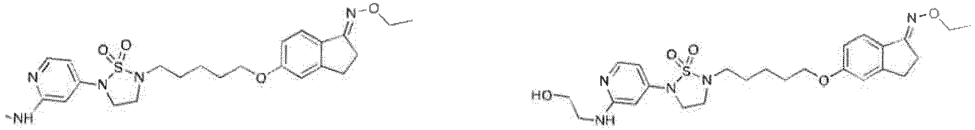

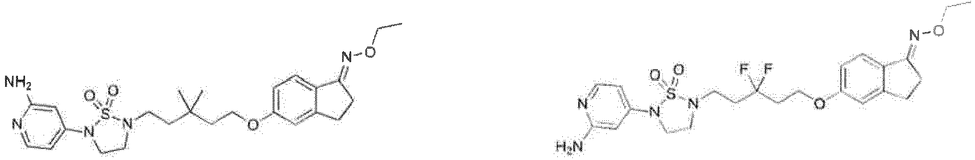

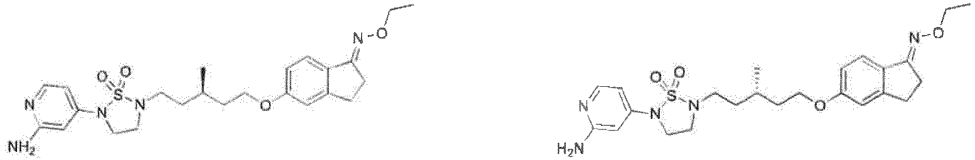

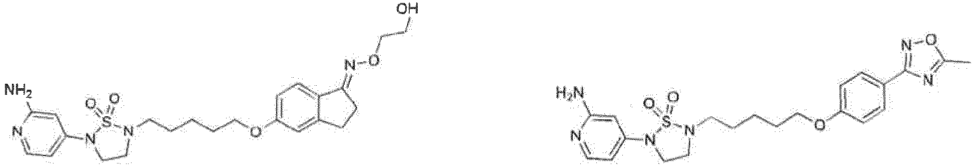

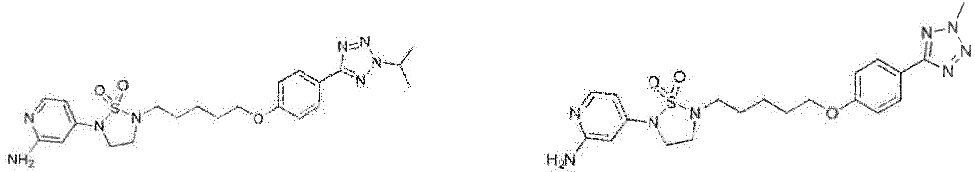

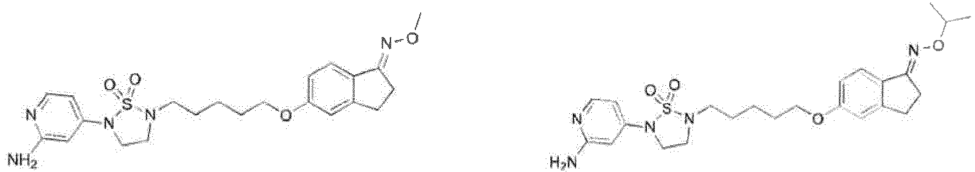

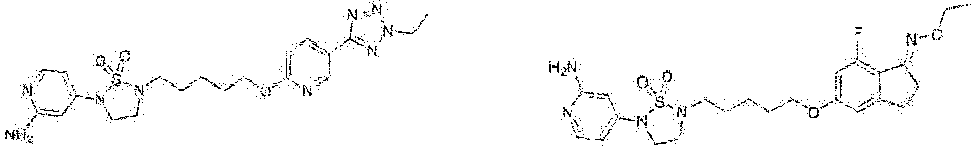

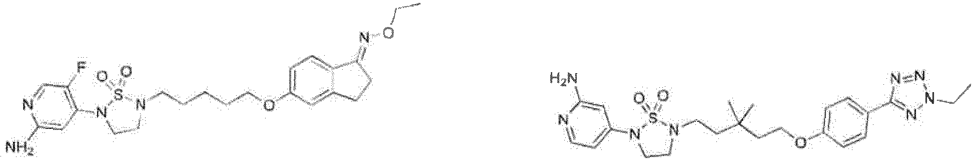

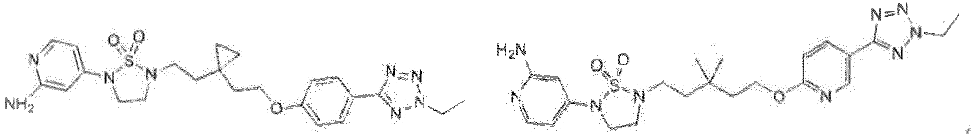

## Patentansprüche

1. Verbindung(en) oder ihr pharmazeutisch akzeptables Salz/ihre pharmazeutisch akzeptablen Salze, wie in Formel (II) gezeigt, 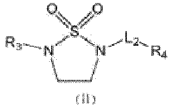
wobei R₃ ausgewählt ist aus 5-gliedrigen Heterocyclen , C₆₋₁₂-Arylgruppen, C₆₋₁₂-Aralkylgruppen, heteroaromatischen C₅₋₁₂-Ringen und C₅₋₁₂-Heteroarylgruppen substituiert mit R₀₁;
L₂ jeweils unabhängig ausgewählt ist aus 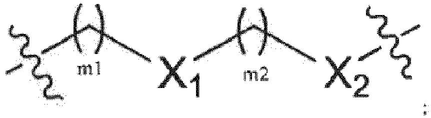
m₁ und m₂ jeweils unabhängig ausgewählt sind aus 1, 2 oder 3;
X₁ und X₂ jeweils unabhängig ausgewählt sind aus -C(R_{d1})(R_{d2})-, -C(=O)N(R_{d3})-, -N(R_{d4})-, -C(=NR_{d5})-, -S(=O)2N(R_{d6})-, -S(=O)N(R_{d7})-, -O-, -S-, -C(=O)O-, -C(=O)-, -C(=S)-, -S(=O)- oder -S(=O)₂-,
R₄ ausgewählt ist aus 5-14-gliedrigen cyclischen Hydrocarbyl- oder Heterocycloalkylgruppen,
R_{d1} und R_{d2} jeweils unabhängig ausgewählt sind aus H, F, Cl, Br, I, CN, OH, SH, NH₂, CHO, COOH oder beliebigen ausgewählt aus C₁₋₁₀-Alkyl- oder Heteroalkylgruppen und cyclischen C₃₋₁₀-Hydrocarbyl- oder Heterocycloalkylgruppen substituiert mit R₀₁, C₁₋₁₀-Alkyl- oder Heteroalkylgruppen substituiert mit C₃₋₁₀-Hydrocarbyl- oder Heterocycloalkylgruppen sind;
R₀₁ ausgewählt ist aus F, Cl, Br, I, CN, OH, SH, NH2, CHO, COOH, R₀₂;
R₀₂ ausgewählt ist aus C₁₋₁₀-Alkylgruppen, C₁₋₁₀-Alkylaminogruppen, N,N-(C₁₋₁₀-Alkyl)aminogruppen, C₁₋₁₀-Alkoxygruppen, C₁₋₁₀-Alkanoylgruppen, C₁₋₁₀-Alkoxycarbonylgruppen, C₁₋₁₀-Alkylsulfonylgruppen, C₁₋₁₀-Alkylsulfinylgruppen, C₃₋₁₀-Cycloalkylgruppen, C₃₋₁₀-Cycloalkylaminogruppen, heterocyclischen C₃₋₁₀-Alkylaminogruppen, C₃₋₁₀-Cycloalkoxygruppen, C₃₋₁₀-Cycloalkylacylgruppen, C₃₋₁₀-Cycloalkoxy-carbonylgruppen, C₃₋₁₀-Cycloalkylsulfonylgruppen und C₃₋₁₀-Cycloalkylsulfinylgruppen,
das "Hetero" für ein Heteroatom oder ein Heteroatomradikal steht, das ausgewählt ist aus -C(=O)N(R_{d3})-, -N(R_{d4})-, -C(=NR_{d5})-, -S(=O)₂N(R_{d6})-, -S(=O)N(R_{d7})-, -O-, -S-, =O, =S, -C(=O)O-, -C(=O)-, -C(=S)-, -S(=O)- und/oder -S(=O)₂;
R_{d3-d7} unabhängig ausgewählt ist aus H, R₀₃;
R₀₃ ausgewählt ist aus C₁₋₁₀-Alkylgmppen, C₁₋₁₀-Alkanoylgruppen, C₁₋₁₀-Alkoxycarbonylgruppen, C₁₋₁₀-Alkylsulfonylgmppen, C₁₋₁₀-Alkylsulfinylgruppen, C₃₋₁₀-Cycloalkylgruppen, C₃₋₁₀-Cycloalkylacylgruppen, C₃₋₁₀-Cycloalkoxy-carbonylgruppen, C₃₋₁₀-Cycloalkylsulfonylgruppen und C₃₋₁₀-Cycloalkylsulfinylgruppen,
R₀₂ und R₀₃ beliebige zwingend mit R₀₀₁ substituierte sind;
R₀₀₁ ausgewählt ist aus F, Cl, Br, I, CN, OH, N(CH₃)₂, NH(CH₃), NH₂, CHO, COOH, einer Trifluormethylgruppe, Aminomethylgruppe, Hydroxymethylgruppe, Methylgruppe, Methoxygruppe, Formylgruppe, Methoxycarbonylgruppe, Methylsulfonylgruppe und Methylsulfinylgruppe;
die Anzahl von R₀₁, R₀₀₁ und Heteroatomen oder Heteroatomradikalen jeweils unabhängig ausgewählt ist aus 0, 1, 2 oder 3;
wobei wahlweise R_{d1} und R_{d2} zu einem 3- oder 4-gliedrigen carbocyclischen oder heterocyclischen Ring verbunden sind.

2. Verbindung(en) oder ihr pharmazeutisch akzeptables Salz/ihre pharmazeutisch akzeptablen Salze nach Anspruch 1, wobei: R₃ ein beliebiges ausgewählt aus substituierter 5-6-gliedriger Aryl- und substituierter Heteroarylgruppe ist;
R₃ vorzugsweise ein beliebiges wahlweise ausgewählt aus einer Pyridylgruppe, Phenylgruppe, Furanylgruppe, Pyrazolylgruppe, Pyrrolylgruppe, Thiazolylgruppe, Pyridazinylgruppe, Pyrimidinylgruppe und Thienylgruppe substituiert durch R₀₁ ist und die Definition von R₀₁ wie in Anspruch 1 ist und die Anzahl von R₀₁ ausgewählt ist aus 0, 1 2 oder 3;
wobei vorzugsweise R₀₁ ausgewählt ist aus F, Cl, Br, I, CN, OH, SH, NH₂, CHO, COOH, Me,

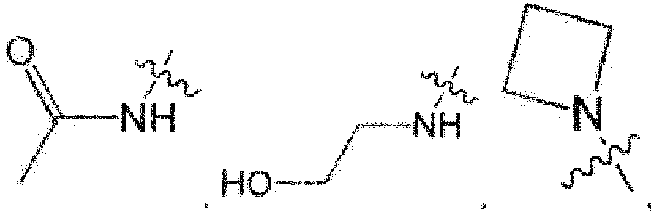

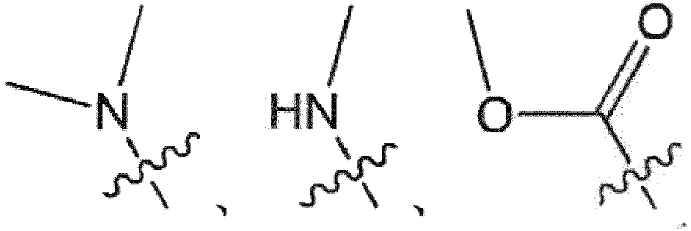

3. Verbindung(en) oder ihr pharmazeutisch akzeptables Salz/ihre pharmazeutisch akzeptablen Salze nach Anspruch 2, wobei: R₃ ausgewählt ist aus:

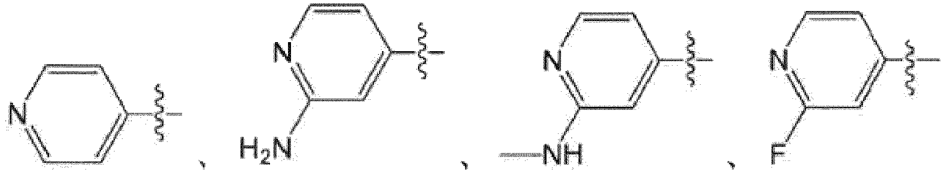

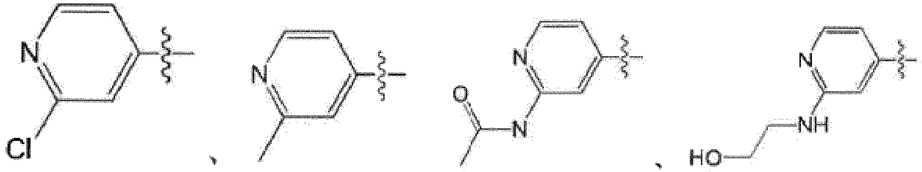

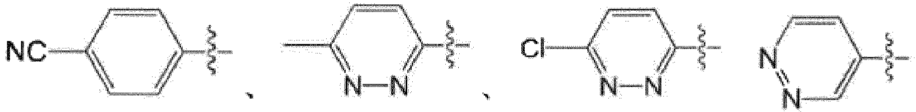

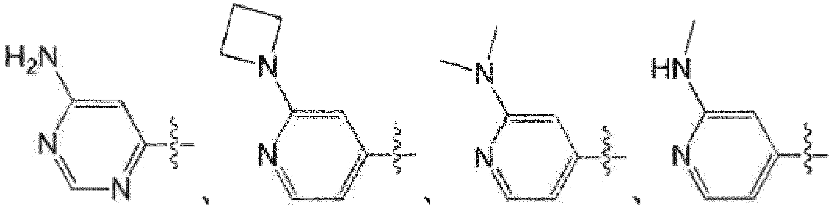

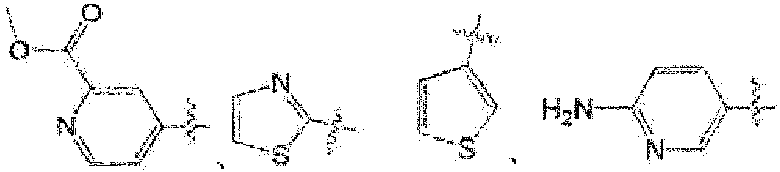

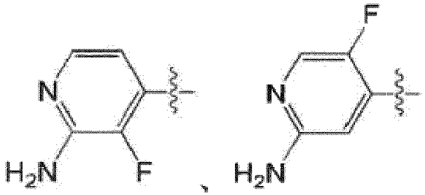

4. Verbindung(en) oder ihr pharmazeutisch akzeptables Salz/ihre pharmazeutisch akzeptablen Salze nach Anspruch 1, wobei:
X₁, X₂ jeweils unabhängig ausgewählt sind aus - O -, -C(=O)-, -CH(CH₃)-, -C(CH₃)₂-, -CF₂-, -CH(F) 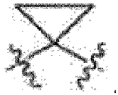 -CH(OH), -CH₂-, -NH- und -N(CH₃)-;
wobei vorzugsweise L₂ ausgewählt ist aus: 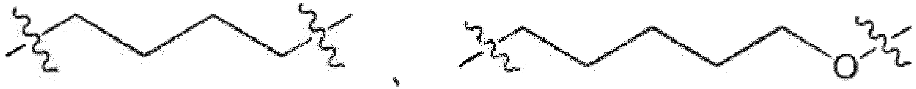 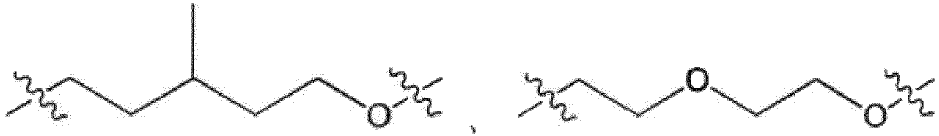 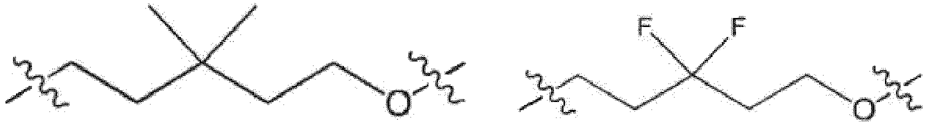 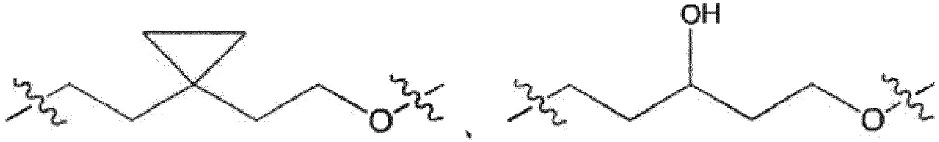 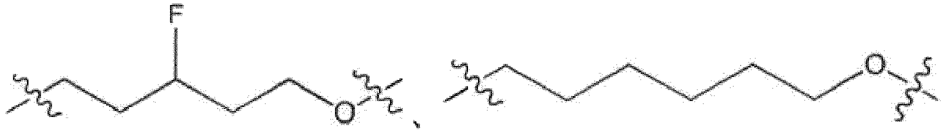 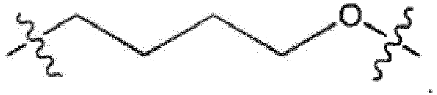 während andere Variablen wie in Anspruch 1 definiert sind.

5. Verbindung(en) oder ihr pharmazeutisch akzeptables Salz/ihre pharmazeutisch akzeptablen Salze nach Anspruch 1, wobei: R₄ ausgewählt ist aus den Folgenden: 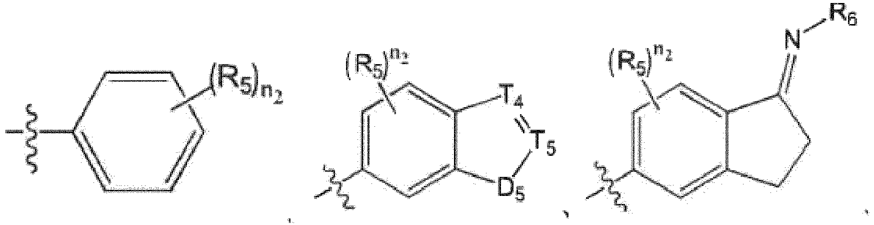 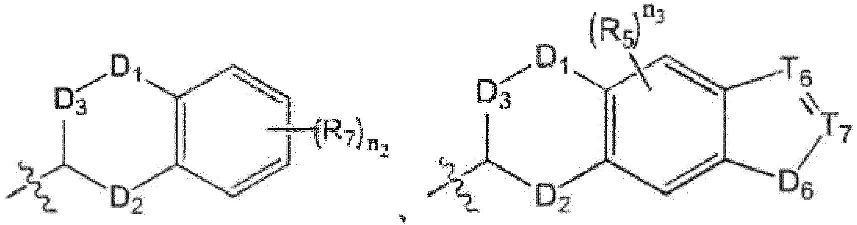 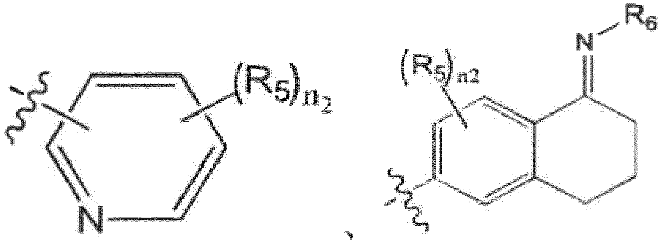
T₄₋₇ jeweils unabhängig ausgewählt ist aus N oder C(Rₜ);
D₁₋₃, D₅ und D₆ jeweils unabhängig ausgewählt sind aus einer Einzelbindung, -C(R_{d1})(R_{d2})-, -C(=O)N(R_{d3})-, -N(R_{d4})-, -C(=NR_{d5})-, -S(= O)₂N(R_{d6})-, -S(=O)N(R_{d7})-, -O-, -S-, -C(=O)O-, -C(= O)-, -C(=S)-, -S(=O)- oder -S(=O)₂;
n₂ ausgewählt ist aus 0, 1, 2 oder 3;
n₃ ausgewählt ist aus 0, 1 oder 2;
R₅, R₆, R₇, Rₜ, R_{d1} und R_{d2} jeweils unabhängig ausgewählt sind aus H, F, Cl, Br, I, CN, OH, SH, NH₂, CHO und COOH oder beliebige ausgewählt aus C₁₋₁₀-Alkyl- oder Heteroalkylgruppen und cyclischen C₃₋₁₀-Hydrocarbyl- oder Heterocycloalkylgruppen substituiert mit R₀₁, C₁₋₁₀-Alkyl- oder Heteroalkylgruppen substituiert mit C₃₋₁₀-Hydrocarbyl- oder Heterocycloalkylgruppen, während andere Variablen wie in Anspruch 1 definiert sind.

6. Verbindung(en) oder ihr pharmazeutisch akzeptables Salz/ihre pharmazeutisch akzeptablen Salze
nach Anspruch 5, wobei: R₄ ausgewählt ist aus den Folgenden: 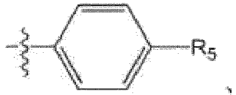 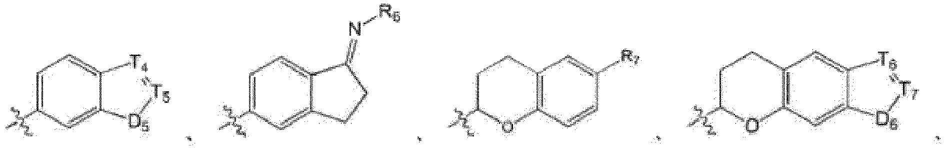 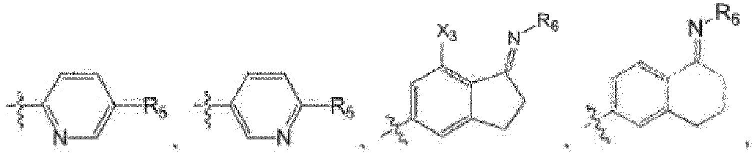 X₃ ausgewählt ist aus F, Cl, Br, I, CN, OH, SH, NH₂, CHO, COOH oder beliebigen ausgewählt aus C₁₋₁₀-Alkyl- oder Heteroalkylgruppen und cyclischen C₃₋₁₀-Hydrocarbyl- oder Heterocycloalkylgruppen substituiert mit R₀₁, C₁₋₁₀-Alkyl- oder Heteroalkylgruppen substituiert mit C₃₋₁₀-Hydrocarbyl- oder Heterocycloalkylgruppen sind;

7. Verbindung(en) oder ihr pharmazeutisch akzeptables Salz/ihre pharmazeutisch akzeptablen Salze nach Anspruch 5 oder Anspruch 6, wobei: R₅₋₇ jeweils unabhängig ausgewählt ist aus: 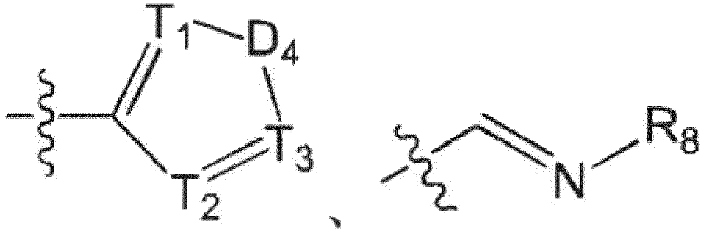 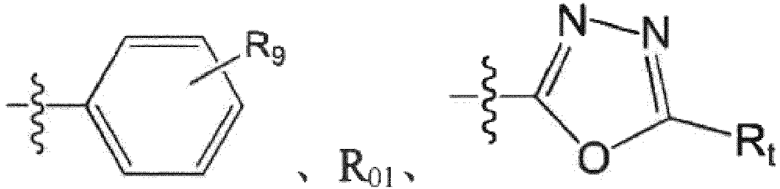 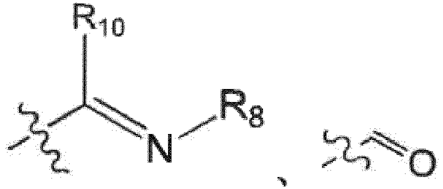 wobei:
T₁₋₃ jeweils unabhängig ausgewählt ist aus N oder C(Rₜ);
D₄ ausgewählt ist aus -C(R_{d1})(R_{d2})-, -C(=O)N(R_{d3})-, -N(R_{d4})-, -C(=NR_{d5})-, -S(=O)2N(R_{d6})-, -S(=O)N(R_{d7})-, -O-, -S-, -C(=O)O-, -C(= O)-, -C(=S)-, -S(=O)- oder -S(=O)₂-;
R₈, R₉ und Rₜ jeweils unabhängig ausgewählt sind aus H, F, Cl, Br, I, CN, OH, SH, NH₂, CHO, COOH oder beliebige ausgewählt aus C₁₋₁₀-Alkyl- oder Heteroalkylgruppen und cyclischen C₃₋₁₀-Hydrocarbyl- oder Heterocycloalkylgruppen substituiert mit R₀₁, C₁₋₁₀-Alkyl- oder Heteroalkylgruppen substituiert mit C₃₋₁₀-Hydrocarbyl- oder Heterocycloalkylgruppen sind;
R₁₀ ausgewählt ist aus F, Cl, Br, I, CN, OH, SH, NH₂, CHO, COOH oder beliebigen ausgewählt aus C₁₋₁₀-Alkyl- oder Heteroalkylgruppen und cyclischen C₃₋₁₀-Hydrocarbyl- oder Heterocycloalkylgruppen substituiert mit R₀₁, C₁₋₁₀-Alkyl- oder Heteroalkylgruppen substituiert mit C₃₋₁₀-Hydrocarbyl- oder Heterocycloalkylgruppen sind;

8. Verbindung(en) oder ihr pharmazeutisch akzeptables Salz/ihre pharmazeutisch akzeptablen Salze nach Anspruch 7, wobei: R₅₋₉, R₀₁ und Rₜ jeweils unabhängig ausgewählt sind aus den Folgenden: 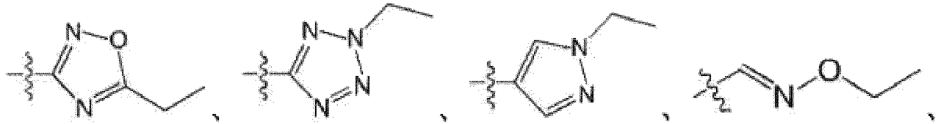 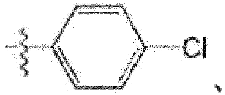 F, Cl, Br, CF₃, -C(C₂H₅)-, -C(OC₂H₅)-, einer Methylgruppe, Ethylgruppe, Propylgruppe, Methoxygruppe, Ethoxygruppe, Propoxygruppe,

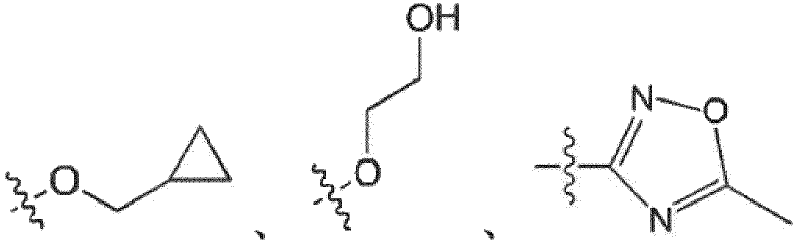

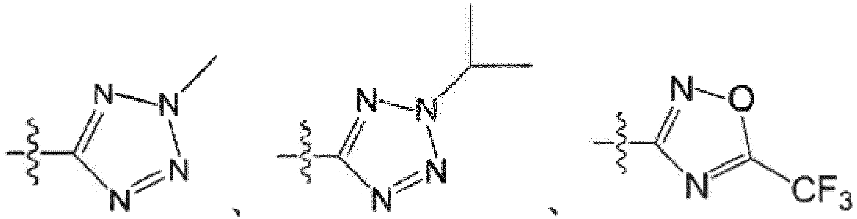

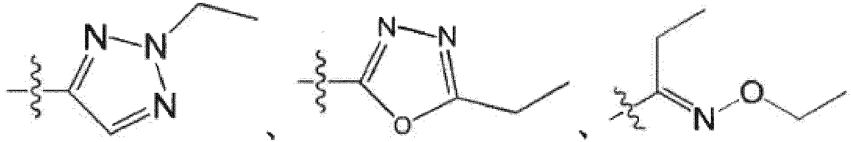

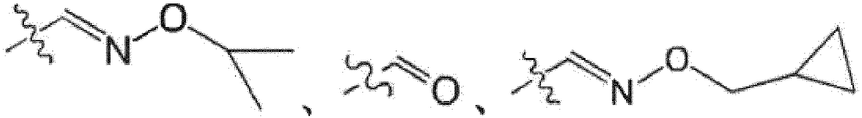

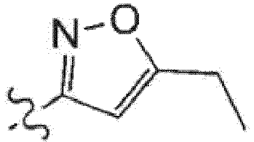

9. Verbindung(en) oder ihr pharmazeutisch akzeptables Salz/ihre pharmazeutisch akzeptablen Salze
nach Anspruch 8, wobei: R₄ ausgewählt ist aus den Folgenden:

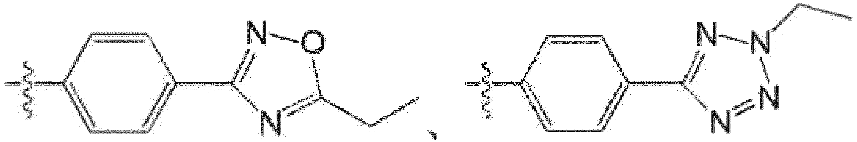

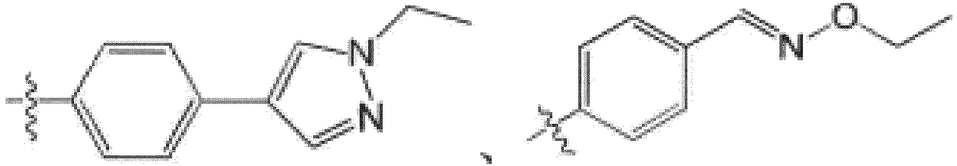

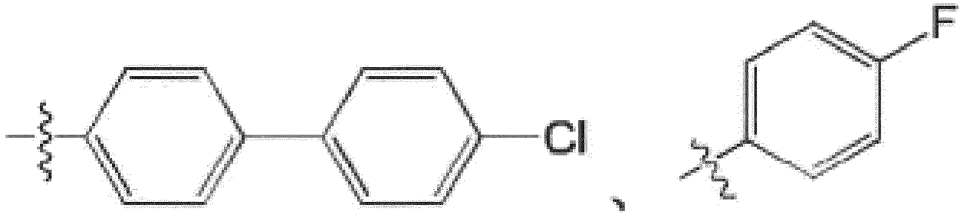

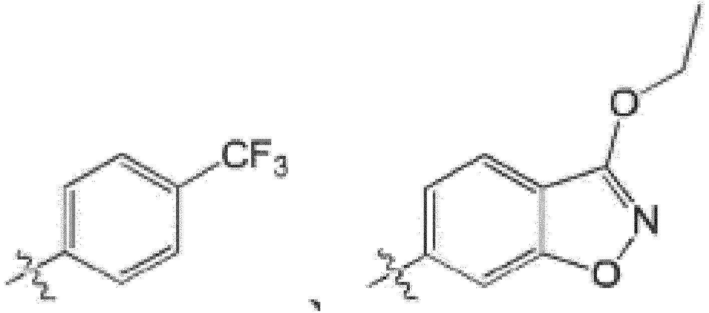

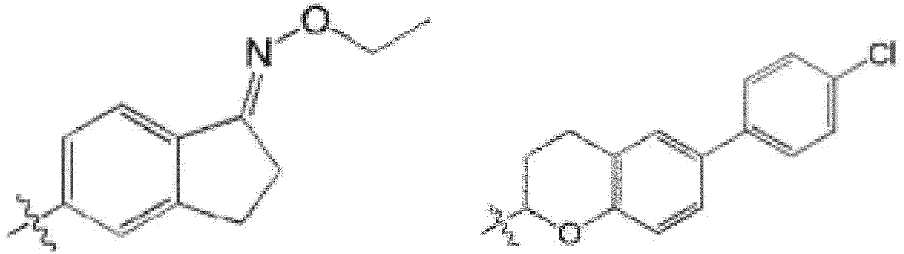

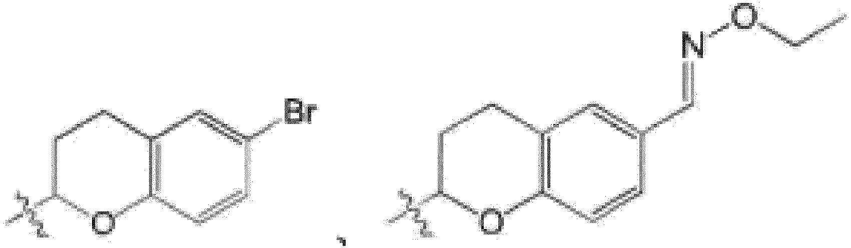

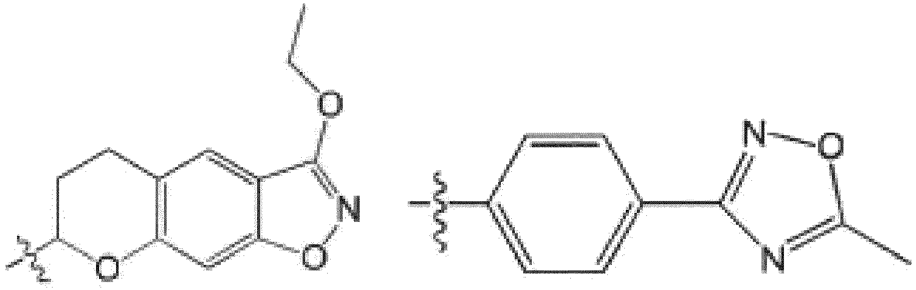

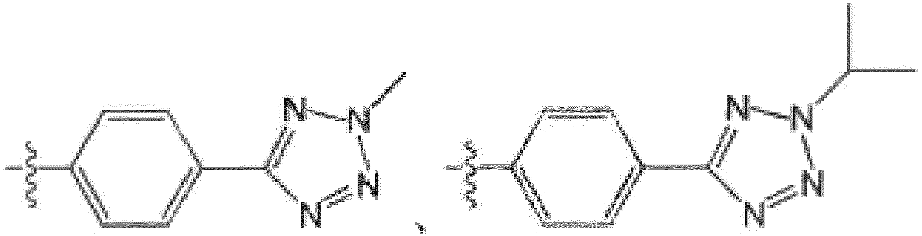

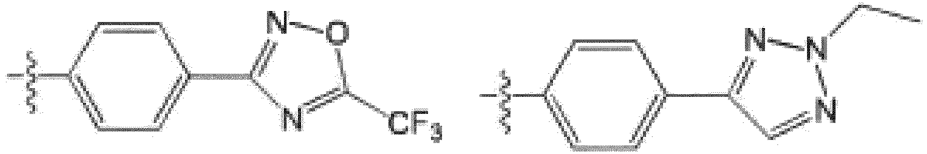

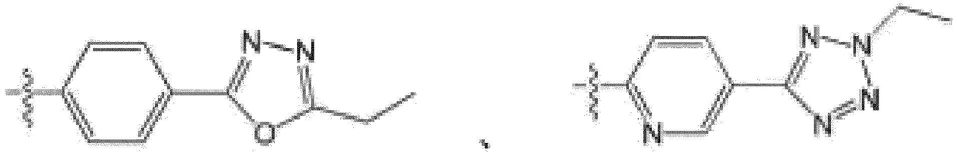

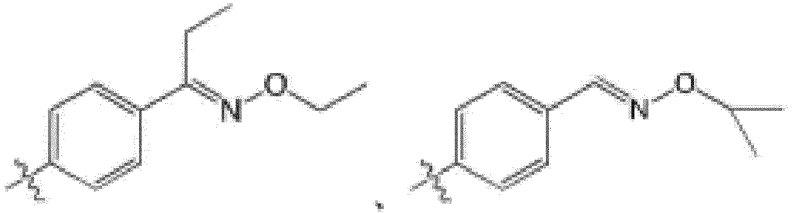

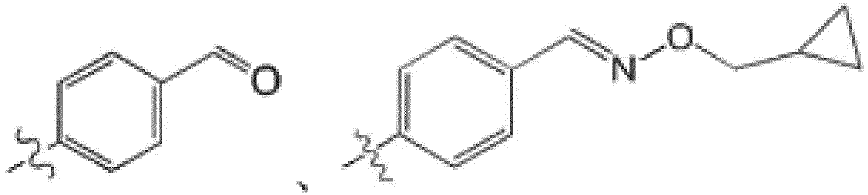

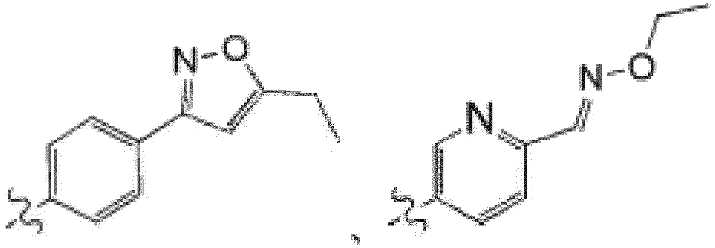

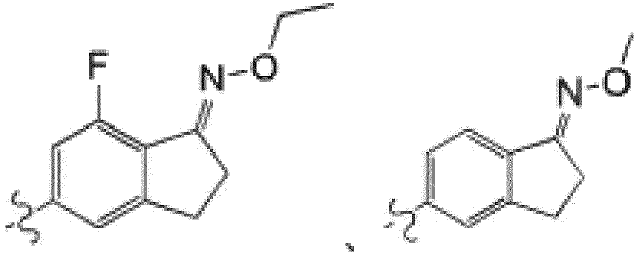

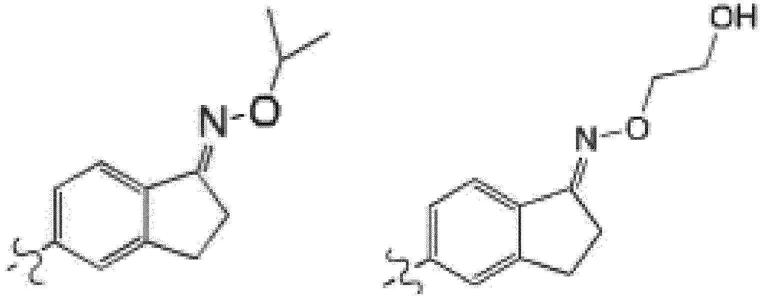

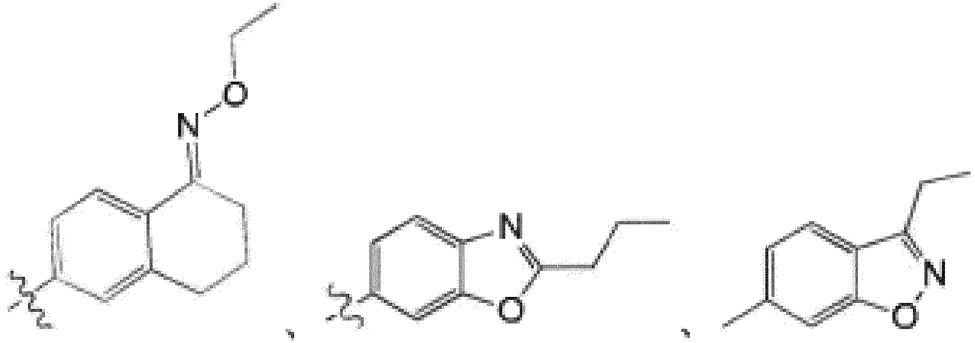

10. Verbindung(en) oder ihr pharmazeutisch akzeptables Salz/ihre pharmazeutisch akzeptablen Salze
nach Anspruch 1, ausgewählt aus den Folgenden

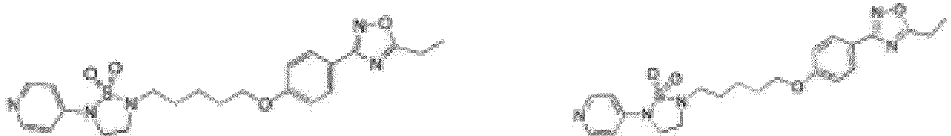

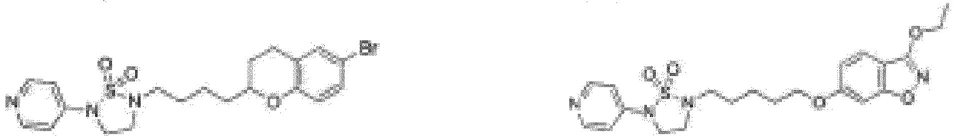

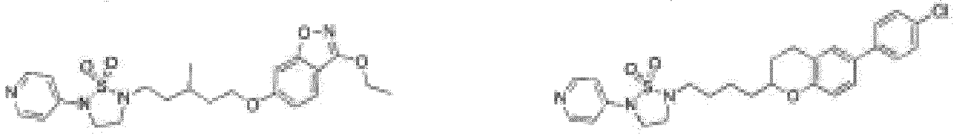

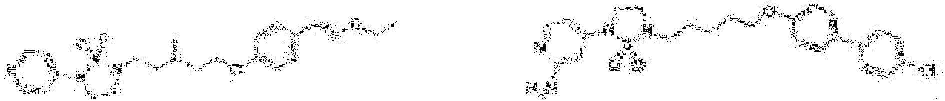

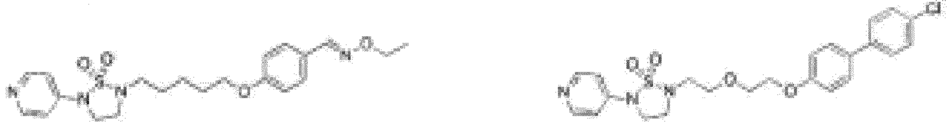

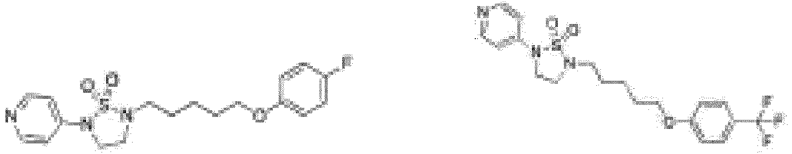

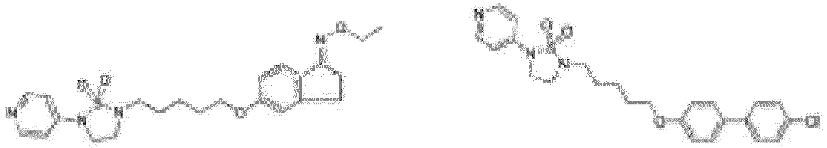

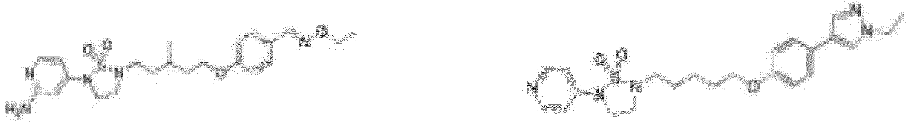

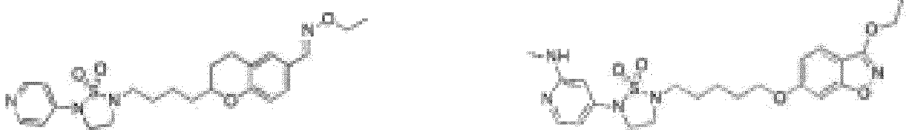

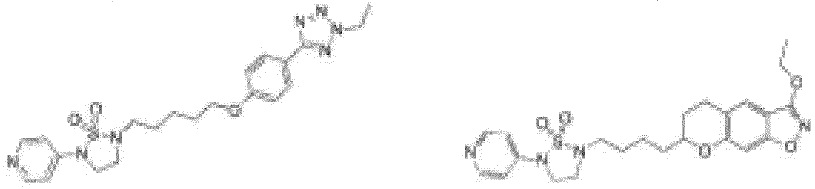

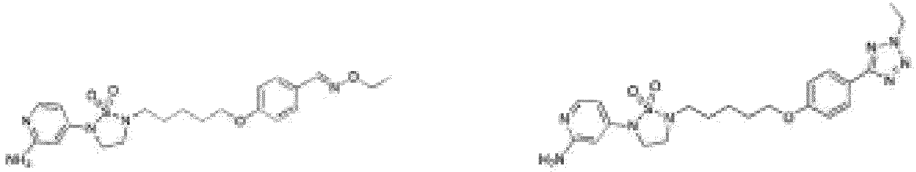

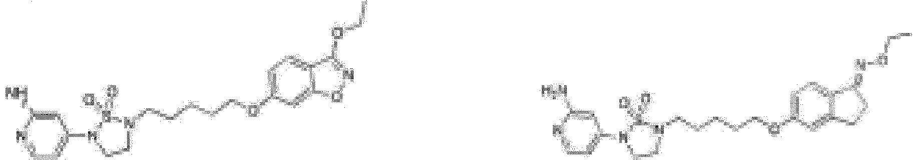

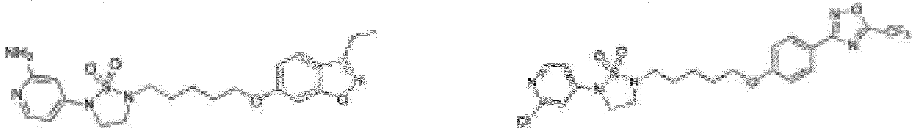

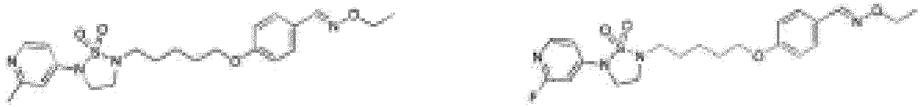

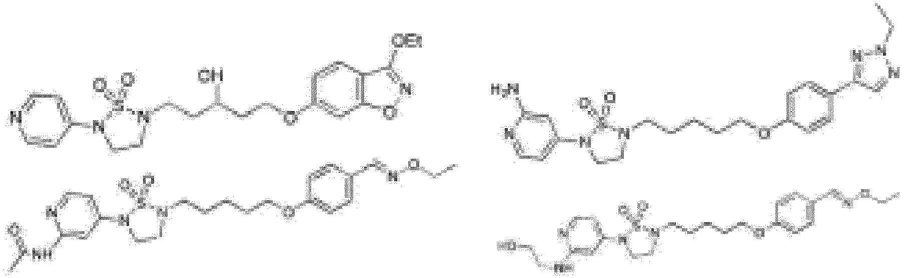

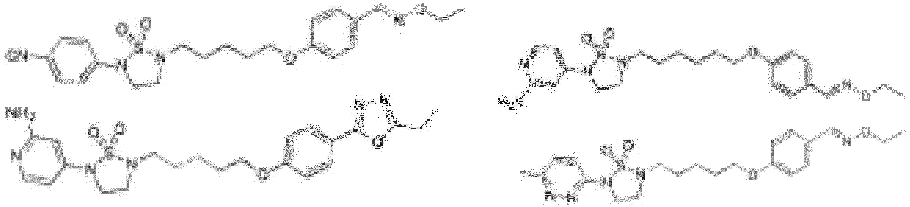

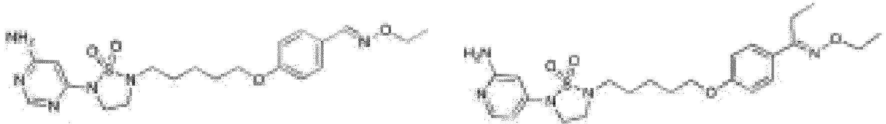

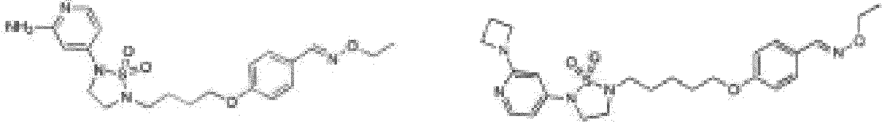

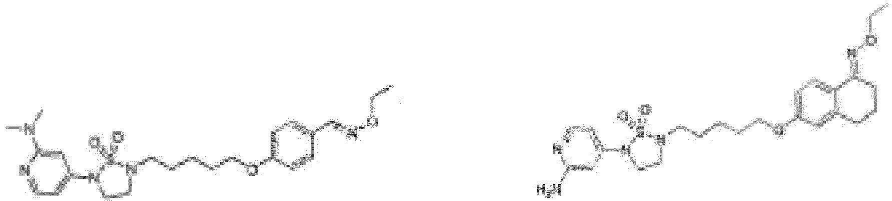

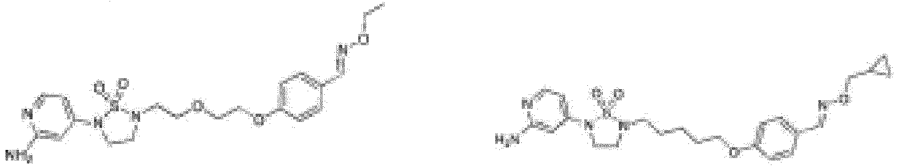

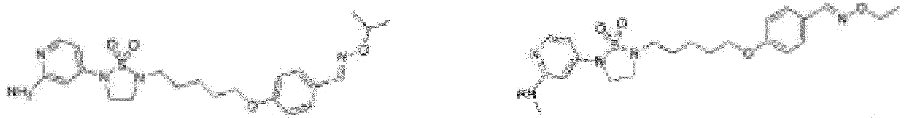

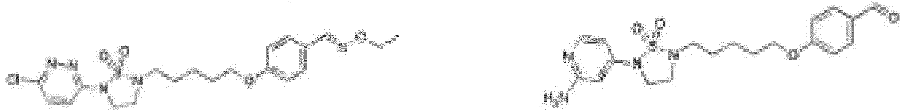

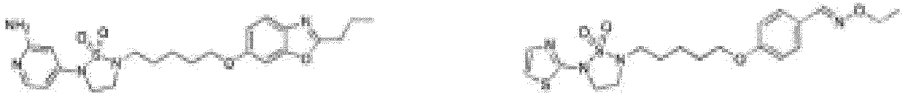

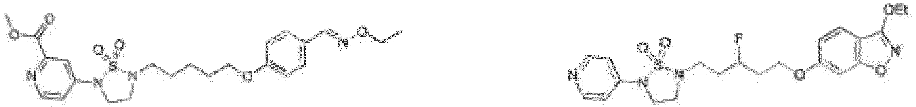

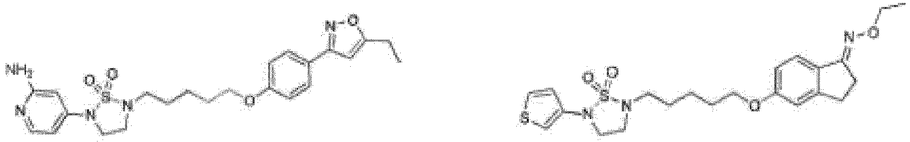

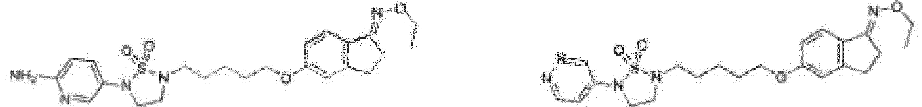

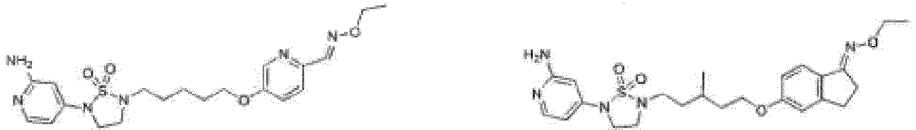

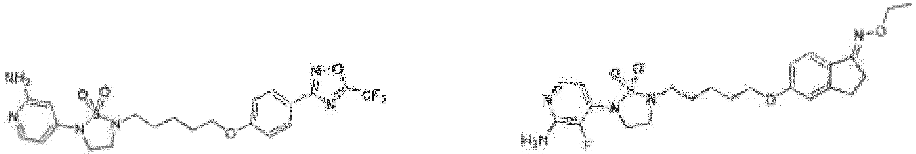

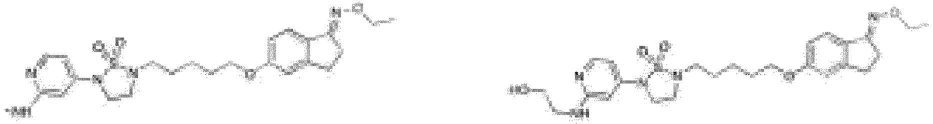

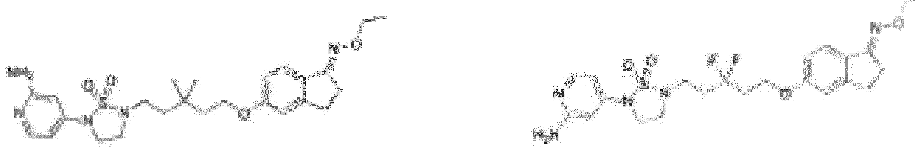

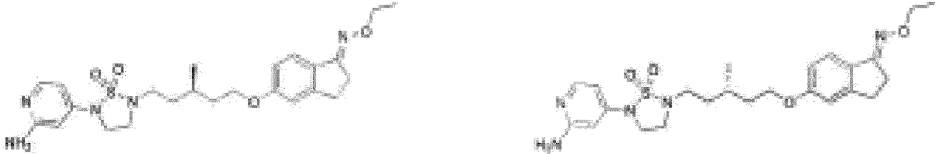

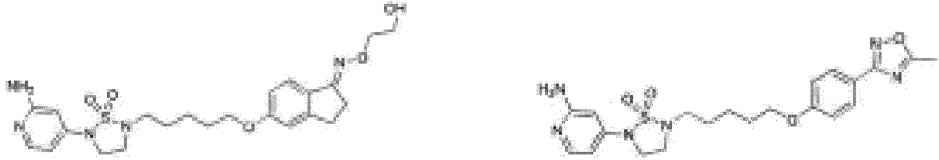

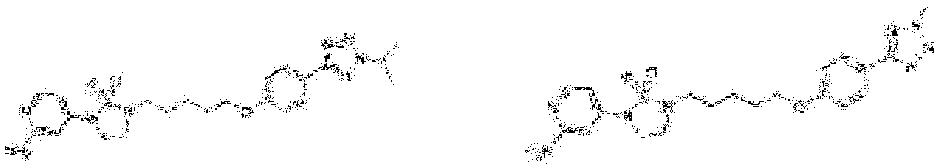

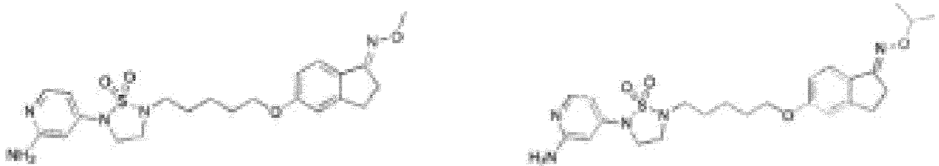

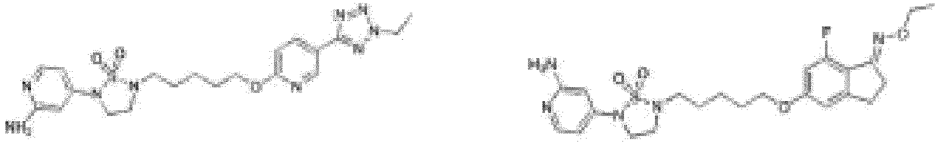

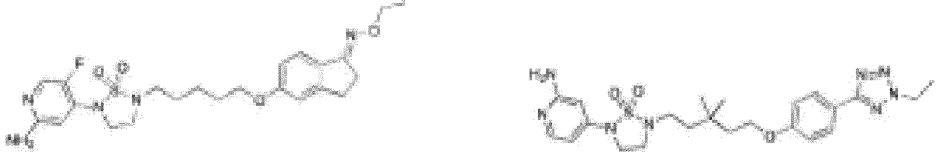

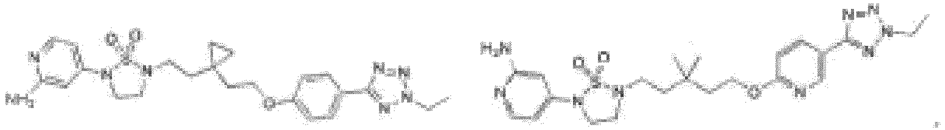

## Revendications

1. Composé(s) ou leurs sels pharmaceutiquement acceptables tel que représenté à la Formule (II), 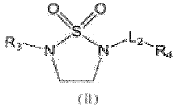
R₃ étant sélectionné parmi 5 hétérocycles, groupes aryles C_{6∼12}, groupes aralkyles C_{6∼12}, cycles hétéroaromatiques C_{5∼12} et hétérogroupes aryles C_{5∼12} remplacés par R₀₁;
L₂ est indépendamment sélectionné respectivement parmi 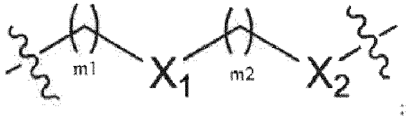
m₁ et m₂ sont indépendamment sélectionnés respectivement entre 1, 2, ou 3 ;
X₁ et X₂ sont indépendamment sélectionnés entre -C(R_{d1})(R_{d2})-, -C(=O)N(R_{d3})-, -N(R_{d4})-, -C(=NR_{d5})-, -S(=O)₂N(R_{d6})-, -S(=O)N(R_{d7})-, -O-, -S-, -C(=O)O-, -C(=O)-, -C(=S)-, -S(=O)- ou-S(=O)₂-,
R₄ est sélectionné parmi 5∼14 groupes hydrocarbyles ou hétérocycloalkyles cycliques,
R_{d1} et R_{d2} sont indépendamment sélectionnés respectivement entre H, F, Cl, Br, I, CN, OH, SH, NH₂, CHO, COOH ou sélectionnés parmi les groupes alkyles ou hétéroalkyles C₁₋₁₀ et les groupes hydrocarbyles ou hétérocycloalkyles cycliques C₃₋₁₀ remplacés par les groupes alkyles ou hétéroalkyles R₀₁, C₁₋₁₀ remplacés par les groupes hydrocarbyles ou hétérocycloalkyles C₃₋₁₀;
R₀₁ est sélectionné entre F, Cl, Br, I, CN, OH, SH, NH2, CHO, COOH, R₀₂ ;
R₀₂ est sélectionné parmi les groupes alcalis C₁₋₁₀, les groupes alkylamino C₁₋₁₀, les groupes amino N,N-(alcali C₁₋₁₀), les groupes alkoxy C₁₋₁₀, les groupes alcanoyles C₁₋₁₀, les groupes alkoxycarbonyles C₁₋₁₀, les groupes alkylsulfonyles C₁₋₁₀, les groupes alkylsulfinyles C₁₋₁₀, les groupes cycloalkyles C₃₋₁₀, les groupes cycloalkylamino C₃₋₁₀, les groupes alkyles amino hétérocycliques C₃₋₁₀, les groupes cycloalkoxy C₃₋₁₀, les groupes cycloalkyles acyles C₃₋₁₀, les groupes cycloalkoxy-carbonyles C₃₋₁₀, les groupes cycloalkylsulfonyles C₃₋₁₀ et les groupes cycloalkylsulfinyles C₃₋₁₀ ;
[0010] « hétéro » représente un hétéroatome ou un radical hétéroatomique sélectionné entre -C(=O)N(R_{d3})-, -N(R_{d4})-, -C(=NR_{d5})-, -S(=O)₂N(R_{d6})-, -S(=O)N(R_{d7})-, -O-, -S-, =O, =S, -C(=O)O-, -C(=O)-, -C(=S)-, -S(=O)- et/ou-S(=O)₂;
R_{d3-d7} est indépendamment sélectionné entre H, R₀₃ ;
R₀₃ est sélectionné parmi les groupes alkyles C₁₋₁₀, les groupes alcanoyles C₁₋₁₀, les groupes alkoxycarbonyles C₁₋₁₀, les groupes alkylsulfonyles C₁₋₁₀, les groupes alkylsulfinyles C₁₋₁₀, les groupes cycloalkyles C₃₋₁₀, les groupes cycloalkyles acyles C₃₋₁₀, les groupes cycloalkoxy-carbonyles C₃₋₁₀, les groupes cycloalkylsulfonyles C₃₋₁₀ et les groupes cycloalkylsulfinyles C₃₋₁₀ ;
R₀₂ et R₀₃ sont remplacés nécessairement par R₀₀₁ ;
R₀₀₁ est sélectionné entre F, Cl, Br, I, CN, OH, N(CH₃)₂, NH(CH₃), NH₂, CHO, COOH, le groupe trifluorométhyle, le groupe aminométhyle, le groupe hydroxyméthyle, le groupe méthyle, le groupe méthoxy, le groupe formyle, le groupe méthoxycarbonyle, le groupe méthylsulfonyle et le groupe méthylsulfinyle ;
le nombre de R₀₁, R₀₀₁ et d'hétéroatomes ou de radicaux hétéroatomiques est indépendamment sélectionné respectivement entre 0, 1, 2 ou 3 ;
éventuellement, R_{d1} et R_{d2} sont interconnectés ensemble pour former un cycle carbocyclique ou hétérocyclique à 3 ou 4 chaînons.

2. Le ou lesdits composé(s) ou leurs sels pharmaceutiquement acceptables selon la revendication 1, où R₃ est sélectionné entre le groupe aryle à 5∼6 chaînons remplacé et le groupe hétéroaryle remplacé ;
[0018] de préférence, R₃ est sélectionné éventuellement entre le groupe pyridyle, le groupe phényle, le groupe furanyle, le groupe pyrazolyle, le groupe pyrrolyle, le groupe thiazolyle, le groupe pyridazinyle, le groupe pyrimidinyle et le groupe thiényle remplacés par R₀₁ et la définition de R₀₁ est selon la revendication 1 et le nombre de R₀₁ est sélectionné parmi 0, 1, 2 ou 3 ;
de préférence, R₀₁ est sélectionné entre F, Cl, Br, I, CN, OH, SH, NH₂, CHO, COOH, Me,

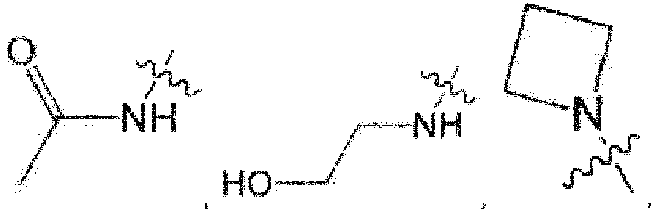

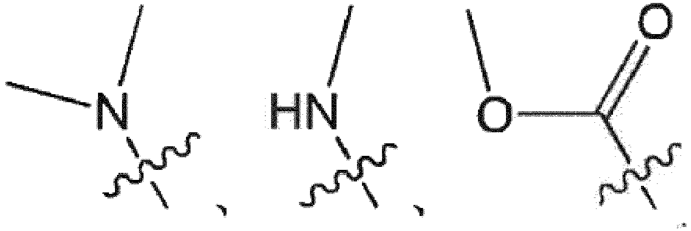

3. Le ou lesdits composé(s) ou leurs sels pharmaceutiquement acceptables selon la revendication 2, où R₃ est sélectionné entre :

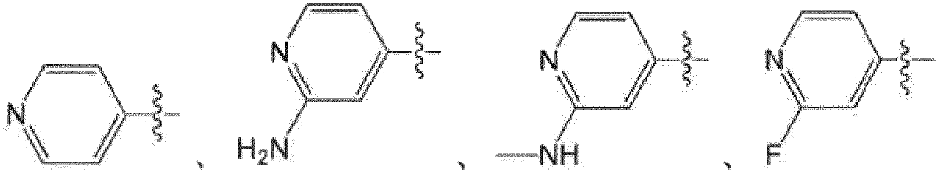

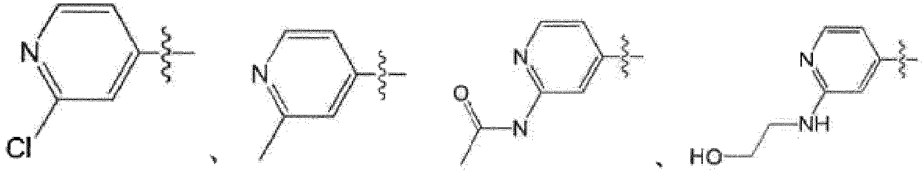

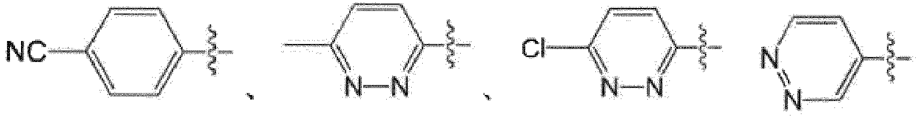

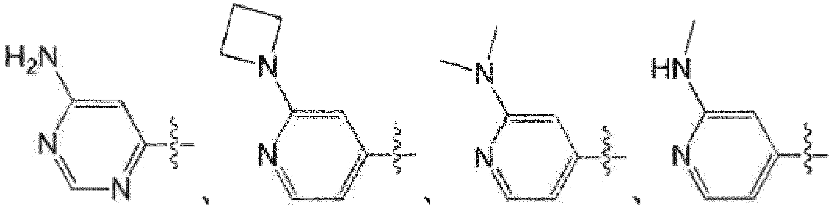

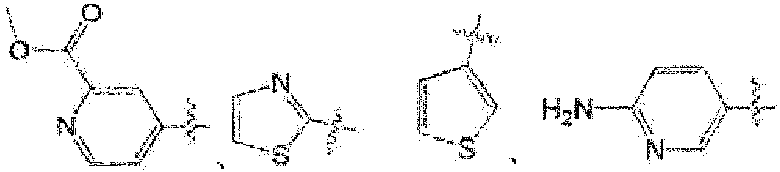

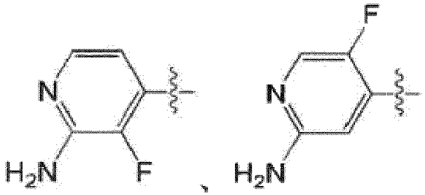

4. Le ou lesdits composé(s) ou leurs sels pharmaceutiquement acceptables selon la revendication 1, dans lesquels :
X₁, X₂ sont indépendamment sélectionnés respectivement entre -O-, -C(=O)-, -CH(CH₃)-, -C(CH₃)₂-, -CF₂-, -CH(F) 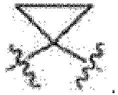 -CH(OH), -CH₂-, -NH- et -N(CH₃)- ;
de préférence, L₂ est sélectionné entre : 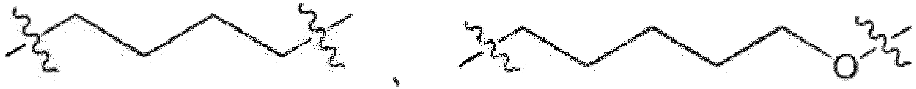 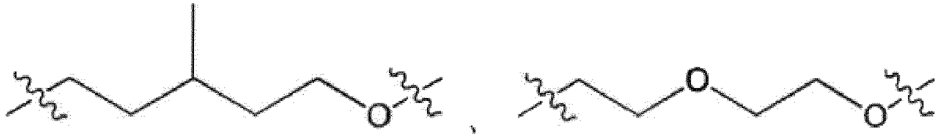 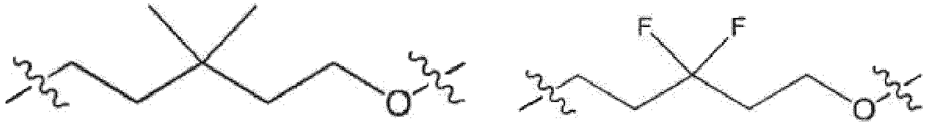 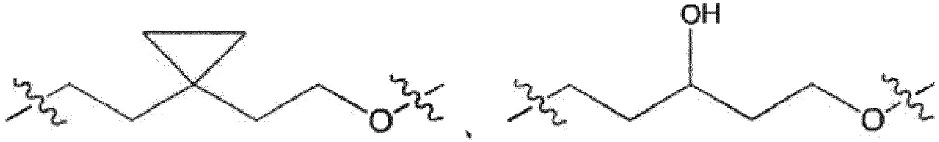 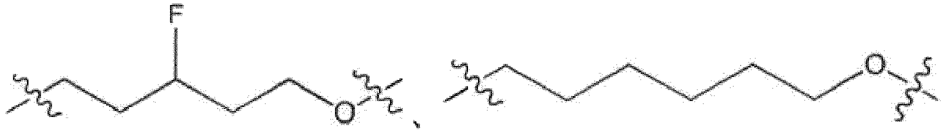 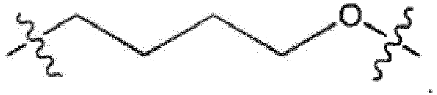 les autres variables sont définies selon la revendication 1.

5. Le ou lesdits composé(s) ou leurs sels pharmaceutiquement acceptables selon la revendication 1, dans lesquels R₄ est sélectionné entre : 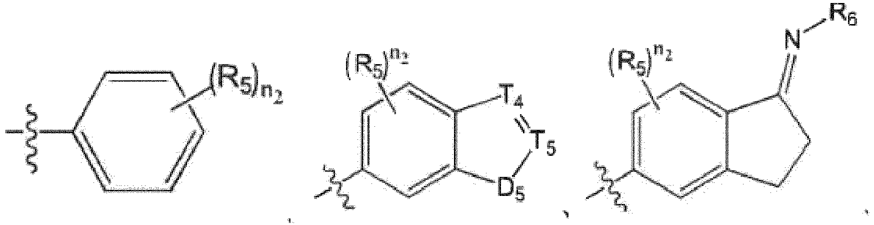 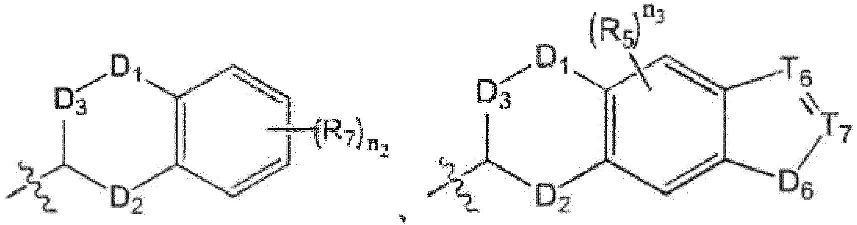 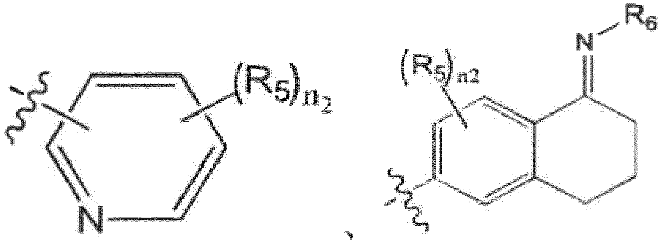
T₄₋₇ est indépendamment sélectionné respectivement entre N ou C(Rₜ) ;
D₁₋₃, D₅ et D₆ sont indépendamment sélectionnés respectivement parmi les liaisons simples, -C(R_{d1})(R_{d2})-, -C(=O)N(R_{d3})-, -N(R_{d4})-, -C(=NR_{d5})-, -S(=O)₂N(R_{d6})-, -S(=O)N(R_{d7})-, -O-, -S-, -C(=O)O-, -C(=O)-, -C(=S)-, -S(=O)- ou -S(=O)₂ ;
n₂ est sélectionné entre 0, 1, 2 ou 3 ;
n₃ est sélectionné entre 0, 1 ou 2 ;
R₅, R₆, R₇, Rₜ, R_{d1} et R_{d2} sont indépendamment sélectionnés respectivement entre H, F, Cl, Br, I, CN, OH, SH, NH₂, CHO et COOH ou sélectionnés parmi les groupes alkyles ou hétéroalkyles C₁₋₁₀ et les groupes hydrocarbyles ou hétérocycloalkyles cycliques C₃₋₁₀ remplacés par R₀₁, les groupes alkyles ou hétéroalkyles C₁₋₁₀ remplacés par les groupes hydrocarbyles ou hétérocycloalkyles C₃₋₁₀, les autres variables étant définies selon la revendication 1.

6. Le ou lesdits composé(s) ou leurs sels pharmaceutiquement acceptables selon la revendication 5, dans
lesquels R₄ est sélectionné entre : 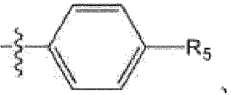 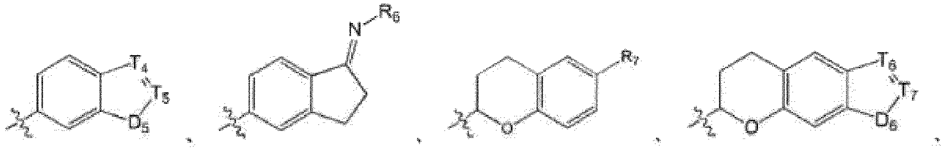 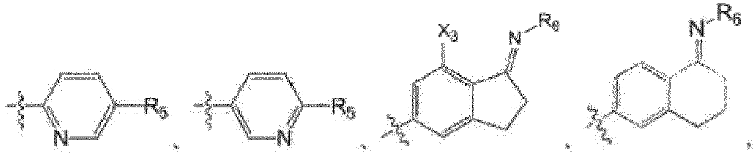 X₃ est sélectionné entre F, Cl, Br, I, CN, OH, SH, NH₂, CHO, COOH ou sélectionné parmi les groupes alkyles ou hétéroalkyles C₁₋₁₀ et les groupes hydrocarbyles ou hétérocycloalkyles cycliques C₃₋₁₀ remplacés par R₀₁, les groupes alkyles ou hétéroalkyles C₁₋₁₀ remplacés par les groupes hydrocarbyles ou hétérocycloalkyles C₃₋₁₀.

7. Le ou lesdits composé(s) ou leurs sels pharmaceutiquement acceptables selon la revendication 5 ou la revendication 6, dans lesquels R₅₋₇ sont indépendamment sélectionnés respectivement entre : 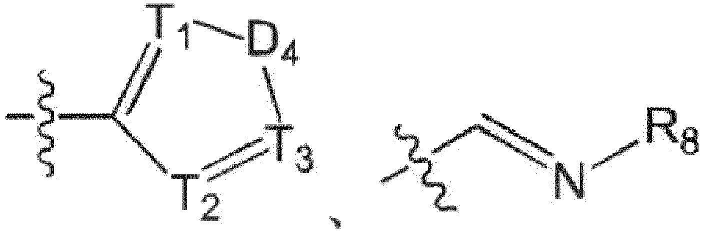 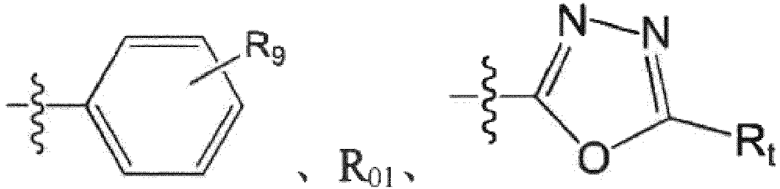 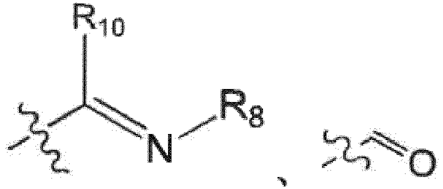 Dans lesquels :
T₁₋₃ est indépendamment sélectionné respectivement entre N ou C(Rₜ) ;
D₄ est sélectionné entre -C(R_{d1})(R_{d2})-, -C(=O)N(R_{d3})-, -N(R_{d4})-, -C(=NR_{d5})-, -S(=O)2N(R_{d6})-, -S(=O)N(R_{d7})-, -O-, -S-, -C(=O)O-, -C(=O)-, -C(=S)-, -S(=O)- ou -S(=O)₂- ;
R₈, R₉ et Rₜ sont indépendamment sélectionnés respectivement entre H, F, Cl, Br, I, CN, OH, SH, NH₂, CHO, COOH ou sélectionnés parmi les groupes alkyles ou hétéroalkyles C₁₋₁₀ et les groupes hydrocarbyles ou hétérocycloalkyles cycliques C₃₋₁₀ remplacés par R₀₁, les groupes alkyles ou hétéroalkyles C₁₋₁₀ remplacés par les groupes hydrocarbyles ou hétérocycloalkyles C₃₋₁₀ ;
R₁₀ est sélectionné entre F, Cl, Br, I, CN, OH, SH, NH₂, CHO, COOH ou sélectionné parmi les groupes alkyles ou hétéroalkyles C₁₋₁₀ et les groupes hydrocarbyles ou hétérocycloalkyles cycliques C₃₋₁₀ remplacés par R₀₁, les groupes alkyles ou hétéroalkyles C₁₋₁₀ remplacés par les groupes hydrocarbyles ou hétérocycloalkyles C₃₋₁₀.

8. Le ou lesdits composé(s) ou leurs sels pharmaceutiquement acceptables selon la revendication 7, dans lesquels R₅₋₉, R₀₁ et Rₜ sont indépendamment sélectionnés respectivement entre : 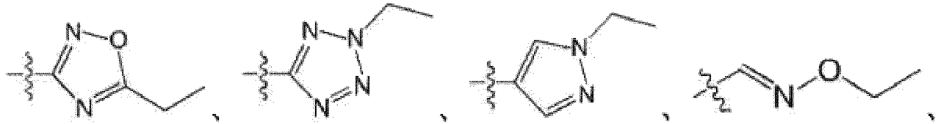 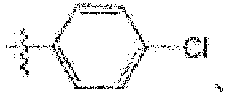 F, Cl, Br, CF₃, -C(C₂H₅)-, -C(OC₂H₅)-, groupe méthyle, groupe éthyle, groupe propyle, groupe méthoxy, groupe éthoxy, groupe propoxy,

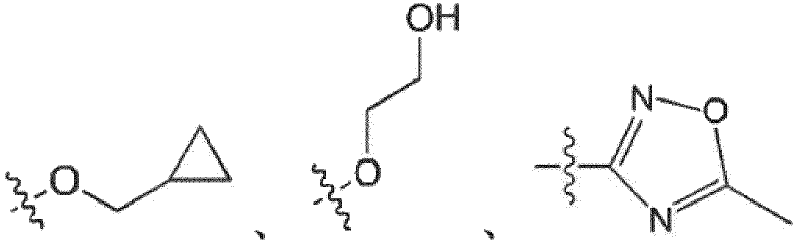

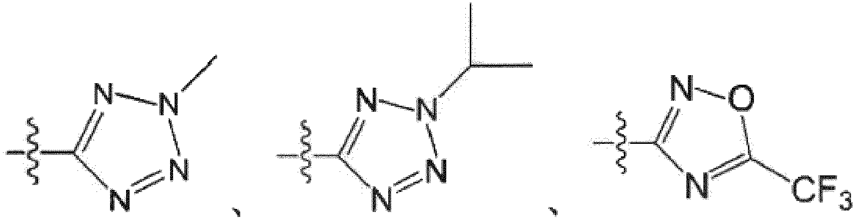

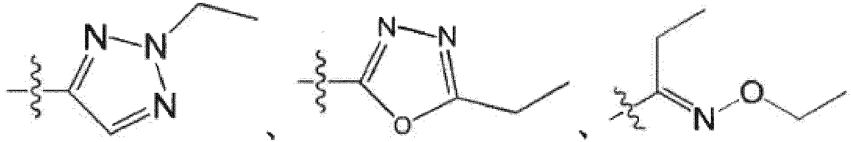

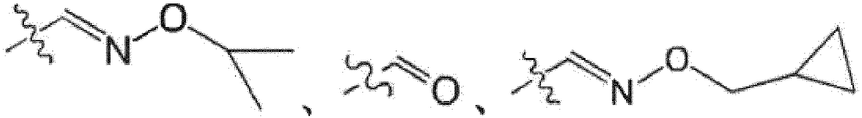

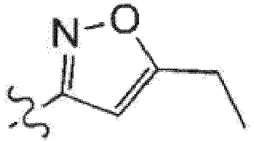

9. Le ou lesdits composé(s) ou leurs sels pharmaceutiquement acceptables selon la revendication 8, dans
lesquels R₄ est sélectionné entre :

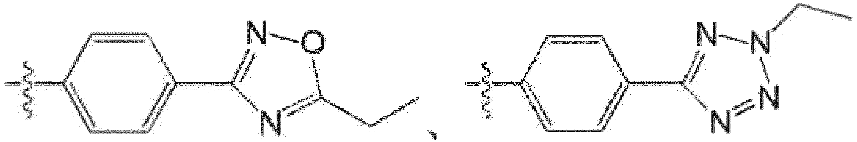

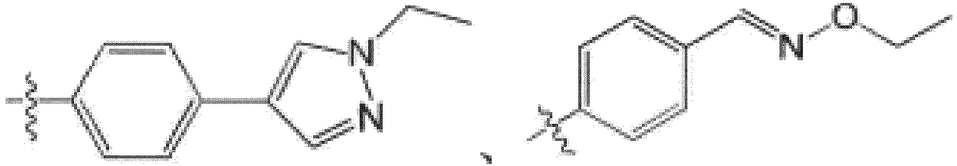

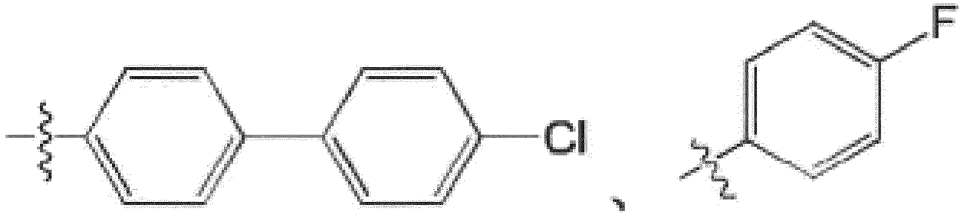

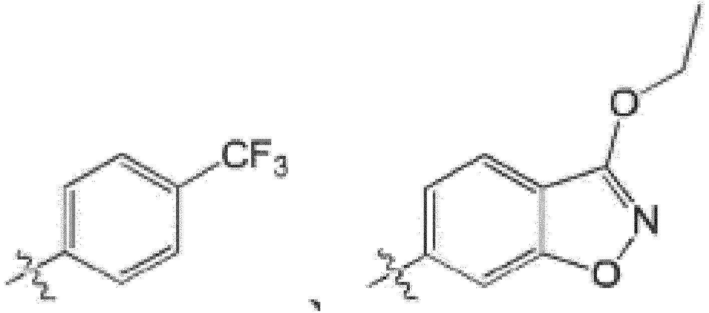

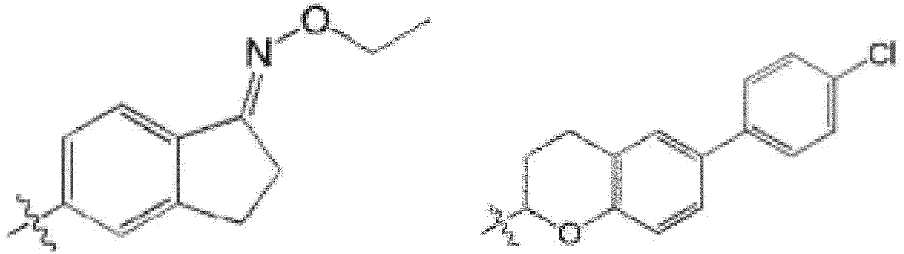

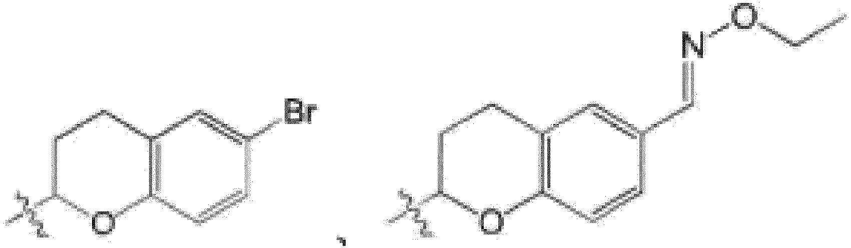

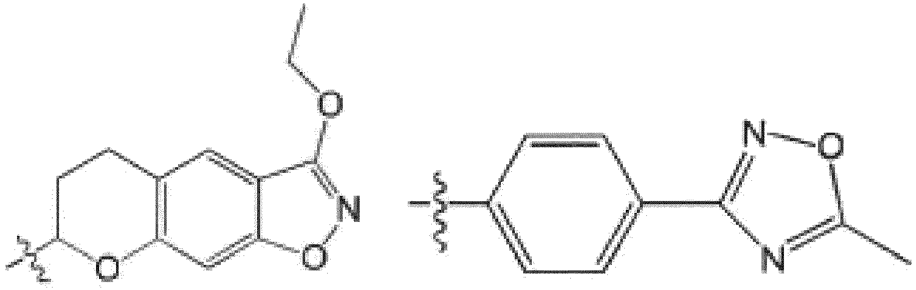

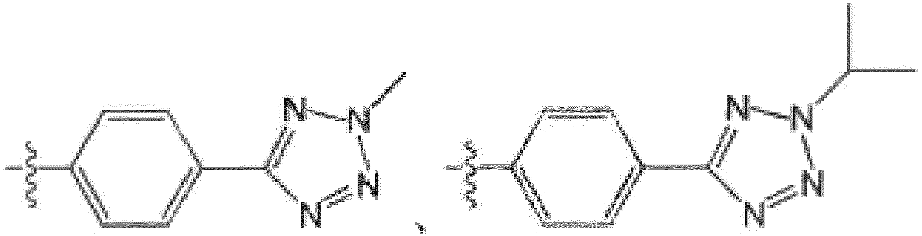

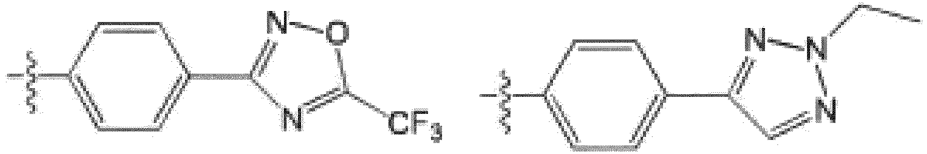

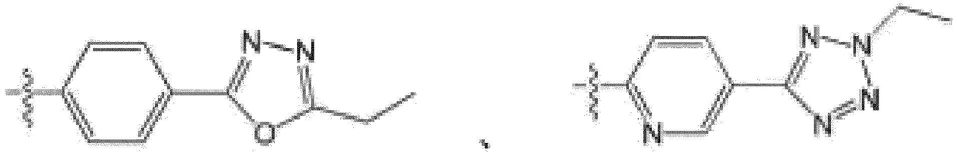

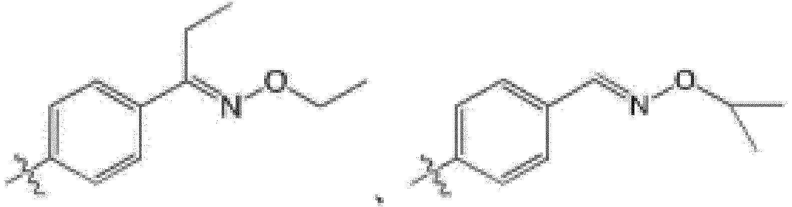

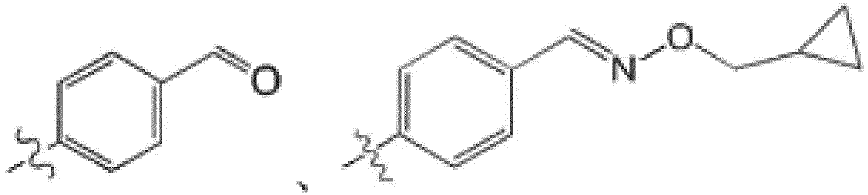

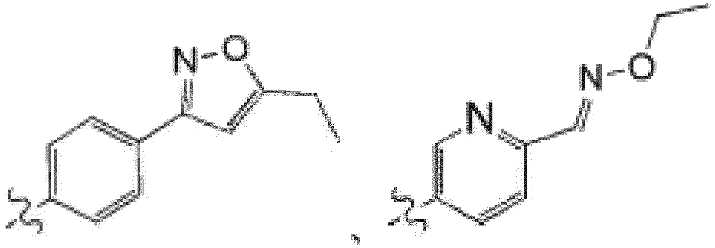

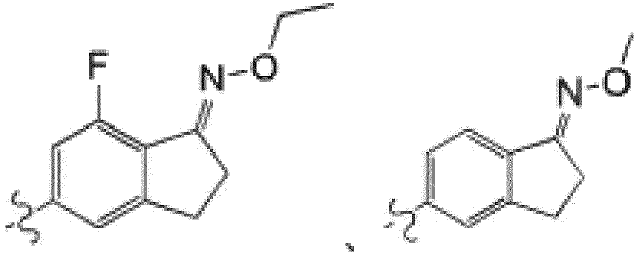

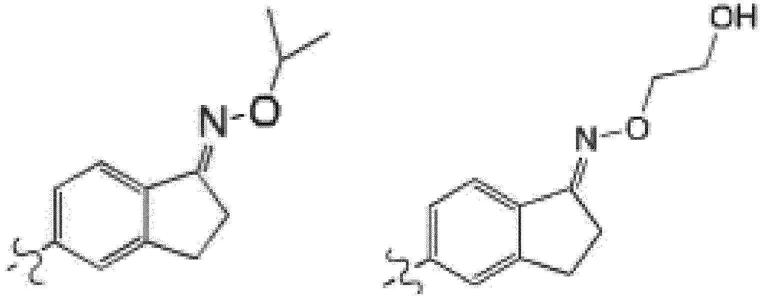

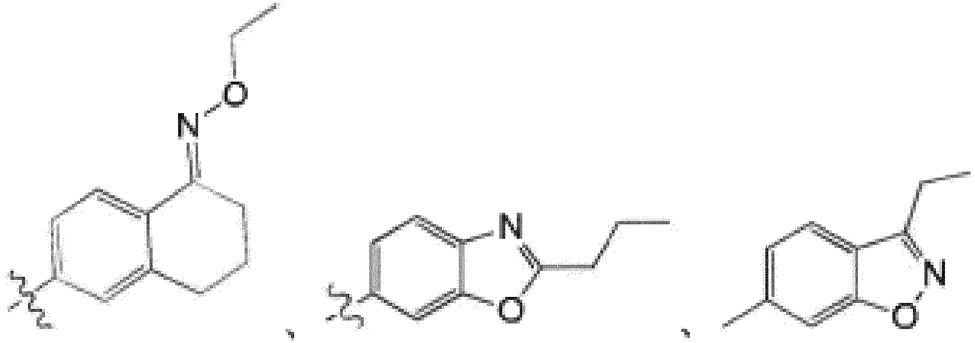

10. Le ou lesdits composé(s) ou leurs sels pharmaceutiquement acceptables selon la revendication 1, sélectionnés entre :
